(19) Europäisches Patentamt European Patent Office Office européen des brevets

(11) **EP 1 626 084 B1**

(12) **EUROPEAN PATENT SPECIFICATION**

(45) Date of publication and mention
of the grant of the patent:
**11.02.2009 Bulletin 2009/07**

(51) Int Cl.:
*C12N 9/02* (2006.01)　　　*C12Q 1/68* (2006.01)
*G01N 33/574* (2006.01)

(21) Application number: **05018353.2**

(22) Date of filing: **11.02.2000**

(54) **Compositions and methods for the diagnosis of tumours**

Zusammensetzungen und Methoden zur Diagnose von Tumoren

Compositions et méthodes pour le diagnostic de tumeurs

(84) Designated Contracting States:
**AT BE CH CY DE DK ES FI FR GB GR IE IT LI LU MC NL PT SE**

(30) Priority:　**08.03.1999 PCT/US99/05028**
**11.03.1999 US 123972 P**
**11.05.1999 US 133459 P**
**02.06.1999 PCT/US99/12252**
**22.06.1999 US 140650 P**
**22.06.1999 US 140653 P**
**20.07.1999 US 144758 P**
**26.07.1999 US 145698 P**
**28.07.1999 US 146222 P**
**17.08.1999 US 149395 P**
**31.08.1999 US 151689 P**
**01.09.1999 PCT/US99/20111**
**15.09.1999 PCT/US99/21090**
**30.11.1999 PCT/US99/28313**
**01.12.1999 PCT/US99/28301**
**01.12.1999 PCT/US99/28634**
**05.01.2000 PCT/US00/00219**

(43) Date of publication of application:
**15.02.2006 Bulletin 2006/07**

(62) Document number(s) of the earlier application(s) in
accordance with Art. 76 EPC:
**00907270.3 / 1 173 563**

(73) Proprietor: **Genentech, Inc.**
**South San Francisco CA 94080-4990 (US)**

(72) Inventors:
• **Ashkenazi, Avi J.**
**San Mateo, CA 94402 (US)**
• **Goddard, Audrey**
**San Francisco, CA 94131 (US)**
• **Godowski, Paul J.**
**Burlingame, CA 94010 (US)**
• **Gurney, Austin L.**
**San Francisco, CA 94114 (US)**
• **Hillan, Kenneth J.**
**San Francisco, CA 94114 (US)**
• **Masters, Scot A.**
**San Carlos, CA 94070 (US)**
• **Pan, James**
**Belmont, CA 94002 (US)**
• **Pitti, Robert M.**
**El Cerrito, CA 94530 (US)**
• **Roy, Margaret Ann**
**San Francisco, CA 94123 (US)**
• **Smith, Victoria**
**Burlingame, CA 94010 (US)**
• **Stone, Donna M.**
**Brisbane, CA 94005 (US)**
• **Watanabe, Colin K.**
**Moraga, CA 94556 (US)**
• **Wood, William I.**
**Cupertino, CA 95014 (US)**

(74) Representative: **Denison, Christopher Marcus et al**
**Mewburn Ellis LLP**
**33 Gutter Lane**
**London**
**EC2V 8AS (GB)**

(56) References cited:
**WO-A-00/04135**　　　**DE-A1- 19 818 620**

- FRANSEN M ET AL: "IDENTIFICATION OF PEROXISOMAL PROTEINS BY USING M13 PHAGE PROTEIN VI PHAGE DISPLAY: MOLECULAR EVIDENCE THAT MAMMALIAN PEROXISOMES CONTAIN A 2,4-DIENOYL-COA REDUCTASE" BIOCHEMICAL JOURNAL, PORTLAND PRESS, LONDON, GB, vol. 340, no. PART 2, June 1999 (1999-06), pages 561-568, XP000862955 ISSN: 0264-6021

- GABRIELLI F ET AL: "A NUCLEAR PROTEIN, SYNTHESIZED IN GROWTH-ARRESTED HUMAN HEPATOBLASTOMA CELLS, IS A NOVEL MEMBER OF THE SHORT-CHAIN ALCOHOL DEHYDROGENASE FAMILY" EUROPEAN JOURNAL OF BIOCHEMISTRY, BERLIN, DE, vol. 232, no. 2, 1995, pages 473-477, XP000862845 ISSN: 0014-2956

**Description**

Field of the Invention

The present invention relates to compositions and methods for the diagnosis and treatment of tumor.

Background of the Invention

Malignant tumors (cancers) are the second leading cause of death in the United States, after heart disease (Boring et al., CA Cancel J. Clin., 43:7 [1993]).

Cancer is characterized by an increase in the number of abnormal, or neoplastic cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites (metastasis). In a cancerous state, a cell proliferates under conditions in which normal cells would not grow. Cancer manifests itself in a wide variety of forms, characterized by different degrees of invasiveness and aggressiveness.

Alteration of gene expression is intimately related to the uncontrolled cell growth and de-differentiation which are a common feature of all cancers. The genomes of certain well studied tumors have been found to show decreased expression of recessive genes, usually referred to as tumor suppression genes, which would normally function to prevent malignant cell growth, and/or overexpression of certain dominant genes, such as oncogenes, that act to promote malignant growth. Each of these genetic changes appears to be responsible for importing some of the traits that, in aggregate, represent the full neoplastic phenotype (Hunter, Cell, 64:1129 [1991] and Bishop, Cell, 64:235-248 [1991]).

A well known mechanism of gene (*e.g.*, oncogene) overexpression in cancer cells is gene amplification. This is a process where in the chromosome of the ancestral cell multiple copies of a particular gene are produced. The process involves unscheduled replication of the region of chromosome comprising the gene, followed by recombination of the replicated segments back into the chromosome (Alitalo et al., Adv. Cancer Res., 47:235-281 [1986]). It is believed that the overexpression of the gene parallels gene amplification, *i.e.*, is proportionate to the number of copies made.

Proto-oncogenes that encode growth factors and growth factor receptors have been identified to play important roles in the pathogenesis of various human malignancies, including breast cancer. For example, it has been found that the human ErbB2 gene (erbB2, also known as her2, or c-erbB-2), which encodes a 185-kd transmembrane glycoprotein receptor (p 185$^{HER2}$; HER2) related to the epidermal growth factor receptor EGFR), is overexpressed in about 25% to 30% of human breast cancer (Slamon et al., Science, 235:177-182 [1987]; Slamon et al., Science, 244:707-712 [1989]).

It has been reported that gene amplification of a proto-oncogene is an event typically involved in the more malignant forms of cancer, and could act as a predictor of clinical outcome (Schwab et al., Genes Chromosomes cancer, 1:181-193 [1990]; Alitalo *et al., supra*). Thus, *erb*B2 overexpression is commonly regarded as a predictor of a poor prognosis, especially in patients with primary disease that involves axillary lymph nodes (Slamon *et al.,* [1987] and [1989], *supra*; Ravdin and Chamness, Gene, 159:19-27 [1995]; and Hynes and Stern, Biochim. Biophys. Acta, 1198: 165-184 [1994]), and has been linked to sensitivity and/or resistance to hormone therapy and chemotherapeutic regimens, including CMF (cyclophosphamide, methotrexate, and fluoruracil) and anthracyclines (Baselga et al., Oncology, 11 (3 Suppl 1):43-48 [1997]). However, despite the association of *erb*B2 overexpression with poor prognosis, the odds of HER2-positive patients responding clinically to treatment with taxanes were greater than three times those of HER2-negative patients (*Ibid*). A recombinant humanized anti-ErbB2 (anti-HER2) monoclonal antibody (a humanized version of the murine anti-ErbB2 antibody 4D5, referred to as rhuMAb HER2 or Herceptin™) has been clinically active in patients with ErbB2-overexpressing metastatic breast cancers that had received extensive prior anticancer therapy. (Baselga et al., J. Clin. Oncol., 14:737-744 [1996]).

In light of the above, there is obvious interest in identifying novel methods and compositions which are useful for diagnosing and treating tumors which are associated with gene amplification.

Summary of the Invention

A. Embodiments

The present invention concerns compositions and methods for the diagnosis and treatment of neoplastic cell growth and proliferation in mammals, including humans. The present invention is based on the identification of genes that are amplified in the genome of tumor cells. Such gene amplification is expected to be associated with the overexpression of the gene product and contribute to tumorigenesis. Accordingly, the proteins encoded by the amplified genes are believed to be useful targets for the diagnosis and/or treatment (including prevention) of certain cancers, and may act as predictors of the prognosis of tumor treatment. The application concerns PRO1800 and subject-matter related thereto. All other PRO designations are included in the description for comparative purposes and background information only.

In one embodiment, the present application concerns an isolated antibody which binds to a polypeptide designated herein as a PRO197, PRO207, PRO226, PRO232, PRO243, PRO256, PRO269, PRO274, PRO304, PR0339, PRO1558, PRO779, PRO1185, PRO245, PRO1759, PRO775, PRO7133, PRO7168, PRO725, PRO02, PRO06, PRO64, PRO313, PRO342, PRO542, PRO773, PRO61, PRO216, PRO686, PRO1800, PRO3562, PRO9850, PRO539, PRO4316 or PRO4980 polypeptide. In one aspect, the isolated antibody specifically binds to a PRO197, PRO207, PRO226, PRO232, PRO243, PRO56, PRO69, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO850, PRO539, PRO4316 or PRO4980 polypeptide. In another aspect, the antibody induces the death of a cell which expresses a PRO97, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide. Often, the cell that expresses the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide is a tumor cell that overexpresses the polypeptide as compared to a normal cell of the same tissue type. In yet another aspect, the antibody is a monoclonal antibody, which preferably has non-human complementarity determining region (CDR) residues and human framework region (FR) residues. The antibody may be labeled and may be immobilized on a solid support In yet another aspect, the antibody is an antibody fragment, a single-chain antibody, or a humanized antibody which binds, preferably specifically, to a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide.

In another embodiment, the application concerns a composition of matter which comprises an antibody which binds, preferably specifically, to a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide in admixture with a pharmaceutically acceptable carrier. In one aspect, the composition of matter comprises a therapeutically effective amount of the antibody. In another aspect, the composition comprises a further active ingredient, which may for example, be a further antibody or a cytotoxic or chemotherapeutic agent. Preferably, the composition is sterile.

In a further embodiment, the application concerns isolated nucleic acid molecules which encode anti-PRO197, anti-PRO207, anti-PRO226, anti-PRO232, anti-PRO243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PRO5775, anti-PRO7133, anti-PRO7168, anti-PRO5725, anti-PRO202, anti-PRO206, anti-PRO264, anti-PR0313, anti-PRO342, anti-PRO542, anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibodies, and vectors and recombinant host cells comprising such nucleic acid molecules.

In a still further embodiment, the application concerns a method for producing an anti-PRO197, anti-PRO207, anti-PRO226, anti-PRO232, anti-PRO1243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PRO5775, anti-PRO7133, anti-PRO7168, anti-PRO5725, anti-PRO202, anti-PRO206, anti-PRO264, anti-PRO313, anti-PRO342, anti-PRO542, anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibody, wherein the method comprises culturing a host cell transformed with a nucleic acid molecule which encodes the antibody under conditions sufficient to allow expression of the antibody, and recovering the antibody from the cell culture.

The application further concerns antagonists of a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PR03562, PR09850, PRO539, PR04316 or PR04980 polypeptide that inhibit one or more of the biological and/or immunological functions or activities of a PRO197, PRO207, PRO226, PR0232, PR0243, PRO256, PRO269, PR0274, PR0304, PR0339, PRO1558, PRO779, PRO1185, PRO1245, PRO1759, PRO5775, PRO7133, PR07168, PRO5725, PRO202, PRO206, PR0264, PRO313, PRO342, PRO542, PRO773, PRO861, PRO1216, PRO1686, PRO1800, PRO3562, PRO9850, PRO539, PR04316 or PR04980 polypeptide.

In a further embodiment, the application concerns an isolated nucleic acid molecule that hybridizes to a nucleic acid molecule encoding a PRO197, PRO207, PR0226, PRO232, PRO243, PRO256, PR0269, PR0274, PR0304, PR0339, PRO1558, PR0779, PRO1185, PRO1245, PRO1759, PR05775, PRO7133, PRO7168, PRO5725, PRO202, PR0206, PR0264, PR0313, PR0342, PRO542, PRO773, PR0861, PRO1216, PRO1686, PRO1800, PRO3562, PRO9850, PRO539, PRO4316 or PRO4980 polypeptide or the complement thereof. The isolated nucleic acid molecule is preferably DNA, and hybridization preferably occurs under stringent hybridization and wash conditions. Such nucleic acid molecules can act as antisense molecules of the amplified genes identified herein, which, in turn, can find use in the modulation of the transcription and/or translation of the respective amplified genes, or as antisense primers in amplification reactions. Furthermore, such sequences can be used as part of a ribozyme and/or a triple helix sequence which in turn, may be used in regulation of the amplified genes.

In another embodiment, the application provides a method for determining the presence of a PRO197, PRO207, PR0226, PR0232, PRO243, PRO256, PRO269, PRO274, PR0304, PR0339, PRO1558, PRO779, PRO1185, PRO1245, PRO1759, PRO5775, PRO7133, PRO7168, PRO5725, PRO202, PRO206, PR0264, PR0313, PRO342, PRO542, PR0773, PRO861, PRO1216, PRO1686, PRO1800, PR03562, PRO9850, PRO539, PRO4316 or PR04980 polypeptide in a sample suspected of containing a PRO197, PRO207, PRO226, PRO232, PR0243, PR0256, PR0269, PR0274, PRO304, PR0339, PRO1558, PRO779, PRO1185, PRO1245, PRO1759, PR05775, PRO7133. PRO7168, PRO5725. PR0202, PRO206, PRO264, PR0313, PR0342, PRO542, PRO773, PR0861, PRO1216, PRO1686, PR01800, PR03562, PR09850, PRO539, PRO4316 or PR04980 polypeptide, wherein the method comprises exposing the sample to an anti-PRO197, anti-PRO207, anti-PRO226, anti-PRO232, anti-PRO243, anti-PR0256, anti-PR0269, anti-PR0274, anti-PRO304, anti-PRO339, anti-PRO1558, anti-PRO779, anti-PRO1185, anti-PRO 1245, anti-PRO 1759, anti-PR05775, anti-PRO7133, anti-PRO7168, anti-PR05725, anti-PRO202, anti-PR0206, anti-PRO264, anti-PR0313, anti-PRO342, anG-PRO542, anti-PRO773, anti-PRO861, anti-PRO1216, anti-PRO1686, anti-PRO1800, anti-PRO3562, anti-PR09850, anti-PRO539, anti-PR04316 or anti-PRO4980 antibody and determining binding of the antibody to a PRO197, PRO207, PRO226, PR0232, PRO243, PRO256, PRO269, PRO274, PRO304, PR0339, PRO1558, PRO779, PRO1185, PRO1245, PRO1759, PR05775, PRO7133, PRO7168. PR05725, PRO202, PRO206, PRO264, PR0313, PR0342, PR0542, PR0773, PRO861, PRO1216. PRO1686, PRO1800, PR03562, PR09850, PRO539, PRO4316 or PRO4980 polypeptide in the sample In another embodiment, the invention provides a method for determining the presence of a PRO197, PR0207, PR0226, PRO232, PRO243, PRO256, PR0269, PRO274, PR0304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PR0773, PRO861, PRO1216, PRO1686, PRO1800, PRO3562, PRO9850, PRO539, PR04316 or PR04980 polypeptide in a cell, wherein the method comprises exposing the cell to an anti-PRO197, anti-PRO207, anti-PRO226, anti-PRO232, anti-PRO243, anti-PRO256, anti-PRO269, anti-PR0274, anti-PRO304, anti-PRO339, anti-PRO1558, anti-PRO779, anti-PRO1185, anti-PRO1245, anti-PRO1759, anti-PR05775, anti-PRO7133, anti-PRO7168, anti-PRO5725, anti-PRO202, anti-PRO206, anti-PRO264, anti-PRO313, anti-PRO342, anti-PRO542, anti-PR0773, anti-PRO861, anti-PRO1216, anti-PRO1686, anti-PRO1800, anti-PRO3562, anti-PRO9850, anti-PRO539, anti-PRO4316 or anti-PRO4980 antibody and determining binding of the antibody to the cell.

In yet another embodiment, the present application concerns a method of diagnosing tumor in a mammal, comprising detecting the level of expression of a gene encoding a PRO197, PRO207, PRO226, PRO232, PR0243, PRO256, PRO269, PRO274, PR0304, PR0339, PRO1558, PRO779, PRO1185. PRO1245, PRO1759, PR05775, PRO7133, PR07168, PRO5725, PRO202, PRO206, PR0264, PR0313, PR0342, PRO542, PR0773, PRO861, PRO1216, PRO1686, PRO1800, PRO3562, PR09850, PRO539, PR04316 or PR04980 polypeptide (a) in a test sample of tissue cells obtained from the mammal, and (b) in a control sample of known normal tissue cells of the same cell type, wherein a higher expression level in the test sample as compared to the control sample, is indicative of the presence of tumor in the mammal from which the test tissue cells were obtained.

In another embodiment, the present application concerns a method of diagnosing tumor in a mammal, comprising (a) contacting an anti-PRO197, anti-PR0207, anti-PR0226, anti-PR0232, anti-PR0243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PRO5775, anti-PRO7133, anti-PRO7168, anti-PRO5725, anti-PRO202, anti-PRO206, anti-PRO264, anti-PR0313, anti-PR0342, anti-PRO542; anti-PRO773, anti-PRO861, anti-PRO1216, anti-PRO1686, anti-PRO1800, anti-PRO3562, anti-PRO9850, anti-PRO539, anti-PRO4316 or anti-PR04980 antibody with a test sample of tissue cells obtained from the mammal, and (b) detecting the formation of a complex between the anti-PRO197, anti-PRO207, anti-PRO226, anti-PRO232, anti-PR0243, anti-PR0256, anti-PRO269, anti-PRO274, anti-PRO304, anti-PRO339, anti-PRO1558. anti-PRO779, anti-PRO1185, anti-PRO1245, anti-PRO1759, anti-PRO5775, anti-PRO7133, anti-PRO7168, anti-PR05725, anti-PRO202, anti-PRO206, anti-PRO264, anti-PRO313, anti-PR0342, anti-PR0542, anti-PRO773, anti-PRO861, anti-PRO1216, anti-PRO1686, anti-PRO1800, anti-PRO3562. anti-PRO9850, anti-PRO539, anti-PRO4316 or anti-PR04980 antibody and a PRO197, PRO207, PRO226, PRO232, PRO243, PR0256, PR0269, PRO274, PR0304, PR0339, PRO1558, PR0779, PRO1185, PRO1245, PRO1759, PR05775, PRO7133. PR07168, PRO5725, PRO202, PR0206, PRO264, PRO313, PRO342, PRO542, PR0773, PRO861, PRO1216, PRO1686, PRO1800, PRO3562, PRO9850, PRO539, PRO4316 or PR04980 polypeptide in the test sample, wherein the formation of a complex is indicative of the presence of a tumor in said mammal. The detection may be qualitative or quantitative, and may be performed in comparison with monitoring the complex formation in a control sample of known normal tissue cells of the same cell type. A larger quantity of complexes formed in the test sample indicates the presence of tumor in the mammal from which the test tissue cells were obtained. The antibody preferably carries a detectable label. Complex formation can be monitored, for example, by light microscopy, flow cytometry, fluorimetry, or other techniques known in the art.

The test sample is usually obtained from an individual suspected to have neoplastic cell growth or proliferation (*e.g.* cancerous cells).

In another embodiment, the present application concerns a cancer diagnostic kit comprising an anti-PRO197, anti-PR0207, anti-PRO226, anti-PRO232. anti-PRO243, anti-PRO256, anti-PRO269, anti-PRO274, anti-PRO304, anti-PRO339, anti-PRO1558, anti-PR0779, anti-PRO1185, anti-PRO1245, anti-PRO1759, anti-PRO5775, anti-PRO7133, anti-PRO7168, anti-PRO5725, anti-PRO202, anti-PRO206, anti-PRO264, anti-PRO313, anti-PRO342, anti-PR0542, anti-PR0773, anti-PRO861, anti-PRO1216, anti-PRO1686, anti-PRO1800, anti-PRO3562, anti-PR09850, anti-PRO539, anti-PR04316 or anti-PR04980 antibody and a carrier (*e.g.*, a buffer) in suitable packaging. The kit preferably contains instructions for using the antibody to detect the presence of a PRO197, PRO207, PRO226, PR0232, PRO243, PRO256, PR0269, PRO274, PR0304, PR0339, PRO1558, PRO779, PRO1185, PRO1245, PRO1759, PRO5775, PR07133, PRO7168, PR05725, PRO202, PRO206, PRO264, PRO313, PRO342, PRO542, PRO773, PR0861, PRO1216, PRO1686, PRO1800, PR03562, PRO9850, PR0539, PRO4316 or PRO4980 polypeptide in a sample suspected of containing the same.

In yet another embodiment, the application concerns a method for inhibiting the growth of tumor cells comprising exposing tumor cells which express a PRO197, PRO207, PRO226, PRO232, PR0243, PRO256, PRO269, PRO274, PRO304, PRO339, PRO1558, PRO779, PRO11185, PRO1245, PRO1759, PRO5775, PRO7133, PR07168, PR05725, PRO202, PRO206, PRO264, PR0313, PRO342, PRO542, PR0773, PRO861, PRO1216, PRO1686, PRO1800, PR03562, PRO9850, PRO539, PR04316 or PRO4980 polypeptide to an effective amount of an agent which inhibits a biological and/or immunological activity and/or the expression of a PR0197, PR0207, PRO226, PRO232, PRO243, PRO256, PRO269, PRO274, PRO304. PR0339, PRO1558, PRO779, PRO1185, PRO1245, PRO1759, PR05775, PRO7133, PRO7168, PRO5725, PRO202, PR0206, PR0264, PR0313, PR0342, PRO542, PRO773, PRO861, PRO1216, PRO1686, PRO1800, PR03562, PRO9850, PRO539, PR04316 or PR04980 polypeptide, wherein growth of the tumor cells is thereby inhibited. The agent preferably is an anti-PRO197, anti-PR0207, anti-PR0226, anti-PRO232, anti-PR0243, anti-PRO256, anti-PRO269, anti-PRO274, anti-PRO304, anti-PR0339, anti-PRO1558, anti-PRO779, anti-PRO1185, anti-PRO1245, anti-PRO1759, anti-PRO5775, anti-PR07133, anti-PRO7168, anti-PRO5725, anti-PRO202, anti-PRO206, anti-PRO264, anti-PRO313, anti-PRO342, anti-PRO542, anti-PRO773, anti-PR0861, anti-PRO1216, anti-PRO1686, anti-PRO1800, anti-PRO3562, anti-PRO9850, anti-PR0539, anti-PRO4316 or anti-PR04980 antibody, a small organic and inorganic molecule, peptide, phosphopeptide, antisense or ribozyme molecule, or a triple helix molecule. In a specific aspect, the agent, e.g., the anti-PRO197, anti-PRO207, anti-PRO226, anti-PRO232, anti-PRO243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PR05775, anti-PRO7133, anti-PR07168, anti-PR05725, anti-PRO202, anti-PRO206, anti-PRO264, anti-PR0313, anti-PRO342, anti-PRO542, anti-PRO773, anti-PRO861, anti-PRO1216, anti-PRO1686, anti-PRO1800, anti-PRO3562, anti-PR09850, anti-PR0539, anti-PRO4316 or anti-PR04980 antibody, induces cell death. In a further aspect, the tumor cells are further exposed to radiation treatment and/or a cytotoxic or chemotherapeutic agent.

In a further embodiment, the application concerns an article of manufacture, comprising:

a container;
a label on the container, and
a composition comprising an active agent contained within the container; wherein the composition is effective for inhibiting the growth of tumor cells and the label on the container indicates that the composition can be used for treating conditions characterized by overexpression of a PRO197, PRO207, PRO226, PRO232, PRO243, PRO256, PRO269, PRO274, PRO304, PRO339, PRO1558, PRO779, PRO1185, PRO1245 , PRO1759, PRO5775, PRO7133, PRO7168, PRO5725, PRO202. PRO206, PRO264, PRO313, PRO342, PRO542, PRO773. PRO861, PRO1216, PRO1686, PRO1800, PRO3562, PRO9850, PRO539, PRO4316 or PRO4980 polypeptide as compared to a normal cell of the same tissue type. In particular aspects, the active agent in the composition is an agent which inhibits an activity and/or the expression of a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264. PRO313, PRO342, PRO542, PRO773, PRO861, PRO1216, PRO1686, PRO1800, PRO3562, PRO9850, PRO539, PRO4316 or PRO4980 polypeptide. In preferred aspects, the active agent is an anti-PRO197, anti-PRO207, anti-PRO226, anti-PRO232, anti-PRO243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PRO5775, anti-PRO7133, anti-PRO7168, anti-PRO5725, anti-PRO202, anti-PRO206, anti-PRO264, anti-PRO313, anti-PR0342, anti-PR0542, anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibody or an antisense oligonucleotide.

The application also provides a method for identifying a compound that inhibits an activity of a PRO197, PRO207, PRO226, PRO232, PRO243, PRO256, PRO269, PRO274, PRO304, PRO339, PRO1558, PRO779, PRO1185, PRO1245. PRO1759, PRO5775, PRO7133, PRO7168, PRO5725, PRO207, PRO206, PRO264, PRO313, PRO342, PRO542, PRO773, PRO861, PRO1216, PRO1686, PRO1800, PRO3562, PRO9850, PRO539, PRO4316 or PRO4980 polypeptide, comprising contacting a candidate compound with a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide under conditions and for a time sufficient to allow these two components to interact and determining whether a biological and/or immunological activity of the PRO197, PRO207, PRO226, PRO232, PRO243, PRO256, PRO269, PRO274, PR0304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide is inhibited. In a specific aspect, either the candidate compound or the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide is immobilized on a solid support In another aspect, the non-immobilized component carries a detectable label. In a preferred aspect, this method comprises the steps of (a) contacting cells and a candidate compound to be screened in the presence of the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686. PRO1800, PRO3562, PRO9850, PRO539, PRO4316 or PRO4980 polypeptide under conditions suitable for the induction of a cellular response normally induced by a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PR04980 polypeptide and (b) determining the induction of said cellular response to determine if the test compound is an effective antagonist.

In another embodiment, the application provides a method for identifying a compound that inhibits the expression of a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide in cells that express the polypeptide, wherein the method comprises contacting the cells with a candidate compound and determining whether the expression of the PRO197, PRO207, PRO226. PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide is inhibited. In a preferred aspect, this method comprises the steps of (a) contacting cells and a candidate compound to be screened under conditions suitable for allowing expression of the PRO197, PRO207, PRO226, PRO232, PRO243, PRO256, PRO269, PRO274, PRO304, PRO339, PRO1558, PRO779, PRO1195, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide and (b) determining the inhibition of expression of said polypeptide.

B. Additional Embodiments

In other embodiments of the present application, the invention provides an isolated nucleic acid molecule comprising a nucleotide sequence that encodes a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PR03562, PRO9850, PRO539, PRO4316 or PRO4980 polypeptide.

In one aspect, the isolated nucleic acid molecule comprises a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91 % sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule encoding a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule comprising the coding sequence of a full-length PRO197, PRO207, PRO226, PRO232, PRO243, PRO256, PRO269, PR0274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide cDNA as disclosed herein, the coding sequence of a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide lacking the signal peptide as disclosed herein, the coding sequence of an extracellular domain of a transmembrane PRO197, PRO207, PRO226, PRO232, PRO243, PRO256, PRO269, PRO274, PRO304, PRO339, PRO1558, PRO719, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide, with or without the signal peptide, as disclosed herein or the coding sequence of any other specifically defined fragment of the full-length amino acid sequence as disclosed herein, or (b) the complement of the DNA molecule of (a).

In a further aspect, the application concerns an isolated nucleic acid molecule comprising a nucleotide sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to (a) a DNA molecule that encodes the same mature polypeptide encoded by any of the human protein cDNAs deposited with the ATCC as disclosed herein, or (b) the complement of the DNA molecule of (a).

Another aspect of the application provides an isolated nucleic acid molecule comprising a nucleotide sequence encoding a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO197, PRO207, PR0226, PR0232, PRO243, PRO256. PR0269, PRO274, PRO304, PRO339, PRO1558. PRO779, PROL185, PRO1245, PRO1759, PRO5775, PRO7133, PRO7168, PRO5725, PRO202, PRO206, PRO264, PRO313, PRO342, PRO542, PRO773, PRO861, PRO1216, PR01686, PRO1800, PRO3562, PRO9850, PRO539, PRO4316 or PRO4980 polypeptides arc contemplated.

Another embodiment is directed to fragments of a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PR0313, PRO342, PRO542, PRO773, PRO861, PRO1216, PRO1686, PRO1800, PRO3562, PRO9850, PRO539, PRO4316 or PRO4980 polypeptide coding sequence, or the complement thereof, that may find use as, for example, hybridization probes, for encoding fragments of a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide that may optionally encode a polypeptide comprising a binding site for an anti-PRO197, anti-PRO207, anti-PRO226, anti-PRO232, anti-PRO243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PRO5775, anti-PRO7133, anti-PRO7168, anti-PRO5725, anti-PRO202, anti-PRO206, anti-PRO264, anti-PRO313, anti-PRO342, anti-PRO542, anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibody or as antisense oligonucleotide probes. Such nucleic acid fragments are usually at least about 20 nucleotides in length, preferably at least about 30 nucleotides in length, more preferably at least about 40 nucleotides in length, yet more preferably at least about 50 nucleotides in length, yet more preferably at least about 60 nucleotides in length, yet more preferably at least about 70 nucleotides in length, yet more preferably at least about 80 nucleotides in length, yet more preferably at least about 90 nucleotides in length, yet more preferably at least about 100 nucleotides in length, yet more preferably at least about 110 nucleotides in length, yet more preferably at least about 120 nucleotides in length, yet more preferably at least about 130 nucleotides in length, yet more preferably at least about 140 nucleotides in length, yet more preferably at least about 150 nucleotides in length, yet more preferably at least about 160 nucleotides in length, yet more preferably at least about 170 nucleotides in length, yet more preferably at least about 180 nucleotides in length, yet more preferably at least about 190 nucleotides in length, yet more preferably at least about 200 nucleotides in length, yet more preferably at least about 250 nucleotides in length, yet more preferably at least about 300 nucleotide in length, yet more preferably at least about 350 nucleotides in length, yet more preferably at least about 400 nucleotides in length, yet more preferably at least about 450 nucleotides in length, yet more preferably at least about 500 nucleotides in length, yet more preferably at least about 600 nucleotides in length, yet more preferably at least about 700 nucleotides in length, yet more preferably at least about 800 nucleotides in length, yet more preferably at least about 900 nucleotides in length and yet more preferably at least about 1000 nucleotides in length, wherein in this context the term "about" means the referenced nucleotide sequence length plus or minus 10% of that referenced length. It is noted that novel fragments of a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypepdde-encoding nucleotide sequence may be determined in a routine manner by aligning the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide-encoding nucleotide sequence with other know nucleotide sequences using any of a number of well known sequence alignment programs and determining which PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide-encoding nucleotide sequence fragment(s) are novel. All of such PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide-encoding nucleotide sequences are contemplated herein. Also contemplated are the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide fragments encoded by these nucleotide molecule fragments, preferably those PRO197, PRO207, PRO226, PRO232, PRO243, PRO256, PRO269, PRO274, PRO304. PRO339, PRO1558, PRO779, PRO1185, PRO1245, PRO1759, PRO5775, PRO7133, PRO7168, PRO5725, PRO202, PRO206, PRO264, PRO313, PRO342, PRO542, PR0773,

PRO861, PRO1216, PRO1686, PRO1800, PRO3562, PRO9850, PRO539, PRO4316 or PRO4980 polypeptide fragments that comprise a binding site for an anti-PRO197, anti-PRO207, anti-PRO226, anti-PRO232, anti-PRO243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PRO5775, anti-PRO7133, anti-PRO7168, anti-PRO5725, anti-PRO202, anti-PRO206, anti-PRO264, anti-PRO313, anti-PRO342, anti-PRO542, anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibody.

In another embodiment, the application provides isolated PRO197, PR0207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide encoded by any of the isolated nucleic acid sequences hereinabove identified.

In a certain aspect, the application concerns an isolated PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide, comprising an amino acid sequence having at least about 80% sequence identity, preferably at least about 81% sequence identity, more preferably at least about 8296 sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91 % sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the application concerns an isolated PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide comprising an amino acid sequence having at least about 80% sequence identity, preferably at least about 81% sequence identity, more preferably at least about 82% sequence identity, yet more preferably at least about 83% sequence identity, yet more preferably at least about 84% sequence identity, yet more preferably at least about 85% sequence identity, yet more preferably at least about 86% sequence identity, yet more preferably at least about 87% sequence identity, yet more preferably at least about 88% sequence identity, yet more preferably at least about 89% sequence identity, yet more preferably at least about 90% sequence identity, yet more preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity and yet more preferably at least about 99% sequence identity to an amino acid sequence encoded by any of the human protein cDNAs deposited with the ATCC as disclosed herein.

In a further aspect, the application concerns an isolated PRO197, PR0207, PR0226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PR0206, PRO264, PR0313, PRO342, PRO542, PR0773, PRO861, PRO1216, PRO1686, PRO1800, PRO3562, PR09850, PRO539, PRO4316 or PRO4980 polypeptide comprising an amino acid sequence scoring at least about 80% positives, preferably at least about 81% positives, more preferably at least about 82% positives, yet more preferably at least about 83% positives, yet more preferably at least about 84% positives, yet more preferably at least about 85% positives, yet more preferably at least about 86% positives, yet more preferably at least about 87% positives, yet more preferably at least about 88% positives, yet more preferably at least about 89% positives, yet more preferably at least about 90% positive, yet more preferably at least about 91% positives, yet more preferably at least about 92% positives, yet more preferably at least about 93% positives, yet more preferably at least about 94% positives, yet more preferably at least about 95% positives, yet more preferably at least about 96% positives, yet more preferably at least about 97% positives, yet more preferably at least about 98% positives and yet more preferably at least about 99% positives when compared with the amino acid sequence of a PRO197, PRO207, PRO226, PRO232, PR0243, PR0256, PRO269, PRO274, PRO304, PRO339, PRO1558, PR0779, PRO1185, PRO1245, PRO1759, PRO5775, PRO7133, PRO7168, PRO5725, PRO202, PRO206, PRO264, PR0313, PRO342, PRO542, PR0773, PRO861, PRO1216, PRO1686, PRO1800, PR03562, PR09850, PR0539, PRO4316 or PRO4980 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a specific aspect, the application provides an isolated PRO197, PRO207, PRO226, PRO232, PRO243, PRO256, PRO269, PRO274, PR0304, PR0339, PRO1558, PRO779, PRO1185, PRO1245, PRO1759, PR05775, PR07133, PRO7168, PRO5725, PR0202, PR0206, PRO264, PRO313, PRO342, PR0542, PR0773, PRO861, PRO1216, PRO1686, PRO1800, PR03562, PR09850, PR0539, PRO4316 or PRO4980 polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PR07168, PRO5725, PRO202, PRO206, PRO264, PRO313, PR0342, PR0542, PR0773, PRO861, PRO1216, PRO1686, PRO1800, PRO3562, PRO9850, PR0539, PRO4316 or PRO4980 polypeptide and recovering the PRO197, PRO207, PRO226, PR0232, PRO243, PRO256, PRO269, PRO274, PRO304, PRO339, PRO1558, PRO779, PRO1185, PRO1245, PRO1759, PRO5775, PR07133, PRO7168, PRO5725, PRO202, PRO206, PRO264, PR0313, PRO342, PRO542, PRO773, PRO861, PRO1216, PRO1686, PRO1800, PRO3562, PRO9850, PRO539, PRO4316 or PR04980 polypeptide from the cell culture.

Another aspect of the application provides an isolated PRO197, PRO207, PRO226, PRO232, PRO243, PRO256, PRO269, PR0274, PR0304, PR0339, PRO1558, PR0779, PRO1185, PRO1245, PRO1759, PRO5775, PRO7133, PRO7168, PR05725, PRO202, PR0206, PR0264, PRO313, PR0342, PR0542, PRO773, PRO861, PRO1216, PRO1686, PRO1800, PR03562, PRO9850, PR0539, PRO4316 or PRO4980 polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO197, PR0207, PR0226, PRO232, PRO243, PRO256, PR0269, PR0274, PR0304, PR0339, PRO1558, PRO779, PRO1185, PRO1245, PRO1759, PRO5775, PR07133, PR07168, PRO5725, PRO202, PRO206, PR0264, PR0313, PRO342, PR0542, PRO773, PRO861, PRO1216, PRO1686, PRO1800, PRO3562, PR09850, PR0539, PR04316 or PR04980 polypeptide and recovering the PRO197, PRO207, PRO226, PRO232, PR0243, PRO256, PRO269, PR0274, PRO304, PRO339, PRO1558, PR0779, PRO1185, PRO1245, PRO1759, PRO5775, PRO7133, PRO7168, PR05725, PRO202, PRO206, PR0264, PR0313, PRO342, PRO542, PRO773, PRO861, PRO1216, PRO1686, PRO1800, PR03562, PRO9850, PRO539, PR04316 or PRO4980 polypeptide from the cell culture.

In yet another embodiment, the application concerns antagonists of a native PRO197, PRO207, PRO226, PRO232, PR0243, PRO256, PRO269, PRO274, PR0304, PRO339, PRO1558, PR0779, PRO1185, PRO1245, PRO1759, PRO5775, PRO7133, PRO7168, PR05725, PRO202, PR0206, PRO264, PRO313, PRO342, PRO542, PR0773, PRO861, PRO1216, PRO1686, PRO1800, PR03562. PRO09850, PR0539. PR04316 or PR04980 polypeptide as defined herein. In a particular embodiment, the antagonist is an anti-PRO197, anti-PRO207, anti-PRO226, anti-PR0232, anti-PRO243, anti-PRO256, anti-PRO269, anti-PR0274, anti-PRO304, anti-PRO339, anti-PRO1558, anti-PR0779, anti-PRO1185, anti-PRO1245, anti-PRO1759, anti-PRO5775, anti-PRO7133, anti-PRO7168, anti-PRO5725, anti-PR0202, anti-PRO206, anti-PRO264, anti-PRO313, anti-PRO342, anti-PR0542, anti-PR0773, anti-PRO861, anti-PRO1216, anti-PRO1686, anti-PRO1800, anti-PR03562, anti-PR09850, anti-PRO539, anti-PR04316 or anti-PR04980 antibody or a small molecule.

In a further embodiment, the application concerns a method of identifying antagonists to a PRO197, PR0207, PRO226, PRO232, PRO243, PRO256, PRO269, PRO274, PR0304, PRO339, PRO1558, PRO779, PRO1185, PRO1245, PRO1759, PR05775, PR07133, PR07168, PR05725, PRO202, PRO206, PR0264, PR0313, PRO342, PRO542, PRO773, PRO861, PRO1216, PRO1686, PRO1800, PR03562, PR09850, PR0539, PRO4316 or PRO4980 polypeptide which comprise contacting the PRO197, PRO207, PR0226, PRO232, PR0243, PRO256, PRO269, PRO274, PR0304, PRO339, PRO1558, PRO779, PRO1185, PRO1245, PRO1759, PRO5775, PRO7133, PR07168, PRO5725, PRO202, PRO206, PR0264, PR0313, PRO342, PRO542, PRO773, PRO861, PRO1216, PRO1686, PRO1800, PRO3562, PRO9850, PRO539, PRO4316 or PRO4980 polypeptide with a candidate molecule and monitoring a biological activity mediated by said PRO197, PRO207, PR0226, PRO232, PRO243, PRO256, PR0269, PRO274, PR0304, PRO339, PRO1558, PRO779, PRO1185, PRO1245, PRO1759, PRO5775, PRO7133, PRO7168, PRO5725, PRO202, PRO206, PRO264, PR0313, PRO342, PRO542, PRO773, PRO861, PRO1216, PRO1686, PRO1800. PRO3562,

PRO9850, PRO539, PRO4316 or PRO4980 polypeptide. Preferably, the PRO197, PR0207, PR0226, PRO232, PRO243, PRO256, PRO269, PR0274, PR0304, PR0339, PRO1558, PRO779, PRO1185, PRO1245, PRO1759, PRO5775, PR07133, PR07168, PRO5725, PR0202, PRO206, PR0264, PRO313, PRO342, PRO542, PRO773, PRO861, PRO1216, PRO1686, PRO1800, PRO3562, PRO9850, PRO539, PRO4316 or PR04980 polypeptide is a native PRO197, PR0207, PRO226, PR0232, PRO243, PRO256, PRO269, PR0274, PRO304, PR0339, PRO1558, PR0779, PRO1185, PRO1245, PRO1759, PRO5775, PRO7133, PRO7168, PRO5725, PRO202, PRO206, PRO264, PR0313, PRO342, PRO542, PR0773, PR0861, PRO1216, PRO1686, PRO1800, PRO3562, PRO9850, PRO539, PR04316 or PR04980 polypeptide.

In a still further embodiment, the application concerns a composition of matter comprising a PRO197, PRO207, PR0226, PRO232, PR0243, PRO256, PRO269, PR0274, PR0304, PR0339, PRO1558, PRO779, PRO1185, PRO1245, PRO1759, PRO5775, PR07133, PR07168, PRO5725, PR0202, PRO206, PRO264, PR0313, PR0342, PRO542, PR0773, PRO861, PRO1216, PRO1686, PRO 1800, PRO3562, PRO9850, PR0539, PRO4316 or PRO4980 polypeptide, or an antagonist of a PRO197, PR0207, PRO226, PRO232, PRO243, PRO256, PRO269, PR0274, PR0304, PR0339, PRO1558, PRO779, PRO1185, PRO1245, PRO1759, PRO5775, PRO7133, PRO7168, PRO5725, PR0202, PRO206, PR0264, PR0313, PRO342, PRO542, PRO773, PRO861, PRO1216, PRO1686, PRO1800, PRO3562, PRO9850, PRO539, PR04316 or PRO4980 polypeptide as herein described, or an anti-PRO197, anti-PRO207, anti-PRO226, anti-PRO232, anti-PRO243, anti-PRO256, anti-PRO269, anti-PR0274, anti-PR0304, anti-PR0339, anti-PRO1558, anti-PR0779, anti-PRO1185, anti-PRO1245, anti-PRO1759, anti-PRO5775, anti-PRO7133, anti-PRO7168, anti-PRO5725, anti-PRO202, anti-PR0206, anti-PRO264, anti-PR0313, anti-PRO342, anti-PRO542, anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibody, in combination with a carrier. Optionally, the carrier is a pharmaceutically /acceptable carrier.

Another embodiment of the present application is directed to the use of a PRO197, PRO207, PRO226, PR0232, PRO243, PRO256, PRO269, PRO274, PRO304, PR0339, PRO1558, PR0779, PRO1185, PRO1245, PRO1759, PRO5775, PRO7133, PRO7168, PRO5725, PRO202, PRO206, PR0264, PR0313, PR0342, PRO542, PR0773, PRO861, PRO1216, PRO1686, PRO1800, PRO3562, PRO9850, PRO539, PR04316 or PR04980 polypeptide, or an antagonist thereof as hereinbefore described, or an anti-PRO 197, anti-PR0207, anti-PRO226, anti-PRO232, anti-PRO243, anti-PR0256, anti-PR0269, anti-PRO274, anti-PRO304, anti-PR0339, anti-PRO1558, anti-PRO779, anti-PRO1185, anti-PRO1245, anti-PRO1759, anti-PRO5775, anti-PRO7133, anti-PR07168, anti-PRO5725, anti-PRO202, anti-PRO206, anti-PR0264, anti-PRO313, anti-PRO342, anti-PRO542, anti-PRO773. anti-PR0861, anti-PRO1216, anti-PRO1686, anti-PRO1800, anti-PRO3562, anti-PRO9850, anti-PRO539, anti-PR04316 or anti-PR04980 antibody, for the preparation of a medicament useful in the treatment of a condition which is responsive to the PRO197, PRO207, PR0226, PR0232, PR0243, PRO256, PRO269, PRO274, PRo304, PR0339, PRO1558, PRO779, PRO1185, PRO1245, PRO1759, PRO5775, PRO7133, PR07168, PRO5725, PRO202, PRO206, PR0264, PRO313, PRO342, PRO542, PRO773, PRO861, PRO1216, PRO1686, PRO1800, PRO3562, PRO9850, PRO539, PRO4316 or PRO4980 polypeptide, an antagonist thereof or an anti-PRO197, anti-PRO207, anti-PRO226, anti-PRO232, anti-PRO243, anti-PRO256, anti-PR0269, anti-PRO274, anti-PR0304, anti-PRO339, anti-PRO1558, anti-PR0779, anti-PRO1185, anti-PRO1245, anti-PRO1759, anti-PRO5175, anti-PRO7133, anti-PRO7168, anti-PRO5725, anti-PR0202, anti-PRO206, anti-PRO264, anti-PRO313, anti-PRO342, anti-PRO542, anti-PR0773, anti-PRO861, anti-PRO1216, anti-PRO1686, anti-PRO1800, anti-PRO3562, anti-PRO9850, anti-PR0539, anti-PRO4316 or anti-PRO4980 antibody.

In other embodiments of the present invention, the invention provides vectors comprising DNA encoding any of the herein described polypeptides. Host cell comprising any such vector are also provided. By way of example, the host cells may be CHO cells, *E. coli,* yeast, or Baculovirus-infected insect cells. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

Brief Description of the Figures

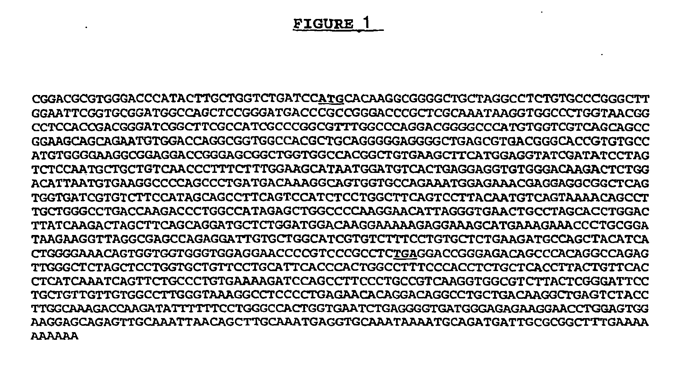

Figure 1 shows the amino acid sequence (SEQ ID NO:60) of a native sequence PRO1800 polypeptide as derived from the coding sequence of SEQ ID NO:59 shown in Figure 59.

Figure 1 shows the nucleotide sequence (SEQ ID NO:61) of a cDNA containing a nucleotide sequence encoding native sequence PRO3562, wherein the nucleotide sequence (SEQ ID NO:61) is a clone designated herein as DNA96791. Also presented in bold font and underlined are the positions of the respective start and stop codons.

Detailed Description of the Invention

I. Definitions

The phrases "gene amplification" and "gene duplication" are used interchangeably and refer to a process by which multiple copies of a gene or gene fragment are formed in a particular cell or cell line. The duplicated region (a stretch of amplified DNA) is often referred to as "amplicon." Usually, the amount of the messenger RNA (mRNA) produced, i.e., the level of gene expression, also increases in the proportion of the number of copies made of the particular gene expressed.

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma, kidney cancer, liver cancer, vulval cancer, thyroid cancer, hepatic carcinoma and various types of bead and neck cancer.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In tumor (*e.g.*, cancer) treatment, a therapeutic agent may directly decrease the pathology of tumor cells, or render the tumor cells more susceptible to treatment by other therapeutic agents, *e.g.*, radiation and/or chemotherapy.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

"Mammal" for purposes of treatment refers to any animal classified as a mammal, including humans, domestic and farm animals, and zoo, sports, or pet animals, such as dogs, horses, cats, cattle, pigs, sheep, etc. Preferably, the mammal is human.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

Administration "in combination with" one or more further therapeutic agents includes simultaneous (concurrent) and consecutive administration in any order.

The term "cytotoxic agent" as used herein refers to a substance that inhibits or prevents the function of cells and/or causes destruction of cells. The term is intended to include radioactive isotopes (*e.g.*, $I^{131}$, $I^{125}$, $Y^{90}$ and $Re^{186}$), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e.g.*, paclitaxel (Taxol, Bristol-Myers Squibb Oncology, Princeton, NJ), and doxetaxel (Taxotere, Rhône-Poulenc Rorer, Antony, Rnace), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), 5-FU, 6-thioguanine, 6-mercaptopurine, actinomycin D, VP-16, chlorambucil, melphalan, and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo*. Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al., (WB Saunders: Philadelphia, 1995), especially p. 13.

"Doxorubicin" is an anthracycline antibiotic. The full chemical name of doxorubicin is (8S-cis)-10-[(3-amino-2,3,6-trideoxy-$\alpha$-L-lyxo-hexapyranosyl)oxy]-7,8,9,10-tetrahydro-6,8,11-trihydroxy-8-(hydroxyacetyl)-1-methoxy-5,12-naphthacenedione.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; mullecian-inhibiting substance; mouse gonadotropin-associated peptide; inhibin; activin; vascular endothelial growth factor; integrin; thrombopoietin (TPO); nerve growth factors such as NGF-$\beta$; platelet-growth factor; transforming growth factors (TGFs) such as PGF-$\alpha$ and TGF-$\beta$; insulin-like growth factor-I and -II; erythropoietin (EPO); osteoinductive factors; interferons such as interferon-$\alpha$, -$\beta$, and -$\gamma$; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1a, IL2, IL3, IL-4, IL-5, IL-6, IL-7, IL-8, IL-9, IL-11, IL-12; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy". Biochemical Society Transactions,14:375-382, 615th Meeting, Belfast (1986), and Stella et al., "Prodrugs: A Chemical Approach to Targeted Drug Delivery", Directed Drug Delivery, Borchardt et al., (ed.), pp.147-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glysocylated prodrugs, $\beta$-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrugs form for use in this invention include, but are not limited to, those chemotherapeutic agents described above.

An "effective amount" of a polypeptide disclosed herein or an antagonist thereof, in reference to inhibition of neoplastic cell growth, tumor growth or cancer cell growth, is an amount capable of inhibiting, to some extent, the growth of target cells. The term includes an amount capable of invoking a growth inhibitory, cytostatic and/or cytotoxic effect and/or apoptosis of the target cells. An "effective amount" of a PRO polypeptide antagonist for purposes of inhibiting neoplastic cell growth, tumor growth or cancer cell growth, may be determined empirically and in a routine manner.

A "therapeutically effective amount", in reference to the treatment of tumor, refers to an amount capable of invoking one or more of the following effects: (1) inhibition, to some extent, of tumor growth, including, slowing down and complete growth arrest; (2) reduction in the number of tumor cells; (3) reduction in tumor size; (4) inhibition (*i.e.*, reduction, slowing down or complete stopping) of tumor cell infiltration into peripheral organs; (5) inhibition (*i.e.*, reduction, slowing down or complete stopping) of metastasis; (6) enhancement of anti-tumor immune response, which may, but does not have to, result in the regression or rejection of the tumor; and/or (7) relief, to some extent, of one or more symptoms associated with the disorder. A "therapeutically effective amount" of a PRO polypeptide antagonist for purposes of treatment of tumor may be determined empirically and in a routine manner.

A "growth inhibitory amount" of a PRO antagonist is an amount capable of inhibiting the growth of a cell, especially tumor, *e.g.*, cancer cell, either *in vitro* or *in vivo*. A "growth inhibitory amount" of a PRO antagonist for purposes of inhibiting neoplastic cell growth may be determined empirically and in a routine manner.

A "cytotoxic amount" of a PRO antagonist is an amount capable of causing the destruction of a cell, especially tumor, *e.g.*, cancer cell, either *in vitro* or *in vivo*. A "cytotoxic amount" of a PRO antagonist for purposes of inhibiting neoplastic cell growth may be determined empirically and in a routine manner.

The terms "PRO polypeptide" and "PRO" as used herein and when immediately followed by a numerical designation refer to various polypeptides, wherein the complete designation (*i.e.*, PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (*e.g.*, an extracellular domain sequence), naturally-occurring variant forms (*e.g.*, alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.590 of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (*e.g.*, Nielsen et al., Prot. Eng,. 10:1-6 (1997) and von Heinje et al., Nucl. Acids Res., 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at-least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and most preferably at least about 99% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 300 amino acids in length, or more.

As shown below, Table 1 provides the complete source code for the ALIGN-2 sequence comparison computer program. This source code may be routinely compiled for use on a UNIX operating system to provide the ALIGN-2 sequence comparison computer program

In addition, Tables 2A-2D show hypothetical exemplifications for using the below described method to determine % amino acid sequence identity (Tables 2A-2B) and % nucleic acid sequence identity (Tables 2C-2D) using the ALIGN-2 sequence comparison computer program, wherein "PRO" represents the amino acid sequence of a hypothetical PRO polypeptide of interest, "Comparison Protein" represents the amino acid sequence of a polypeptide against which the "PRO" polypeptide of interest is being compared. "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, "X". "Y", and "Z" each represent different hypothetical amino acid residues and "N", "L" and "V" each represent different hypothetical nucleotides.

## Table 1

```
/*
 *
 * C-C increased from 12 to 15
 * Z is average of EQ
 * B is average of ND
 * match with stop is _M; stop-stop = 0; J (joker) match = 0
 */
#define _M      -8        /* value of a match with a stop */

int      _day[26][26] = {
/*    A  B  C  D  E  F  G  H  I  J  K  L  M  N  O  P  Q  R  S  T  U  V  W  X  Y  Z */
/* A */   { 2, 0,-2, 0, 0,-4, 1,-1,-1, 0,-1,-2,-1, 0,_M, 1, 0,-2, 1, 1, 0, 0,-6, 0,-3, 0},
/* B */   { 0, 3,-4, 3, 2,-5, 0, 1,-2, 0, 0,-3,-2, 2,_M,-1, 1, 0, 0, 0, 0,-2,-5, 0,-3, 1},
/* C */   {-2,-4,15,-5,-5,-4,-3,-3,-2, 0,-5,-6,-5,-4,_M,-3,-5,-4, 0,-2, 0,-2,-8, 0, 0,-5},
/* D */   { 0, 3,-5, 4, 3,-6, 1, 1,-2, 0, 0,-4,-3, 2,_M,-1, 2,-1, 0, 0, 0,-2,-7, 0,-4, 2},
/* E */   { 0, 2,-5, 3, 4,-5, 0, 1,-2, 0, 0,-3,-2, 1,_M,-1, 2,-1, 0, 0, 0,-2,-7, 0,-4, 3},
/* F */   {-4,-5,-4,-6,-5, 9,-5,-2, 1, 0,-5, 2, 0,-4,_M,-5,-5,-4,-3,-3, 0,-1, 0, 0, 7,-5},
/* G */   { 1, 0,-3, 1, 0,-5, 5,-2,-3, 0,-2,-4,-3, 0,_M,-1,-1,-3, 1, 0, 0,-1,-7, 0,-5, 0},
/* H */   {-1, 1,-3, 1, 1,-2,-2, 6,-2, 0, 0,-2,-2, 2,_M, 0, 3, 2,-1,-1, 0,-2,-3, 0, 0, 2},
/* I */   {-1,-2,-2,-2,-2, 1,-3,-2, 5, 0,-2, 2, 2,-2,_M,-2,-2,-2,-1, 0, 0, 4,-5, 0,-1,-2},
/* J */   { 0, 0, 0, 0, 0, 0, 0, 0, 0, 0, 0, 0, 0, 0,_M, 0, 0, 0, 0, 0, 0, 0, 0, 0, 0, 0},
/* K */   {-1, 0,-5, 0, 0,-5,-2, 0,-2, 0, 5,-3, 0, 1,_M,-1, 1, 3, 0, 0, 0,-2,-3, 0,-4, 0},
/* L */   {-2,-3,-6,-4,-3, 2,-4,-2, 2, 0,-3, 6, 4,-3,_M,-3,-2,-3,-3,-1, 0, 2,-2, 0,-1,-2},
/* M */   {-1,-2,-5,-3,-2, 0,-3,-2, 2, 0, 0, 4, 6,-2,_M,-2,-1, 0,-2,-1, 0, 2,-4, 0,-2,-1},
/* N */   { 0, 2,-4, 2, 1,-4, 0, 2,-2, 0, 1,-3,-2, 2,_M,-1, 1, 0, 1, 0, 0,-2,-4, 0,-2, 1},
/* O */   {_M,_M,_M,_M,_M,_M,_M,_M,_M,_M,_M,_M,_M,_M, 0,_M,_M,_M,_M,_M,_M,_M,_M,_M,_M,_M},
/* P */   { 1,-1,-3,-1,-1,-5,-1, 0,-2, 0,-1,-3,-2,-1,_M, 6, 0, 0, 1, 0, 0,-1,-6, 0,-5, 0},
/* Q */   { 0, 1,-5, 2, 2,-5,-1, 3,-2, 0, 1,-2,-1, 1,_M, 0, 4, 1,-1,-1, 0,-2,-5, 0,-4, 3},
/* R */   {-2, 0,-4,-1,-1,-4,-3, 2,-2, 0, 3,-3, 0, 0,_M, 0, 1, 6, 0,-1, 0,-2, 2, 0,-4, 0},
/* S */   { 1, 0, 0, 0, 0,-3, 1,-1,-1, 0, 0,-3,-2, 1,_M, 1,-1, 0, 2, 1, 0,-1,-2, 0,-3, 0},
/* T */   { 1, 0,-2, 0, 0,-3, 0,-1, 0, 0, 0,-1,-1, 0,_M, 0,-1,-1, 1, 3, 0, 0,-5, 0,-3, 0},
/* U */   { 0, 0, 0, 0, 0, 0, 0, 0, 0, 0, 0, 0, 0, 0,_M, 0, 0, 0, 0, 0, 0, 0, 0, 0, 0, 0},
/* V */   { 0,-2,-2,-2,-2,-1,-1,-2, 4, 0,-2, 2, 2,-2,_M,-1,-2,-2,-1, 0, 0, 4,-6, 0,-2,-2},
/* W */   {-6,-5,-8,-7,-7, 0,-7,-3,-5, 0,-3,-2,-4,-4,_M,-6,-5, 2,-2,-5, 0,-6,17, 0, 0,-6},
/* X */   { 0, 0, 0, 0, 0, 0, 0, 0, 0, 0, 0, 0, 0, 0,_M, 0, 0, 0, 0, 0, 0, 0, 0, 0, 0, 0},
/* Y */   {-3,-3, 0,-4,-4, 7,-5, 0,-1, 0,-4,-1,-2,-2,_M,-5,-4,-4,-3,-3, 0,-2, 0, 0,10,-4},
/* Z */   { 0, 1,-5, 2, 3,-5, 0, 2,-2, 0, 0,-2,-1, 1,_M, 0, 3, 0, 0, 0, 0,-2,-6, 0,-4, 4}
};
```

```
/*
 */
#include <stdio.h>
#include <ctype.h>

#define  MAXJMP    16         /* max jumps in a diag */
#define  MAXGAP    24         /* don't continue to penalize gaps larger than this */
#define  JMPS      1024       /* max jmps in an path */
#define  MX        4          /* save if there's at least MX-1 bases since last jmp */

#define  DMAT      3          /* value of matching bases */
#define  DMIS      0          /* penalty for mismatched bases */
#define  DINS0     8          /* penalty for a gap */
#define  DINS1     1          /* penalty per base */
#define  PINS0     8          /* penalty for a gap */
#define  PINS1     4          /* penalty per residue */

struct jmp {
        short           n[MAXJMP];      /* size of jmp (neg for dely) */
        unsigned short  x[MAXJMP];      /* base no. of jmp in seq x */
};                                      /* limits seq to 2^16 -1 */

struct diag {
        int             score;          /* score at last jmp */
        long            offset;         /* offset of prev block */
        short           ijmp;           /* current jmp index */
        struct jmp      jp;             /* list of jmps */
};

struct path {
        int     spc;            /* number of leading spaces */
        short   n[JMPS];/* size of jmp (gap) */
        int     x[JMPS];/* loc of jmp (last elem before gap) */
};

char            *ofile;                 /* output file name */
char            *namex[2];              /* seq names: getseqs() */
char            *prog;                  /* prog name for err msgs */
char            *seqx[2];               /* seqs: getseqs() */
int             dmax;                   /* best diag: nw() */
int             dmax0;                  /* final diag */
int             dna;                    /* set if dna: main() */
int             endgaps;                /* set if penalizing end gaps */
int             gapx, gapy;             /* total gaps in seqs */
int             len0, len1;             /* seq lens */
int             ngapx, ngapy;           /* total size of gaps */
int             smax;                   /* max score: nw() */
int             *xbm;                   /* bitmap for matching */
long            offset;                 /* current offset in jmp file */
struct  diag    *dx;                    /* holds diagonals */
struct  path    pp[2];                  /* holds path for seqs */

char            *calloc(), *malloc(), *index(), *strcpy();
char            *getseq(), *g_calloc();
```

```
/* Needleman-Wunsch alignment program
 *
 * usage: progs file1 file2
 *    where file1 and file2 are two dna or two protein sequences.
 *    The sequences can be in upper- or lower-case an may contain ambiguity
 *    Any lines beginning with ';', '>' or '<' are ignored
 *    Max file length is 65535 (limited by unsigned short x in the jmp struct)
 *    A sequence with 1/3 or more of its elements ACGTU is assumed to be DNA
 *    Output is in the file "align.out"
 *
 * The program may create a tmp file in /tmp to hold info about traceback.
 * Original version developed under BSD 4.3 on a vax 8650
 */
#include "nw.h"
#include "day.h"

static    _dbval[26] = {
          1,14,2,13,0,0,4,11,0,0,12,0,3,15,0,0,0,5,6,8,8,7,9,0,10,0
};

static    _pbval[26] = {
          1, 2|(1<<('D'-'A'))|(1<<('N'-'A')), 4, 8, 16, 32, 64,
          128, 256, 0xFFFFFFF, 1<<10, 1<<11, 1<<12, 1<<13, 1<<14,
          1<<15, 1<<16, 1<<17, 1<<18, 1<<19, 1<<20, 1<<21, 1<<22,
          1<<23, 1<<24, 1<<25|(1<<('E'-'A'))|(1<<('Q'-'A'))
};

main(ac, av)                                                              main
          int       ac;
          char      *av[];
{
          prog = av[0];
          if (ac != 3) {
                    fprintf(stderr,"usage: %s file1 file2\n", prog);
                    fprintf(stderr,"where file1 and file2 are two dna or two protein sequences.\n");
                    fprintf(stderr,"The sequences can be in upper- or lower-case\n");
                    fprintf(stderr,"Any lines beginning with ';' or '<' are ignored\n");
                    fprintf(stderr,"Output is in the file \"align.out\"\n");
                    exit(1);
          }
          namex[0] = av[1];
          namex[1] = av[2];
          seqx[0] = getseq(namex[0], &len0);
          seqx[1] = getseq(namex[1], &len1);
          xbm = (dna)? _dbval : _pbval;

          endgaps = 0;                    /* 1 to penalize endgaps */
          ofile = "align.out";            /* output file */

          nw();           /* fill in the matrix, get the possible jmps */
          readjmps();     /* get the actual jmps */
          print();        /* print stats, alignment */

          cleanup(0);     /* unlink any tmp files */
}
```

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                                              nw
{
    char        *px, *py;           /* seqs and ptrs */
    int         *ndely, *dely;      /* keep track of dely */
    int         ndelx, delx;        /* keep track of delx */
    int         *tmp;               /* for swapping row0, row1 */
    int         mis;                /* score for each type */
    int         ins0, ins1;         /* insertion penalties */
    register    id;                 /* diagonal index */
    register    ij;                 /* jmp index */
    register    *col0, *col1;       /* score for curr, last row */
    register    xx, yy;             /* index into seqs */

    dx = (struct diag *)g_calloc("to get diags", len0+len1+1, sizeof(struct diag));

    ndely = (int *)g_calloc("to get ndely", len1+1, sizeof(int));
    dely = (int *)g_calloc("to get dely", len1+1, sizeof(int));
    col0 = (int *)g_calloc("to get col0", len1+1, sizeof(int));
    col1 = (int *)g_calloc("to get col1", len1+1, sizeof(int));
    ins0 = (dna)? DINS0 : PINS0;
    ins1 = (dna)? DINS1 : PINS1;

    smax = -10000;
    if (endgaps) {
            for (col0[0] = dely[0] = -ins0, yy = 1; yy <= len1; yy++) {
                    col0[yy] = dely[yy] = col0[yy-1] - ins1;
                    ndely[yy] = yy;
            }
            col0[0] = 0;        /* Waterman Bull Math Biol 84 */
    }
    else
            for (yy = 1; yy <= len1; yy++)
                    dely[yy] = -ins0;

    /* fill in match matrix
     */
    for (px = seqx[0], xx = 1; xx <= len0; px++, xx++) {
            /* initialize first entry in col
             */
            if (endgaps) {
                    if (xx == 1)
                            col1[0] = delx = -(ins0+ins1);
                    else
                            col1[0] = delx = col0[0] - ins1;
                    ndelx = xx;
            }
            else {
                col1[0] = 0;
                delx = -ins0;
                ndelx = 0;
            }
```

...nw

```
for (py = seqx[1], yy = 1; yy < = len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 > = dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) > = dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 > = delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) > = delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

...nw

```
                id = xx - yy + len1 - 1;
                If (mis > = delx && mis > = dely[yy])
                        col1[yy] = mis;
                else if (delx > = dely[yy]) {
                        col1[yy] = delx;
                        ij = dx[id].ijmp;
                        if (dx[id].jp.n[0] && (!dna || (ndelx > = MAXJMP
                        && xx > dx[id].jp.x[ij]+MX) || mis > dx[id].score+DINS0)) {
                                dx[id].ijmp++;
                                If (++ij > = MAXJMP) {
                                        writejmps(id);
                                        ij = dx[id].ijmp = 0;
                                        dx[id].offset = offset;
                                        offset += sizeof(struct jmp) + sizeof(offset);
                                }
                        }
                        dx[id].jp.n[ij] = ndelx;
                        dx[id].jp.x[ij] = xx;
                        dx[id].score = delx;
                }
                else {
                        col1[yy] = dely[yy];
                        ij = dx[id].ijmp;

        if (dx[id].jp.n[0] && (!dna || (ndely[yy] > = MAXJMP
                        && xx > dx[id].jp.x[ij]+MX) || mis > dx[id].score+DINS0)) {
                                dx[id].ijmp++;
                                if (++ij > = MAXJMP) {
                                        writejmps(id);
                                        ij = dx[id].ijmp = 0;
                                        dx[id].offset = offset;
                                        offset += sizeof(struct jmp) + sizeof(offset);
                                }
                        }
                        dx[id].jp.n[ij] = -ndely[yy];
                        dx[id].jp.x[ij] = xx;
                        dx[id].score = dely[yy];
                }
                if (xx == len0 && yy < len1) {
                        /* last col
                        */
                        if (endgaps)
                                col1[yy] -= ins0+ins1*(len1-yy);
                        if (col1[yy] > smax) {
                                smax = col1[yy];
                                dmax = id;
                        }
                }
        }
        if (endgaps && xx < len0)
                col1[yy-1] -= ins0+ins1*(len0-xx);
        If (col1[yy-1] > smax) {
                smax = col1[yy-1];
                dmax = id;
        }
        tmp = col0; col0 = col1; col1 = tmp;
        }
        (void) free((char *)ndely);
        (void) free((char *)dely);
        (void) free((char *)col0);
        (void) free((char *)col1);
}
```

```
/*
 *
 * print() -- only routine visible outside this module
 *
 * static:
 * getmat() -- trace back best path, count matches: print()
 * pr_align() -- print alignment of described in array p[]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC     3
#define P_LINE  256     /* maximum output line */
#define P_SPC   3       /* space between name or num and seq */

extern  _day[26][26];
int     olen;           /* set output line length */
FILE    *fx;            /* output file */

print()
{
        int     lx, ly, firstgap, lastgap;      /* overlap */

        if ((fx = fopen(ofile, "w")) == 0) {
                fprintf(stderr, "%s: can't write %s\n", prog, ofile);
                cleanup(1);
        }
        fprintf(fx, "<first sequence: %s (length = %d)\n", namex[0], len0);
        fprintf(fx, "<second sequence: %s (length = %d)\n", namex[1], len1);
        olen = 60;
        lx = len0;
        ly = len1;
        firstgap = lastgap = 0;
        if (dmax < len1 - 1) {          /* leading gap in x */
                pp[0].spc = firstgap = len1 - dmax - 1;
                ly -= pp[0].spc;
        }
        else if (dmax > len1 - 1) {     /* leading gap in y */
                pp[1].spc = firstgap = dmax - (len1 - 1);
                lx -= pp[1].spc;
        }
        if (dmax0 < len0 - 1) {         /* trailing gap in x */
                lastgap = len0 - dmax0 -1;
                lx -= lastgap;
        }
        else if (dmax0 > len0 - 1) {    /* trailing gap in y */
                lastgap = dmax0 - (len0 - 1);
                ly -= lastgap;
        }
        getmat(lx, ly, firstgap, lastgap);
        pr_align();
}
```

**print**

```
/*
 * trace back the best path, count matches
 */
static

getmat(lx, ly, firstgap, lastgap)
        int     lx, ly;                     /* "core" (minus endgaps) */
        int     firstgap, lastgap;          /* leading trailing overlap */
{
        int             nm, i0, i1, siz0, siz1;
        char            outx[32];
        double          pct;
        register        n0, n1;
        register char   *p0, *p1;

        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;

        nm = 0;
        while ( *p0 && *p1 ) {
                if (siz0) {
                        p1++;
                        n1++;
                        siz0--;
                }
                else if (siz1) {
                        p0++;
                        n0++;
                        siz1--;
                }
                else {
                        if (xbm[*p0-'A']&xbm[*p1-'A'])
                                nm++;
                        if (n0++ == pp[0].x[i0])
                                siz0 = pp[0].n[i0++];
                        if (n1++ == pp[1].x[i1])
                                siz1 = pp[1].n[i1++];
                        p0++;
                        p1++;
                }
        }

        /* pct homology:
         * if penalizing endgaps, base is the shorter seq
         * else, knock off overhangs and take shorter core
         */
        if (endgaps)
                lx = (len0 < len1)? len0 : len1;
        else
                lx = (lx < ly)? lx : ly;
        pct = 100.*(double)nm/(double)lx;
        fprintf(fx, "\n");
        fprintf(fx, "< %d match%s in an overlap of %d: %.2f percent similarity\n",
                nm, (nm == 1)? "" : "es", lx, pct);
```

getmat

23

```
        fprintf(fx, " < gaps in first sequence: %d", gapx);
        if (gapx) {
                (void) sprintf(outx, " (%d %s%s)",
                        ngapx, (dna)? "base":"residue", (ngapx == 1)? "":"s");
                fprintf(fx," %s", outx);
        }

        fprintf(fx, ", gaps in second sequence: %d", gapy);
        if (gapy) {
                (void) sprintf(outx, " (%d %s%s)",
                        ngapy, (dna)? "base":"residue", (ngapy == 1)? "":"s");
                fprintf(fx," %s", outx);
        }
        if (dna)
                fprintf(fx,
                "\n < score: %d (match = %d, mismatch = %d, gap penalty = %d + %d per base)\n",
                smax, DMAT, DMIS, DINS0, DINS1);
        else
                fprintf(fx,
                "\n < score: %d (Dayhoff PAM 250 matrix, gap penalty = %d + %d per residue)\n",
                smax, PINS0, PINS1);
        if (endgaps)
                fprintf(fx,
                " < endgaps penalized. left endgap: %d %s%s, right endgap: %d %s%s\n",
                firstgap, (dna)? "base" : "residue", (firstgap == 1)? "" : "s",
                lastgap, (dna)? "base" : "residue", (lastgap == 1)? "" : "s");
        else
                fprintf(fx, " < endgaps not penalized\n");
}

static          nm;             /* matches in core -- for checking */
static          lmax;           /* lengths of stripped file names */
static          ij[2];          /* jmp index for a path */
static          nc[2];          /* number at start of current line */
static          ni[2];          /* current elem number -- for gapping */
static          siz[2];
static char     *ps[2];         /* ptr to current element */
static char     *po[2];         /* ptr to next output char slot */
static char     out[2][P_LINE]; /* output line */
static char     star[P_LINE];   /* set by stars() */

/*
* print alignment of described in struct path pp[]
*/
static
pr_align()
{
        int             nn;     /* char count */
        int             more;
        register        i;

        for (i = 0, lmax = 0; i < 2; i++) {
                nn = stripname(namex[i]);
                if (nn > lmax)
                        lmax = nn;

                nc[i] = 1;
                ni[i] = 1;
                siz[i] = ij[i] = 0;
                ps[i] = seqx[i];
                po[i] = out[i];
        }
```

...getmat

pr_align

```
for (nn = nm = 0, more = 1; more; ) {                                      ...pr_align
        for (i = more = 0; i < 2; i++) {
                /*
                 * do we have more of this sequence?
                 */
                if (!*ps[i])
                        continue;

                more++;

                if (pp[i].spc) {        /* leading space */
                        *po[i]++ = ' ';
                        pp[i].spc--;
                }
                else if (siz[i]) {      /* in a gap */
                        *po[i]++ = '-';
                        siz[i]--;
                }
                else {                  /* we're putting a seq element
                                         */
                        *po[i] = *ps[i];
                        if (islower(*ps[i]))
                                *ps[i] = toupper(*ps[i]);
                        po[i]++;
                        ps[i]++;

                        /*
                         * are we at next gap for this seq?
                         */
                        if (ni[i] == pp[i].x[ij[i]]) {
                                /*
                                 * we need to merge all gaps
                                 * at this location
                                 */
                                siz[i] = pp[i].n[ij[i]++];
                                while (ni[i] == pp[i].x[ij[i]])
                                        siz[i] += pp[i].n[ij[i]++];
                        }
                        ni[i]++;
                }
        }
        if (++nn == olen || !more && nn) {
                dumpblock();
                for (i = 0; i < 2; i++)
                        po[i] = out[i];
                nn = 0;
        }
    }
}

/*
 * dump a block of lines, including numbers, stars: pr_align()
 */
static
dumpblock()                                                               dumpblock
{
        register  i;

        for (i = 0; i < 2; i++)
                *po[i]-- = '\0';
```

```
            (void) putc('\n', fx);
            for (i = 0; i < 2; i++) {
                    if (*out[i] && (*out[i] != ' ' || *(po[i]) != ' ')) {
                            if (i == 0)
                                    nums(i);
                            if (i == 0 && *out[1])
                                    stars();
                            putline(i);
                            if (i == 0 && *out[1])
                                    fprintf(fx, star);
                            if (i == 1)
                                    nums(i);
                    }
            }
    }

    /*
     * put out a number line: dumpblock()
     */
    static
    nums(ix)
            int      ix;        /* index in out[] holding seq line */
    {
            char            nline[P_LINE];
            register        i, j;
            register char   *pn, *px, *py;

            for (pn = nline, i = 0; i < lmax+P_SPC; i++, pn++)
                    *pn = ' ';
            for (i = nc[ix], py = out[ix]; *py; py++, pn++) {
                    if (*py == ' ' || *py == '-')
                            *pn = ' ';
                    else {
                            if (i%10 == 0 || (i == 1 && nc[ix] != 1)) {
                                    j = (i < 0)? -i : i;
                                    for (px = pn; j; j /= 10, px--)
                                            *px = j%10 + '0';
                                    if (i < 0)
                                            *px = '-';
                            }
                            else
                                    *pn = ' ';
                            i++;
                    }
            }
            *pn = '\0';
            nc[ix] = i;
            for (pn = nline; *pn; pn++)
                    (void) putc(*pn, fx);
            (void) putc('\n', fx);
    }

    /*
     * put out a line (name, [num], seq, [num]): dumpblock()
     */
    static
    putline(ix)
            int      ix;
    {
```

```c
        int             i;
        register char   *px;

        for (px = namex[ix], i = 0; *px && *px != ':'; px++, i++)
                (void) putc(*px, fx);
        for (; i < lmax+P_SPC; i++)
                (void) putc(' ', fx);

        /* these count from 1:
         * ni[] is current element (from 1)
         * nc[] is number at start of current line
         */
        for (px = out[ix]; *px; px++)
                (void) putc(*px&0x7F, fx);
        (void) putc('\n', fx);
}


/*
 * put a line of stars (seqs always in out[0], out[1]): dumpblock()
 */
static
stars()
```
```c
{
        int             i;
        register char   *p0, *p1, cx, *px;

        if (!*out[0] || (*out[0] == ' ' && *(po[0]) == ' ') ||
            !*out[1] || (*out[1] == ' ' && *(po[1]) == ' '))
                return;
        px = star;
        for (i = lmax+P_SPC; i; i--)
                *px++ = ' ';

        for (p0 = out[0], p1 = out[1]; *p0 && *p1; p0++, p1++) {
                if (isalpha(*p0) && isalpha(*p1)) {

                        if (xbm[*p0-'A']&xbm[*p1-'A']) {
                                cx = '*';
                                nm++;
                        }
                        else if (!dna && _day[*p0-'A'][*p1-'A'] > 0)
                                cx = '.';
                        else
                                cx = ' ';
                }
                else
                        cx = ' ';
                *px++ = cx;
        }
        *px++ = '\n';
        *px = '\0';
}
```

stars

```
/*
 * strip path or prefix from pn, return len: pr_align()
 */
static
stripname(pn)
        char    *pn;    /* file name (may be path) */
{
        register char   *px, *py;

        py = 0;
        for (px = pn; *px; px++)
                if (*px == '/')
                        py = px + 1;
        if (py)
                (void) strcpy(pn, py);
        return(strlen(pn));

}
```

stripname

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char     *jname = "/tmp/homgXXXXXX";              /* tmp file for jmps */
FILE     *fj;

int      cleanup();                               /* cleanup tmp file */
long     lseek();

/*
 * remove any tmp file if we blow
 */
cleanup(i)
        int      i;
{
        if (fj)
                (void) unlink(jname);
        exit(i);
}

/*
 * read, return ptr to seq, set dna, len, maxlen
 * skip lines starting with ';', '<', or '>'
 * seq in upper or lower case
 */
char     *
getseq(file, len)
        char     *file;    /* file name */
        int      *len;     /* seq len */
{
        char             line[1024], *pseq;
        register char    *px, *py;
        int              natgc, tlen;
        FILE             *fp;

        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0) {
                fprintf(stderr,"%s: malloc() failed to get %d bytes for %s\n", prog, tlen+6, file);
                exit(1);
        }
        pseq[0] = pseq[1] = pseq[2] = pseq[3] = '\0';
```

cleanup

getseq

```
        py = pseq + 4;
        *len = tlen;
        rewind(fp);

        while (fgets(line, 1024, fp)) {
                if (*line == ';' || *line == '<' || *line == '>')
                        continue;
                for (px = line; *px != '\n'; px++) {
                        if (isupper(*px))
                                *py++ = *px;
                        else if (islower(*px))
                                *py++ = toupper(*px);
                        if (index("ATGCU",*(py-1)))
                                natgc++;
                }
        }
        *py++ = '\0';
        *py = '\0';
        (void) fclose(fp);
        dna = natgc > (tlen/3);
        return(pseq+4);
}
```

<div align="right">

g_calloc

</div>

```
char    *
g_calloc(msg, nx, sz)
        char    *msg;           /* program, calling routine */
        int     nx, sz;         /* number and size of elements */
{
        char            *px, *calloc();

        if ((px = calloc((unsigned)nx, (unsigned)sz)) == 0) {
                if (*msg) {
                        fprintf(stderr, "%s: g_calloc() failed %s (n=%d, sz=%d)\n", prog, msg, nx, sz);
                        exit(1);
                }
        }
        return(px);
}

/*
 * get final jmps from dx[] or tmp file, set pp[], reset dmax: main()
 */
```

<div align="right">

readjmps

</div>

```
readjmps()
{
        int             fd = -1;
        int             siz, i0, i1;
        register i, j, xx;

        if (fj) {
                (void) fclose(fj);
                if ((fd = open(jname, O_RDONLY, 0)) < 0) {
                        fprintf(stderr, "%s: can't open() %s\n", prog, jname);
                        cleanup(1);
                }
        }
        for (i = i0 = i1 = 0, dmax0 = dmax, xx = len0; ; i++) {
                while (1) {
                        for (j = dx[dmax].ijmp; j >= 0 && dx[dmax].jp.x[j] >= xx; j--)
                                ;
```

```
                              if (j < 0 && dx[dmax].offset && fj) {
                                      (void) lseek(fd, dx[dmax].offset, 0);
                                      (void) read(fd, (char *)&dx[dmax].jp, sizeof(struct jmp));
                                      (void) read(fd, (char *)&dx[dmax].offset, sizeof(dx[dmax].offset));
                                      dx[dmax].ijmp = MAXJMP-1;
                              }
                              else
                                      break;
                      }
                      if (i >= JMPS) {
                              fprintf(stderr, "%s: too many gaps in alignment\n", prog);
                              cleanup(1);
                      }
                      if (j >= 0) {
                              siz = dx[dmax].jp.n[j];
                              xx = dx[dmax].jp.x[j];
                              dmax += siz;
                              if (siz < 0) {                  /* gap in second seq */
                                      pp[1].n[i1] = -siz;
                                      xx += siz;

                                      /* id = xx - yy + len1 - 1
                                       */
                                      pp[1].x[i1] = xx - dmax + len1 - 1;
                                      gapy++;
                                      ngapy -= siz;
            /* ignore MAXGAP when doing endgaps */
                                      siz = (-siz < MAXGAP || endgaps)? -siz : MAXGAP;
                                      i1++;
                              }
                              else if (siz > 0) {  /* gap in first seq */
                                      pp[0].n[i0] = siz;
                                      pp[0].x[i0] = xx;
                                      gapx++;
                                      ngapx += siz;
            /* ignore MAXGAP when doing endgaps */
                                      siz = (siz < MAXGAP || endgaps)? siz : MAXGAP;
                                      i0++;
                              }
                      }
                      else
                              break;
              }

              /* reverse the order of jmps
               */
              for (j = 0, i0--; j < i0; j++, i0--) {
                      i = pp[0].n[j]; pp[0].n[j] = pp[0].n[i0]; pp[0].n[i0] = i;
                      i = pp[0].x[j]; pp[0].x[j] = pp[0].x[i0]; pp[0].x[i0] = i;
              }
              for (j = 0, i1--; j < i1; j++, i1--) {
                      i = pp[1].n[j]; pp[1].n[j] = pp[1].n[i1]; pp[1].n[i1] = i;
                      i = pp[1].x[j]; pp[1].x[j] = pp[1].x[i1]; pp[1].x[i1] = i;
              }
              if (fd >= 0)
                      (void) close(fd);
              if (fj) {
                      (void) unlink(jname);
                      fj = 0;
                      offset = 0;
              }
      }
```

```
/*
 * write a filled jmp struct offset of the prev one (if any): nw()
 */
writejmps(ix)
        int     ix;
{
        char    *mktemp();

        if (!fj) {
                if (mktemp(jname) < 0) {
                        fprintf(stderr, "%s: can't mktemp() %s\n", prog, jname);
                        cleanup(1);
                }
                if ((fj = fopen(jname, "w")) == 0) {
                        fprintf(stderr, "%s: can't write %s\n", prog, jname);
                        exit(1);
                }
        }
        (void) fwrite((char *)&dx[ix].jp, sizeof(struct jmp), 1, fj);
        (void) fwrite((char *)&dx[ix].offset, sizeof(dx[ix].offset), 1, fj);
}
```

writejmps

Table 2A

| PRO | XXXXXXXXXXXXXXX | (Length = 15 amino acids) |
| Comparison Protein | XXXXXYYVYYYY | (Length = 12 amino acids) |

% amino acid sequence identity =

(the number of identically matching amino acid residues between the two polypeptide sequences as determined by ALIGN-2) divided by (the total number of amino acid residues of the PRO polypeptide) =

5 divided by 15 = 33.3%

Table 2B

| PRO | XXXXXXXXXX | (Length = 10 amino acids) |
| Comparison Protein | XXXXXYYYYYYZZYZ | (Length = 15 amino acids) |

% amino acid sequence identity =

(the number of identically matching amino acid residues between the two polypeptide sequences as determined by ALIGN-2) divided by (the total number of amino acid residues of the PRO polypeptide) =

5 divided by 10 = 50%

Table 2C

| PRO-DNA | NNNNNNNNNNNNNN | (Length = 14 nucleotides) |
|---|---|---|
| Comparison DNA | NNNNNNLLLLLLLLLL | (Length = 16 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

6 divided by 14 = 42.9%

Table 2D

| PRO-DNA | NNNNNNNNNNNN | (Length = 12 nucleotides) |
|---|---|---|
| Comparison DNA | NNNNLLLW | (Length = 9 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

4 divided by 12 = 33.3%

"Percent (%) amino acid sequence identity" with respect to the PRO polypeptide sequences identified herein is defined as the percentage of amino acid residues in a candidate sequence that are identical with the amino acid residues in a PRO sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST. BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2, wherein the complete source code for the ALIGN-2 program is provided in Table 1. The ALIGN-2 sequence comparison computer program was authored by Genentech, Inc., and the source code shown in Table 1 has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations, Tables 2A-2B demonstrate how to calculate the % amino acid sequence identity of the amino acid sequence designated "Comparison Protein" to the amino acid sequence designated "PRO".

Unless specifically stated otherwise, all % amino acid sequence identity values used herein are obtained as described above using the ALIGN-2 sequence comparison computer program. However. % amino acid sequence identity may also be determined using the sequence comparison program NCBI-BLAST2 (Altschul et al., Nucleic Acids Res.,. 25:3389-3402 (1997)). The NCBI-BLAST2 sequence comparison program may be downloaded from http://www.ncbi.nlm.nih-gov. NCBI-BLAST2 uses several search parameters, wherein all of those search parameters are set to default values including, for example, unmask = yes, strand = all, expected occurrences =10, minimum low complexity length = 1515, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

In addition, % amino acid sequence identity may also be determined using the WU-BLAST-2 computer program (Altschul et al., Methods in Enzymology, 266:460-480 (1996)). Most of the WU-BLAST-2 search parameters are set to the default values. Those not set to default values, *i.e.,* the adjustable parameters, are set with the following values: overlap span = 1, overlap fraction = 0.125, word threshold (T) =11, and scoring matrix = BLOSUM62. For purposes herein, a % amino acid sequence identity value is determined by dividing (a) the number of matching identical amino acids residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. Forexample, in the statement "a polypeptide comprising an amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

"PRO variant polypeptide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, more preferably at least about 81% nucleic acid sequence identity, more preferably at least about 82% nucleic acid sequence identity, more preferably at least about 83% nucleic acid sequence identity, more preferably at least about 84% nucleic acid sequence identity, more preferably at least about 85% nucleic acid sequence identity, more preferably at least about 86% nucleic acid sequence identity, more preferably at least about 87% nucleic acid sequence identity, more preferably at least about 88% nucleic acid sequence identity, more preferably at least about 89% nucleic acid sequence identity, more preferably at least about 90% nucleic acid sequence identity, more preferably at least about 91% nucleic acid sequence identity, more preferably at least about 92% nucleic acid sequence identity, more preferably at least about 93% nucleic acid sequence identity, more preferably at least about 94% nucleic acid sequence identity, more preferably at least about 95% nucleic acid sequence identity, more preferably at least about 96% nucleic acid sequence identity, more preferably at least about 97% nucleic acid sequence identity, more preferably at least about 98% nucleic acid sequence identity and yet more preferably at least about 99% nucleic acid sequence identity with the nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, often at least about 60 nucleotides in length, more often at least about 90 nucleotides in length, more often at least about 120 nucleotides in length, more often at least about 150 nucleotides in length, more often at least about 180 nucleotides in length, more often at least about 210 nucleotides in length, more often at least about 240 nucleotides in length, more often at least about 270 nucleotides in length, more often at least about 300 nucleotides in length, more often at least about 450 nucleotides in length, more often at least about 600 nucleotides in length, more often at least about 900 nucleotides in length, or more.

"Percent (%) nucleic acid sequence identity" with respect to the PRO polypeptide-encoding nucleic acid sequences identified herein is defined as the percentage of nucleotides in a candidate sequence that are identical with the nucleotides in a PRO polypeptide-encoding nucleic acid sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % nucleic acid sequence identity values are obtained as described below by using the sequence comparison computer program ALIGN-2, wherein the complete source code for the ALIGN-2 program is provided in Table 1. The ALIGN-2 sequence comparison computer program was authored by Genentech, Inc., and the source code shown in Table 1 has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z$$

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 2C-2D demonstrate how to calculate the % nucleic acid sequence identity of the nucleic acid sequence designated "Comparison DNA" to the nucleic acid sequence designated "PRO-DNA".

Unless specifically stated otherwise, all % nucleic acid sequence identity values used herein are obtained as described above using the ALIGN-2 sequence comparison computer program. However, % nucleic acid sequence identity may also be determined using the sequence comparison program NCBI-BLAST2 (Altschul et al., Nucleic Acids Res., 25:3389-3402 (1997)). The NCBI-BLAST2 sequence comparison program may be downloaded from http://www.ncbi.nlm.nih.gov. NCBI-BLAST2 uses several search parameters, wherein all of those search parameters are set to default values including, for example, unmask = yes, strand = all, expected occurrences = 10, minimum low complexity length = 15/5, multi-pass c-value =0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z$$

where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In addition, to nucleic acid sequence identity values may also be generated using the WU-BLAST-2 computer program (Altschul et al., Methods in Enzymology, 266:460-480 (1996)). Most of the WU-BLAST-2 search parameters are set to the default values. Those not set to default values; *i.e.*, the adjustable parameters, are set with the following values: overlap span = 1. overlap fraction = 0.125, word threshold (T) = 11, and scoring matrix = BLOSUM62. For purposes herein, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-ettcoding nucleic acid and the comparison nucleic acid molecule of interest (*i.e.*, the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

In other embodiments, PRO variant polynucleotides are nucleic acid molecules that encode an active PRO polypeptide and which are capable of hybridizing, preferably under stringent hybridization and wash conditions, to nucleotide sequences encoding the full-length PRO polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), or Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO: 64), Figure 66 (SEQ ID NO:66), Figure 68 (SEQ ID NO:68) or Figure 70 (SEQ ID NO:70), respectively. PRO variant polypeptides may be those that are encoded by a PRO variant polynucleotide.

The term "positives", in the context of the amino acid sequence identity comparisons performed as described above, includes amino acid residues in the sequences compared that are not only identical, but also those that have similar properties. Amino acid residues that score a positive value to an amino acid residue of interest are those that are either identical to the amino acid residue of interest or are a preferred substitution (as defined in Table 3 below) of the amino acid residue of interest.

For purposes herein, the % value of positives of a given amino acid sequence A to, with, or against a given amino acid sequence B (which can alternatively be phrased as a given amino acid sequence A that has or comprises a certain % positives to, with, or against a given amino acid sequence B) is calculated as follows:

$$100 \text{ times the fraction } X/Y$$

where X is the number of amino acid residues scoring a positive value as defined above by the sequence alignment program ALIGN-2 in that program's alignment of A and B. and where Y is the total number of amino acid residues in B. It will be appreciated that where the length of amino acid sequence A is not equal to the length of amino acid sequence B, the % positives of A to B will not equal the % positives of B to A.

"Isolated," when used to describe the various polypeptides disclosed herein, means polypeptide that has been identified and separated and/or recovered from a component of its natural environment. Preferably, the isolated polypeptide is free of association with all components with which it is naturally associated. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO natural environment will not be present. Ordinarily, however, isolated polypeptide will be prepared by at least one purification step.

An "isolated" nucleic acid molecule encoding a PRO polypeptide or an "isolated" nucleic acid encoding an anti-PRO antibody, is a nucleic acid molecule that is identified and separated from at least one contaminant nucleic acid molecule with which it is ordinarily associated in the natural source of the PRO-encoding nucleic acid or the anti-PRO-encoding nucleic acid. Preferably, the isolated nucleic acid is free of association with all components with which it is naturally associated. An isolated PRO-encoding nucleic acid molecule or an anti-PRO-encoding nucleic acid molecule is other than in the form or setting in which it is found in nature. Isolated nucleic acid molecules therefore are distinguished from the PRO-encoding nucleic acid molecule or the anti-PRO-encoding nucleic acid molecule as it exists in natural cells. However, an isolated nucleic acid molecule encoding a PRO polypeptide or an anti-PRO antibody includes PRO-nucleic acid molecules and anti-PRO-nucleic acid molecules contained in cells that ordinarily express PRO polypeptides or express anti-PRO antibodies where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO197, anti-PRO207, anti-PRO226, anti-PRO232, anti-PR0243, anti-PRO256, anti-PRO269, anti-PRO274, anti-PRO304, anti-PRO339, anti-PRO1558. anti-PRO779, anti-PRO1185, anti-PRO1245, anti-PRO1759, anti-PRO5775, anti-PRO7133, anti-PRO7168, anti-PRO5725. anti-PRO202, anti-PR0206, anti-PRO264, anti-PR0313, anti-PRO342, anti-PRO542, anti-PRO773, anti-PRO861, anti-PRO1216, anti-PRO1686, anti-PRO1800, anti-PRO3562, anti-PRO9850, anti-PRO539, anti-PR04316 or anti-PR04980 monoclonal antibodies (including antagonist, and neutralizing antibodies), anti-PRO197, anti-PRO207, anti-PRO226, anti-PRO232, anti-PRO243, anti-PR0256, anti-PRO269, anti-PR0274, anti-PR0304, anti-PR0339, anti-PRO1558, anti-PR0779, anti-PRO1185, anti-PRO1245, anti-PRO1759, anti-PRO5775, anti-PR07133, anti-PR07168, anti-PRO5725, anti-PRO202, anti-PRO206, anti-PRO264, anti-PRO313, anti-PRO342, anti-PRO542, anti-PRO773, anti-PRO861, anti-PRO1216, anti-PR01686, anti-PRO1800, anti-PRO3562, anti-PRO9850, anti-PRO539, anti-PRO4316 or anti-PRO4980 antibody compositions with polyepitopic specificity, single chain anti-PRO197, anti-PRO207, anti-PRO226, anti-PRO232, anti-PRO243, anti-PRO256, anti-PRO269, anti-PRO274, anti-PR0304, anti-PRO339, anti-PRO1558, anti-PRO779, anti-PRO1185, anti-PRO1245, anti-PRO1759, anti-PRO5775, anti-PRO7133, anti-PR07168, anti-PR05725, anti-PRO202, anti-PRO206, anti-PRO264, anti-PR0313, anti-PRO342, anti-PR0542, anti-PRO773, anti-PRO861, anti-PRO1216, anti-PRO1686, anti-PRO1800, anti-PR03562, anti-PRO9850, anti-PRO539, anti-PRO4316 or anti-PRO4980 antibodies, and fragments of anti-PRO197, anti-PRO207, anti-PRO226, anti-PRO232, anti-PRO243, anti-PRO256, anti-PR0269, anti-PRO274, anti-PRO304, anti-PR0339, anti-PRO1558, anti-PRO779, anti-PRO1185, anti-PRO1245, anti-PRO1759, anti-PRO5775, anti-PRO7133, anti-PR07168, anti-PRO5725, anti-PRO202, anti-PRO206, anti-PRO264, anti-PRO313, anti-PRO342, anti-PRO542, anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibodies (see below). The term "monoclonal antibody" as used herein refers to an antibody obtained from a population of substantially homogeneous antibodies, Le., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are presentin an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, *see* Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50˚C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42˚C; or (3) employ 50% formamide. 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42˚C, with washes at 42˚C in 0.2 x SSC (sodium chloride/sodium citrate) and 50% formamide at 55˚C, followed by a high-stringency wash consisting of 0.1 x SSC containing EDTA at 55˚C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent than those described above. An example of moderately stringent conditions is overnight incubation at 37˚C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 35˚C-50˚C. The skilled artisan will recognize how to adjust the temperature, ionic strength, etc. as necessary to accommodate factors such as probe length and the like.

The term "epitope tagged" when used herein refers to a chimeric polypeptide comprising a PRO197, PR0207, PR0226, PRO232, PRO243, PRO256, PR0269, PR0274, PR0304, PR0339, PRO1558. PR0779, PRO1185, PRO1245, PRO1759, PRO5775, PRO7133, PRO7168, PRO5725, PRO202, PRO206, PRO264, PR0313, PR0342, PRO542, PR0773, PRO861, PRO1216, PRO1686, PRO1800, PRO3562, PRO9850, PRO539, PRO4316 or PRO4980 polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

"Active" or "activity" for the purposes herein refers to form(s) of PRO197, PRO207, PRO226, PRO232, PRO243, PRO256, PRO269. PRO274, PR0304, PR0339, PRO1558, PRO779, PRO1185. PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PR03562, PRO9850, PRO539, PRO4316 or PR04980 polypeptides which retain a biological and/or an immunological activity/property of a native or naturally-occurring PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PR04980 polypeptide, wherein "biological" activity refers to a function (either inhibitory or stimulatory) caused by a native or naturally-occurring PRO197, PRO207, PR0226, PRO232, PR0243, PRO256, PRO269, PRO274, PRO304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PR03562, PRO9850, PR0539, PRO4316 or PRO4980 polypeptide other than the ability to induce the production of an antibody against an antigenic epitope possessed by a native or naturally-occurring PRO197, PRO207, PRO226, PRO232, PRO243, PRO256, PRO269, PRO274, PRO304, PRO339, PRO1558, PRO779, PRO1185, PRO1245. PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide and an "immunological" activity refers to the ability to induce the production of an antibody against an antigenic epitope possessed by a native or naturally-occurring PRO197. PRO207, PRO226, PRO232, PRO243, PRO256, PR0269, PR0274, PRO304, PRO339, PRO1558, PRO779, PRO1185, PRO1245, PRO1759, PRO5775, PR07133, PRO7168, PRO5725, PRO202, PRO206, PRO264, PR0313, PR0342, PRO542, PR0773, PRO861, PRO1216, PRO1686, PRO1800, PRO3562, PRO9850, PRO539, PR04316 or PRO4980 polypeptide.

"Biological activity" in the context of an antibody or another antagonist molecule that can be identified by the screening assays disclosed herein (*e.g.*, an organic or inorganic small molecule, peptide, etc.) is used to refer to the ability of such molecules to bind or complex with the polypeptides encoded by the amplified genes identified herein, or otherwise interfere with the interaction of the encoded polypeptides with other cellular proteins or otherwise interfere with the transcription or translation of a PRO197, PRO207, PRO226, PRO232, PRO243, PRO256, PR0269, PR0274, PO0304, PR0339, PRO1558, PRO779, PRO1185, PRO1245, PRO1759, PRO5775, PR07133, PRO7168, PRO5725, PRO202, PRO206, PRO264, PR0313, PRO342, PR0542, PR0773, PRO861, PRO1216, PRO1686, PRO1800, PR03562, PR09850, PRO539, PRO4316 or PRO4980 polypeptide. A preferred biological activity is growth inhibition of a target tumor cell. Another preferred biological activity is cytotoxic activity resulting in the death of the target tumor cell.

The term "biological activity" in the context of a PRO197, PRO207, PRO226, PR0232, PR0243, PR0256, PR0269, PR0274, PR0304, PR0339, PRO1558, PR0779, PRO1185, PRO1245, PRO1759, PR05775, PR07133, PRO7168, PRO5725, PRO202, PRO206, PRO264, PRO313, PR0342, PRO542, PR0773, PRO861, PRO1216, PRO1686, PRO1800, PRO3562, PRO9850, PRO539, PRO4316 or PRO4980 polypeptide means the ability of a PRO197, PRO207, PRO226, PRO232, PRO243, PRO256, PRO269, PRO274, PR0304, PR0339, PRO1558, PR0779, PRO1185, PRO1245, PRO1759, PRO5775, PR07133, PRO7168, PR05725, PRO202, PR0206, PRO264, PRO313, PR0342, PR0542, PRO773, PRO861, PRO1216, PRO1686, PRO1800, RO3562, PRO9850, PRO539, PR04316 or PRO4980 polypeptide to induce neoplastic cell growth or uncontrolled cell growth.

The phrase "immunological activity" means immunological cross-reactivity with at least one epitope of a PRO197, PRO207, PRO226, PRO232, PRO243, PRO256, PR0269, PRO274, PRO304, PRO339, PRO1558, PR0779, PRO1185, PRO1245, PRO1759, PRO5775, PR07133, PR07168, PRO5725, PRO202, PR0206, PR0264, PRO313, PRO342, PRO542, PRO773, PRO861, PRO1216, PRO1686, PRO1800, PR03562, PRO9850, PRO539, PRO4316 or PRO4980 polypeptide.

"Immunological cross-reactivity" as used herein means that the candidate polypeptide is capable of competitively inhibiting the qualitative biological activity of a PRO197, PRO207, PRO226, PRO232, PR0243, PRO256, PRO269, PRO274, PR0304, PRO339, PRO1558, PR0779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PR03562, PRO9850, PRO539, PRO4316 or PRO4980 polypeptide having this activity with polyclonal antisera raised against the known active PRO197, PRO207, PRO226, PRO232, PRO243, PRO256, PR0269, PR0274, PR0304, PR0339, PRO1558, PRO779, PRO1185, PRO1245, PRO1759, PRO5775, PRO7133, PR07168, PRO5725, PRO202, PRO206, PRO264, PR0313, PRO342, PRO542, PRO773, PRO861, PRO1216, PRO1686, PRO1800, PRO3562, PRO9850, PRO539, PRO4316 or PRO4980 polypeptide. Such antisera are prepared in conventional fashion by injecting goats or rabbits, for example, subcutaneously with the known active analogue in complete Freund's adjuvant, followed by booster intraperitoneal or subcutaneous injection in incomplete Freunds. The immunological cross-reactivity preferably is "specific", which means that the binding affinity of the immunologically cross-reactive molecule (*e.g.*, antibody) identified, to the corresponding PRO197, PRO207, PR0226, PR0232, PRO243, PRO256, PRO269, PR0274, PR0304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide is significantly higher (preferably at least about 2-times, more preferably at least about 4-times, even more preferably at least about 8-times, most preferably at least about 10-times higher) than the binding affinity of that molecule to any other known native polypeptide.

The term "antagonist" is used in the broadest sense, and includes any molecule that partially or fully blocks, inhibits, or neutralizes a biological activity of a native PRO197, PRO207, PRO226, PR0232, PRO243, PRO256, PR0269, PRO274, PRO304, PRO339, PRO1558, PRO779,PRO1185, PRO1245, PRO1759, PR05775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide disclosed herein or the transcription or translation thereof. Suitable antagonist molecules specifically include antagonist antibodies or antibody fragments, fragments, peptides, small organic molecules, anti-sense nucleic acids, etc. Included are methods for identifying antagonists of a PRO197, PRO207, PRO226, PRO232, PRO243, PRO256, PRO269, PRO274, PR0304, PRO339. PRO1558, PR0779, PRO1185, PRO1245, PRO1759, PRO5775, PRO7133, PR07168, PR05725, PR0202, PRO206, PRO264, PRO313, PR0342, PR0542, PR0773, PRO861, PRO1216, PRO1686, PRO1800, PRO3562, PRO9850, PRO539, PR04316 or PRO4980 polypeptide with a candidate antagonist molecule and measuring a detectable change in one or more biological activities normally associated with the PRO197, PR0207, PR0226, PRO232, PRO243, PRO256, PRO269, PRO274, PRO304, PRO339, PRO1558, PRO779, PRO1185, PRO1245, PRO1759, PRO5775, PR07133, PR07168, PR05725, PRO202, PR0206, PRO264, PRO313, PRO342, PRO542, PRO773, PRO861, PRO1216, PRO1686, PRO1800, PRO3562, PRO9850, PR0539, PR04316 or PR04980 polypeptide.

A "small molecule" is defined herein to have a molecular weight below about 500 Daltons.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myeloma. The term "antibody" is used in the broadest sense and specifically covers, without limitation, intact monoclonal antibodies, polyclonal antibodies, multispecific antibodies (*e.g.*, bispecific antibodies) formed from at least two intact antibodies, and antibody fragments so long as they exhibit the desired biological activity.

"Native antibodies* and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR) regions. The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat et al., NIH Publ. No.91-3242, Vol. I, pages 647-669 (1991)). The constant domains are not involved directly in binding an antibody to an antigen, but exhibit various effector functions, such as participation of the antibody in antibody-dependent cellular toxicity.

The term "hypervariable region" when used herein refers to the amino acid residues of an antibody which are responsible for antigen-binding. The hypervariable region comprises amino acid residues from a "complementarity determining region" or "CDR" (*i.e.*, residues 24-34 (L1), 50-56 (L2) and 89-97 (L3) in the light chain variable domain and 31-35 (H1), 50-65 (H2) and 95-102 (H3) in the heavy chain variable domain; Kaba et al., Sequences of Proteins of Immunological Interest, 5th Ed. Public Health Service, National Institute of Health, Bethesda, MD. [1991]) and/or those residues from a "hypervariable loop" (*i.e.*, residues 26-32 (L1), 50-52 (L2) and 91-96 (L3) in the light chain variable domain and 26-32 (H1), 53-55 (H2) and 96-101 (H3) in the heavy chain variable domain ; Clothia and Lesk, J. Mol. Biol., 196:901-917 [1987]). "Framework" or "FR" residues are those variable domain residues other than the hypervariable region residues as herein defined.

"Antibody fragments" comprise a portion of an intact antibody, preferably the antigen binding or variable region of the intact antibody. Examples of antibody fragments include Fab, Fab', $F(ab')_2$, and Fv fragments; diabodies; linear antibodies (Zapata et al., Protein Eng., 8(10):1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an $F(ab')_2$ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the $V_H$-$V_L$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), *e.g.*, IgG1, IgG2, IgG3, IgG4, IgA, and IgA2. The beavy-chain constant domains that correspond to the different classes of immunoglobulins are called α, δ, ε, γ, and μ, respectively. The subunit structures and three-dimensional configurations of different classes of immunoglobulins are well known.

The term "monoclonal antibody" as used herein refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 [1975], or may be made by recombinant DNA methods (*see, e.g.,* U.S. Patent No. 4,816,567). The "monoclonal antibodies" may also be isolated from phage antibody libraries using the techniques described in Clackson et al., Nature, 352:624-628 [1991] and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA 81:6851-6855 [1984]).

"humanized" forms of non-human (*e.g.*, murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, *see,* Jones et al., Nature, 321:522-525 (1986); Reichmann et al., Nature, 332: 323-329 [1988]; and Presta, Curr. Op. Struct. Biol., 2:593-596 (1992). The humanized antibody includes a PRIMATIZED™ antibody wherein the antigen-binding region of the antibody is derived from an antibody produced by immunizing macaque monkeys with the antigen of interest.

"Single-chain Fv" or "sFv" antibody fragments comprise the $V_H$ and $V_L$ domains of antibody, wherein these domains are present in a single polypeptide chain. Preferably, the Fv polypeptide further comprises a polypeptide linker between the $V_H$ and $V_L$ domains which enables the sFv to form the desired structure for antigen binding. For a review of sFv *see* Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain ($V_H$) connected to a light-chain variable domain ($V_L$) in the same polypeptide chain ($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90: 6444-6448 (1993).

An "isolated" antibody is one which has been identified and separated and/or recovered from a component of its natural environment. Contaminant components of its natural environment are material which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labeled" antibody. The label may be detectable by itself (*e.g.*, radioisotope labels or fluorescent labels) or, in the case of an enzymatic label, may catalyze chemical alteration of a substrate compound or composition which is detectable. Radionuclides that can serve as detectable labels include, for example, I-131, I-123, I-125, Y-90, Re-188, Re-186, At-211, Cu-67, Bi-212, and Pd-109. The label may also be a non-detectable entity such as a toxin.

By "solid phase" is meant a non-aqueous matrix to which the antibody of the present invention can adhere. Examples of solid phases encompassed herein include those formed partially or entirely of glass (*e.g.*, controlled pore glass), polysaccharides (*e.g.*, agarose), polyacrylamides, polystyrene, polyvinyl alcohol and silicones. In certain embodiments, depending on the context, the solid phase can comprise the well of an assay plate; in others it is a purification column (*e.g.*, an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO197. PRO207, PR0226, PR0232, PRO243, PRO256, PRO269, PR0274, PR0304, PRO339, PRO1558, PRO779, PRO1185, PRO1245, PRO1759, PRO5775, PR07133, PRO7168, PRO5725, PRO202, PRO206, PRO264, PRO313, PRO342, PRO542, PRO773, PRO861, PRO1216, PRO1686, PRO1800, PR03562, PRO9850, PRO539, PRO4316 or PRO4980 polypeptide or antibody thereto and, optionally, a chemotherapeutic agent) to a mammal. The components of the liposome are commonly arranged in a bilayer formation, similar to the lipid arrangement of biological membranes.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i.e.*, is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

II. Compositions and Methods of the Invention

A. Full-length PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 and PRO4980 polypeptides

The present application provides newly identified and isolated nucleotide sequences encoding polypeptides referred to in the present application as PRO197, PRO207, PRO226, PRO232, PRO243, PRO256, PR0269, PRO274, PRO304, PRO339, PRO1558, PRO779, PRO1185, PRO1245, PRO1759, PRO5775, PRO7133, PR07168, PRO5725, PRO202, PRO206, PR0264, PRO313, PRO342, PRO542, PR0773, PRO861, PRO1216, PRO1686, PRO1800, PRO3562, PRO9850, PRO539, PRO4316 and PRO4980. In particular, cDNA encoding PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PR0264, PRO313, PRO342, PRO542, PRO773, PRO861, PRO1216, PRO1686, PRO1800, PRO3562, PRO9850, PRO539, PRO4316 and PR04980 polypeptides has been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the proteins encoded by the herein disclosed nucleic acid sequences as well as all further native homologues and variants included in the foregoing definition of PRO197, PRO207, PRO226, PRO232, PRO243, PRO256, PRO269, PRO274, PRO304, PRO339, PRO1558, PR0779, PRO1185, PRO1245, PRO1759, PRO5775, PR07133, PR07168, PR05725, PRO202, PR0206, PRO264, PRO313, PRO342, PRO542, PRO773, PRO861, PRO1216, PRO1686, PRO1800, PRO3562, PRO9850, PRO539, PRO4316 and PRO4980 will be referred to as "PRO197", "PRO207", "PRO226", "PRO232", "PRO243", "PR0256", "PRO269", "PR0274", "PR0304", "PR0339", "PRO1558", "PRO779", "PRO1185", "PRO1245", "PRO1759", "PRO5775", "PRO7133", "PRO7168", "PRO5725", "PRO202", "PRO206", "PRO264", "PRO313", "PRO342", "PRO542", "PRO773", "PRO861 ", "PRO1216", "PRO1686", "PRO1800", "PRO3562", "PRO9850", "PRO539", "PRO4316" or "PRO4980", regardless of their origin or mode of preparation.

As disclosed in the Examples below, cDNA clones have been deposited with the ATCC, with the exception of known clones: DNA30869, DNA34405, DNA36995, DNA43320, DNA38649, DNA56505, DNA48303, DNA50798, DNA66489, DNA80896, DNA96791, and DNA58725. The actual nucleotide sequence of the clones can readily be determined by the skilled artisan by sequencing of the deposited clone using routine methods in the art. The predicted amino acid sequences can be determined from the nucleotide sequences using routine skill. For the PRO197, PRO207, PRO226, PRO232, PRO243, PRO256, PRO269, PRO274, PR0304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PR04316 or PR04980 polypeptides and encoding nucleic acid described herein, Applicants have identified what are believed to be the reading frames best identifiable with the sequence information available at the time.

B. PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 and PRO4980 Variants

In addition to the full-length native sequence PRO197, PRO207, PRO226, PR0232, PRO243, PRO256, PRO269, PRO274, PR0304, PRO339, PRO1558, PRO779, PRO1185, PRO1245, PRO1759, PRO5775, PRO7133, PRO7168, PRO5725, PRO202, PRO206, PRO264, PR0313, PRO342, PRO542, PRO773, PR0861, PRO1216, PRO1686, PRO1800, PRO3562, PRO9850, PRO539, PRO4316 and PRO4980 polypeptides described herein, it is contemplated that PRO197, PRO207, PR0226, PRO232, PRO243, PRO256, PRO269, PR0274, PR0304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PR0773, PRO861, PRO1216, PRO1686, PRO1800, PRO3562, PRO9850, PR0539, PRO4316 and PRO4980 variants can be prepared. PRO197, PRO207, PRO226, PRO232, PRO243, PRO256, PRO269, PRO274, PR0304, PRO339, PRO1558, PR0779, PRO1185, PRO1245, PRO1759, PRO5775, PR07133, PRO7168, PRO5725, PRO202, PRO206, PRO264, PRO313, PRO342, PRO542, PRO773, PRO861, PRO1216, PRO1686, PRO1800, PR03562, PRO9850, PRO539, PR04316 and PR04980 variants can be prepared by introducing appropriate nucleotide changes into the PRO197, PR0207, PRO226, PR0232, PRO243, PRO256, PRO269, PRO274, PR0304, PR0339, PRO1558, PR0779, PRO1185, PRO1245, PRO1759, PRO5775, PRO7133, PRO7168, PRO5725, PRO202, PRO206, PRO264, PRO313, PRO342, PRO542, PRO773, PRO861, PRO1216. PRO1686, PRO1800, PRO3562, PRO9850, PR0539, PRO4316 or PRO4980 DNA, and/or by synthesis of the desired PRO197, PR0207, PRO226, PR0232, PRO243, PR0256, PRO269, PR0274, PRO304, PR0339, PRO1558, PR0779, PRO1185, PRO1245, PRO1759, PRO5775, PRO7133, PRO7168, PR05725, PRO202, PRO206, PRO264, PRO313, PRO342, PRO542, PR0773, PRO861, PRO1216, PRO1686, PRO1800, PR03562, PR09850, PR0539, PRO4316 or PR04980 polypeptide. Those skilled in the art will appreciate that amino acid changes may alter post-translational processes of the PRO197, PR0207, PRO226, PR0232, PRO243, PR0256, PRO269, PR0274, PR0304, PR0339, PRO1558, PRO779, PRO1185, PRO1245, PRO1759, PRO5775, PRO7133, PRO7168. PR05725, PRO202, PRO206, PR0264, PRO313, PRO342, PRO542, PRO773, PRO861, PRO1216, PRO1686, PRO1800, PRO3562, PR09850, PR0539, PRO4316 or PRO4980, such as changing the number or position of glycosylation sites or altering the membrane anchoring characteristics.

Variations in the native full-length sequence PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PR07168, PRO5725, PRO202, PRO206, PRO264, PRO313, PRO342, PRO542, PRO773, PRO861, PRO1216. PRO1686, PRO1800, PRO3562, PRO9850, PRO539, PRO4316 or PRO4980 or in various domains of the PRO197, PRO207, PRO226, PRO232, PRO243, PRO256, PRO269, PRO274, PRO304, PRO339, PRO1558, PRO779, PRO1185, PRO1245, PRO1759, PRO5775, PR07133, PRO7168, PR05725, PR0202, PRO206, PR0264, PRO313, PRO342, PRO542, PRO773, PRO861, PRO1216, PRO1686, PRO1800, PRO3562, PRO9850, PRO539, PR04316 or PRO4980 described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO197, PRO207, PR0226, PRO232, PRO243, PRO256, PRO269, PR0274, PR0304, PR0339, PRO1559, PRO779, PRO1185, PRO1245, PRO1759, PRO5775, PRO7133, PRO7168, PRO5725, PRO202, PRO206, PRO264, PRO313, PRO342, PRO542, PRO773, PR0861, PRO1216, PRO1686, PRO1800, PR03562, PRO9850, PR0539, PRO4316 or PRO4980 that results in a change in the amino acid sequence of the PRO197, PRO207, PRO226, PRO232, PRO243, PRO256, PRO269, PRO274, PRO304, PRO339, PRO1558, PRO779, PRO1185, PRO1245, PRO1759, PR05775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 as compared with the native sequence PRO197, PRO207, PRO226, PRO232, PRO243, PRO256, PR0269, PRO274, PRO304, PRO339, PRO1558. PRO779, PRO1185, PRO1245, PRO1759, PRO5775, PRO7133, PR07168, PRO5725, PRO202, PRO206, PRO264, PR0313, PRO342, PRO542, PR0773, PRO861, PRO1216, PRO1686, PRO1800, PRO3562, PRO9850, PRO539, PRO4316 or PRO4980. Optionally the variation is by substitution of at least one amino acid with any other amino acid in one or more of the domains of the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PR05725, PRO202, PRO206, PR0264, PR0313, PR0342, PRO542, PR0773, PR0861, PRO1216, PRO1686, PRO1800, PRO3562, PR09850, PRO539, PRO4316 or PR04980. Guidance in determining which amino acid residue may be inserted, substituted or deleted without adversely affecting the desired activity may be found by comparing the sequence of the PRO197, PRO207, PRO226, PRO232, PRO243, PRO256, PRO269, PRO274, PR0304, PR0339, PRO1558, PR0779, PRO1185, PRO1245, PRO1759, PRO5775, PR07133, PR07168, PRO5725, PRO202, PRO206, PRO264, PR0313, PR0342, PRO542, PRO773, PR0861, PRO1216, PRO1686, PRO1800, PR03562, PR09850, PR0539, PRO4316 or PR04980 with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO197, PRO207, PR0226, PRO232, PRO243, PRO256, PRO269, PRO274, PR0304, PR0339, PRO1558, PR0779, PRO1185, PRO1245, PRO1759, PR05775, PRO7133, PR07168, PRO5725, PRO202, PRO206, PRO264, PR0313, PRO342, PRO542, PR0773, PRO861, PRO1216, PRO1686, PRO1800, PR03562, PR09850, PR0539, PRO4316 and PRO4980 polypeptide fragments are provided herein. Such fragments may be truncated at the N-terminus or C-terminus, or may lack internal residues, for example, when compared with a full-length native protein. Certain fragments lack amino acid residues that are not essential for a desired biological activity of the PRO197, PR0207, PRO226, PRO232, PRO243, PRO256, PRO269, PRO274, PRO304, PRO339, PRO1558, PR0779, PRO1185, PRO1245, PRO1759, PR05775, PRO7133, PRO7168, PR05725, PRO202, PRO206, PRO264, PR0313, PR0342, PRO542, PR0773, PRO861, PRO1216, PRO1686, PRO1800, PR03562, PR09850, PRO539, PR04316 or PRO4980 polypeptide.

PRO197, PRO207, PRO226, PRO232, PR0243, PRO256, PRO269, PRO274, PRO304, PR0339, PRO1558, PR0779, PRO1185, PRO1245, PRO1759, PRO5775, PRO7133, PR07168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 fragments may be prepared by any or a number of conventional techniques. Desired peptide fragments may be chemically synthesized. An alternative approach involves generating PRO197, PRO207, PR0226, PRO232, PRO243, PRO256, PRO269, PRO274, PR0304, PR0339, PRO1558, PR0779, PRO1185, PRO1245, PRO1759, PRO5775, PRO7133, PR07168, PRO5725, PRO202, PRO206. PRO264, PR0313, PR0342, PRO542, PRO773, PRO861, PRO1216, PRO1686, PRO1800, PRO3562, PRO9850, PRO539, PRO4316 or PRO4980 fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO197, PRO207, PRO226, PRO232, PRO243, PRO256, PRO269, PRO274, PR0304, PRO339, PRO1558, PRO779, PRO1185, PRO1245, PRO1759, PRO5775, PRO7133, PRO7168, PRO5725, PR0202, PRO206, PRO264, PR0313, PR0342, PRO542, PRO773, PRO861, PRO1216, PRO1686, PRO1800, PRO3562. PR09850, PR0539, PRO4316 or PRO4980 polypeptide fragments share at least one biological and/or immunological activity with the native PRO197, PRO207, PRO226, PRO232, PR0243, PRO256, PRO269, PRO274, PR0304, PR0339, PRO1558, PR0779, PRO1185, PRO1245, PRO1759, PRO5775, PRO7133, PRO7168, PR05725, PRO202, PRO206, PR0264, PR0313, PRO342, PRO542, PRO773, PRO861, PRO1216, PRO1686, PRO1800, PRO3562, PRO9850, PRO539, PR04316 or PR04980 polypeptide.

In particular embodiments, conservative substitutions of interest are shown in Table 3 under the heading of preferred substitutions. If such substitutions result in a change in biological activity, then more substantial changes, denominated exemplary substitutions in Table 3, or as further described below in reference to amino acid classes, are introduced and the products screened.

Table 3

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe, thr; ser | phe |

(continued)

| Original Residue | Exemplary Substitutions | Preferred Substitutions |
|---|---|---|
| Val (V) | ile; leu; met; phe; ala; norleucine | leu |

Substantial modifications in function or immunological identity of the polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.
The variations can be made using methods known in the art such as oligonucleotide-mediated (site-directed) mutagenesis, alanine scanning, and PCR mutagenesis. Site-directed mutagenesis [Carter et al., Nucl. Acids Res., 13: 4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO197, PRO207, PRO226, PRO232, PRO243, PRO256, PRO269, PRO274, PR0304, PR0339, PRO1558, PRO779, PRO1185, PRO1245, PRO1759, PRO5775, PRO7133, PRO7168, PRO5725, PRO202, PRO206, PRO264, PR0313, PR0342, PR0542, PRO773, PRO861, PRO1216, PRO1686, PRO1800, PR03562, PR09850, PR0539, PRO4316 or PRO4980 variant DNA.
Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

C. Modifications of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 and PRO4980

Covalent modifications of PRO197, PRO207, PRO226, PRO232, PRO243, PRO256, PRO269, PRO274, PR0304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 and PRO4980 are included within the scope of this invention. One type of covalent modification includes reacting targeted amino acid residues of a PRO197, PRO207, PR0226, PRO232, PRO243, PRO256, PR0269, PRO274, PRO304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PR03562, PRO9850, PR0539, PR04316 or PR04980 polypeptide with an organic derivatizing agent that is capable of reacting with selected side chains or the N- or C-terminal residues of the PRO197, PRO207, PRO226, PRO232, PRO243, PRO256, PR0269, PRO274, PRO304, PR0339, PRO1558, PRO779, PRO1185, PRO1245, PRO1759, PRO5775, PR07133, PRO7168, PRO5725, PRO202, PRO206, PRO264, PR0313, PRO342, PRO542, PRO773, PRO861, PRO1216, PRO1686, PRO1800, PR03562, PR09850, PR0539, PRO4316 or PRO4980. Derivatization with bifunctional agents is useful, for instance, for crosslinking PRO197, PRO207, PRO226, PRO232, PRO243, PRO256, PR0269, PRO274, PR0304, PRO339, PRO1558, PRO779, PRO1185, PRO1245, PRO1759,

PRO5775, PRO7133, PRO7168, PRO5725, PR0202, PRO206, PRO264, PR0313, PRO342, PRO542, PRO773, PRO861, PRO1216, PRO1686, PRO1800, PRO3562, PRO9850, PRO539, PR04316 or PRO4980 to a water-insoluble support matrix or surface for use in the method for purifying anti-PRO197, anti-PRO207, anti-PRO226, anti-PRO232, anti-PRO243, anti-PRO256, anti-PRO269, anti-PRO274, anti-PR0304, anti-PRO339, anti-PRO1558, anti-PRO779, anti-PRO1185, anti-PRO1245, anti-PRO1759, anti-PRO5775, anti-PR07133, anti-PRO7168, anti-PRO5725, anti-PRO202, anti-PRO206, anti-PRO264, anti-PRO313, anti-PRO342, anti-PRO542, anti-PRO773, anti-PRO861, anti-PRO1216, anti-PRO1686, anti-PRO 1800, anti-PRO3562, anti-PRO9850, anti-PR0539, anti-PRO4316 or anti-PRO4980 antibodies, and vice-versa. Commonly used crosslinking agents include, *e.g.*, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of prone and lysine, phosphorylation of hydroxyl groups of seryl or threonyl residues, methylation of the α-amino groups of lysine, arginine, and histidine side chains [T.E. Creighton, Proteins: Structure and Molecular Properties. W.H. Freeman & Co., San Francisco, pp. 79-86 (1983)], acetylation of the N-terminal amine, and amidation of any C-terminal carboxyl group.

Another type of covalent modification of the PRO197, PRO207, PR0226, PRO232, PRO243, PRO256, PRO269, PRO274, PRO304, PRO339, PRO1558, PRO779, PRO1185, PRO1245, PRO1759, PRO5775, PRO7133, PR07168, PRO5725, PRO202, PRO206, PRO264, PRO313, PR0342, PRO542, PRO773, PRO861, PRO1216, PRO1686, PRO1800, PRO3562, PRO9850, PRO539, PRO4316 or PRO4980 polypeptide included within the scope of this invention comprises altering the native glycosylation pattern of the polypeptide. "Altering the native glycosylation pattern" is intended for purposes herein to mean deleting one or more carbohydrate moieties found in native sequence PRO197, PRO207, PR0226, PRO232, PR0243, PRO256, PR0269, PRO274, PR0304, PRO339, PRO1558, PRO779, PRO1185, PRO1245, PRO1759, PRO5775, PRO7133, PRO7168. PRO5725, PRO202, PRO206, PRO264, PRO313, PRO342, PRO542, PRO773, PRO861, PRO1216, PRO1686, PRO1800, PRO3562, PR09850, PRO539, PRO4316 or PRO4980 (either by removing the underlying glycosylation site or by deleting the glycosylation by chemical and/or enzymatic means), and/or adding one or more glycosylation sites that are not present in the native sequence PRO197, PRO207, PR0226, PRO232, PR0243, PRO256, PRO269, PRO274, PRO304, PR0339, PRO1558, PR0779, PRO1185, PRO1245, PRO1759, PRO5775, PRO7133, PRO7168, PRO5725, PRO202, PRO206, PRO264, PR0313, PR0342, PRO542, PRO773, PRO861, PRO1216, PRO1686, PRO1800, PR03562, PRO9850, PRO539, PRO4316 or PRO4980. In addition, the phrase includes qualitative changes in the glycosylation of the native proteins, involving a change in the nature and proportions of the various carbohydrate moieties present.

Addition of glycosylation sites to the PRO197, PRO207, PRO226, PRO232, PRO243, PRO256, PRO269, PRO274, PRO304, PR0339, PRO1558, PRO779, PRO1185, PRO1245, PRO1759, PR05775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide may be accomplished by altering the amino acid sequence. The alteration may be made, for example, by the addition of, or substitution by, one or more serine or threonine residues to the native sequence PRO197, PRO207, PR0226, PRO232, PR0243, PRO256, PRO269, PRO274, PR0304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 (for O-linked glycosylation sites). The PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PR0264, PR0313, PRO342, PRO542, PRO773, PRO861, PRO1216, PRO1686, PRO1800, PRO3562, PRO9850, PRO539, PRO4316 or PRO4980 amino acid sequence may optionally be altered through changes at the DNA level, particularly by mutating the DNA encoding the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PR04980 polypeptide at preselected bases such that codons are generated that will translate into the desired amino acids.

Another means of increasing the number of carbohydrate moieties on the PRO197, PR0207, PR0226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide is by chemical or enzymatic coupling of glycosides to the polypeptide. Such methods are described in the art, *e.g.*, in WO 87/05330 published 11 September 1987, and in Aplin and Wriston, CRC Crit. Rev. Biochem., pp. 259-306 (1981).

Removal of carbohydrate moieties present on the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133,

PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and cxo-glycosidases as described by Thotakura et al., Meth. Enzymol., 138:350 (1987).

Another type of covalent modification of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 comprises linking the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PR0313, PRO342, PRO542, PRO773, PRO861, PRO1216, PRO1686, PRO1800, PR03562, PR09850, PR0539, PR04316 or PRO4980 polypeptide to one of a variety of nonproteinaceous polymers, *e.g.*, polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO197, PRO207, PRO226, PRO232, PRO243, PRO256, PR0269, PRO274, PR0304, PR0339, PRO1558, PR0779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 of the present invention may also be modified in a way to form a chimeric molecule comprising PRO197, PRO207, PR0226, PRO232, PRO243, PR0256, PR0269, PR0274, PRO304, PRO339, PRO1558, PRO779, PRO1185, PRO1245, PRO1759, PRO5775, PRO7133, PRO7168, PR05725, PRO202. PRO206, PR0264, PR0313, PRO342, PRO542, PR0773, PR0861, PRO1216, PRO1686, PRO1800, PRO3562, PR09850, PRO539, PR04316 or PRO4980 fused to another, heterologous polypeptide or amino acid sequence.

In one embodiment, such a chimeric molecule comprises a fusion of the PRO197, PRO207, PR0226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PR0264, PR0313, PR0342, PRO542, PR0773, PR0861, PRO1216, PRO1686, PRO1800, PR03562, PR09850, PRO539, PRO4316 or PR04980 with a tag polypeptide which provides an epitope to which an anti-tag antibody can selectively bind. The epitope tag is generally placed at the amino- or carboxyl-terminus of the PRO197, PRO207, PR0226, PR0232, PRO243, PRO256, PRO269, PR0274, PR0304, PRO339, PRO1558, PR0779, PRO1185, PRO1245, PRO1759, PRO5775, PRO7133, PR07168, PRO5725, PRO202, PRO206, PR0264, PR0313, PRO342, PR0542, PRO773, PRO861, PRO1216, PRO1686, PRO1800, PR03562, PRO9850, PRO539, PRO4316 or PRO4980, The presence of such epitope-tagged forms of the PRO197, PRO207, PR0226, PRO232, PRO243, PRO256, PRO269, PRO274, PRO304, PR0339, PRO1558, PR0779, PRO1185, PRO1245, PRO1759, PRO5775, PR07133, PR07168, PRO5725, PRO202, PRO206, PRO264, PR0313, PR0342, PRO542, PR0773, PRO861, PRO1216, PRO1686, PRO1800, PRO3562, PRO9850, PRO539, PRO4316 or PR04980 can be detected using an antibody against the tag polypeptide. Also, provision of the epitope tag enables the PRO197, PR0207, PR0226, PRO232, PRO243, PRO256, PR0269, PR0274, PRO304, PRO339, PRO1558, PRO779, PRO1185, PRO1245, PRO1759, PRO5775, PRO7133, PRO7168, PRO5725, PRO202, PR0264, PRO313, PRO342, PRO542, PR0773, PRO861, PRO1216, PRO1686, PRO1800, PR03562, PR09850, PRO539, PR04316 or PRO4980 to be readily purified by affinity purification using an anti-tag antibody or another type of affinity matrix that binds to the epitope tag. Various tag polypeptides and their respective antibodies are well known in the art. Examples include polyhistidine (poly-His) or poly-histidine-glycine (poly-His-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:1:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6: 1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO197, PRO207, PRO226, PRO232, PRO243, PRO256, PRO269, PR0274, PRO304, PRO339, PRO1558, PR0779, PRO1185, PRO1245, PRO1759, PRO5775, PR07133, PR07168, PRO5725, PR0202, PRO206, PRO264, PR0313, PR0342, PRO542, PRO773, PR0861, PRO1216, PRO1686, PRO1800, PRO3562, PRO9850, PRO539, PRO4316 or PRO4980 with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO197, PRO207, PRO226, PRO232, PRO243, PRO256, PRO269, PRO274, PRO304, PRO339, PRO1558, PRO779, PRO1185, PRO1245, PRO1759, PR05775, PR07133, PR07168, PRO5725, PRO202, PRO206, PRO264, PRO313, PRO342, PRO542,

PRO773, PRO861, PRO1216, PRO1686, PRO1800, PRO3562, PR09850. PR0539, PRO4316 or PRO4980 polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions *see* also, US Patent No. 5,428,130 issued June 27, 1995.

D. Preparation of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 Polypeptides

The description below relates primarily to production of PRO197, PRO207, PR0226, PR0232, PR0243, PRO256, PRO269, PRO274, PR0304, PR0339, PRO1558, PRO779, PRO1185, PRO1245, PRO1759, PRO5775, PRO7133, PR07168, PRO5725, PRO202, PRO206, PRO264, PR0313, PR0342, PRO542, PRO773, PR0861, PRO1216, PRO1686, PRO1800, PRO3562, PRO9850, PR0539, PR04316 or PRO4980 by culturing cells transformed or transfected with a vector containing PRO197, PRO207, PR0226, PR0232, PR0243, PRO256, PRO269, PRO274, PR0304, PR0339, PRO1558, PR0779, PRO1185, PRO1245, PRO1759, PRO5775, PRO7133, PRO7168, PRO5725, PRO202, PRO206, PRO264, PRO313, PR0342. PR0542. PRO773, PRO861, PRO1216, PRO1686, PRO1800, PRO3562, PRO9850, PRO539, PR04316 or PR04980 nucleic acid. It is, of course, contemplated that alternative methods, which are well known in the art, may be employed to prepare PRO197, PRO207, PR0226, PRO232, PRO243, PRO256, PRO269, PRO274, PR0304, PRO339, PRO1558, PR0779, PRO1185, PRO1245, PRO1759, PRO5775, PRO7133, PRO7168, PRO5725, PRO202, PRO206, PR0264, PRO313, PR0342, PR0542, PRO773, PRO861, PRO1216, PRO1686, PRO1800, PRO3562, PRO9850, PRO539, PR04316 or PR04980. For instance, the PRO197, PRO207, PRO226, PRO232, PRO243, PRO256, PRO269, PRO274, PRO304, PRO339, PRO1558, PR0779, PRO1185, PRO1245, PRO1759, PRO5775, PRO7133, PRO7168, PR05725, PRO202, PRO206, PRO264, PR0313, PR0342, PRO542, PRO773, PRO861, PRO1216, PRO1686, PRO1800, PRO3562, PRO9850, PRO539, PRO4316 or PRO4980 sequence, or portions thereof, may be produced by direct peptide synthesis using solid-phase techniques [*see, e.g.,* Stewart et al., Solid-Phase Peptide Synthesis, W.H. Freeman Co., San Francisco, CA (1969); Merrifield, J. Am. Chem. Soc., 85:2149-2154(1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO197, PRO207, PR0226, PRO232, PRO243, PR0256, PRO269, PRO274, PRO304, PRO339, PRO1558, PRO779, PRO1185, PRO1245, PRO1759, PRO5175, PR07133, PRO7168, PRO5725, PRO202, PRO206, PRO264, PR0313, PRO342, PRO542, PRO773, PR0861, PRO1216, PRO1686, PRO1800, PRO3562, PRO9850, PRO539, PRO4316 or PRO4980 may be chemically synthesized separately and combined using chemical or enzymatic methods to produce the full-length PRO197, PRO207, PRO226, PR0232, PRO243, PRO256, PRO269, PR0274, PRO304, PRO339, PRO1558, PRO779, PRO1185, PRO1245, PRO1759, PRO5775, PRO7133, PR07168, PRO5725, PRO202, PRO206, PRO264, PR0313, PR0342, PRO542, PR0773, PRO861, PRO1216, PRO1686, PRO1800, PR03562, PRO9850, PRO539, PRO4316 or PRO4980.

a. Isolation of DNA Encoding a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 Polypeptide

DNA encoding PRO197, PRO207, PRO226, PRO232, PRO243, PRO256, PRO269, PR0274, PRO304, PR0339, PRO1558, PRO779, PRO1185, PRO1245, PRO1759, PRO5775, PR07133, PRO7168, PRO5725, PRO202, PRO206, PRO264, PRO313, PRO342, PRO542, PRO773, PR0861, PRO1216, PRO1686, PRO1800, PRO3562, PR09850, PR0539, PR04316 or PRO4980 may be obtained from a cDNA library prepared from tissue believed to possess the PRO197, PRO207, PR0226, PRO232, PRO243, PRO256, PRO269, PR0274, PR0304, PRO339, PRO1558, PR0779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 mRNA and to express it at a detectable level. Accordingly, human PRO197, human PRO207, human PRO226, human PRO232, human PR0243, human PR0256, human PRO269, human PRO274, human PRO304, human PRO339, human PRO1558, human PRO779, human PRO1185, human PRO1245, human PRO1759, human PRO5775, human PR07133, human PRO7168, human PR05725, human PRO202, human PRO206, human PRO264, human PR0313, human PRO342, human PRO542, human PR0773, human PRO861, human PRO1216, human PRO1686, human PRO1800, human PRO3562, human PR09850, human PRO539, human PRO43160 or human PR04980 DNA can beconveniently obtained from a cDNA library prepared from human tissue, such as described in the Examples. PRO197-, PR0207-, PRO226-, PRO232-, PRO243-, PR0256-, PR0269-, PRO0274-, PRO304-, PR0339-, PRO1558-, PRO779-, PRO1185-, PRO1245-, PRO1759-, PRO5775-, PRO7133-, PRO7168-, PRO5725-, PRO202-, PRO206-, PRO264-, PRO313-, PRO342-, PRO542-, PRO773-, PRO861-, PRO1216-, PRO1686-, PRO1800-, PRO3562-, PRO9850-, PRO539-, PRO4316- or PRO4980-encoding gene may also be obtained from a genomic library or by oligonucleotide synthesis.

Libraries can be screened with probes (such as antibodies to the PRO197, PR0207, PR0226, PRO232, PRO243, PRO256, PRO269, PRO274, PRO304, PRO339, PRO1558, PR0779, PRO1185, PRO1245, PRO1759, PRO5775, PRO7133, PRO7168, PRO5725, PRO202, PRO206, PRO264, PR0313, PR0342, PRO542, PRO773, PRO861, PRO1216, PRO1686, PRO1800, PRO3562, PRO9850, PRO539, PRO4316 or PR04980 polypeptide, or oligonucleotides of at least about 20-80 bases) designed to identify the gene of interest or the protein encoded by it. Screening the cDNA or genomic library with the selected probe may be conducted using standard procedure, such as described in Sambrook et al., Molecular Cloning: A Laboratory Manual (New York: Cold Spring Harbor Laboratory Press, 1989). An alternative means to isolate the gene encoding PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PR0539, PRO4316 or PRO4980 is to use PCR methodology [Sambrook *et al., supra*; Dieffenbach et al., PCR Primer: A Laboratory Manual (Cold Spring Harbor Laboratory Press. 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labelling are well known in the art, and include the use of radiolabels like $^{32}$P-labeled ATP, biotinylation or enzyme labeling. Hybridization conditions, including moderate stringency and high stringency, are provided in Sambrook *et al., supra.*

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook *et al., supra,* to detect precursors and processing intermediates of mRNA that may not have been reverse-transcribed into cDNA.

b. Selection and Transformation of Host Cells

Hostcells are transfected or transformed with expression or cloning vectors described herein for PRO197, PRO207, PRO226, PRO232, PR0243, PRO256, PR0269, PRO274, PRO304, PR0339, PRO1558, PRO779, PRO1185, PRO1245, PRO1759, PRO5775, PRO7133, PRO7168, PRO5725, PRO202, PRO206, PRO264, PRO313, PR0342, PRO542, PRO773, PRO861, PRO1216, PRO1686, PRO1800, PRO3562, PR09850, PRO539, PRO4316 or PRO4980 production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, $CaCl_2$, $CAPO_4$, liposome-mediated and electroporation. Depending on the host cell used, transformation is performed using standard techniques appropriate to such cells. The calcium treatment employing calcium chloride, as described in Sambrook *et al., supra,* or electroporation is generally used for prokaryotes. Infection with *Agrobacterium tumefaciens* is used for transformation of certain plant cells, as described by Shaw et al., Gene, 23: 315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact.,130:946 (1977) and Hsiao et al., Proc. Natl. Acad.-Sci. (USA), 76:3829 (1979). However, other methods for introducing DNA into cells, such as by nuclear microinjection, electroporation, bacterial protoplast fusion with intact cells, or polycations, *e.g*., polybrene, polyornithine, may also be used. For various techniques for transforming mammalian cells, *see,* Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E.*

*coli* K12 strain MM294 (ATCC 31,446); *E. coli* X1776 (ATCC 31,537); *E. coli* strain W3110 (ATCC 27,325) and *E. coli* strain K5 772 (ATCC 53,635). Other suitable prokaryotic host cells include Enterobacteriaceae such as *Escherichia, e.g., E. coli, Enterobacter, Erwinia, Klebsiella, Proteus, Salmonella, e.g., Salmonella typhimurium, Serratia, e.g., Serratia marcescans,* and *Shigella,* as well as *Bacilli* such as *B. subtilis* and *B. licheniformis* (*e.g., B. licheniformis* 41P disclosed in DD 266,710 published 12 April 1989). *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2. which has the complete genotype *tonA* ; *E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan$^r$; E. coli* W3110 strain 37D6, which has the complete genotype *tonA ptr3 phoA E15 (argF-lac)169degP ompT rbs7 ilvG kan$^r$; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO197-, PRO207-, PRO226-, PRO232-. PRO243-, PRO256-, PRO269-, PRO274-, PRO304, PR0339-, PRO1558-, PRO779-, PRO1185-, PRO1245-, PRO1759-, PRO5775-, PRO7133-, PRO7168-, PRO5725-, PR0202-, PRO206-, PRO264-, PRO313-, PRO342-, PRO542-, PRO773-, PRO861-, PRO1216, PRO1686-, PRO1800-, PRO3562-, PRO9850-, PRO539-, PRO4316- or PRO4980- encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology 9:968-975 (1991)) such as, *e.g., K. lactis* (MW98-8C, CBS683, CBS4574; Louvencourt et al., J. Bacteriol., 737 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Vanden Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76: 5259-5263 [1979]); *Schwanniomyces* such as *Schwanniomyces occidentalis* (EP 394,538 published 31 October 1990); and filamentous fungi such as, *e.g., Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81:1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO197, PRO207, PR0226, PRO232, PRO243, PRO256, PRO269, PRO274, PRO304, PRO339, PRO1558, PRO779, PRO1185, PRO1245, PRO1759, PRO5775, PR07133, PRO7168, PRO5725, PR0202, PR0206, PR0264, PR0313, PR0342, PRO542, PRO773, PRO861, PRO1216, PRO1686, PRO1800, PR03562, PRO9850, PRO539, PRO4316 or PRO4980 are derived from multicellular organisms. Examples of invertebrate cells include insect cells such as Drosophila S2 and Spodoptera Sf9. as well as plant cells. Examples of useful mammalian host cell lines include Chinese hamster ovary (CHO) and COS cells. More specific examples include monkey kidney CV1 line transformed by SV40 (COS-7. ATCC CRL 1651); human embryonic kidney line (293 or 293 cells subcloned for growth in suspension culture, Graham et al., J. Gen. Virol., 36:59 (1977)); Chinese hamster ovary cells/-DHFR (CHO), Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver celts (Hep G2, HB 8065); and mouse mammary tumor (MMT 060562, ATCC CCL51). The selection of the appropriate host cell is deemed to be within the skill in the art.

c. Selection and Use of a Replicable Vector

The nucleic acid (*e.g.*, cDNA or genomic DNA) encoding PRO 197, PRO207, PR0226, PRO232, PRO243, PR0256, PR0269, PR0274, PR0304, PR0339, PRO1558, PRO779, PRO1185, PRO1245, PRO1759, PRO5775, PRO7133, PRO7168, PRO5725, PRO202, PRO206, PRO264, PRO313, PR0342, PRO542, PRO773, PRO861, PRO1216, PRO1686, PRO1800. PR03562, PRO9850, PR0539, PR04316 or PR04980 may be inserted into a replicate vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO197, PRO207, PRO226, PRO232, PRO243, PRO256, PRO269, PR0274, PR0304, PR0339, PRO1558, PRO779, PRO1185, PRO1245, PRO1759, PRO5775, PRO7133, PRO7168, PRO5725, PRO202, PRO0206, PRO264, PRO313, PR0342, PRO542, PRO773, PRO861, PRO1216, PRO1686, PRO1800, PRO3562, PRO9850, PR0539, PRO4316 or PRO4980 may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO197-, PRO207-, PRO226-, PRO232-, PRO243-, PRO256-, PRO269-, PR0274-, PRO304-, PR0339-, PRO1558-, PRO779-, PRO1185-, PRO1245-, PRO1759-, PRO5775-, PRO7133-, PR07168-, PRO5725-, PRO202-. PRO206-, PRO264-, PRO313-, PR0342-, PRO542-, PR0773-, PRO861-, PRO1216-, PRO1686-, PRO1800-, PRO3562-, PRO9850-, PRO539-, PRO4316- or PRO4980-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, *e.g.*, the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus. VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.*, ampicillin, neomycin, methotrexate, or tetracycline, (b) complement auxotrophic deficiencies, or (c) supply critical nutrients not available from complex media, *e.g.,* the gene encoding D-alanine racemase for *Bacilli.*

An example of suitable selectable markers for mammalian cells are those that enable the identification of cells competent to take up the PRO197-, PRO207-, PRO226-, PR0232-, PRO243-, PRO256-, PRO269-, PRO274-, PR0304-, PR0339-, PRO1558-, PRO779-, PRO1185-, PRO1245-, PRO1759-, PRO5775-, PR07133-, PR07168-, PRO5725-, PRO202-, PRO206-, PRO264-, PRO313-, PRO342-, PR0542-, PRO773-, PR0861-, PRO1216-, PRO1686-, PRO1800-, PRO3562-, PRO9850-, PRO539-, PRO4316- or PRO4980-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA. 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85: 12 (1977)].

Expression and cloning vectors usually contain a promoter operably linked to the PRO197-, PR0207-, PRO226-, PRO232-, PRO243-, PRO256-, PRO269-, PRO274-, PRO304-, PR0339-, PRO1558-, PRO779-, PRO1185-, PRO1245-, PRO1759-, PRO5775-, PRO7133-, PRO7168-, PRO5725-, PRO202-, PRO206-, PRO264-, PRO313-, PRO342-, PRO542-, PRO773-, PRO861-, PRO1216-, PRO1686-, PRO1800-, PRO3562-, PRO9850-, PRO539-, PRO4316- or PRO4980-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO197, PRO207, PRO226, PR0232, PRO243, PRO256, PR0269, PRO274, PRO304, PRO339, PRO1558, PRO779, PRO1185, PRO1245, PRO1759, PRO5775, PRO7133, PR07168, PRO5725, PR0202, PR0206, PRO264, PR0313, PR0342, PRO542, PR0773, PRO861, PRO1216, PRO1686, PRO1800, PRO3562, PRO9850, PRO539, PRO4316 or PRO4980.

Examples of suitable promoting sequences for use with yeast hosts include the promoters for 3-phosphoglycerate kinase [Hitzeman et al., J. Biol. Chem. 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO197, PRO207, PRO226, PR0232, PRO243, PRO256, PR0269, PR0274, PR0304, PR0339, PRO1558, PRO779, PRO1185, PRO1245, PRO1759, PRO5775, PRO7133, PRO7168, PRO5725, PR0202, PRO206, PRO264, PRO313, PR0342, PRO542, PR0773, PRO861, PRO1216, PRO1686, PRO1800, PRO3562, PR09850, PR0539, PR04316 or PRO4980 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g.*, the actin promoter or an immunoglobulin promoter, and from heat-shock promoters, provided such promoters are compatible with the host cell systems.

Transcription of a DNA encoding the PRO 197, PRO207, PRO226, PRO232, PRO243, PRO256, PRO269, PRO274, PR0304, PR0339, PRO1558, PRO779. PRO1185, PRO1245, PRO1759, PR05775, PRO7133, PRO7168, PRO5725, PR0202, PRO206, PR0264, PR0313, PR0342, PRO542, PRO773, PR0861, PRO1216, PRO1686, PRO1800, PR03562, PRO9850, PRO539, PRO4316 or PRO4980 by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, $\alpha$-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO197, PRO207, PRO226, PRO232, PRO243, PRO256, PRO269, PRO274, PRO304, PR0339, PRO1558, PR0779, PRO1185. PRO1245, PRO1759, PRO5775, PR07133, PR07168, PRO5725, PRO202, PRO206, PRO264, PRO313, PR0342, PRO542, PRO773, PRO861, PRO1216. PRO1686, PRO1800, PRO3562, PR09850, PRO539, PRO4316 or PRO4980 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO197, PRO207, PRO226, PR0232, PRO243, PRO256, PRO269, PR0274, PR0304, PRO339, PRO1558, PR0779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980.

Still other methods, vectors, and host cells suitable for adaptation to the synthesis of PRO197, PRO207, PR0226, PRO232, PR0243, PRO256, PRO269, PRO274, PRO304. PR0339, PRO1558, PR0779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PR04980 in recombinant vertebrate cell culture are described in Gething et al., Nature, 293:620-625 (1981); Mantei et al., Nature, 281: 40-46 (1979); EP 117,060; and EP 117,058.

d. Detecting Gene Amplication/Expression

Gene amplifications and/or expression may be measured in a sample directly, for example, by conventional Southern blotting, Northern blotting to quantitate the transcription of mRNA (thomas, Proc. Natl. Acad. Sci. USA, 77: 5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO197, PRO207, PRO226, PRO232, PRO243, PRO256, PRO269, PRO274, PRO304, PRO339, PRO1558, PRO179, PRO1185, PRO1245, PRO1759, PRO5775, PRO7133, PRO7168, PRO5725, PRO202, PRO206, PRO264, PR0313, PR0342, PRO542, PRO773, PRO861, PRO1216, PRO1686, PRO1800, PRO3562, PRO9850, PRO539,

PRO4316 or PRO4980 polypeptide or against a synthetic peptide based on the DNA sequences provided herein or against an exogenous sequence fused to PRO197, PR0207, PRO226, PRO232, PRO243, PR0256, PRO269, PR0274, PR0304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PR0773, PRO861, PRO1216, PRO1686, PRO1800, PRO3562, PRO9850, PRO539, PRO4316 or PRO4980 DNA and encoding a specific antibody epitope.

e. Purification of Polypeptide

Forms of PRO197, PR0207, PR0226, PRO232, PRO243, PRO256, PRO269, PRO274, PRO304, PR0339, PRO1558, PR0779, PRO1185, PRO1245, PRO1759, PR05775, PR07133, PR07168, PRO5725, PRO202, PRO206, PRO264. PRO313, PR0342, PRO542, PR0773, PRO861, PRO1216, PRO1686, PRO1800, PRO3562, PRO9850, PR0539, PR04316 or PRO4980 may be recovered from culture medium or from host cell lysates. If membrane-bound, it can be released from the membrane using a suitable detergent solution (e.g., Triton-X 100) or by enzymatic cleavage. Cells employed in expression of PRO 197, PRO207, PRO226, PR0232, PRO243, PRO256, PRO269, PRO274, PR0304, PRO339, PRO1558, PRO779, PRO1185, PRO1245, PRO1759, PRO5775, PRO7133, PR07168, PRO5725, PRO202, PRO206, PR0264, PR0313, PR0342, PR0542, PRO773, PRO861, PRO1216, PRO1686, PRO1800, PRO3562, PR09850, PRO539, PRO4316 or PRO4980 can be disrupted by various physical or chemical means, such as freeze-thaw cycling, sonication, mechanical disruption, or cell lysing agents.

It may be desired to purify PRO197, PRO207, PRO226, PRO232, PRO243, PR0256, PR0269, PR0274, PR0304, PR0339, PRO1558, PR0779, PRO1185, PRO1245, PRO1759, PRO5775, PR07133, PRO7168, PRO5725, PRO202, PRO206, PRO264, PRO313, PR0342, PRO542, PR0773, PRO861, PRO1216, PRO1686 PRO1800, PRO3562, PRO9850, PRO539, PR04316 or PR04980 from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO 197, PR0207, PRO226, PRO232, PRO243, PRO256, PR0269, PR0274, PRO304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PR0539, PRO4316 or PRO4980. Various methods of protein purification may be employed and such methods are known in the art and described for example in Deutscher, Methods in Enzymology, 182(1990); Scopes, Protein Purification: Principles and Practice, Springer-Verlag, New York (1982). The purification step(s) selected will depend, for example, on the nature of the production process used and the particular PRO197, PRO207, PRO226, PRO232, PRO243, PRO256, PRO269, PRO274, PR0304, PR0339, PRO1558, PRO779, PRO1185, PRO1245, PRO1759, PRO5775, PRO7133, PRO7168, PRO5725, PRO202, PRO206, PR0264, PRO313, PRO342, PRO542, PRO773, PRO861, PRO1216, PRO1686, PRO1800, PRO3562, PRO9850, PRO539, PRO4316 or PR04980 produced.

E. Amplification of Genes Encoding PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO541, PRO773, PRO861, PRO1216 PRO1686, PRO1800, PRO3562, PRO9850, PRO539, PRO4316 or PRO4980 Polypeptides in Tumor Tissues and Cell Lines

The present invention is based on the identification and characterization of genes that are amplified in certain cancer cells.

The genome of prokaryotic and eukaryotic organisms is subjected to two seemingly conflicting requirements. One is the preservation and propagation of DNA as the genetic information in its original form, to guarantee stable inheritance through multiple generations. On the other hand, cells or organisms must be able to adapt to lasting environmental changes. The adaptive mechanisms can include qualitative or quantitative modifications of the genetic material. Qualitative modifications include DNA mutations, in which coding sequences are altered resulting in a structurally and/or functionally different protein. Gene amplification is a quantitative modification, whereby the actual number of complete coding sequence, *i.e.*, a gene, increases, leading to an increased number of available templates for transcription, an increased number of translatable transcripts, and, ultimately, to an increased abundance of the protein encoded by the amplified gene.

The phenomenon of gene amplification and its underlying mechanisms have been investigated *in vitro* in several prokaryotic and eukaryotic culture systems. The best-characterized example of gene amplification involves the culture of eukaryotic cells in medium containing variable concentrations of the cytotoxic drug methotrexate (MTX). MTX is a folic acid analogue and interferes with DNA synthesis by blocking the enzyme dihydrofolate reductase (DHFR). During the initial exposure to low concentrations of MTX most cells (>99.9%) will die. A small number of cells survive, and are capable of growing in increasing concentrations of MTX by producing large amounts of DHFR-RNA and protein. The basis of this overproduction is the amplification of the single DHFR gene. The additional copies of the gene are found as extrachromosomal copies in the form of small, supernumerary chromosomes (double minutes) or as integrated chromosomal copies.

Gene amplification is most commonly encountered in the development of resistance to cytotoxic drugs (antibiotics for bacteria and chemotherapeutic agents for eukaryotic cells) and neoplastic transformation. Transformation of a eukaryotic cell as a spontaneous event or due to a viral or chemical/environmental insult is typically associated with changes in the genetic material of that cell. One of the most common genetic changes observed in human malignancies are mutations of the p53 protein. p53 controls the transition of cells from the stationary (G1) to the replicative (S) phase and prevents this transition in the presence of DNA damage. In other words, one of the main consequences of disabling p53 mutations is the accumulation and propagation of DNA damage, *i.e.*, genetic changes. Common types of genetic changes in neoplastic cells are, in addition to point mutations, amplifications and gross, structural alterations, such as translocations.

The amplification of DNA sequences may indicate a specific functional requirement as illustrated in the DHFR experimental system Therefore, the amplification of certain oncogenes in malignancies points toward a causative role of these genes in the process of malignant transformation and maintenance of the transformed phenotype. This hypothesis has gained support in recent studies. For example, the *bcl*-2 protein was found to be amplified in certain types of non-Hodgkin's lymphoma. This protein inhibits apoptosis and leads to the progressive accumulation of neoplastic cells. Members of the gene family of growth factor receptors have been found to be amplified in various types of cancers suggesting that overexpression of these receptors may make neoplastic cells less susceptible to limiting amounts of available growth factor. Examples include the amplification of the androgen receptor in recurrent prostate cancer during androgen deprivation therapy and the amplification of the growth factor receptor homologue ERB2 in breast cancer. Lastly, genes involved in intracellular signaling and control of cell cycle progression can undergo amplification during malignant transformation. This is illustrated by the amplification of the *bcl-I* and *ras* genes in various epithelial and lymphoid neoplasms.

These earlier studies illustrate the feasibility of identifying amplified DNA sequences in neoplasms, because this approach can identify genes important for malignant transformation. The case of ERB2 also demonstrates the feasibility from a therapeutic standpoint, since transforming proteins may represent novel and specific targets for tumor therapy.

Several different techniques can be used to demonstrate amplified genomic sequences. Classical cytogenetic analysis of chromosome spreads prepared from cancer cells is adequate to identify gross structural alterations, such as translocations, deletions and inversions. Amplified genomic regions can only be visualized, if they involve large regions with high copy numbers or are present as extrachromosomal material. While cytogenetics was the first technique to demonstrate the consistent association of specific chromosomal changes with particular neoplasms, it is inadequate for the identification and isolation of manageable DNA sequences. The more recently developed technique of comparative genomic hybridization (CGH) has illustrated the widespread phenomenon of genomic amplification in neoplasms. Tumor and normal DNA are hybridized simultaneously onto metaphases of normal cells and the entire genome can be screened by image analysis for DNA sequences that are present in the tumor at an increased frequency. (WO93/18,186; Gray et al., Radiation Res.,137:275-289 [1994]). As a screening method, this type of analysis has revealed a large number of recurring amplicons (a stretch of amplified DNA) in a variety of human neoplasms. Although CGH is more sensitive than classical cytogenetic analysis in identifying amplified stretches of DNA, it does not allow a rapid identification and isolation of coding sequences within the amplicon by standard molecular genetic techniques.

The most sensitive methods to detect gene amplification are polymerase chain reaction (PCR)-based assays. These assays utilize very small amount of tumor DNA as starting material, are exquisitely sensitive, provide DNA that is amenable to further analysis, such as sequencing and are suitable for high-volume throughput analysis.

The above-mentioned assays are not mutually exclusive, but are frequently used in combination to identify amplifications in neoplasms. While cytogenetic analysis and CGH represent screening methods to survey the entire genome for amplified regions, PCR-based assays are most suitable for the final identification of coding sequences, *i.e.*, genes in amplified regions.

According to the present invention, such genes have been identified by quantitative PCR (S. Gelmini et al., Clin. Chem., 43:752 [1997]), by comparing DNA from a variety of primary tumors, including breast, lung, colon, prostate, brain, liver, kidney, pancreas, spleen, thymus, testis, ovary, uterus, etc., tumor, or tumor cell lines, with pooled DNA from healthy donors. Quantitative PCR was performed using a TaqMan™ instrument (ABI). Gene-specific primers and fluorogenic probes were designed based upon the coding sequences of the DNAs.

Human lung carcinoma cell lines include A549 (SRCC768), Calu-1(SRCC769), Calu-6 (SRCC770), H157 (SRCC771), H441 (SRCC772), H460 (SRCC773), SKMES-1 (SRCC774), SW900 (SRCC775), H522 (SRCC832),and H810(SRCC833), all available from ATCC. Primary human lung tumor cells usually derive from adenocarcinomas, squamous cell carcinomas, large cell carcinomas, non-small cell carcinomas, small cell carcinomas, and broncho alveolar carcinomas, and include, for example, SRCC724 (adenocarcinoma, abbreviated as "AdenoCa')(LT1), SRCC725 (squamous cell carcinoma, abbreviated as "SqCCa)(LT1a), SRCC726 (adenocarcinoma)(LT2). SRCC727 (adenocarcinoma)(LT3). SRCC728 (adenocarcinoma)(LT4), SRCC729 (squamous cell carcinoma)(LT6), SRCC730 (adeno/squamous cell carcinoma)(LT7). SRCC731 (adenocarcinoma)(LT9), SRCC732 (squamous cell carcinoma)(LT10), SRCC733 (squamous cell carcinoma)(LT11), SRCC734 (adenocarcinoma)(LT12), SRCC735 (adeno/squamous cell carcinoma) (LT13), SRCC736 (squamous cell carcinoma)(LT15), SRCC737 (squamous cell carcinoma)(LT16), SRCC738 (squamous cell carcinoma)(LT17), SRCC739 (squamous cell carcinoma)(LT18), SRCC740 (squamous cell carcinoma)(LT19), SRCC741 (lung cell carcinoma, abbreviated as "LCCa")(LT21), SRCC811 (adenocarcinoma)(LT22), SRCC825 (adenocarcinoma)(LT8), SRCC886 (adenocarcinoma)(LT25), SRCC887 (squamous cell carcinoma) (LT26), SRCC888 (adeno-BAC carcinoma) (LT27), SRCC889 (squamous cell carcinoma) (LT28), SRCC890 (squamous cell carcinoma) (LT29), SRCC891 (adenocarcinoma) (LT30). SRCC892 (squamous cell carcinoma) (LT31), SRCC894 (adenocarcinoma) (LT33). Also included are human lung tumors designated SRCC1125 [HP-000631]. SRCC1127 [HF-000641], SRCC1129 [HF-000643], SRCC1133 [HF-000840], SRCC1135 [HF-000842]. SRCC1227 [HF-001291], SRCC1229 [HF-001293], SRCC1230 [HF-001294], SRCC1231 [HF-001295], SRCC1232 [HF-001296], SRCC1233 [HF-001297], SRCC1235 [HF-001299], and SRCC1236 [HF-001300].

Colon cancer cell lines include, for example, ATCC cell lines SW480 (adenocarcinoma, SRCC776), SW620 (lymph node metastasis of colon adenocarcinoma. SRCC777), Colo320 (carcinoma, SRCC778), HT29 (adenocarcinoma, SRCC779), HM7 (a high mucin producing variant of ATCC colon adenocarcinoma cell line, SRCC780, obtained from Dr. Robert Warren, UCSF), CaWiDr (adenocarcinoma, SRCC781), HCT116 (carcinoma, SRCC782), SKCO1 (adenocarcinoma, SRCC783), SW403 (adenocarcinoma. SRCC784), LS174T (carcinoma, SRCC785), Colo205 (carcinoma, SRCC828), HCT15 (carcinoma, SRCC829), HCC2998 (carcinoma, SRCC830), and KM12 (carcinoma, SRCC831). Primary colon tumors include colon adenocarcinomas designated CT2 (SRCC742), CT3 (SRCC743), CT8 (SRCC744), CT10 (SRCC745), CT12 (SRCC746), CT14 (SRCC747), CT15 (SRCC748), CT16 (SRCC749), CT17 (SRCC750), CT1 (SRCC751), CT4 (SRCC752), CT5 (SRCC753), CT6 (SRCC754), CT7 (SRCC755), CT9 (SRCC756), CT11 (SRCC757), CT18 (SRCC758), CT19 (adenocarcinoma, SRCC906, CT20 (adenocarcinoma, SRCC907), CT21 (adenocarcinoma, SRCC908), CT22 (adenocarcinoma, SRCC909), CT23 (adenocarcinoma, SRCC910), CT24 (adenocarcinoma, SRCC911), CT25 (adenocarcinoma, SRCC912), CT26 (adenocarcinoma, SRCC913), CT27 (adenocarcinoma, SRCC914),CT28 (adenocarcinoma, SRCC915), CT29 (adenocarcinoma, SRCC916), CT30 (adenocarcinoma, SRCC917), CT31 (adenocarcinoma, SRCC918), CT32 (adenocarcinoma, SRCC919), CT33 (adenocarcinoma, SRCC920), CT35 (adenocarcinoma, SRCC921), and CT36 (adenocarcinoma, SRCC922). Also included are human colon tumor centers designated SRCC1051 [HF-000499], SRCC1052 [HF-000539], SRCC1053 [HF-000575], SRCC1054 [HF-000698], SRCC1059 [HP-000755], SRCC1060 [HF000756], SRCC1142 [HF-000762], SRCC1144 [HF-000789], SRCC1146 [HF-000795] and SRCC1148[HF-000811].

Human breast carcinoma cell lines include, for example, HBL100 (SRCC759), MB435s (SRCC760), T47D (SRCC761), MB468(SRCC762), MB 175 (SRCC763), MB361 (SRCC764), BT20 (SRCC765), MCF7 (SRCC766), and SKBR3 (SRCC767), and human breast tumor center designated SRCC1057 [HF-000545]. Also included are human breast tumors designated SRCC1094, SRCC1095, SRCC1096, SRCC1097, SRCC1098, SRCC1099, SRCC1100, SRCC1101, and human breast-met-lung-NS tumor designated SRCC893 [LT 32].

Human rectum tumors include SRCC981 [HF-000550] and SRCC982 [HF-000551].

Human kidney tumor centers include SRCC989 [HF-000611] and SRCC1014 [HF-000613].

Human testis tumor center include SRCC1001 [HF-000733] and testis tumor margin SRCC999 [HF-000716].

Human parathyroid tumors include SRCC1002 [HF-000831] and SRCC1003 [HF-000832].

Human lymph node tumors include SRCC1004 [HF-00854], SRCC1005 [HF-000855], and SRCC1006 [HF-000856].

F. Tissue Distribution

The results of the gene amplification assays herein can be verified by further studies, such as, by determining mRNA expression in various human tissues.

As noted before, gene amplification and/or gene expression in various tissues may be measured by conventional Southern blotting, Northern blotting to quantitate the transcription of mRNA (Thomas, Proc. Natl. Acad. Sci. USA, 77: 5201-5205 [1980]), dot blotting (DNA analysis), or *in situ* hybridization, usingan appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO197, PRO207, PRO226, PRO232, PRO243, PRO256, PRO269, PRO274, PR0304, PRO339, PRO1558, PRO779, PRO1185, PRO1245, PRO1759, PRO5775, PRO7133, PRO7168, PRO5725, PRO202, PRO206, PRO264, PR0313, PRO342, PRO542, PRO773, PRO861, PRO1216, PRO1686, PRO1800, PR03562, PR09850, PR0539, PRO4316 or PRO4980 polypeptide or against a synthetic peptide based on the DNA sequences provided herein or against exogenous sequence fused to sequence PRO197, PRO207, PRO226, PRO232, PRO243, PRO256, PRO269, PRO274, PR0304, PR0339, PRO1558, PR0779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PR03562, PRO9850, PR0539, PR04316 or PRO4980 DNA and encoding a specific antibody epitope. General techniques for generating antibodies, and special protocols for Northern blotting and *in situ* hybridization are provided hereinbelow.

G. Chromosome Mapping

If the amplification of a given gene is functionally relevant, then that gene should be amplified more than neighboring genomic regions which are not important for tumor survival. To test this, the gene can be mapped to a particular chromosome, *e.g.*, by radiation-hybrid analysis. The amplification level is then determined at the location identified, and at the neighboring genomic region. Selective or preferential amplification at the genomic region to which the gene has been mapped is consistent with the possibility that the gene amplification observed promotes tumor growth or survival. Chromosome mapping includes both framework and epicenter mapping. For further details *see*, *e.g..* Stewart et al., Genome Research, 7:422-433 (1997).

H. Antibody Binding Studies

The results of the gene amplification study can be further verified by antibody binding studies, in which the ability of anti-PRO 197, anti-PRO207, anti-PRO226, anti-PR232, anti-PRO243, anti-PR256, anti-PR0269, anti-PR0274, anti-PRO304, anti-PRO339, anti-PRO1558, anti-PRO779, anti-PRO1185, anti-PRO1245, anti-PRO1759, anti-PR05775, anti-PRO7133, anti-PR07168, anti-PRO5725, anti-PRO202," anti-PR0206, anti-PRO264, anti-PRO313, anti-PR0342, anti-PRO542, anti-PRO773, anti-PR0861, anti-PRO1216, anti-PRO1686, and-PRO1800, anti-PRO3562, anti-PR09850, anti-PR0539, anti-PRO4316 or anti-PRO4980 antibodies to inhibit the expression of PRO197, PRO207, PRO226, PRO232, PRO243. PRO256, PR0269, PRO274, PRO304, PRO339q, PRO1558, PRO779. PRO1185, PRO1245, PRO1759, PRO5775, PRO7133, PRO7168, PRO572.5, PRO202, PRO206, PRO264, PR0313, PR0342, PRO542, PRO773, PRO861, PRO1216, PRO1686, PRO1800, PRO3562, PRO9850, PRO539, PRO4316 or PR04980 polypeptides on tumor (cancer) cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein (encoded by a gene amplified in a tumor cell) in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. *See, e.g.*, U.S. Patent No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tumor sample may be fresh or frozen or may be embedded in paraffin and fixed with a preservative such as formalin, for example.

1. Cell-Based Tumor Assays

Cell-based assays and animal models for tumors (*e.g.*, cancers) can be used to verify the findings of the gene amplification assay, and further understand the relationship between the genes identified herein and the development and pathogenesis of neoplastic cell growth. The role of gene products identified herein in the development and pathology of tumor or cancer can be tested by using primary tumor cells or cells lines that have been identified to amplify the genes herein. Such cells include, for example, the breast, colon and lung cancer cells and cell lines listed above.

In a different approach, cells of a cell type known to be involved in a particular tumor are transfected with the cDNAs herein, and the ability of these cDNAs to induce excessive growth is analyzed. Suitable cells include, for example, stable tumor cells lines such as, the B104-1-1 cell line (stable NIH-3T3 cell line transfected with the neu protooncogene) and *ras*-transfected NIH-3T3 cells, which can be transfected with the desired gene, and monitored for tumorigenic growth. Such transfected cell lines can then be used to test the ability of poly- or monoclonal antibodies or antibody compositions to inhibit tumorigenic cell growth by exerting cytostatic or cytotoxic activity on the growth of the transformed cells, or by mediating antibody-dependent cellular cytotoxicity (ADCC). Cells transfected with the coding sequences of the genes identified herein can further be used to identify drug candidates for the treatment of cancer.

In addition, primary cultures derived from tumors in transgenic animals (as described below) can be used in the cell-based assays herein, although stable cell lines are preferred. Techniques to derive continuous cell lines from transgenic animals are well known in the art (*see*, *e.g.,* Small et al., Mol. Cell. Biol., 5:642-648 [1985]).

J. <u>Animal Models</u>

A variety of well known animal models can be used to further understand the role of the genes identified herein in the development and pathogenesis of tumors, and to test the efficacy of candidate therapeutic agents, including antibodies, and other antagonists of the native polypeptides, including small molecule antagonists. The *in vivo* nature of such models makes them particularly predictive of responses in human patients. Animal models of tumors and cancers (*e.g*., breast cancer, colon cancer, prostate cancer, lung cancer, etc.) include both non-recombinant and recombinant (transgenic) animals. Non-recombinant animal models include, for example, rodent, *e.g*., murine models. Such models can be generated by introducing tumor cells into syngeneic mice using standard techniques, *e.g*., subcutaneous injection, tail vein injection, spleen implantation, intraperitoneal implantation, implantation under the renal capsule, or orthopin implantation, *e.g*., colon cancer cells implanted in colonic tissue. (*See, e-g*., PCT publication No. WO 97/33551, published September 18, 1997).

Probably the most often used animal species in oncological studies are immunodeficient mice and, in particular, nude mice. The observation that the nude mouse with hypo/aplasia could successfully act as a host for human tumor xenografts has lead to its widespread use for this purpose. The autosomal recessive nu gene has been introduced into a very large number of distinct congenic strains of nude mouse, including, for example, ASW, A/He, AKR. BALB/c, B10.LP, C17, C3H, C57BL, C57, CBA, DBA, DDD, I/st, NC, NFR, NFS, NFS/N, NZB, NZC, NZW. P, RIII and SJL. In addition, a wide variety of other animals with inherited immunological defects other than the nude mouse have been bred and used as recipients of tumor xenografts. For further details *see, e.g.,* The Nude Mouse in Oncology Research, E. Boven and B. Winograd, eds., CRC Press, Inc., 1991.

The cells introduced into such animals can be derived from known tumor/cancer cell lines, such as, any of the above-listed tumor cell lines, and, for example, the B104-1-1 cell line (stable NIH-3T3 cell line transfected with the *neu* protooncogene); *ras*-transfected NIH-3T3 cells; Caco-2 (ATCC HTB-37); a moderately well-differentiated grade II human colon adenocarcinoma cell line, HT-29 (ATCC HTB-38), or from tumors and cancers. Samples of tumor or cancer cells can be obtained from patients undergoing surgery, using standard conditions, involving freezing and storing in liquid nitrogen (Karmali et al., Br. J. Cancer. 48:689-696 [1983]).

Tumor cells can be introduced into animals, such as nude mice, by a variety of procedures. The subcutaneous (s.c.) space in mice is very suitable for tumor implantation. Tumors can be transplanted s.c.as solid blocks, as needle biopsies by use of a trochar, or as cell suspensions. For solid blocker trochar implantation, tumor tissue fragments of suitable size are introduced into the s.c. space. Cell suspensions are freshly prepared from primary tumors or stable tumor cell lines, and injected subcutaneously. Tumor cells can also be injected as subdermal implants. In this location, the inoculum is deposited between the lower part of the dermal connective tissue and the s.c. tissue. Boven and Winograd (1991), *supra.*

Animal models of breast cancer can be generated, for example, by implanting rat neuroblastoma cells (from which the *neu* oncogen was initially isolated), or *neu*-transformed NIH-3T3 cells into nude mice, essentially as described by Drebin et al., PNAS USA, 83:9129-9133 (1986).

Similarly, animal models of colon cancer can be generated by passaging colon cancer cells in animals, e.g., nude mice, leading to the appearance of tumors in these animals. An orthotopic transplant model of human colon cancer in nude mice has been described, for example, by Wang et al., Cancer Research, 54:4726-4728 (1994) and Too et al., Cancer Research, 55:681-684 (1995). This model is based on the so-called "METAMOUSE" sold by AntiCancer, Inc., (San Diego, California).

Tumors that arise in animals can be removed and cultured *in vitro.* Cells from the *in vitro* cultures can then be passaged to animals. Such tumors can serve as targets for further testing or drug screening. Alternatively, the tumors resulting from the passage can be isolated and RNA from pre-passage cells and cells isolated after one or more rounds of passage analyzed for differential expression of genes of interest. Such passaging techniques can be performed with any known tumor or cancer cell lines.

For example, Meth A, CMS4, CMS5, CMS21, and WEHI-164 are chemically induced fibrosarcomas of BALB/c female mice (DeLeo et al., J. Exp. Med., 146:720 [1977]), which provide a highly controllable model system for studying the anti-tumor activities of various agents (Palladino et al., J. Immunol., 138:4023-4032 [1987]), Briefly, tumor cells are propagated *in vitro* in cell culture. Prior to injection into the animals, the cell lines are washed and suspended in buffer, at a cell density of about $10x10^6$ to $10x10^7$ cells/ml. The animals are then infected subcutaneously with 10 to 100μl of the cell suspension, allowing one to three weeks for a tumor to appear.

In addition, the Lewis lung (3LL) carcinoma of mice, which is one of the most thoroughly studied experimental tumors, can be used as an investigational tumor model. Efficacy in this tumor model has been correlated with beneficial effects in the treatment of human patients diagnosed with small cell carcinoma of the lung (SCCL). This tumor can be introduced in normal mice upon injection of tumor fragments from an affected mouse or of cells maintained in culture (Zupi et al., Br.J. Cancer, 41:suppl. 4:309 [1980]), and evidence indicates that tumors can be started from injection of even a single cell and that a very high proportion of infected tumor cells survive. For further information about this tumor model *see*, Zacharski, Haemostasis, 16:300-320 [1986]).

One way of evaluating the efficacy of a test compound in an animal model on an implanted tumor is to measure the size of the tumor before and after treatment. Traditionally, the size of implanted tumors has been measured with a slide caliper in two or three dimensions. The measure limited to two dimensions does not accurately reflect the size of the tumor, therefore, it is usually converted into the corresponding volume by using a mathematical formula. However, the measurement of tumor size is very inaccurate. The therapeutic effects of a drug candidate can be better described as treatment-induced growth delay and specific growth delay. Another important variable in the description of tumor growth is the tumor volume doubling time. Computer programs for the calculation and description of tumor growth are also available, such as the program reported by Rygaard and Spang-Thomsen, Proc. 6th Int. Workshop on Immune-Deficient Animals. Wu and Sheng eds., Basel, 1989, 301. It is noted, however, that necrosis and inflammatory responses following treatment may actually result in an increase in tumor size, at least initially. Therefore, these changes need to be carefully monitored, by a combination of a morphometric method and flow cytometric analysis.

Recombinant (transgenic) animal models can be engineered by introducing the coding portion of the genes identified herein into the genome of animals of interest, using standard techniques for producing transgenic animals. Animals that can serve as a target for transgenic manipulation include, without imitation, mice, rats, rabbits, guinea pigs, sheep, goats, pigs, and non-human primates, *e.g.*, baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*e.g.*, Van der Putten et al., Proc. Natl. Acad. Sci. USA, 82:6148-615 [1985]); gene targeting in embryonic stem cells (Thompson et al., Cell, 56:313-321 [1989]); electroporation of embryos (Lo, Mol. Cell Biol., 3:1803-1814 [1983]); sperm-mediated gene transfer (Lavitrano et al., Cell. 57:717-73 [1989]). For review, see, for examples, U.S. Patent No. 4,736,866.

For the purpose of the present invention, transgenic animals include those that carry the transgene only in part of their cells ("mosaic animals"). The transgene can be integrated either as a single transgene, or in concatamers, *e.g.*, head-to-head or head-to-tail tandems. Selective introduction of a transgene into a particular cell type is also possible by following, for example, the technique of Lasko et al., Proc. Natl. Acad. Sci. USA. 89:6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry. The animals are further examined for signs of tumor or cancer development.

Alternatively, "knock out" animals can be constructed which have a defective or altered gene encoding a PRO197, PRO207, PRO226, PRO232, PRO243, PRO256, PRO269, PRO274, PRO304, PR0339, PRO1558, PRO779, PRO1185, PRO1245, PRO1759, PRO5775, PR07133, PR07168, PRO5725, PR0202, PRO206, PRO264, PRO313, PRO342, PRO542, PRO773, PRO861, PRO1216, PRO1686, PRO1800, PRO3562, PRO9850, PRO539, PR04316 or PR04980 polypeptide identified herein, as a result of homologous recombination between the endogenous gene encoding the polypeptide and altered genomic DNA encoding the same polypeptide introduced into an embryonic cell of the animal. For example, cDNA encoding a PRO197, PR0207, PR0226, PR0232, PRO243, PRO256, PRO269, PRO274, PR0304, PRO339, PRO1558, PRO779, PRO1185, PRO1245, PRO1759, PRO5775, PRO7133, PR07168, PRO5725, PR0202, PRO206, PRO264, PR0313, PR0342, PRO542, PRO773, PRO861, PRO1216, PRO1686, PRO1800, PR03562, PR09850, PR0539, PRO4316 or PRO4980 polypeptide can be used to clone genomic DNA encoding that polypeptide in accordance with established techniques. A portion of the genomic DNA encoding a particular PRO197, PR0207, PR0226, PRO232, PRO243, PRO256, PRO269, PRO274, PRO304, PRO339, PRO1558, PRO779, PRO1185, PRO1245, PRO1759, PRO5775, PR07133, PR07168, PRO5725, PR0202, PRO206, PRO264, PR0313, PRO342, PRO542, PRO773, PRO861, PRO1216, PRO1686, PRO1800, PR03562, PR09850, PRO539, PR04316 or PRO4980 polypeptide can be deleted or replaced with another gene, such as a gene encoding a selectable marker which can be used to monitor integration. Typically, several kilobases of unaltered flanking DNA (both at the 5' and 3' ends) are included in the vector [*see, e.g.*, Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [*see. e.g.,* Li et al., Cell, 69:915 (1992)]. The selected cells are then injected into a blastocyst of an animal (*e.g.*, a mouse or rat) to form aggregation chimeras [*see. e.g.,* Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, by their ability to defend against certain pathological conditions and by their development of pathological conditions due to absence of the PRO197, PRO207, PR0226, PRO232, PRO243, PRO256, PRO269, PR0274, PR0304, PRO339, PRO1558, PR0779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide.

The efficacy of antibodies specifically binding the polypeptides identified herein and other drug candidates, can be tested also in the treatment of spontaneous animal tumors. A suitable target for such studies is the feline oral squamous cell carcinoma (SCC). Feline oral SCC is a highly invasive, malignant tumor that is the most common oral malignancy of cats, accounting for over 60% of the oral tumors reported in this species. It rarely metastasizes to distant sites, although this low incidence of metastasis may merely be a reflection of the short survival times for cats with this tumor. These tumors are usually not amenable to surgery, primarily because of the anatomy of the feline oral cavity. At present, there is no effective treatment for this tumor. Prior to entry into the study, each cat undergoes complete clinical examination, biopsy, and is scanned by computed tomography (CT). Cats diagnosed with sublingual oral squamous cell tumors are excluded from the study. The tongue can become paralyzed as a result of such tumor, and even if the treatment kills the tumor, the animals may not be able to feed themselves. Each cat is treated repeatedly, over a longer period of time. Photographs of the tumors will be taken daily during the treatment period, and at each subsequent recheck. After treatment, each cat undergoes another CTscan. CT scans and thoracic radiograms are evaluated every 8 weeks thereafter. The data are evaluated for differences in survival, response and toxicity as compared to control groups. Positive response may require evidence of tumor regression, preferably with improvement of quality of life and/or increased life span.

In addition, other spontaneous animal tumors, such as fibrosarcoma, adenocarcinoma, lymphoma, chrondroma, leiomyosarcoma of dogs, cats, and baboons can also be tested. Of these mammary adenocarcinoma in dogs and cats is a preferred model as its appearance and behavior are very similar to those in humans. However, the use of this model is limited by the rare occurrence of this type of tumor in animals.

K. <u>Screening Assays for Drug Candidates</u>

Screening assays for drug candidates are designed to identify compounds that bind or complex with the polypeptides encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art.

All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e.g.*, on a microtiter plate, by covalent or non-covalent attachments. Non-covalent attachment generally is accomplished by coating the solid surface with a solution of the polypeptide and drying. Alternatively, an immobilized antibody, *e.g.*, a monoclonal antibody, specific for the polypeptide to be immobilized can be used to anchor it to a solid surface. The assay is performed by adding the non-immobilized component, which may be labeled by a detectable label, to the immobilized component, *e.g.*, the coated surface containing the anchored component. When the reaction is complete, the non-reacted components are removed, *e.g.*, by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide encoded by a gene identified herein, its interaction with that polypeptide can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers (Fields and Song, Nature, 340:245-246 (1989); Chien et al., Proc. Natl. Acad. Sci. USA, 88: 9578-9582 (1991)] as disclosed by Chevray and Nathans, Proc. Natl. Acad. Sci. USA, 89:5789-5793 (1991)]. Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a PRO197-, PR0207-, PRO226-, PRO232-, PRO243-, PRO256-, PR0269-, PRO274-. PR0304-, PR0339-, PRO1558-, PRO779-, PRO1185-, PRO1245-, PRO1759-, PRO5775-, PRO7133-, PRO7168-, PRO5725-, PRO202-, PRO206-, PRO264-, PRO313-, PRO342-, PRO542-, PRO773-, PRO861-, PRO1216-, PRO1686-, PRO1800-, PRO3562-, PRO9850-, PRO539-, PRO4316- or PRO4980-encoding gene identified herein and other intra- or extracellular components can be tested as follows: usually a reaction mixture is prepared containing the product of the amplified gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the PRO197. PRO207, PRO226, PRO232, PRO243, PRO256, PRO269, PRO274, PRO304, PRO339, PRO1558, PRO779, PRO1185, PRO1245, PRO1759. PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide may be added to a cell along with the compound to be screened for a particular activity and the ability of the compound to inhibit the activity of interest in the presence of the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide indicates that the compound is an antagonist to the PRO197, PRO207, PRO226, PRO232, PRO243, PRO256, PR0269, PR0274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide. Alternatively, antagonists may be detected by combining the PRO 197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide and a potential antagonist with membrane-bound PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide receptors or recombinant receptors under appropriate conditions for a competitive inhibition assay. The PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216,

PRO1686, PRO1800, PRO3562, PRO9850, PRO539, PRO4316 or PRO4980 polypeptide can be labeled, such as by radioactivity, such that the number of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the PRO197, PRO207, PRO226, PRO232, PRO243, PRO256, PRO269, PRO274, PRO304, PRO339, PRO1558, PRO779, PRO1185, PRO1245. PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide and a cDNA library created from this RNA is divided into pools and used to transect COS cells or other cells that are not responsive to the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide. Transfected cells that are grown on glass slides are exposed to labeled PRO197, PRO207, PRO226, PRO232, PRO243, PRO256. PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide. The PRO197, PRO207, PRO226, PRO232, PRO243, PRO256, PRO269, PRO274, PRO304, PRO339, PRO1558, PRO779, PRO1185, PRO1245, PRO1759, PR05775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO530, PRO4316 or PRO4980 polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, labeled PRO197. PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide can be photoaffinity-linked with cell membrane or extract preparations that express the receptor molecule. Cross-linked material is resolved by PAGE and exposed to X-ray film The labeled complex containing the receptor can be excised, resolved into peptide fragments, and subjected to protein micro-sequencing. The amino acid sequence obtained from micro-sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide in the presence of the candidate compound. The ability of the compound to enhance or block this interaction could then be measured.

More specific examples of potential antagonists include an oligonucleotide that binds to the fusions of immunoglobulin with the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PRO197, PRO207, PRO226, PRO232, PRO243, PRO256, PRO269, PRO274, PRO304, PRO339, PRO1558. PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850. PRO539, PRO4316 or PRO4980 polypeptide that recognizes the receptor but imparts no effect, thereby competitively inhibiting the action of the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide.

Another potential PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide antagonist is an antisense RNA or DNA construct prepared using antisense technology, where, *e.g.*, an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide herein, is used to design an antisense RNA oligonucleotide of from about 10 to 40 base pairs in length. A DNA oligonucleotide is designed to be complementary to a region of the gene involved in transcription (triple helix - *see*, Lee et al., Nucl. Acids Res., 6:3073 (1979); Cooney et al., Science, 241: 456 (1988); Dervan et al., Science, 251: 1360 (1991)), thereby preventing transcription and the production of the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide. The antisense RNA oligonucleotide hybridizes to the mRNA *in vivo* and blocks translation of the mRNA molecule into the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed in vivo to inhibit production of the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide. When antisense DNA is used, oligodeoxyribonucleotides derived from the translation-initiation site, *e.g.*, between about -10 and +10 positions of the target gene nucleotide sequence, are preferred.

Antisense RNA or DNA molecules are generally at least about 5 bases in length, about 10 bases in length, about 15 bases in length, about 20 bases in length, about 25 bases in length, about 30 bases in length, about 35 bases in length, about 40 bases in length, about 45 bases in length, about 50 bases in length, about 55 bases in length, about 60 bases in length, about 65 bases in length, about 70 bases in length, about 75 bases in length, about 80 bases in length, about 85 bases in length, about 90 bases in length, about 95 bases in length, about 100 bases in length, or more.

Potential antagonists include small molecules that bind to the active site, the receptor binding site, or growth factor or other relevant binding site of the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide, thereby blocking the normal biological activity of the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details *see, e.g.,* Rossi, Current Biology, 4:469-471 (1994), and PCT publication No. WO 97/33551 (published September 18. 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details *see, e.g.,* PCT publication No. WO 97/33551, *supra.*

These small molecule can be identified by any one or more of the screening assays discussed hereinabove and/or by any other screening techniques well known for those skilled in the art.

L. Compositions and Methods for the Treatment of Tumors

The compositions useful in the treatment of tumors associated with the amplification of the genes identified herein include, without limitation, antibodies, small organic and inorganic molecules, peptides, phosphopeptides, antisense and ribozyme molecules, triple helix molecules, etc., that inhibit the expression and/or activity of the target gene product.

For example, antisense RNA and RNA molecules act to directly block the translation of mRNA by hybridizing to targeted mRNA and preventing protein translation. When antisense DNA is used, oligodeoxyribonucleotides derived from the translation initiation site, *e.g.*, between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details *see, e.g.,* Rossi, Current Biology, 4:469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details *see, e.g.,* PCT publication No. WO 97/33551, *supra.*

These molecules can be identified by any or any combination of the screening assays discussed hereinabove and/or by any other screening techniques well known for those skilled in the art.

M. Antibodies

Some of the most promising drug candidates according to the present invention are antibodies and antibody fragments which may inhibit the production or the gene product of the amplified genes identified herein and/or reduce the activity of the gene products

1. Polyclonal Antibodies

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO197, PRO207, PRO226, PRO232, PRO243, PRO256, PRO269, PRO274, PRO304, PRO339, PRO1558, PRO779, PRO1185, PRO1245, PRO1759, PRO5775. PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

2. Monoclonal Antibodies

The anti-PRO197, anti-PRO207, anti-PRO226, anti-PRO232, anti-PRO243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PRO5775, anti-PRO7133, anti-PRO7168, anti-PRO5725, anti-PRO202, anti-PRO206, anti-PRO264, anti-PR0313, anti-PRO342, anti-PRO542, anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent Alternatively, the lymphocytes may be immunized *in vitro.*

The immunizing agent will typically include the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide including fragments, or a fusion protein of such protein or a fragment thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection (ATCC), Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, *supra*]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (*e.g.*, by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al*., supra*] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

3. Human and Humanized Antibodies

The anti-PRO197, anti-PRO207. anti-PRO226, anti-PRO232, anti-PRO243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PRO5775, anti-PRO7133, anti-PR07168, anti-PRO5725, anti-PRO202, anti-PRO206. anti-PRO264. anti-PRO313, anti-PRO342, anti-PRO542. anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibodies may further comprise humanized antibodies or human antibodies. Humanized forms of non-human (*e.g.*, murine) antibodies are chimeric immunoglobulins, immunoglobulin chains or fragments thereof (such as Fv, Fab, Fab', F(ab')$_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of' at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-325 (1986); Riechmann et al., Nature, 332:323-329 (1988); and Presta, Curr. Op. Struct. Biol., 2 :593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321: 522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole *et al.,* and Boerner *et al.,* are also available for the preparation of human monoclonal antibodies (Cole et al., Monoclonal Antibodies and Cancer Therapy, Alan R. Liss, p. 77 (1985) and Boerner et al., J. Immunol., 147(1): 86-95 (1991)]. Similarly, human antibodies can be made by introducing of human immunoglobulin loci into transgenic animals, *e.g.,* mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5.661,016, and in the following scientific publications: Marks et al., Bio/Technology, 10:779-783 (1992); Lonberg et al., Nature, 368:856-859 (1994); Morrison, Nature, 368:812-13 (1994); Fishwild et al., Nature Biotechnology, 14:845-51 (1996); Neuberger, Nature Biotechnology, 14-826 (1996); Lonberg and Huszar, Intern. Rev. Immunol., 13:65-93 (1995).

4. Antibody Dependent Enzyme Mediated Prodrug Therapy (ADEPT)

The antibodies of the present invention may also be used in ADEPT by conjugating the antibody to a prodrug-activating enzyme which converts a prodrug (*e.g.,* a peptidyl chemotherapeutic agent, *see* WO 81/01145) to an active anti-cancer drug. *See,* for example, WO 88/07378 and U. S. Patent No. 4,975,278.

The enzyme component of the immunoconjugate useful for ADEPT includes any enzyme capable of acting on a prodrugs in such as way so as to convert it into its more active, cytotoxic form.

Enzymes that are useful in the method of this invention include, but are not limited to, glycosidase, glucose oxidase, human lysozyme, human glucuronidase, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug 5-fluorouracil; proteases, such as serratia protease, thermolysin, subtilisin, carboxypeptidases (*e.g.*, carboxypeptidase G2 and carboxypeptidase A) and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galaclosidase and neuraminidase useful for converting glycosylated prodrugs into free drugs; β-lactamase useful for converting drugs derivatized with β-lactams into free drugs; and penicillin amidases, such as penicillin Vamidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes" can be used to convert the prodrugs of the invention into free active drugs (*see, e.g.*, Massey, Nature, 328:457-458 (1987)). Antibody-abzyme conjugates can be prepared as described herein for delivery of the abzyme to a tumor cell population.

The enzymes of this invention can be covalently bound to the anti-PRO197, anti-PRO207, anti-PRO226. anti-PRO232, anti-PRO243, anti-PRO256, anti-PRO269, and-PRO274, anti-PRO304, anti-PRO339, anti-PRO1558, anti-PRO779, anti-PRO1185, anti-PRO1245, anti-PRO1759, anti-PRO5775, anti-PRO7133, anti-PRO7168, anti-PRO5725, anti-PRO202, anti-PRO206. anti-PRO264. anti-PRO313, anti-PRO342, and-PRO542, anti-PR0773, and-PRO861, anti-PRO1216, anti-PRO1686, anti-PRO1800, anti-PR03562, anti-PRO9850, anti-PRO539, anti-PRO4316 or anti-PRO4980 antibodies by techniques well known in the art such as the use of the heterobifunctional cross-linking agents discussed above. Alternatively, fusion proteins comprising at least the antigen binding region of the antibody of the invention linked to at least a functionally active portion of an enzyme of the invention can be constructed using recombinant DNA techniques well known in the art (*see. e.g..* Neuberger et al., Nature, 312:604-608 (1984)).

5. Bispecific Antibodies

Bispecific antibodies are monoclonal, preferably human or humanized, antibodies that have binding specificities for at least two different antigens. In the present case, one of the binding specificities is for the PRO197, PRO207, PRO226, PRO232, PRO243, PRO256, PRO269, PRO274, PRO304, PRO339, PRO1558, PRO779. PRO1185. PRO1245, PRO1759, PRO5775, PRO7133. PRO7168, PRO5725, PRO202, PRO206, PRO264, PRO313, PRO342, PRO542, PRO773, PRO861, PRO1216, PRO1686, PRO1800, PRO3562, PRO9850, PRO539. PR04316 or PRO4980 the other one is for any other antigen, and preferably for a cell-surface protein or receptor or receptor subunit

Methods for making bispecific antibodies are known in the art. Traditionally, the recombinant production of bispecific antibodies is based on the co-expression of two immunoglobulin heavy-chain/flight-chain pairs, where the two heavy chains have different specificities (Milstein and Cuello, Nature, 305:537-539 [1983]). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies *see,* for example, Suresh et al., Methods in Enzymology,121:210(1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (*e.g.*, tyrosine or tryptophan). Compensatory "cavities" of identical or similar size to the large side chain(s) are created on the interface of the second antibody molecule by replacing large amino acid side chains with smaller ones (*e.g.*, alanine or threonine). This provides a mechanism for increasing the yield of the heterodimer over other unwanted end-products such as homodimers.

Bispecific antibodies can be prepared as full length antibodies or antibody fragments (*e.g.*, F(ab')$_2$ bispecific antibodies). Techniques for generating bispecific antibodies from antibody fragments have been described in the literature. For example, bispecific antibodies can be prepared using chemical linkage. Brennan et al., Science, 229:81 (1985) describe a procedure wherein intact antibodies are proteolytically cleaved to generate F(ab')$_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate(TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med., 175:217-225 (1992) describe the production of a fully humanized bispecific antibody F(ab')$_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain ($V_H$ connected to a light-chain variable domain ($V_L$) by a linker which is too short to allow pairing between the two domains on the same chain. Accordingly, the $V_H$ and $V_L$, domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. *See,* Gruber el al.; J. Immunol., 152:5368 (1994).

Antibodies with more than two valencies are contemplated. For example, trispecific antibodies can be prepared. Tutt et al., J. Immunol., 147:60 (1991).

Exemplary bispecific antibodies may bind to two different epitopes on a given polypeptide herein. Alternatively, an anti-polypeptide arm may be combined with an arm which binds to a triggering molecule on a leukocyte such as a T-cell receptor molecule (e.g., CD2, CD3, CD28, or B7), or Fc receptors for IgG (FcγR), such as FcγRI (CD64). FcγRII (CD32) and FcγRIII (CD16) so as to focus cellular defense mechanisms to the cell expressing the particular polypeptide. Bispecific antibodies may also be used to localize cytotoxic agents to cells which express a particular polypeptide. These antibodies possess a polypeptide-binding arm and an arm which binds a cytotoxic agent or a radionuclide chelator, such as EOTUBE, DPTA, DOTA, or TETA. Another bispecific antibody of interest binds the polypeptide and further binds tissue factor (TF).

### 6. Heteroconjugate Antibodies

Heteroconjugate antibodies are composed of two covalently joined antibodies. Such antibodies have, for example, been proposed to target immune system cells to unwanted cells [U.S. Patent No. 4,676,980], and for treatment of HIV infection [WO91/00360; WO92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 7. Effector function engineering

It may be desirable to modify the antibody of the invention with respect to effector function, so as to enhance the effectiveness of the antibody in treating cancer, for example. For example, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). *See,* Caron et al., J. Exp. Med. 176:1191-1195 (1992) and shopes, J. Immunol., 148:2918-2922 (1992). Homodimeric antibodies with enhanced anti-tumor activity may also be prepared using heterobifunctional cross-linkers as described in Wolff et al., Cancer Research, 53:2560-2565 (1993). Alternatively, an antibody can be engineered which has dual Fc regions and may thereby have enhanced complement lysis and ADCC capabilities. *See*, Stevenson et al., Anti-Cancer Drug Design, 3:219-230 (1989).

### 8. Immunoconjugates

The invention also pertains to immunoconjugates comprising an antibody conjugated to a cytotoxic agent such as a chemotherapeutic agent, toxin (*e.g.*, an enzymatically active toxin of bacterial, fungal, plant or animal origin, or fragments thereof; or a small molecule toxin), or a radioactive isotope (*i.e.*, a radioconjugate).

Chemotherapeutic agents useful in the generation of such immunoconjugates have been described above. Enzymatically active protein toxins and fragments thereof which can be used include diphtheria A chain, nonbinding active fragments of diphtheria toxin, cholera toxin, botulinus toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, saporin, mitogellin, restrictocin, phenomycin, enomycin and the tricothecenes. Small molecule toxins include, for example, calicheamicins, maytansinoids, palytoxin and CC1065. A variety of radionuclides are available for the production of radioconjugated antibodies. Examples include $^{212}$Bi, $^{131}$I, $^{131}$In, $^{90}$Y and $^{186}$Re.

Conjugates of the antibody and cytotoxic agent are made using a variety of bifunctional protein coupling agents such as N-succimidyl-3(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. *See*, WO94/11026.

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (*e.g.,* avidin) which is conjugated to a cytotoxic agent (*e.g.,* a radionucleotide).

9. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl. Acad. Sci, USA, 77:4030 (1980); and U.S. Patent Nos. 4,485,045 and 4,544,545. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem., 257:286-288 (1982) via a disulfide interchange reaction. A chemotherapeutic agent (such as Doxorubicin) is optionally contained within the liposome. *See*, Gabizon et al., J. National Cancer Inst., 81(19):1484 (1989).

N. Pharmaceutical Compositions

Antibodies specifically binding the product of an amplified gene identified herein, as well as other molecules identified by the screening assays disclosed hereinbefore, can be administered for the treatment of tumors, including cancers, in the form of pharmaceutical compositions.

If the protein encoded by the amplified gene is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (*see, e.g.,* Marasco et al., Proc. Natl. Acad. Sci. USA, 90-7889-7893 [1993]).

Therapeutic formulations of the antibody are prepared for storage by mixing the antibody having the desired degree of purity with optional pharmaceutically acceptable carriers, excipients or stabilizers (Remington's Pharmaceutical Sciences, 16th edition, Osol, A. ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine: preservatives (such as octadecyldimethylbenzyl ammoniumchloride; hexamethonium chloride; benzalkonium chloride, benzethionium chloride; phenol, butyl or benzyl alcohol; alkyl parabens such as methyl or propyl paraben; catechol; resorcinol; cyclohexanol; 3-pentanol; and *m*-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins ; chelati ng agents such as EDTA; sugars such as sucrose, mannitol, trebalose or sorbitol; salt-forming counter-ions such as sodium; metal complexes (*e.g.*, Zn-protein complexes); and/or non-ionic surfactants such as TWEEN™, PLURONICS™ or polyethylene glycol (PEG).

Non-antibody compounds identified by the screening assays of the present invention can be formulated in an analogous manner, using standard techniques well known in the art.

The formulation herein may also contain more than one active compound as necessary for the particular indication being treated, preferably those with complementary activities that do not adversely affect each other. Alternatively, or in addition, the composition may comprise a cytotoxic agent, cytokineor growth inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. ed. (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g.*, films or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT ™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37˚C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

O. Methods of Treatment

It is contemplated that the antibodies and other anti-tumorcompounds of the present invention may be used to treat various conditions, including those characterized by overexpression and/or activation of the amplified genes identified herein. Exemplary conditions or disorders to be treated with such antibodies and other compounds, including, but not limited to, small organic and inorganic molecules, peptides, antisense molecules, etc., include benign or malignant tumors (*e.g.*, renal, liver, kidney, bladder, breast, gastric, ovarian, colorectal, prostate, pancreatic, lung, vulval, thyroid, hepatic carcinomas; sarcomas; glioblastomas; and various head and neck tumors); leukemias and lymphoid malignancies; other disorders such as neuronal, glial, astrocytal, hypothalamic and other glandular, macrophagal, epithelial, stromal and blastocoelic disorders; and inflammatory, angiogenic and immunologic disorders.

The anti-tutnor agents of the present invention, *e.g.*, antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred.

Other therapeutic regimens may be combined with the administration of the anti-cancer agents, *e.g.*, antibodies of the instant invention. For example, the patient to be treated with such anti-cancer agents may also receive radiation therapy. Alternatively, or in addition, a chemotherapeutic agent may be administered to the patient Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the anti-tumor agent, *e.g.*, antibody, or may be given simultaneously therewith. The antibody may be combined with an anti-oestrogen compound such as tamoxifen or an anti-progesterone such as onapristone (*see*, EP 616812) in dosages known for such molecules.

It may be desirable to also administer antibodies against other tumor associated antigens, such as antibodies which bind to the ErbB2, EGFR. ErbB3, ErbB4, or vascular endothelial factor (VEGF). Alternatively, or in addition, two or more antibodies binding the same or two or more different antigens disclosed herein may be co-administered to the patient Sometimes, it may be beneficial to also administer one or more cytokines to the patient. In a preferred embodiment, the antibodies herein are co-administered with a growth inhibitory agent. For example, the growth inhibitory agent may be administered first, followed by an antibody of the present invention. However, simultaneous administration or administration of the antibody of the present invention first is also contemplated. Suitable dosages for the growth inhibitory agent are those presently used and may be lowered due to the combined action (synergy) of the growth inhibitory agent and the antibody herein.

For the prevention or treatment of disease, the appropriate dosage of an anti-tumor agent, e.g., an antibody herein will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments.

For example, depending on the type and severity of the disease, about 1 μg/kg to 15 mg/kg (*e.g.*, 0.1-20 mg/kg) of antibody is an initial candidate dosage for administration to the patient, whether, for example, by one or more separate administrations, or by continuous infusion. A typical daily dosage might range from about 1 $\mu$g/kg to 100 mg/kg or more, depending on the factors mentioned above. For repeated administration over several days or longer, depending on the condition, the treatment is sustained until a desired suppression of disease symptoms occurs. However, other dosage regimens may be useful. The progress of this therapy is easily monitored by conventional techniques and assays.

P. Articles of Manufacture

In another embodiment of the invention, an article of manufacture containing materials useful for the diagnosis or treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually an anti-tumor agent capable of interfering with the activity of a gene product identified herein, *e.g.*, an antibody. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

Q. Diagnosis and Prognosis of Tumors

While cell surface proteins, such as growth receptors overexpressed in certain tumors are excellent targets for drug candidates or tumor (*e.g.*, cancer) treatment, the same proteins along with secreted proteins encoded by the genes amplified in tumor cells find additional use in the diagnosis and prognosis of tumors. For example; antibodies directed against the protein products of genes amplified in tumor cells can be used as tumor diagnostics or prognostics.

For example, antibodies, including antibody fragments, can be used to qualitatively or quantitatively detect the expression of proteins encoded by the amplified genes ("marker gene products"). The antibody preferably is equipped with a detectable, *e.g.*, fluorescent label, and binding can be monitored by light microscopy, flow cytometry, fluorimetry, or other techniques known in the art. These techniques are particularly suitable, if the amplified gene encodes a cell surface protein, *e.g.*, a growth factor. Such binding assays are performed essentially as described in section 5 above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for in situ detection.

The following examples are offered for illustrative purposes only, and are not intended to limit the scope of the present invention in any way.

All patent and literature references cited in the present specification are hereby incorporated by reference in their entirety.

EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, 10801 University Blvd, Manassas, VA 20110-2209. All original deposits referred to in the present application were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc., and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR § 1.14 with particular reference to 886 OG 638).

Unless otherwise noted, the present invention uses standard procedures of recombinant DNA technology, such as those described hereinabove and in the following textbooks: Sambrook et al., Molecular Cloning,: A Laboratory Manual. Cold Spring Harbor Press N.Y., 1989; Ausubel et al.. Current Protocols in Molecular Biolol!V Green Publishing Associates and Wiley Interscience, N.Y.,1989; Innis et al., PCR Protocols: A Guide to Methods and Applications, Academic Press, Inc., N.Y., 1990; Harlow et al.. Antibodies: A Laboratory Manual, Cold Spring Harbor Press, Cold Spring Harbor,1988; Gait, Oligonucleotide Synthesis, IRL Press, Oxford, 1984; R.I. Freshney, Animal Cell Culture, 1987; Coligan et al., Current Protocols in Immunology, 1991.

EXAMPLE 1

Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (*e.g.,* Dayhoff, GenBank), and proprietary databases (*e.g.* LIFESEQ®, Incyte Pharmaceuticals, Palo Alto, CA). The search was performed using the computer program BLAST or BLAST-2 (Altschul et al, Methods in Enzymology, 266:460-480 (1996)) as a comparison of the ECD protein sequences to a 6 frame translation of the EST sequences. Those comparisons with a BLAST score of 70 (or in some cases 90) or greater that did not encode known proteins were clustered and assembled into consensus DNA sequences with the program "phrap" (Phil Green, University of Washington, Seattle, Washington).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5 kbp. In order to screen several libraries for a full-length clone, DNA from the libraries was screened by PCR amplification, as per Ausubel *et al,* Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al, Science 253:1278-1280 (1991)) in the unique XhoI and NotI sites.

EXAMPLE 2

Isolation of cDNA Clones Using Signal Algorithm Analysis

Various polypeptide-encoding nucleic acid sequences were identified by applying a proprietary signal sequence finding algorithm developed by Genentech, Inc., (South San Francisco, CA) upon ESTs as well as clustered and assembled EST fragments from public (*e.g.*, GenBank) and/or private (LIFESEQ® Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

EXAMPLE 3

Isolation of cDNA clones encoding Human PRO197

Pyro197 was identified by screening the GenBank database using the computer program BLAST (Altschul *et al*., Methods in Enzymology, 266:460-480 (1996)). The PRO197 sequence was shown to have homology with known EST sequences T08223. AA 122061, and M62290. None of the known EST sequences have been identified as full-length sequences, or described as ligands associated with TIE receptors. Following identification, PRO 197 was cloned from a human fetal lung library prepared from mRNA purchased from Clontech, Inc., (Palo Alto, CA), catalog # 6528-1, following the manufacturer's instructions. The library was screened by hybridization with synthetic oligonucleotide probes. Based on the ESTs found in the GenBank database, the oligonucleotide sequences used were as follows:

5'-ATGAGGTGGCCAAGCCTGCCCGAAGAAAGAGGC-3'        (SEQ ID NO:71)
5'-CAACTGGCTGGGCCATCTCGGGCAGCCTCTTTCTTCGGG-3'        (SEQ ID NO:72).
5'-CCCAGCCAGAACTCGCCGTGGGGA-3'        (SEQ ID NO:73)

A cDNA clone was identified and sequenced in entirety. Clone DNA22780-1078 contains a single open reading frame with an apparent translational initiation site at nucleotide positions 23-25, and a stop codon at nucleotide positions 1382-1384. The predicted polypeptide precursor is 453 amino acids long.

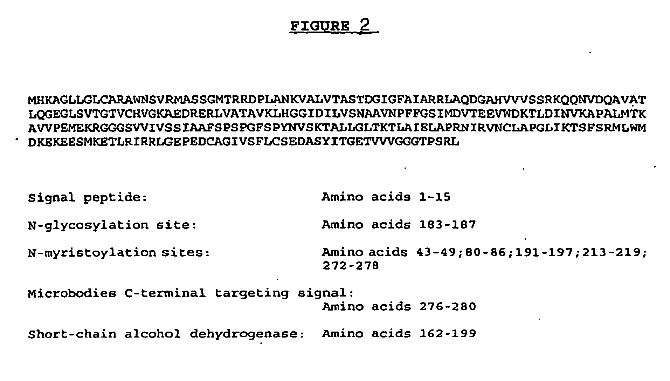

Analysis of the full-length PRO197 evidences the presence of important polypeptide domains, wherein the locations given for those important polypeptide domains are approximate as described above. Analysis of the full-length PRO197 sequence shown in Figure 2 evidences the presence of the following: a transmembrane domain from about amino acid 51 to about amino acid 70; an N-glycosylation site from about amino acid 224 to about amino acid 228; cAMP- and cGMP-dependent protein kinase phosphorylation sites from about amino acid 46 to about amino acid 50 and from about amino acid 118 to about amino acid 122; N-myristoylation sites from about amino acid 50 to about amino acid 56, from about amino acid 129 to about amino acid 135, from about amino acid 341 to about amino acid 347, and from about amino acid 357 to about amino acid 363; and a fibrinogen beta and gamma chains C-terminal domain signature from about amino acid 396 to about amino acid 409.

Clone DNA22780-1078 has been deposited with ATCC on September 18, 1997 and is assigned ATCC deposit no. 209284. It is understood that the deposited clone has the actual correct sequence rather than the representations provided herein.

An analysis of the Dayhoff database (version 35.45 SwissProt 35), using the ALIGN-2 sequence alignment analysis of full-length sequence, evidenced homology between the PRO197 amino acid sequence and ligands associated with TIE receptors. The abbreviation "TIE" is an acronym which stands for "tyrosine kinase containing Ig and EGF homology domains" and was coined to designate a new family of receptor tyrosine kinases.

### EXAMPLE 4

Isolation of cDNA clones Encoding Human PRO207

An expressed sequence tag(EST) DNA database (LIFESEQ®, Incyte Pharmaceuticals, Palo Alto, CA) was searched and an EST was identified which showed homology to human Apo-2 ligand. A human fetal kidney cDNA library was then screened. mRNAisolated from human fetal kidney tissue (Clontech) was used to prepare the cDNA library. This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites. The library was screened by hybridization with a synthetic oligonucleotide probe:

5'-CCAGCCCTCTGCGCTACAACCGCCAGATCGGGGAGTTTATAGTCACCCGG-3' (SEQ ID NO:74) based on the EST.

A cDNA clone was sequenced in entirety. Clone DNA30879-1152 contains a single open reading frame with an apparent translational initiation site at nucleotide positions 58-60 and an apparent stop codon at nucleotide positions 805-807. The predicted polypeptide precursor is 249 amino acids long.

Analysis of the full-length PR0207 sequence evidences the presence of important polypeptide domains, wherein the locations given for those important polypeptide domains are approximate as described above. Analysis of the full-length PRO207 sequence shown in Figure 4 evidences the presence of the following: a signal peptide from about amino acid 1 to about amino acid 40; an N-glycosylation site from about amino acid 139 to about amino acid 143; N-myristoylation sites from about amino acid 27 to about amino acid 33, from about amino acid 29 to about amino acid 35, from about amino acid 36 to about amino acid 42, from about amino acid 45 to about amino acid 51, from about amino acid 118 to about amino acid 124, from about amino acid 121 to about amino acid 127, from about amino acid 125 to about amino acid 131, and from about amino acid 128 to about amino acid 134; amidation sites from about amino acid 10 to about amino acid 14 and from about amino acid 97 to about amino acid 101; and a prokaryotic membrane lipoprotein lipid attachment site from about amino acid 24 to about amino acid 35. Clone DNA30879-1152 has been deposited with ATCC on October 10. 1997 and is assigned ATCC deposit no. 209358.

Based on a BLAST and FastA sequence alignment analysis (using the ALIGN-2 computer program) of the full-length PRO207sequence, PRO207 shows amino acid sequence identity to several members of the TNF cytokine family, and particularly, to human lymphotoxin-beta (23.4%) and human CD40 ligand (19.8%).

EXAMPLE 5

Isolation of cDNA Clones Encoding Human PRO226

A consensus DNA sequence was assembled relative to other EST sequences using phrap as described in Example 1 above. This assembled consensus sequence encoding an EGF-like homologue is herein identified as DNA28744. Based on the DNA28744 consensus sequence, oligonucleotides were synthesized: 1) to identify by PCR a cDNA library that contained the sequence of interest, and 2) for use as probes to isolate a clone of the full-length coding sequence for PRO226.

PCR primers (forward and reverse) were synthesized:

forward PCR primer (28744.f) (OLI556):

    5'-ATTCTGCGTGAACACTGAGGGC-3     ' (SEQ ID NO:75)

reverse PCR primer (28744.r) (OLI557):

    5'-ATCTGCTTGTAGCCCTCGGCAC-3'    (SEQ ID NO:76)

Additionally, a synthetic oligonucleotide hybridization probe was constructed from the DNA28744 consensus sequence which had the following nucleotide sequence:
hybridization probe (28744p) (OLIS55):

    5'-CCTGGCTATCAGCAGGTGGGCTCCAAGTGTCTCGATGTGGATOAGTGTOA-3'    (SEQ ID NO:77)

In order to screen several libraries for a source of a full-length clone, DNA from the libraries was screened by PCR amplification with the PCR primer pairs identified above. A positive library was then used to isolate clones encoding the PR0226 gene using the probe oligonucleotide and one of the PCR primers. RNA for construction of the cDNA libraries was isolated from human fetal lung tissue.

DNA sequencing of the isolated clones isolated as described above gave the full-length DNA sequence for DNA33460-1166; and the derived protein sequence for PRO226.

Clone DNA33460-1166 contains a single open reading frame with an apparent translational initiation site at nucleotide positions 62-64, and an apparent stop codon at nucleotide positions 1391-1393. The predicted polypeptide precursor is 443 amino acids long. Analysis of the full-length PRO226 sequence evidences the presence of a variety of important polypeptide domains, wherein the locations given for those important polypeptide domains are approximate as described above. Analysis of the full-length PR0226 polypeptide evidences the presence of the following: a signal peptide from about amino acid 1 to about amino acid 25; N-glycosylation sites from about amino acid 198 to about amino acid 202 and from about amino acid 394 to about amino acid 398; N-myristoylation sites from about amino acid 76 to about amino acid 82, from about amino acid 145 to about amino acid 151, from about amino acid 182 to about amino acid 188, from about amino acid 222 to about amino acid 228, from about amino acid 290 to about amino acid 296, from about amino acid 305 to about amino acid 311, from about amino acid 371 to about amino acid 377 and from about amino acid 381 to about amino acid 387; and aspartic acid and asparagine hydroxylation sites from about amino acid 140 to about amino acid 152, from about amino acid 177 to about amino acid 189, from about amino acid 217 to about amino acid 229, and from about amino acid 258 to about amino acid 270. Clone DNA33460-1166 has been deposited with the ATCC on October 16, 1997 and is assigned ATCC deposit no. 209376.

Based on a BLAST and FastA sequence alignment analysis of the full-length PR0226 sequence, EGF-like homolog DNA33460-1166 shows amino acid sequence identity to HT protein and/or Fibulin (49% and 38%, respectively).

EXAMPLE 6

Isolation of cDNA Clones Encoding Human PRO232

A consensus DNA sequence was assembled relative to other EST sequences using phrap as described in Example 1 above. This assembled consensus sequence is herein identified as DNA30935, wherein the polypeptide showed similarity to one or more stem cell antigens. Based on the DNA30935 consensus sequence, oligonucleotides were synthesized: 1) to identify by PCR a cDNA library that contained the sequence of interest, and 2) for use as probes to isolate a clone of the full-length coding sequence for PR0232.

PCR primers (forward and reverse) were synthesized:

> forward PCR primer:
>
> 5'-TGCTGTGCTACTCCTGCAAAGCCC-3'        (SEQ ID NO:78)
>
> reverse PCR primer:
>
> 5'-TGCACAAGTCGGTGTCACAGCACG-3'        (SEQ ID NO:79)

Additionally, a synthetic oligonucleotide hybridization probe was constructed from the DNA30935 consensus sequence which had the following nucleotide sequence:
hybridization probe:

> 5'-AGCAACGAGGACTGCCTGCAGGTGGAGAACTGCACCCAGCTGGG-3'        (SEQ ID NO:80)

In order to screen several libraries for a source of a full-length clone, DNA from the libraries was screened by PCR amplification with the PCR primer pairs identified above. A positive library was then used to isolate clones encoding the PR0232 gene using the probe oligonucleotide and one of the PCR primers. RNA for construction of the cDNA libraries was isolated from human fetal kidney tissue.

DNA sequencing of the isolated clones isolated as described above gave the full-length DNA sequence for DNA34435-1140 [SEQ ID NO:7]; and the derived protein sequence for PR0232.

Clone DNA34435-1140 contains a single open reading frame with apparent stop codon at nucleotide positions 359-361. The predicted polypeptide precursor is 119 amino acids long. Analysis of the full-length PR0232 sequence evidences the presence of a variety of important polypeptide domains, wherein the locations given for those important polypeptide domains are approximate as described above. Analysis of the full-length PR0232 polypeptide evidences the presence of the following: a signal peptide from about amino acid 1 to about amino acid 16; N-glycosylation sites from about amino acid 36 to about amino acid 40, from about amino acid 79 to about amino acid 83, and from about amino acid 89 to about amino acid 93; an N-myristoylation site from about amino acid 61 to about amino acid 67; and an amidation site from about amino acid 75 to about amino acid 79. Clone DNA34435-1140 has been deposited with the ATCC on September 16,1997 and is assigned ATCC deposit no. 209250.

An analysis of the full-length PR0232 sequence suggests that it possesses 35% sequence identity with a stem cell surface antigen from Gallus gallus.

EXAMPLE 7

Isolation of cDNA Clones Encoding Human PRO243 by Genomic Walking

Introduction:

Human thrombopoietin (THPO) is a glycosylated hormone of 352 amino acids consisting of two domains. The N-terminal domain, sharing 50% similarity to erythropoietin, is responsible for the biological activity. The C-terminal region is required for secretion. The gene for thrombopoietin (THPO) maps to human chromosome 3q27-q28 where the six exons of this gene span 7 kilobase base pairs of genomic DNA (Gurney et al, Blood, 85:981-988 (1995). In order to determine whether there were any genes encoding THPO homologues located in close proximity to THPO, genomic DNA fragments from this region were identified and sequenced. Three P1 clones and one PAC clone (Genome Systems, Inc., St. Louis. MO; cat. Nos. P1-2535 and PAC-6539) encompassing the THPO locus were isolated and a 140 kb region was sequenced using the ordered shotgun strategy (Chen et al. Genomics. 17:651 - 656 (1993)), coupled with a PCR-based gap filling approach. Analysis reveals that the region is gene-rich with four additional genes located very close to THPO: tumor necrosis factor-receptor type 1 associated protein 2 (TRAP2) and elongation initiation factor gamma (e1F4g), chloride channel 2 (CLCN2) and RNA polymerase II subunit hRPB 17. While no THPO homolog was found in the region, four novel genes have been predicted by computer-assisted gene detection (GRAIL)(Xu et al. Gen. Engin., 16:241-253 (1994), the presence of CpG islands (Cross, S. and Bird, A., Curr. Opin. Genet. & Devel., 5:109-314 (1995), and homology to known genes (as detected by WU-BLAST2.0) (Altschul and Gish, Methods Enzymol., 266:460-480 (1996)).

Procedures:

P1 and PAC clones:

The initial human P1 clone was isolated from a genomic P1 library (Genome Systems, Inc., St. Louis. MO; cat no.: P1-2535) screened with PCR primers designed from the THPO genomic sequence (A. L. Gurney, et al, Blood, 85: 981-988 (1995). PCR primers were designed from the end sequences derived from this P1 clone were then used to screen P1 and PAC libraries (Genome Systems, Cat Nos.: P1-2535 & PAC-6539) to identify overlapping clones.

Ordered Shotgun Strategy:

The Ordered Shotgun Strategy (OSS) (Chen et al, Genomics,17:651-656 (1993)) Involves the mapping and sequencing of large genomic DNA clones with a hierarchical approach. The P1 or PAC clone was sonicated and the fragments subcloned into lambda vector (λBluestar) (Novagen, Inc., Madison, WI; cat no. 69242-3). The lambda subclone inserts were isolated by long-range PCR (Barnes, W., Proc. Natl, Acad. Sci. USA, 91:2216-2220 (1994) and the ends sequenced. The lambda-end sequences were overlapped to create a partial map of the original clone. Those lambda clones with overlapping end-sequences were identified, the insets subcloned into a plasmid vector (pUC9 or pUC18) and the ends of the plasmid subclones were sequenced and assembled to generate a contiguous sequence. This directed sequencing strategy minimizes the redundancy required while allowing one to scan for and concentrate on interesting regions.

In order to identify better the THPO locus and to search for other genes related to the hematopoietin family, four genomic clones were isolated from this region by PCR screening of human P1 and PAC libraries (Genome System, Inc., Cat. Nos.: P1-2535 and PAC-6539). The sizes of the genomic fragments are as follows: P1.t is 40 kb; P1.g is 70 kb; P1.u is 70 kb; and PAC.z is 200 kb. Approximately 80% of the 200 kb genomic DNA region was sequenced by the Ordered Shotgun Strategy (OSS) (Chen et al., Genomics, 17:651-56 (1993) and assembled into contigs using AutoAssembler™ (Applied Biosystems, Perkin Elmer, Foster City. CA, cat no. 903227). The preliminary order of these contigs was determined by manual analysis. There were 46 contigs and filling in the gaps was employed. Table 4 summarizes the number and sizes of the gaps.

Table 4

Summary of the gaps in the 140 kb region

| Size of gap | Number |
| --- | --- |
| <50 bp | 13 |
| 50-150 bp | 7 |
| 150-300 bp | 7 |
| 300-1000 bp | 10 |
| 1000-5000 bp | 7 |
| >5000 bp | 2 (=15,000 bp) |

DNA sequencing:

ABI DYE-primer™ chemistry (PE Applied Biosystems, Foster City, CA; Cat. No.: 402112) was used to end-sequence the lambda and plasmid subclones. ABIDYE-terminator™ chemistry (PE Applied Biosystems, Foster City, CA, Cat. No: 403044) was used to sequence the PCR products with their respective PCR primers. The sequences were collected with an ABI377 instrument. For PCR products larger than 1kb, walking primers were used. The sequences ofcontigs generated by the OSS strategy in AutoAssembler™ (PE AppliedBiosysterns, Foster City, CA; Cat. No: 903227) and the gap-filling sequencing trace files were imported into Sequencher™ (Gene Codes Corp., Ann Arbor, MI) for overlapping and editing.

PCR-Based gap filling Strategy:

Primers were designed based on the 5'- and 3'-end sequence of each contig, avoiding repetitive and low quality sequence regions. All primers were designed to be 19-24-mers with 50%-70% G/C content. Oligos were synthesized and gel-purified by standard methods.

Since the orientation and order of the contigs were unknown, permutations of the primers were used in the amplification reactions. Two PCR kits were used: first, XL PCR kit (Perkin Elmer, Norwalk, CT; Cat No.: N8080205), with extension times of approximately 10 minutes; and second, the Taq polymerase PCR kit (Qiagen, Inc., Valencia, CA; Cat. No.: 201223) was used under high stringency conditions if smeared or multiple products were observed with the XL PCR kit. The main PCR product from each successful reaction was extracted from a 0.9% low melting agarose gel and purified with the Geneclean DNA Purification kit prior to sequencing.

Analysis:

The identification and characterization of coding regions was carried out as follows: First, repetitive sequences were masked using RepeatMasker (A.F.A. Smit & P.Green, http://ftp.genome.washington.edu/RMIRM_details.html) which screens DNA sequences in FastA format against a library of repetitive elements and returns a masked query sequence. Repeats not masked were identified by comparing the sequence to the GenBank database using WUBLAST (Altschul, S. & Gish, W., Methods Enzymol., 266:460-480 (1996)) and were masked manually.

Next, known genes were revealed by comparing the genomic regions against Genentech's protein database using the WUBLAST2.0 algorithm and then annotated by aligning the genomic and cDNA sequences for each gene, respectively, using a Needleman-Wunch (Needleman and Wunsch, J. Mol. Biol., 48:443-453 (1970)) algorithm to find regions of local identity between sequences which are otherwise largely dissimilar. The strategy results in detection of all exons of the five known genes in the region, THPO, TRAP2, e1 F4g, CLCN2, and hRPB 17 (Table 5).

Table 5
Summary of known genes located in the 140 kb region analyzed

| Known genes | Map position |
| --- | --- |
| eukaryotic translation initiation factor 4 gamma | 3q27-qter |
| thrombopoietin | 3q26-q27 |
| chloride channel 2 | 3q26-qter |
| TNF receptor associated protein 2 | not previously mapped |
| RNA polymerase II subunit hRPB 17 | not previously mapped |

Finally, novel transcription units were predicted using a number of approaches. CpG islands (S. Cross & Bird, A., Curr. Opin. Genet. Dev., 5:109-314 (1995)) islands were used to define promoter regions and were identified as clusters of sites cleaved by enzymes recognizing GC-rich, 6 or 8-mer palindromic sequences. CpG islands are usually associated with promoter regions of genes. WUBLAST20 analysis of short genomic regions (10-20 kb) versus GenBaak revealed matches to ESTs. The individual EST sequences (or where possible, their sequence chromatogram files) were retrieved and assembled with Sequencher to provide a theoretical cDNA sequence (DNA34415). GRAIL2 (ApoCom, Inc., Knoxville, TN, command line version for the DEC alpha) was used to predict a novel exon. The five known genes in the region served as internal controls for the success of the GRAIL algorithm.

Isolation:

Chordin cDNA clones were isolated from an oligo-dT-primed human fetal lung library. Human fetal lung polyA$^+$RNA was purchased from Clontech (cat#6528-1, lot#43777) and 5 mg used to construct a cDNA library in pRK5B (Genentech, LIB26). The 3'-primer:

pGACTAGTTCTAGATCGCGAGCGGCCGCCCTTTTTTTTTTTTTTTT (SEQ ID NO:81)

and the 5'-linker:

pCGGACGCGTGGGGCCTGCGCACCCAGCT (SEQ ID NO:82)

were designed to introduce SalI and NotI restriction sites. Clones were screened with oligonucleotide probes designed from the putative human chordin cDNA sequence (DNA34415) deduced by manually "splicing" together the proposed genomic exons of the gene. PCR primers flanking the probes were used to confirm the identity of the cDNA clones prior to sequencing.

The screening oligonucleotide probes were the following.

OLI5640 34415.p1:

5'-GCCGCTCCCCGAACGGGCAGCGGCTCCTTCTCAGAA-3'         (SEQ ID NO:83)

OLI5642 34415.p2:

5'-GGCGCACAGCACGCAGCGCATCACCCCGAATGGCTC-3'         (SEQ ID NO:84)

and the flanking probes used were the following:

OLI5639 34415.ft:

5'-GTGCTGCCCATCCGTTCTGAGAAGGA-3'         (SEQ ID NO:85)

OLI5643 34415.r:

5'-GCAGGGTGCTCAAACAGGACAC-3'         (SEQ ID NO:86)

Clone DNA35917-1207 contains a single open reading frame with an apparent translational initiation site at nucleotide positions 137-139 and with apparent stop codon at nucleotide positions 2999-3001. The predicted polypeptide precursor is 954 amino acids long. Analysis of the full-length PR0243 sequence (SEQ ID NO: 10) evidences the presence of a variety of important polypeptide domains, wherein the locations given for those important polypeptide domains are approximate as described above. Analysis of the full-length PR0243 polypeptide evidences the presence of the following: a signal peptide from about amino acid 1 to about amino acid 23; N-glycosylation sites from about amino acid 217 to about amino acid 221, from about amino acid 351 to about amino acid 355, from about amino acid 365 to about amino acid 369, and from about amino acid 434 to about amino acid 438; tyrosine kinase phosphorylation sites from about amino acid 145 to about amino acid 153 and from about amino acid 778 to about amino acid 786; N-myristoylation sites from about amino acid 20 to about amino acid 26, from about amino acid 47 to about amino acid 53, from about amino acid 50 to about amino acid 56, from about amino acid 69 to about amino acid 75, from about amino acid 73 to about amino acid 79, from about amino acid 232 to about amino acid 238, from about amino acid 236 to about amino acid 242, from about amino acid 390 to about amino acid 396, from about amino acid 422 to about amino acid 428, from about amino acid 473 to about amino acid 479, from about amino acid 477 to about amino acid 483, from about amino acid 483 to about amino acid 489, from about amino acid 489 to about amino acid 495, from about amino acid 573 to about amino acid 579, from about amino acid 576 to about amino acid 582, from about amino acid 580 to about amino acid 586, from about amino aacid 635 to about amino acid 641, from about amino acid 670 to about amino acid 676, from about amino acid 773 to about amino acid 779, from about amino acid 807 to about amino acid 813, from about amino acid 871 to about amino acid 877, and from about amino acid 905 to about amino acid 911; an amidation site from about amino acid 87 to about amino acid 91; a cell attachment sequence from about amino acid 165 to about amino acid 168; and a leucine zipper pattern from about amino acid 315 to about amino acid 337. Clone DNA35917-1207 has been deposited with the ATCC on September 3, 1997 and is assigned ATCC deposit no. 209508. The full-length PR0243 protein has an estimated molecular weight of about 101,960 daltons and a pI of about 8.21.

EXAMPLE 8

Isolation of CDYA Clones Encodina_Human PRO256

A consensus DNA sequence was assembled relative to other EST sequences using phrap as described in Example 1 above. This assembled consensus sequence is herein identified as DNA28725. Based on the DNA28725 consensus sequence, oligonucleotides were synthesized: 1) to identify by PCR a cDNA library that contained the sequence of interest, and 2) for use as probes to isolate a clone of the full-length coding sequence for PR0256.

A pair of PCR primers (forward and reverse) were synthesized:

forward PCR primer:

5'-TGTCCACCAAGCAGACAGAAG-3' (SEQ ID NO:87)

reverse PCR primer:

5'-ACTGGATGGCGCCTTTCCATG-3' (SEQ ID NO:88)

Additionally, two synthetic oligonucleotide hybridization probes were constructed from the consensus DNA28725 sequence which had the following nucleotide sequences:

hybridization probes:

5'-GTGACAGTGAGTAGCTCAGACCACCCAGAGGACACGGCCAACGTCACAGT-3' (SEQ ID NO:89)
5'-GGGCTCTTTCCCACGCTGGTACTATGACCCCACGGAGCAGATCTG-3' (SEQ ID NO:90)

In order to screen several libraries for a source of a full-length clone, DNA from the libraries was screened by PCR amplification with the PCR primer pair identified above. A positive library was then used to isolate clones encoding the PR0256 gene using one of the probe oligonucleotides and one of the PCR primers.

RNA for construction of the cDNA libraries was isolated from human placenta tissue. The cDNA libraries used to isolate the cDNA clones were constructed by standard methods using commercially available reagents such as those from Invitrogen. San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI sites.

DNA sequencing of the clones isolated as described above gave the full-length DNA sequence for PRO256, herein designated as DNA35880-1160 [SEQ ID NO: 1] and the derived protein sequence for PRO256.

Clone DNA35880-1160 contains a single open reading frame with an apparent translational initiation site at nucleotide positions 188-190 and ending at the stop codon at nucleotide positions 1775-1777. The predicted polypeptide, precursor is 529 amino acids long. Analysis of the full-length PRO256 sequence (SEQ ID NO: 12) evidences the presence of a variety of important polypeptide domains, wherein the locations given for those important polypeptide domains are approximate as described above. Analysis of the full-length PR0256 polypeptide evidences the presence of the following: a signal peptide from about amino acid 1 to about amino acid 35; a transmembrane domain from about amino acid 466 to about amino acid 483; N-glycosylation sites from about amino acid 66 to about amino acid 70, from about amino acid 235 to about amino acid 239, and from about amino acid 523 to about amino acid 527; N-myristoylation sites from about amino acid 29 to about amino acid 35, from about amino acid 43 to about amino acid 49, from about amino acid 161 to about amino acid 167, from about amino acid 212 to about amino acid 218, from about amino acid 281 to about amino acid 287, from about amino acid 282 to about amino acid 288, from about amino acid 285 to about amino acid 291, from about amino acid 310 to about amino acid 316, from about amino acid 313 to about amino acid 319, from about amino acid 422 to about amino acid 428, from about amino acid 423 to about amino acid 429, and from about amino acid 426 to about amino acid 432; a cell attachment sequence from about amino acid 193 to about amino acid 199; and pancreatic trypsin inhibitor (Kunitz) family signatures from about amino acid 278 to about amino acid 298 and from about amino acid 419 to about amino acid 438. Clone DNA35880-1160 has been deposited with ATCC on October 16, 1997 and is assigned ATCC deposit no. 209379.

Analysis of the amino acid sequence of the full-length PR0256 polypeptide suggests that portions of it possess significant homology to the human bikunin protein, thereby indicating that PRO256 may be a novel proteinase inhibitor.

EXAMPLE 9

Isolation of cDNA Clones Encoding Human PRO269

A consensus DNA sequence was assembled relative to other EST sequences using phrap as described in Example 1 above. This consensus sequence is designated herein as DNA35705. Based on the assembled DNA35705 consensus sequence, oligonucleotides were synthesized: 1) to identify by PCR a cDNA library that contained the sequence of interest, and 2) for use as probes to isolate a clone of the full-length coding sequence for PR0269.

PCR primers (three forward and two reverse) were synthesized:

forward PCR primer 1:

5'-TGGAAGGAGATGCGATGCCACCTG-3'     (SEQ ID NO:91)

forward PCR primer 2:

    5'-TGACCAGTGGGGAAGGACAG 3'     (SEQ ID NO:92)

forward PCR primer 3:

    5'-ACAGAGCAGAGGGTGCCTrG-3'     (SEQ ID NO:93)

reverse PCR primer 1

    5'-7CAGGGACAAGTGGTGTCTCTCCC-3'     (SEQ ID NO:94)

reverse PCR primer 2:

    5'-TCAGGGAAGGAGTGTGCAGTTCTG-3'     (SEQ ID NO:95)

Additionally, a synthetic oligonucleotide hybridization probe was constructed from the DNA35705 consensus sequence which had the following nucleotide sequence:
hybridization probe:

    5'-ACAGCTCCCGATCTCAGTTACTTGCATCGCGGACGAAATCGGCGCTCGCT-3' (SEQ ID NO:96)

In order to screen several libraries for a source of a full-length clone, DNA from the libraries was screened by PCR amplification with the PCR primers identified above. A positive library was then used to isolate clones encoding the PR0269 gene using the probe oligonucleotide and one of the PCR primers. RNA for construction of the cDNA libraries was isolated from human fetal kidney tissue.

DNA sequencing of the isolated clones isolated as described above gave the full-length DNA sequence for DNA38260-1180 [SEQ ID NO:13]; and the derived protein sequence for PRO269.

Clone DNA38260-1180 contains a single open reading frame with an apparent translational initiation site at nucleotide positions 314-316, and an apparent stop codon at nucleotide positions 1784-1786. The predicted polypeptide precursor is 490 amino acids long with a molecular weight of approximately 51,636 daltons and an estimated pI of about 6.29. Analysis of the full-length PR0269 sequence (SEQ ID NO: 14) evidences the presence of variety of important polypeptide domains, wherein the locations given for those important polypeptide domains are approximate as described above. Analysis of the full-length PR0269 polypeptide evidences the presence of the following: a signal peptide from about amino acid 1 to about amino acid 16; a transmembrane domain from about amino acid 397 to about amino acid 418; N-glycosylation sites from about amino acid 189 to about amino acid 193, and from about amino acid 381 to about amino acid 385; a glycosaminoglycan attachment site from about amino acid 289 to about amino acid 293; cAMP- and cGMP-dependent protein kinase phosphorylation sites from about amino acid 98 to about amino acid 102, and from about amino acid 434 to about amino acid 438; N-myristoylation sites from about amino acid 30 to about amino acid 36, from about amino acid 35 to about amino acid 41, from about amino acid 58 to about amino acid 64, from about amino acid 59 to about amino acid 65, from about amino acid 121 to about amino acid 127, from about amino acid 151 to about amino acid 157, from about amino acid 185 to about amino acid 191, from about amino acid 209 to about amino acid 215, from about amino acid 267 to about amino acid 273, from about amino acid 350 to about amino acid 356, from about amino acid 374 to about amino acid 380, from about amino acid 453 to about amino acid 459, from about amino acid 463 to about amino acid 469, and from about amino acid 477 to about amino acid 483; and an aspartic acid and asparagine hydroxylation site from about amino acid 262 to about amino acid 274. Clone DNA38260-1180 has been deposited with the ATCC on October 17, 1997 and is assigned ATCC deposit no. 209397.

Analysis of the amino acid sequence of the full-length PR0269 sequence (SEQ ID NO:14), suggests that portions of it possess significant homology to the human thrombomodulin proteins, thereby indicating that PR0269 may possess one or more thrombomodulin-like domains.

EXAMPLE 10

Isolation of cDNA Clones Encoding Human PRO274

A consensus DNA sequence was assembled relative to other EST sequences using phrap as described in Example 1 above. This consensus sequence is designated herein as DNA36469. The DNA36469 consensus sequence was then extended using repeated cycles of BLAST and phrap to extend the consensus sequence as far as possible using the sources of EST sequences discussed above. The extended assembly consensus sequence is herein designated <consen01>. ESTs proprietary to Genentech were employed in the second consensus assembly and are herein designated DNA17873, DNA36157 and DNA28929. Based on the assembled DNA36469 and <consen01> consensus sequences, oligonucleotides were synthesized: 1) to identify by PCR a cDNA library that contained the sequence of interest, and 2) for use as probes to isolate a clone of the full-length coding sequence for PR0274.

Pairs of PCR primers (forward and reverse) were synthesized:

forward PCR primer 1 (36469.f1):

5'-CTGATCCGGTTCTTGGTGCCCCTG-3.'        (SEQ ID NO:97)

forward PCR primer 2 (36469.f2):

5'-GCTCTGTCACTCACGCTC-3'        (SEQ ID NO:98)

forward PCR primer 3 (36469.f3):

5'-TCATCTCTTCCCTCTCCC-3'        (SEQ ID NO:99)

forward PCR primer 4 (36469.f4):

5'-CCTTCCGCCACGGAGTTC-3'        (SEQ ID NO:100)

reverse PCR primer 1 (36469.r1):

5'-GCAAAGTCCACTCCGATGATGTC-3'        (SEQ ID NO:101)

reverse PCR primer 2 (36469.r2):

5'-GCCTGCTGTGGTCACAGGTCTCCG-3'        (SEQ ID NO:102)

Additionally, a synthetic oligonucleotide hybridization probe was constructed from the DNA36469 and <consen01> consensus sequences which had the following nucleotide sequence:

hybridization probe (36469.p1):

5'-TCGGGGAGCAGGCCTTGAACCGGGGCATTGCTGCTGTCAAGGAGG-3' (SEQ ID NO:103)

In order to screen several libraries for a source of a full-length clone, DNA from the libraries was screened by PCR amplification with the PCR primers identified above. A positive library was then used to isolate clones encoding the PR0274 gene using the probe oligonucleotide and one of the PCR primers. RNA for construction of the cDNA libraries was isolated from human fetal liver tissue (LIB229).

DNA sequencing of the isolated clones isolated as described above gave the full-length DNA sequence for DNA39987-1184, SEQ ID NO:15]; and the derived protein sequence for PRO274.

Clone DNA39987- 184 contains a single open reading frame with an apparent translational initiation site at nucleotide positions 83-85, and an apparent stop codon at nucleotide positions 1559-1561. The predicted polypeptide precursor is 492 amino acids long with a molecular weight of approximately 54,241 daltons and an estimated pI of about 8.21. Analysis of the full-length PRO274 sequence (SEQ ID NO:16) evidences the presence of a variety of important polypeptide domains, wherein the locations given for those important polypeptide domains are approximate as described above. Analysis of the full-length PRO274 polypeptide shown in Figure 16 evidences the presence of the following: transmembrane domains from about amino acid 86 to about amino acid 105, from about amino acid 162 to about amino acid 178, from about amino acid 327 to about amino acid 345, from about amino acid 359 to about amino acid 374, and from about amino acid 403 to about amino acid 423; N-glycosylation sites from about amino acid 347 to about amino acid 351, and from about amino acid 461 to about amino acid 465; a cAMP- and cGMP-dependent protein kinase phosphorylation site from about amino acid 325 to about amino acid 329; and N-myristoylation sites from about amino acid 53 to about amino acid 59, from about amino acid 94 to about amino acid 100, from about amino acid 229 to about amino acid 235, from about amino acid 267 to about amino acid 273, from about amino acid 268 to about amino acid 274, from about amino acid 358 to about amino acid 364, from about amino acid 422 to about amino acid 428, from about amino acid 425 to about amino acid 431, and from about amino acid 431 to about amino acid 437. Clone DNA39987-1184 has been deposited with the ATCC on April 21, 1998 and is assigned ATCC deposit no. 209786.

Analysis of the amino acid sequence of the full-length PRO274 sequence (SEQ ID NO:16), suggests that portions of it possess significant homology to the Fn54 protein. More specifically, an analysis of the Dayhoff database (version 35.45 SwissProt 35) evidenced significant homology between the PR0274 amino acid sequence and the following Dayhoff sequences: MMFN54S2_1. MMFN54S1_1, CELF48C1_8, CEF38B7_6, PRP3_RAT, INL3_PIG, MTCY07A7_13, YNAX_KLEAE A47234 and HME2_MOUSE.

EXAMPLE 11

Isolation of cDNA Clones Encoding Human PRO304

A consensus DNA sequence was assembled relative to other EST sequences using phrap as described in Example 1 above. This consensus sequence is designated herein as DNA35958. Based on the assembled DNA35958 consensus sequence, oligonucleotides were synthesized: 1) to identify by PCR a cDNA library that contained the sequence of interest, and 2) for use as probes to isolate a clone of the full-length coding sequence for PR0304.

Pairs of PCR primers (forward and reverse) were synthesized:

forward PCR primer 1:

5'-GCGGAAGGGCAGAATGGGACTCCAAG-3'       (SEQ ID NO: 104)

forward PCR primer 2:

5'-CAGCCCTGCCACATGTGC-3'       (SEQ ID NO: 105)

forward PCR primer 3:

5'-TACTGGGTGGTCAGCAAC-3'       (SEQ ID NO: 106)

reverse PCR primer 1:

5'-GGCGAAGAGCAGGGTGAGACCCCG-3'       (SEQ ID NO: 107)

Additionally, a synthetic oligonucleotide hybridization probe was constructed from the DNA35958 consensus sequence which had the following nucleotide sequence:

hybridization probe:

5'-GCCCTCATCCTCTCTGGCAAATGCAGTTACAGCCCGGAGCCCGAC-3' (SEQ 10 NO:108)

In order to screen several libraries for a source of a full-length clone, DNA from the libraries was screened by PCR amplification with the PCR primers identified above. A positive library was then used to isolate clones encoding the PR0304 gene using the probe oligonucleotide and one of the PCR primers. RNA for construction of the cDNA libraries was isolated from 22 week human fetal brain tissue (LIB153).

DNA sequencing of the isolated clones isolated as described above gave the full-length DNA sequence for DNA39520-1217 [SEQ ID NO:17]; and the derived protein sequence for PRO304.

Clone DNA39520-1217 contains a single open reading frame with an apparent translational initiation site at nucleotide positions 34-36, and an apparent stop codon at nucleotide positions 1702-1704. The predicted polypeptide precursor is 556 amino acids long. Analysis of the full-length PR0304 sequence (SEQ ID NO: 18) evidences the presence of a variety of important polypeptide domains, wherein the locations given for those important polypeptide domains are approximate as described above. Analysis of the full-length PR0304 polypeptide evidences the presence of the following: a signal sequence from about amino acid 1 to about amino acid 16; N-glycosylation sites from about amino acid 210 to about amino acid 214, from about amino acid 222 to about amino acid 226, from about amino acid 286 to about amino acid 290, from about amino acid 313 to about amino acid 317, and from about amino acid 443 to about amino acid 447; glycosaminoglycan attachment sites from about amino acid 361 to about amino acid 365, from about amino acid 408 to about amino acid 412, and from about amino acid 538 to about amino acid 542; and N-myristoylation sites from about amino acid 2 to about amino acid 8, from about amino acid 107 to about amino acid 113, from about amino acid 195 to about amino acid 201, from about amino acid 199 to about amino acid 205, from about amino acid 217 to about amino acid 223, from about amino acid 219 to about amino acid 225, from about amino acid 248 to about amino acid 254, from about amino acid 270 to about amino acid 276, from about amino acid 284 to about amino acid 290, from about amino acid 409 to about amino acid 415, from about amino acid 410 to about amino acid 416, from about amino acid 473 to about amino acid 479, from about amino acid 482 to about amino acid 488, from about amino acid 521 to about amino aciid 527, from about amino acid 533 to about amino acid 539, and from about amino acid 549 to about amino acid 555. Clone DNA39520-1217 has been deposited with the ATCC on November 21, 1997 and is assigned ATCC deposit no. 209482.

EXAMPLE 12

Isolation of cDNA Clones Encoding Human PR0339

An expressed sequence tag (EST) DNA database ( LIFESEQ®, Incyte Pharmaceuticals, Palo Alto, CA) was searched and an EST was identified. An assembly of Incyte clones and a consensus sequence was formed from which 4 forward primers, two reverse primers and another primer was formed. Human fetal liver cDNA libraries were screened by hybridization with a synthetic oligonucleotide probe based on the identified EST. The cDNA libraries used to isolate the cDNA clones encoding human PR0339 were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the Sfil site; *see,* Holmes et al., Science, 253:1278-1280 (1991)) in the unique XhoI and NotI.
The following oligonucleotide probes were used:

forward PCR primer 1:

　　5'-GGGATGCAGGTGGTGTCTCATGGGG-3'　　　(SEQ ID NO: 109)

forward PCR primer 2:

　　5'-CCCTCATGTACCGGCTCC-3'　　　(SEQ ID NO:110)

forward PCR primer 3:

　　5'-GTGTGACACAGCGTGGGC-3'　　　(SEQ ID NO:111)

forward PCR primer 4:

　　5'-GACCGGCAGGCTTCTGCG-3'　　　(SEQ ID NO:112)

reverse PCR primer 1:

　　5'-CAGCAGCTTCAGCCACCAGGAGTGG-3'　　　(SEQ ID NO:113)

reverse PCR primer 2:

　　5'-CTGAGCCGTGGGCTGCAGTCTCGC-3'　　　(SEQ ID NO:114)

primer:

　　5'-CGACTACGACTGGTTCTTCATCATGCAGGATGACACATATGTGC-3'　　　(SEQ ID NO:115)

A full length clone DNA43466-1225 [SEQ ID NO:19] was identified and sequenced in entirety that contained a single open reading frame with an apparent translational initiation site at nucleotide positions 333-335 and a stop signal at nucleotide positions 2649-2651 (SEQ ID NO:19). The predicted polypeptide precursor is 772 amino acids long and has a calculated molecular weight of approximately 86,226 daltons. Analysis of the full-length PRO339 sequence evidences the presence of a variety of important polypeptide domains, wherein the locations given for those important polypeptide domains are approximate as described above. Analysis of the full-length PR0339 polypeptide evidences the presence of the following: a signal sequence from about amino acid 1 to about amino acid 15; a transmembrane domain from about amino acid 489 to about amino acid 510; N-glycosylation sites from about amino acid 121 to about amino acid 125 and from about amino acid 342 to about amino acid 346; cAMP- and cGMP-dependent protein kinase phosphorylation sites from about amino acid 319 to about amino acid 323 and from about amino acid 464 to about amino acid 468; a tyrosine kinase phosphorylation site from about amino acid 736 to about amino acid 743; N-myristoylation sites from about amino acid 19 to about amino acid 25, from about amino acid 23 to about amino acid 29, from about amino acid 136 to about amino acid 142, from about amino acid 397 to about amino acid 403, from about amino acid 441 to about amino acid 447, from about amino acid 544 to about amino acid 550, from about amino acid 558 to about amino acid 564, from about amino acid 651 to about amino acid 657, from about amino acid 657 to about amino acid 663, and from about amino acid 672 to about amino acid 678; a prokaryotic membrane lipoprotein lipid attachment site from about amino acid 14 to about amino acid 25; and a cell attachment site from about amino acid 247 to about amino acid 250. Clone DNA43466-1225 has been deposited with ATCC on November 21, 1997 and is assigned ATCC deposit no. 209490.

Based on a BLAST and FastA sequence alignment analysis of the full-length sequence (SEQ ID NO:20), PRO339 shows amino acid sequence identity to *C. elegans* proteins and collagen-like polymer sequences as well as to fringe, thereby indicating that PR0339 may be involved in development or tissue growth.

<u>EXAMPLE 13</u>

<u>Isolation of cDNAs Encoding Human PRO1558</u>

DNA71282-1668 was identified by applying the proprietary signal sequence finding algorithm described in Example 2 above. Use of the above described signal sequence algorithm allowed identification of an EST cluster sequence from the LIFESEQ® database, Incyte Pharmaceuticals, Palo Alto, CA, designated Incyte EST cluster no. 86390. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA58842.

In light of an observed sequence homology between the DNA58842 sequence and Incyte EST clone no. 3746964, Incyte EST no. 3746974 was purchased and the cDNA insert was obtained and sequenced. The sequence of this cDNA insert is (SEQ ID NO:21) and is herein designated as DNA71282-1668.

Clone DNA71282-1668 contains a single open reading frame with an apparent translational initiation site at nucleotide positions 84-86 and ending at the stop codon at nucleotide positions 870-872 (Figure 21). The predicted polypeptide precursor is 262 amino acids long (SEQ ID NO:22). The full-length PRO1558 protein shown in Figure 22 has an estimated molecular weight of about 28,809 daltons and a pI of about 8.80. Analysis of the full-length PRO1558 sequence (SEQ ID NO:22) evidences the presence of a variety of important polypeptide domains, wherein the locations given for those important polypeptide domains are approximate as described above. Analysis of the full-length PRO1558 sequence shown in Figure 22 evidences the presence of the following: a signal peptide from about amino acid 1 to about amino acid 25; transmembrane domains from about amino acid 8 to about amino acid 30 and from about amino acid 109 to about amino acid 130; an N-glycosylation site from about amino acid 190 to about amino acid 194; a tyrosine kinase phosphorylation site from about amino acid 238 to about amino acid 247; N-myristoylation sites from about amino acid 22 to about amino acid 28, from about amino acid 28 to about amino acid 34, from about amino acid 110 to about amino acid 116, from about amino acid 205 to about amino acid 211, and from about amino acid 255 to about amino acid 261; and amidation sites from about amino acid 31 to about amino acid 35 and from about amino acid 39 to about amino acid 43. Clone DNA71282-1668 has been deposited with ATCC on October 6, 1998 and is assigned ATCC deposit no. 203312.

An analysis of the Dayhoff database (version 35.45 SwissProt 35), using a WU-BLAST2 sequence alignment analysis of the full-length sequence (SEQ ID NO:22), evidenced significant sequence identity between the PRO1558 amino acid sequence and the following Dayhoff sequences: AF075724_2, MXU24657_3, CAMT_EUCGU, MSU20736_1 , P_R29515, B70431, JC4004, CEY32B12A_3, CELF53B3_2 and P_R13543.

EXAMPLE 14

Isolation of cDNA Clones Encoding Human PR0779

Human fetal heart and human fetal lung 1gt10 bacteriophage cDNA libraries (both purchased from Clontech) were screened by hybridization with synthetic oligonucleotide probes based on an EST (GenBank locus W71984), which showed some degree of homology to the intracellular domains (ICD) of human TNFR1 and CD95. W71984 is a 523 bp EST, which in its -1 reading frame has 27 identities to a 43 amino acid long sequence in the ICD of human TNFR1. The oligonucleotide probes used in the screening were 27 and 25 bp long, respectively, with the following sequences:

    5'-GGCGCTCTGGTGGCCCTTGCAGAAGCC-3'        (SEQ ID NO:116)
    5-TTCGGCCGAGAAGTTGAGAAATGTC-3'           (SEQ ID NO:117)

Hybridization was done with a 1:1 mixture of the two probes overnight at room temperature in buffer containing 20% formamide, 5X SSC, 10% dextran sulfate, 0.1% $NaPiPO_4$,) 0.05 M $NaPO_4$, 0.05 mg salmon sperm DNA, and 0.1% sodium dodecyl sulfate (SDS), followed consecutively by one wash at room temperature in 6X SSC, two washes at 37°C in 1X SSC/0.1% SDS, two washes at 37°C in 0.5X SSC/0.1% SDS, and two washes at 37°C in 0.2X SSC/0.1% SDS. One positive clone from each of the fetal heart (FH20A.57) and fetal lung (FL8A.53) libraries were confused to be specific by PCR using the respective above hybridization probes as primers. Single phage plaques containing each of the positive clones were isolated by limiting dilution and the DNA was purified using a Wizard lambda prep DNA purification kit (Promega).

The cDNA inserts were excised from the lambda vector arms by digestion with EcoRI, gel-purified, and subcloned into pRK5 that was predigested with EcoRI. The clones were then sequenced in entirety.

Clone (FH20A.57) DNA58801-1052 (also referred to as Apo 3 clone FH20.57 deposited as ATCC 55820, as indicated below) contains a single open reading frame with an apparent translational initiation site at nucleotide positions 103-105 and ending at the stop codon found at nucleotide positions 1354-1356 [SEQ ID NO:23]. The predicted polypeptide precursor is 417 amino acids long (SEQ ID NO:24). The full-length PR0779 protein shown in Figure 24 has an estimated molecular weight of about 45,000 daltons and a pl of about 6.40. Analysis of the full-length PRO779 sequence (SEQ ID NO:24) evidences the presence of a variety of important polypeptide domains, wherein the locations given for those important polypeptide domains are approximate as described above. Analysis of the full-length PRO779 sequence evidences the presence of the following: a signal peptide from about amino acid 1 to about amino acid 24; a transmembrane domain from about amino acid 199 to about amino acid 219; N-glycosylation sites from about amino acid 67 to about amino acid 71 and from about amino acid 106 to about amino acid 110; a cAMP- and cGMP-dependent protein kinase phosphorylation site from about amino acid 157 to about amino acid 161; a tyrosine kinase phosphorylation site from about amino acid 370 to about amino acid 377; N-myristoylation sites from about amino acid 44 to about amino acid 50, from about amino acid 50 to about amino acid 56, from about amino acid 66 to about amino acid 72, from about amino acid 116 to about amino acid 122, from about amino acid 217 to about amino acid 223, from about amino acid 355 to about amino acid 361, from about amino acid 391 to about amino acid 397, and from about amino acid 401 to about amino acid 407; and a prokaryotic membrane lipoprotein lipid attachment site from about amino acid 177 to about amino acid 188. Clone DNA58801-1052 has been deposited with ATCC on September 5, 1996 and is assigned ATCC deposit no. 55820.

The ECD contains 4 cysteine-rich repeats which resemble the corresponding regions of human TNFR1 (4 repeats), of human CD95 (3 repeats) and of the other known TNFR family members. The ICD contains a death domain sequence that resembles the death domains found in the ICD of TNFR1 and CD95 and in the cytoplasmic death signalling proteins such as human FADD/MORT1, TRADD, RIP, and Drosophila Reaper. Both globally and in individual regions, PR0779 (Apo 3) is more closely related to TNFR1 than to CD95; the respective amino acid identities are 29.3% and 22.8% overall, 28.2% and 24.7% in the ECD, 31.6% and 18.3% in the ICD, and 47.5% and 20% in the death domain.

EXAMPLE 15

Isolation of cDNA Clones Encoding Human PRO1185

DNA62881-1515 was identified by applying the proprietary signal sequence finding algorithm described in Example 2 above. Use of the above described signal sequence algorithm allowed identification of an EST cluster sequence from the LIFESEQ® database, Incyte Pharmaceuticals, Palo Alto, CA. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington. Seattle, Washington). The consensus sequence obtained therefrom is herein designated as DNA56426.

In light of an observed sequence homology between the DNA56426 sequence and Incyte EST 3284411, the clone including this Incyte EST 3284411 (from a library constructed of RNA from aortic tissue) was purchased and the cDNA insert was obtained and sequenced. The sequence of this cDNA insert is shown in (SEQ ID NO:25) and is herein designated as DNA62881-1515.

Clone DNA62881-1515 contains a single open reading frame with an apparent translational initiation site at nucleotide positions 4-6 and ending at the stop codon at nucleotide positions 598-600. The predicted polypeptide precursor is 198 amino acids long (SEQ ID NO:26). The full-length PRO1185 protein shown in Figure 26 has an estimated molecular weight of about 22,105 daltons and a pI of about 7.73. Analysis of the full-length PRO1185 sequence (SEQ ID NO:26) evidences the presence of a variety of important polypeptide domains, wherein the locations given for those important polypeptide domains are approximate as described above. Analysis of the full-length PRO1185 sequence evidences the presence of the following: a signal peptide from about amino acid 1 to about amino acid 21; and N-myristoylation sites from about amino acid 46 to about amino acid 52, from about amino acid 51 to about amino acid 57, and from about amino acid 78 to about amino acid 84. Clone DNA62881-1515 has been deposited with ATCC on August 4, 1998 and is assigned ATCC deposit no. 203096.

An analysis of the Dayhoff database (version 35.45 SwissProt 35), using a WU-BLAST2 sequence alignment analysis of the full-length sequence (SEQ ID NO:26), evidenced significant sequence identity between the PRO1185 amino acid sequence and the following Dayhoff sequences: TUP1_YEAST, AF041382-1, MAOM_SOLTU, SPPBPHU9_1, EPCPLCFAIL_1, HSPLEC_1, YKL4_CAEEL, A44643, and TGU65922_1.

EXAMPLE 16

Isoladon of cDNA Clones Encoding Human PRO1245

DNA64884-1527 was identified by applying the proprietary signal sequence finding algorithm described in Example 2 above. Use of the above described signal sequence algorithm allowed identification of an EST cluster sequence from the LIFESEQ® database, Incyte Pharmaceuticals, Palo Alto, CA, designated Incyte EST Cluster No. 46370. This EST cluster sequence was then compared to a variety of expressed sequence tag (EST) databases which included public EST databases (*e.g.*, GenBank) and a proprietary EST DNA database LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). One or more of the ESTs used in the assembly was derived from a library constructed from tissue obtained from the parotid (salivary) gland of a human with parotid cancer. The consensus sequence obtained therefrom is herein designated as DNA56019.

In light of an observed sequence homology between the DNA56019 sequence and Incyte EST clone no. 1327836, Incyte EST clone no. 1327836 was purchased and the cDNA insert was obtained and sequenced. The sequence of this cDNA insert (SEQ ID NO:27) is herein designated as DNA64884-1527.

Clone DNA64884-1527 contains a single open reading frame with an apparent translational initiation site at nucleotide positions 79-81 and ending at the stop codon at nucleotide positions 391-393. The predicted polypeptide precursor is 104 amino acids long (SEQ ID NO:28). The full-length PRO1245 protein shown in Figure 28 has an estimated molecular weight of about 10,100 daltons and a pI of about 8.76. Analysis of the full-length PRO1245 sequence (SEQ ID NO:28) evidences the presence of a variety of important polypeptide domains, wherein the locations given for those important polypeptide domains are approximate as described above. Analysis of the full-length PRO1245 sequence evidences the presence of the following: a signal peptide from about amino acid 1 to about amino acid 18; N-myristoylation sites from about amino acid 8 to about amino acid 14, from about amino acid 65 to about amino acid 71, from about amino acid 74 to about amino acid 80, and from about amino acid 88 to about amino acid 94; and a prokaryotic membrane lipoprotein lipid attachment site from about amino acid 5 to about amino acid 16. Clone DNA64884-1527 has been deposited with ATCC on August 25, 1998 and is assigned ATCC deposit no. 203155.

An analysis of the Dayhoff database (version 35.45 SwissProt 35), using a WU-BLAST2 sequence alignment analysis of the full-length sequence (SEQ ID NO:28), evidenced some homology between the PRO1245 amino acid sequence and the following Dayhoff sequences: SYA_THETH, GEN11167, MTV044_4, AB011151_1, RLAJ2750_3, SNELIPTRA_1, S63624, C28391, A37907, and S14064.

EXAMPLE 17

Isolation of cDNA Clones Encoding Human PRO1759

DNA76531-1701 was identified by applying the proprietary signal sequence finding algorithm described in Example 2 above. Use of the above described signal sequence algorithm allowed identification of an EST cluster sequence from the LIFESEQ® database. Incyte Pharmaceuticals, Palo Alto. CA, designated DNA 10571. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-180 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). One or more of the ESTs used in the assembly was derived from pooled eosinophils of allergic asthmatic patients. The consensus sequence obtained therefrom is herein designated as DNA57313.

In light of an observed sequence homology between the DNA57313 sequence and Incyte EST2434255. the clone including this Incyte EST2434255 was purchased and the cDNA insert was obtained and sequenced. The sequence of this cDNA insert is (SEQ ID NO:29) and is herein designated as DNA76531-1701.

Clone DNA76531-1701 contains a single open reading frame with an apparent translational initiation site at nucleotide positions 125-127 and ending at the stop codon at nucleotide positions 1475-1477. The predicted polypeptide precursor is 450 amino acids long (SEQ ID NO:30). The full-length PRO1759 protein shown in Figure 30 has an estimated molecular weight of about 49,765 daltons and a pI of about 8.14. Analysis of the full-length PRO1759 sequence (SEQ ID NO:30) evidences the presence of a variety of important polypeptide domains, wherein the locations given for those important polypeptide domains are approximate as described above. Analysis of the full-length PRO1759 sequence evidences the presence of the following: a signal peptide from about amino acid 1 to about amino acid 18; transmembrane domains from about amino acid 41 to about amino acid 55, from about amino acid 75 to about amino acid 94, from about amino acid 127 to about amino acid 143, from about amino acid 191 to about amino acid 213, from about amino acid 249 to about amino acid 270, from about amino acid 278 to about amino acid 299, from about amino acid 314 to about amino acid 330, from about amino acid 343 to about amino acid 359, from about amino acid 379 to about amino acid 394, and from about amino acid 410 to about amino acid 430; a cAMP- and cGMP-dependent protein kinase phosphorylation site from about amino acid 104 to about amino acid 108; N-myristoylation sites from about amino acid 11 to about amino acid 17, from about amino acid 18 to about amino acid 24, from about amino acid 84 to about amino acid 90, from about amino acid 92 to about amino acid 98, from about amino acid 137 to about amino acid 143, from about amino acid 138 to about amino acid 144, from about amino acid 238 to about amino acid 244, from about amino acid 253 to about amino acid 259, from about amino acid 278 to about amino acid 284, and from about amino acid 282 to about amino acid 288; an amidation site from about amino acid 102 to about amino acid 106; and a prokaryotic membrane lipoprotein lipid attachment site from about amino acid 6 to about amino acid 17. Clone DNA76531-1701 has been deposited with ATCC on November 17, 1998 and is assigned ATCC deposit no. 203465.

An analysis of the Dayhoff database (version 35.45 SwissProt 35), using a WU-BLAST2 sequence alignment analysis of the full-length sequence (SEQ ID NO:30), evidenced sequence identity between the PRO1759 amino acid sequence and the following Dayhoff sequences: OPDE_PSEAE, TH11_TRYBB, S67684, RGT2_YEAST, S68362, ATSUGTRPR_1, P_W17836 (Patent application WO9715668-A2), F69587, A48076, and A45611.

EXAMPLE 18

Isolation of cDNA Clones Encoding Human PRO5775

DNA96869-2673 was identified by applying the proprietary signal sequence finding algorithm described in Example 2 above. Use of the above described signal sequence algorithm allowed identification of an EST cluster sequence from the LIFESEQ® database, Incyte Pharmaceuticals, Palo Alto, CA, designated herein as CLU86443. This EST cluster sequence was then compared to a variety of expressed sequence tag (EST) databases which included public EST databases (*e.g.*, GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al, Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA79860.

In light of an observed sequence homology between the DNA79860 sequence and an Incyte EST sequence encompassed within clone no. 1614726H1 from the LIFESEQ®, Incyte Pharmaceuticals, Palo Alto, CA database, clone no. 1614726H1 was purchased and the cDNA insert was obtained and sequenced. The sequence of this cDNA insert (SEQ ID NO:31) is herein designated as DNA96869-2673.

Clone DNA96869-2673 contains a single open reading frame with an apparent translational initiation site at nucleotide positions 193-195 and ending at the stop codon at nucleotide positions 1660-1662. The predicted polypeptide precursor is 489 amino acids long (SEQ ID NO:32). The full-length PRO5775 protein has an estimated molecular weight of about 53,745 daltons and a pI of about 8.36. Analysis of the full-length PRO5775 sequence (SEQ ID NO:32) evidences the presence of a variety of important polypeptide domains, wherein the locations given for those important polypeptide domains are approximate as described above. Analysis of the full-length PRO5775 sequence evidences the presence of the following: a signal peptide from about amino acid 1 to about amino acid 29; a transmembrane domain from about amino acid 381 to about amino acid 399; N-glycosylation sites from about amino acid 133 to about amino acid 137, from about amino acid 154 to about amino acid 158, from about amino acid 232 to about amino acid 236, from about amino acid 264 to about amino acid 268, from about amino acid 386 to about amino acid 390, from about amino acid 400 to about amino acid 404, from about amino acid 410 to about amino acid 414, and from about amino acid 427 to about amino acid 431; and N-myristoylation sites from about amino acid 58 to about amino acid 64, from about amino acid 94 to about amino acid 100, from about amino acid 131 to about amino acid 137, from about amino acid 194 to about amino acid 200, from about amino acid 251 to about amino acid 257, from about amino acid 277 to about amino acid 283, from about amino acid 281 to about amino acid 287, from about amino acid 361 to about amino acid 367, from about amino acid 399 to about amino acid 405, from about amino acid 440 to about amino acid446, from about amino acid 448 to about amino acid 454, and from about amino acid 478 to about amino acid 484. Clone DNA96869-2673 has been deposited with ATCC on June 22, 1999 and is assigned ATCC deposit no. PTA-255.

An analysis of the Dayhoff database (version 35.45 SwissProt 35), using a WU-BLAST2 sequence alignment analysis of the full-length sequence (SEQ ID NO:32), evidenced sequence identity between the PRO5775 amino acid sequence and the following Dayhoff sequences: U94848_12, P_W57899, CV41KBPL_33, HSU60644_1, CVORF1L5L_3, VK04_VACCV, CVGRI90_41, VK04_VACCC, and AF026124_1.

EXAMPLE 19

Isolation of cDNA Clones Encoding a Human PR07133

Clone DNA128450-2739 was pulled out by a CARD homolog screen, and the sequence was used as a probe to isolate a clone of the full-length coding sequence for PRO7133 using traditional low stringency and hybridization. To identify the full ORF for the PR07133 cDNA, the CARD domain containing molecule; a cDNA fragment encoding the N-terminal portion of SOCA-1; was used to screen a human fetal kidney library. Several positive clones were picked up, and the DNA was prepared and sequenced.

forward primer:

  5'-GCCGGATCCACAATGGCTACCGAGAGTACTCC-3'        (SEQ ID NO:118)

reverse primer:

  5'-GCGGAATTCACAGATCCTCTTCTGAGATGAGTTTCTGTTCTCCTCCAATGAAAGGC-3'        (SEQ ID NO: 119)

The probe DNA (soca-1) had the following nucleotide sequence:

5'CGCGTACGTAAGCTCGGAATTCGGCTCGAGGGAACAATGGCTACCGAGAGTACTCCCTCAGAG
ATCATAGAACTGGTGAAGAACCAAGTTATGAGGGATCAGAAACCAGCCTTTCATTGGAGGAGGA
ACAGGAGAAAAGTATAAAAAAAAAAAAAAAAAGGGCGGCCGCCGACTAGTGAGCTCGTCGACCCG
GGAATTAATTCCGGACCGGTACCTGCAGGCGTACCAGCTTTCCCTATAGTAGTG-3'
(SEQ ID NO:120)

DNA sequencing revealed that one of the cDNA clones contains a full-length ORF that encodes a protein significantly homologous to the human Sab protein; the PR07133 polypeptide (designated herein as DNA 128451-2739 [SEQ ID NO:33] and the derived protein sequence for that PRO7133 polypeptide.

Clone DNA 128451-2739 contains a single open reading frame with an apparent translational initiation site at nucleotide positions 501-503 and ending at the stop codon at nucleotide positions 1680-1682. The predicted polypeptide precursor is 393 amino acids long (SEQ ID NO:34). The full-length PRO7133 protein has an estimated molecular weight of about 43,499 daltons and a pI of about 5.75. Analysis of the full-length PR07133 sequence (SEQ ID NO:34) evidences the presence of a variety of important polypeptide domains, wherein the locations given for those important polypeptide domains are approximate as described above. Analysis of the full-length PRO7133 sequence evidences the presence of the following: cAMP- and cGMP-dependent protein kinase phosphorylation sites from about amino acid 287 to about amino acid 291 and from about amino acid 375 to about amino acid 379; N-myristoylation sites from about amino acid 37 to about amino acid 43, from about amino acid 38 to about amino acid 44, from about amino acid 39 to about amino acid 45, from about amino acid 40 to about amino acid 46, from about amino acid 103 to about amino acid 109, from about amino acid 307 to about amino acid 313, from about amino acid 310 to about amino acid 316, from about amino acid 315 to about amino acid 321, from about amino acid 365 to about amino acid 371, from about amino acid 369 to about amino acid 375, from about amino acid 373 to about amino acid 379, fromabout amino acid 377 to about amino acid 383, from about amino acid 380 to about amino acid 386, and from about amino acid 381 to about amino acid 387; and an amidation site from about amino acid 373 to about amino acid 377. Clone DNA128451-2739 has been deposited with ATCC on August 31, 1999 and is assigned ATCC deposit no. PTA-618.

EXAMPLE 20

Isolation of cDNA Clones Encoding Human PR07168

DNA 102846-2742 was identified by applying the proprietary signal sequence finding algorithm described in Example 2 above. Use of the above described signal sequence algorithm allowed identification of an EST cluster sequence from the LTFESEQ® database, Incyte Pharmaceuticals, Palo Alto, CA, designated herein as CLU122441. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al, Methods in Enzymology, 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated as DNA57953.

In light of an observed sequence homology between the DNA57953 sequence and an Incyte EST sequence encompassed within clone no. 4181351 from the LIFESEQ®, Incyte Pharmaceuticals, Palo Alto, CA database, clone no. 4181351 was purchased and the cDNA insert was obtained and sequenced. The sequence of this cDNA insert (SEQ ID NO:35) is herein designated as DNA 102846-2742.

Clone DNA 102846-2742 contains a single open reading frame with an apparent translational initiation site at nucleotide positions 23-25 and ending at the stop codon at nucleotide positions 2540-2542. The predicted polypeptide precursor is 839 amino acids long (SEQ ID NO:36). The full-length PR07168 protein has an estimated molecular weight of about 87,546 daltons and a pI of about 4.84. Analysis of the full-length PR07168 sequence (SEQ ID NO:36) evidences the presence of a variety of important polypeptide domains, wherein the locations given for those important polypeptide domains are approximate as described above. Analysis of the full-length PRO7168 sequence shown in Figure 36 evidences the presence of the following: a signal peptide from about amino acid 1 to about amino acid 25; a transmembrane domain from about amino acid 663 to about amino acid 686; N-glycosylation sites from about amino acid 44 to about amino acid 48, from about amino acid 140 to about amino acid 144, from about amino acid 198 to about amino acid 202, from about amino acid 297 to about amino acid 301, from about amino acid 308 to about amino acid 312, from about amino acid 405 to about amino acid 409, and from about amino acid 520 to about amino acid 524; glycosaminoglycan attachment sites from about amino acid 490 to about amino acid 494, from about amino acid 647 to about amino acid 651 and from about amino acid 813 to about amino acid 817; a cAMP- and cGMP-dependent protein kinase phosphorylation site from about amino acid 655 to about amino acid 659; tyrosine kinase phosphorylation sites from about amino acid 154 to about amino acid 163 and from about amino acid 776 to about amino acid 783; N-myristoylation sites from about amino acid 57 to about amino acid 63, from about amino acid 102 to about amino acid 108, from about amino acid 255 to about amino acid 261, from about amino acid 294 to about amino acid 300, from about amino acid 366 to about amino acid 372, from about amino acid 426 to about amino acid 432, from about amino acid 441 to about amino acid 447, from about amino acid 513 to about amino acid 519, from about amino acid 517 to about amino acid 523, from about amino acid 530 to about amino acid 536, from about amino acid 548 to about amino acid 554, from about amino acid 550 to about amino acid 556, from about amino acid 581 to about amino acid 587, from about amino acid 592 to about amino acid 598, from about amino acid 610 to about amino acid 616, from about amino acid 612 to about amino acid 618, from about amino acid 623 to about amino acid 629, from about amino acid 648 to about amino acid 654, from about amino acid 666 to about amino acid 672, from about amino acid 667 to about amino acid 673, from about amino acid 762 to about amino acid 768, from about amino acid 763 to about amino acid 769, from about amino acid 780 to about amino acid 786, from about amino acid 809 to about amino acid 815, from about amino acid 821 to about amino acid 827, and from about amino acid 833 to about amino acid 839; and a cadherins extracellular repeated domain signature from about amino acid 112 to about amino acid 123. Clone DNA102846-2742 has been deposited with ATCC on August 17, 1999 and is assigned ATCC deposit no. PTA-545.

An analysis of the Dayhoff database (version 35.45 SwissProt 35), using a WU-BLAST2 sequence alignment analysis of the full-length sequence (SEQ ID NO:36), evidenced sequence identity between the PRO7169 amino acid sequence and the following Dayhoff sequences: CELT22D1_9, B48013, AF100960_1, MUC2_HUMAN, PRP3_MOUSE S53363,A39066, HUMSPRPA_1, AF053091_1, andS80905_1.

EXAMPLE 21

Isolation of cDNA Clones Encoding Human PRO5725

An expressed sequence tag (EST) DNA database (LIFESEQ®, Incyte Pharmaceuticals, Palo Alto, CA) was searched and an EST was identified which showed homology to Neuritin. Incyte ESTclone no. 3705684 was then purchased from LIFESEQ®, Incyte Pharmaceuticals, Palo Alto, CA and the cDNA insert of that clone (designated herein as DNA92265-2669) was obtained and sequenced in entirety [SEQ ID NO:37].

The full-length clone [DNA92265-2669; SEQ ID NO:37] contains a single open reading frame with an apparent translational initiation site at nucleotide positions 27-29 and a stop signal at nucleotide positions 522-524 (SEQ ID NO: 37). The predicted polypeptide precursor is 165 amino acids long and has a calculated molecular weight of approximately 17,786 daltons and an estimated pl of approximately 8.43. Analysis of the full-length PRO5725 sequence (SEQ ID NO: 38) evidences the presence of a variety of important polypeptide domains, wherein the locations given for those important polypeptide domains are approximate as described above. Analysis of the full-length PR05725 polypeptide shown in Figure 38 evidences the presence of the following: a signal sequence from about amino acid 1 to about amino acid 35; a transmembrane domain from about amino acid 141 to about amino acid 157; an N-myristoylation site from about amino acid 127 to about amino acid 133; and a prokaryotic membrane lipoprotein lipid attachment site from about amino acid 77 to about amino acid 88. Clone DNA92265-2669 has been deposited with ATCC on June 22,1999 and is assigned ATCC deposit no. PTA-256.

An analysis of the Dayhoff database (version 35.45 SwissProt 35), using a WU-BLAST2 sequence alignment analysis of the full-length sequence (SEQ ID NO:38), evidenced sequence identity between the PR05725 amino acid sequence and the following Dayhoff sequences: RNU88958_1, P_W37859, P_W37858, JC6305, HGS_RE778, HGS_ RE777, P_W27652, P_W44088, HGS_RE776, and HGS_RE425.

EXAMPLE 22

Isolation of cDNA Clones Encoding Human PRO1800

A consensus DNA sequence was assembled relative to other EST sequences using phrap as described in Example 1 above. This consensus sequence is designated herein as DNA30934. Based on the assembled DNA30934 consensus sequence, oligonucleotides were synthesized: 1) to identify by PCR a cDNA library that contained the sequence of interest, and 2) for use as probes to isolate a clone of the full-length coding sequence for PRO1800.

PCR primers (forward and reverse) were synthesized:

forward PCR primer (30934.f1):

    5'-GCATAATGGATGTCACTGAGG-3'    (SEQ ID NO:121)

reverse PCR primer (30934.r1):

    5'-AGAACAATCCTGCTGAAAGCTAG-3'    (SEQ ID NO: 122)

Additionally, a synthetic oligonucleotide hybridization probe was constructed from the DNA30934 consensus sequence which had the following nucleotide sequence:

hybridization probe (30934.p1):

5'-GAAACGAGGAGGCGGCTCAGTGGTGATCGTGTCTTCCATAGCAGCC-3' (SEQ ID NO:123)

In order to screen several libraries for a source of a full-length clone, DNA from the libraries was screened by PCR amplification with the PCR primers identified above. A positive library was then used to isolate clones encoding the PRO1800 gene using the probe oligonucleotide and one of the PCR primers. RNA for construction of the cDNA libraries was isolated from human fetal liver tissue.

DNA sequencing of the isolated clones isolated as described above gave the full-length DNA sequence for DNA35672-2508 [Figure 1, SEQ ID NO:59]; and the derived protein sequence for PRO1800.

The entire coding sequence of DNA35672-2508 is included in Figure 1 (SEQ ID NO:59). Clone DNA35672-2508 contains a single open reading frame with an apparent translational initiation site at nucleotide positions 36-38, and an apparent stop codon at nucleotide positions 870-872. The predicted polypeptide precursor is 278 amino acids long and has an estimated molecular weight of about 29,537 daltons and a pI of about 8.97. Analysis of the full-length PRO1800 sequence shown in Figure 2 (SEQ ID NO:60) evidences the presence of a variety of important polypeptide domains, wherein the locations given for those important polypeptide domains are approximate as described above. Analysis of the full-length PRO1800 polypeptide shown in Figure 2 evidences the presence of the following: a signal sequence from about amino acid 1 to about amino acid 15; an N-glycosylation site from about amino acid 183 to about amino acid 187; N-myristoylation sites from about amino acid 43 to about amino acid 49, from about amino acid 80 to about amino acid 86, from about amino acid 191 to about amino acid 197, from about amino acid 213 to about amino acid 219, and from about amino acid 272 to about amino acid 278: a microbodies C-terminal targeting signal from about amino acid 276 to about amino acid 280; and a short-chain alcohol dehydrogenase sequence from about amino acid 162 to about amino acid 199. Clone DNA35672-2508 has been deposited with the ATCC on December 15, 1998 and is assigned ATCC deposit no. 203538.

An analysis of the Dayhoff database (version 35.45 SwissProt 35), using a WU-BLAST2 sequence alignment analysis of the full-length sequence shown in Figure 2 (SEQ ID NO:60), evidenced significant homology between the PRO1800 amino acid sequence and the following Dayhoff sequences: HE27_HUMAN, CELF36H9_1, CEF54F3_3, A69621, AP000007_27, UCPA_ECOLI, F69868, Y4LA_RHISN, DHK2_STRVN, and DHG1_BACME.

DE 198 18 620 describes a nucleic acid sequence with SEQ.ID.10 with a high degree of identity to that in present figure 1, encoding PRO1800. It is shown to be differentially expressed in various tumours, whereby both up- and downregulation are observed depending on the tumour type. In WO 00/04135, ScRM-1 is described, which has a high degree of identity to PR01800, both at the nucleic acid and at the protein level. Use of antibodies in detecting differences in expression levels of ScRM-1 are suggested in the diagnosis of proliferative disorders.

EXAMPLE 23

Isolation of cDNA Clones Encoding Human PRO539

An expressed sequence tag (EST) DNA database (LIFESEQ®, Incyte Pharmaceuticals, Palo Alto, CA) was searched and an EST (1299359) was identified which showed homology to Costal-2 protein of Drosophila melanogaster. This EST sequence was then compared to various EST databases including public EST databases (eg., GenBank), and a proprietary EST database (LIFESEQ®, Incyte Pharmaceuticals, Palo Alto, CA) to identify homologous EST sequences. The comparison was performed using the computer program BLAST or BLAST2 (Altschul et al., Methods in Enzymology, 266:460-480 (1996)) and another sequence EST. The comparisons were clustered and assembled into a consensus DNA sequence with the program "phrap" (Phil Green, University of Washington, Seattle, Washington). This consensus sequence is herein designated "consensus".

Based on the assembled "consensus" sequence, oligonucleotides were synthesized: 1) to identify by PCR a cDNA library that contained the sequence of interest, and 2) for use as probes to isolate a clone of the full-length coding sequence for PRO539. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5 kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

PCR primers (forward and reverse) were synthesized:

forward PCR primer (hcos2.F):

       5'-GATGAGGCCATCGAGGCCGG-3'       (SEQ ID NO:124)

reverse PCR primer (hcos2.R):

5'-TCTCGGAGCGTCACCACCTTGTC-3'        (SEQ ID NO:125)

Additionally, a synthetic oligonucleotide hybridization probe was constructed from the "consensus" sequence which had the following nucleotide sequence:
Hybridization probe (hcos2.P):

5'-CTGGATGCTGCCATTGAGTATAAGAATGAGGCCATCACA-3' (SEQ ID NO: 126)

RNA for construction of the cDNA libraries was isolated from human fetal kidney tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI sites.
DNA sequencing of the isolated clones isolated as described above gave the full-length DNA sequence for DNA47465-1561 [SEQ ID NO:65]; and the derived protein sequence for PRO539.
Clone DNA47465-1561 contains a single open reading frame with an apparent translational initiation site at nucleotide positions 186-188, and an apparent stop codon at nucleotide positions 2676-2678. The predicted polypeptide precursor is 830 amino acids long and has an estimated molecular weight of about 95,029 daltons and a pI of about 8.26. Analysis of the full-length PRO539 sequence (SEQ ID NO:66) evidences the presence of a variety of important polypeptide domains, wherein the locations given for those important polypeptide domains are approximate as described above. Analysis of the full-length PRO539 polypeptide evidences the presence of the following: leucine zipper patterns from about amino acid 557 to about amino acid 579 and from about amino acid 794 to about amino acid 816; N-glycosylation sites from about amino acid 133 to about amino acid 137 and from about amino acid 383 to about amino acid 387; and a kinesin related protein Kif-4 coiled-coil domain from about amino acid 231 to about amino acid 672. Clone DNA47465-1561 has been deposited with the ATCC on February 9, 1999 and is assigned ATCC deposit no. 203661.
An analysis of the Dayhoff database (version 35.45 SwissProt 35), using a WU-BLAST2 sequence alignment analysis of the full-length sequence (SEQ ID NO:66), evidenced significant homology between the PRO539 amino acid sequence and the following Dayhoff sequences: AFO19250_1, KIF4_MOUSE, TRHY_HUMAN, A56514, G02520, MYSP_HUMAN, AF041382_1,A45592, HS125H2_1, and HS6802_2.

EXAMPLE 24

Isolation of cDNA Clones Encoding Human PRO4316

A cDNA clone designated herein as DNA80935 was identified by a yeast screen, in a human adrenal gland cDNA library that preferentially represents the 5'ends of the primary cDNA clones. This cDNA was then compared to other known EST sequences, wherein the comparison was performed using the computer program BLAST or BLAST2 (Altschul et al., Methods in Enzymology, 266:460-480 (1996)]. Those comparisons resulting in a BLAST score of 70 (or in some cases, 90) or greater that did not encode known proteins were clustered and assembled into a consensus DNA sequence with the program "phrap" (PhilGreen, University of Washington, Seattle, Washington). Ths consensus sequence is herein designated DNA83527.
PCR primers (forward and reverse) were synthesized based upon the DNA83527 sequence:

forward PCR primer:

5'-TGGACGACCAGGAGAAGCTGC-3' (SEQ ID NO: 127)

reverse PCR primer.

5'-CTCCACTTGTCCTCTGGAAGGTGG-3' (SEQ ID NO:128)

Additionally, a synthetic oligonucleotide hybridization probe was constructed from the DNA83527 consensus sequence which had the following nucleotide sequence:

hybridization probe:

5'-GCAAGAGGCAGAAGCCATGTTAGATGAGCCTCAGGAACAAGCGG-3' (SEQ ID NO:129)

RNA for construction of the cDNA libraries was isolated from human adrenal gland tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D thatdoes not contain the SfiI site; *see,* Holmes et al., Science 253:1278-1280 (1991)) in the unique XhoI and NotI sites.

The full-length DNA94713-2561 clone obtained from this screen [SEQ ID NO:67] contains a single open reading frame with an apparent translational initiation site at nucleotide positions 293-295, and an apparent stop codon at nucleotide positions 1934-1936. The predicted polypeptide precursor is 547 amino acids long. The full-length PRO4316 protein has an estimated molecular weight of about 61,005 daltons and a pI of about 6.34. Analysis of the full-length PRO4316 sequence (SEQ ID NO:68) evidences the presence of a variety of important polypeptide domains, wherein the locations given for those important polypeptide domains are approximate as described above. Analysis of the full-length PRO4316 polypeptide evidences the presence of the following: a signal peptide from about amino acid 1 to about amino acid 23; transmembrane domains from about amino acid 42 to about amino acid 60 and from about amino acid 511 to about amino acid 530; N-glycosylation sites from about amino acid 259 to about amino acid 263 and from about amino acid 362 to about amino acid 366; casein kinase II phosphorylation sites from about amino acid 115 to about amino acid 119, from about amino acid 186 to about amino acid 190, from about amino acid 467 to about amino acid 471, and from about amino acid 488 to about amino acid 494; N-myristoylation sites from about amino acid 255 to about amino acid 261, from about amino acid 304 to about amino acid 310, and from about amino acid 335 to about amino acid 341; and amidation sites from about amino acid 7 to about amino acid 11 and from about amino acid 174 to about amino acid 178. Clone DNA94713-2561 has been deposited with the ATCC on March 9,1999 and is assigned ATCC deposit no. 203835.

An analysis of the Dayhoff database (version 35.45 SwissProt 35), using a WU-BLAST2 sequence alignment analysis of the full-length sequence (SEQ ID NO:68), evidenced significant homology between the PRO4316 amino acid sequence and the following Dayhoff sequences: YDA9_SCHPO, S67452, S69714, DP27_CAEEL, S47053, CEY43F8C_4, VP2_BRD, and SPCC895_9.

EXAMPLE 25

Isolation of cDNA Clones Encoding Human PR04980

An initial DNA sequence, referred to herein as DNA81573 was identified by a yeast screen, in a human cDNA library that preferentially represents the 5' ends of the primary cDNA clones. This cDNA was then compared to ESTs from public databases (e.g., GenBank), and a proprietary EST database (LIFESEQ®, Incyte Pharmaceuticals, Palo Alto, CA), using the computer program BLAST or BLAST2 [Altschul et al., Methods in Enzymology, 266:460-480(1996)]. The ESTs were clustered and assembled into a consensus DNA sequence with the program "phrap" (Phil Green, University of Washington, Seattle, Washington). Ths consensus sequence is herein designated DNA90613.

PCR primers (forward and reverse) were synthesized based upon the DNA90613 sequence for use as probes to isolate a clone of the full-length coding sequence for PRO4480 from a human aortic endothelial cell cDNA library:

forward PCR primer:

5'-CAACCGTATGGGACCGATACTCG-3'          (SEQ ID NO: 130)

reverse PCR primer:

5'-CACGCTDCAACGAGTCTTCATG-3          ' (SEQ ID NO:131)

hybridization probe:

5'-GTGGCCCTCGCAGTGCAGGCCTTCTACGTCCAATACAAGTG-3' (SEQ ID NO:132)

RNA for construction of the cDNA libraries was isolated from human aortic endothelial cell tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI sites.

The full-length DNA97003-2649 clone obtained from this screen [SEQ ID NO:69] contains a single open reading frame with an apparent translational initiation site at nucleotide positions 286-288, and an apparent stop codon at nucleotide positions 1900-1902. The predicted polypeptide precursor is 538 amino acids long. The full-length PRO4980 protein has an estimated molecular weight of about 59,268 daltons and a pI of about 8.94. Analysis of the full-length PRO4980 sequence (SEQ ID NO:70) evidences the presence of a variety of important polypeptide domains, wherein the locations given for those important polypeptide domains are approximate as described above. Analysis of the full-length PRO4980 polypeptide evidences the presence of the following: a signal peptide from about amino acid 1 to about amino acid 36; transmembrane domains from about amino acid 77 to about amino acid 95, from about amino acid 111 to about amino acid 133, from about amino acid 161 to about amino acid 184, from about amino acid 225 to about amino acid 248, from about amino acid 255 to about amino acid 273, from about amino acid 299 to about amino acid 314, from about amino acid 348 to about amino acid 373, from about amino acid 406 to about amino acid 421, from about amino acid 435 to about amino acid 456, and from about amino acid 480 to about amino acid 497; an N-glycosylation site from about amino acid 500 to about amino acid 504; a cAMP-and cGMP-dependent protein kinase phosphorylation site from about amino acid 321 to about amino acid 325; N-myristoylation sites from about amino acid 13 to about amino acid 19, from about amino acid 18 to about amino acid 24, from about amino acid 80 to about amino acid 86, from about amino acid 111 to about amino acid 117, from about amino acid 118 to about amino acid 124, from about amino acid 145 to about amino acid 151, from about amino acid 238 to about amino acid 244, from about amino acid 251 to about amino acid 257, from about amino acid 430 to about amino acid 436, from about amino acid 433 to about amino acid 439, from about amino acid 448 to about amino acid 454, from about amino acid 458 to about amino acid 464, from about amino acid 468 to about amino acid 474, from about amino acid 475 to about amino acid 481, from about amino acid 496 to about amino acid 502, and from about amino acid 508 to about amino acid 514; and a prokaryotic membrane lipoprotein lipid attachment site from about amino acid 302 to about amino acid 313. Clone DNA97003-2649 has been deposited with the ATCC on May 11, 1999 and is assigned ATCC deposit no. PTA-43.

An analysis of the Dayhoff database (version 35.45 SwissProt 35), using a WU-BLAST2 sequence alignment analysis of the full-length sequence (SEQ ID NO:70), evidenced significant homology between the PRO4980 amino acid sequence and the following Dayhoff sequences: SC59_YEAST, S76857, CELF31F4_12, AC002464_1, NU5M_CHOCR, S59109, SAY10108_2, AF055482_2, F69049, and G70433.


EXAMPLE 26


Gene Amplification


This example shows that the PRO197-, PRO207-, PRO226-, PRO232-, PRO243-, PRO256-, PRO269-, PRO274-, PRO304-, PRO339-, PRO1558-, PRO779-, PRO1185-, PRO1245-, PRO1759-, PRO5775-, PRO7133-, PRO7168-, PRO5725-, PRO202-, PRO206-, PRO264-, PRO313-, PRO342-, PRO542-, PRO773-, PRO861-, PRO1216-, PRO1686-, PRO1800-, PRO3562-, PRO9850-, PRO539-, PRO4316- or PRO4980-encoding genes are amplified in the genome of certain human lung, colon and/or breast cancers and/or cell lines. Amplification is associated with overexpression of the gene product, indicating that the polypeptides are useful targets for therapeutic intervention in certain cancers such as colon, lung, breast and other cancers. Therapeutic agents may take the form of antagonists of PRO197, PRO207, PRO226, PRO232, PRO243, PRO256, PRO269, PRO274, PRO304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptides, for examples, murine-human chimeric, humanized or human antibodies against a PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 polypeptide.

The starting material for the screen was genomic DNA isolated from a variety of cancers. The DNA is quantitated precisely, e.g., fluorometrically. As a negative control, DNA was isolated from the cells of ten normal healthy individuals which was pooled and used as assay controls for the gene copy in healthy individuals (not shown). The 5' nuclease assay (for example, TaqMan™) and real-time quantitative PCR (for example, ABI Prizm 7700 Sequence Detection System™ (Perkin Elmer, Applied Biosystems Division, Foster City, CA)), were used to find genes potentially amplified in certain cancers. The results were used to determine whether the DNA encoding PRO197, PRO207, PR0226, PRO232, PRO243, PRO256, PRO269, PR0274, PR0304, PR0339, PRO1558, PRO779, PRO1185, PRO1245, PRO1759, PRO5775, PR07133, PRO7168, PRO5725, PRO202, PR0206, PR0264, PR0313, PRO342, PRO542, PRO773, PRO861, PRO1216, PRO1686, PRO1800, PR03562, PR09850, PR0539, PRO4316 or PRO4980 is over-represented in any of the primary lung or colon cancers or cancer cell lines or breast cancer cell lines that were screened. The primary lung cancers were obtained from individuals with tumors of the type and stage as indicated in Table 6. An explanation of the abbreviations used for the designation of the primary tumors listed in Table 6 and the primary tumors and cell lines referred to throughout this example has been given hereinbefore.

The results of the TaqMan™ are reported in delta (Δ) Ct units. One unit corresponds to 1 PCR cycle or approximately a 2-fold amplification relative to normal, two units corresponds to 4-fold, 3 units to 8-fold amplification and so on. Quantitation was obtained using primers and a TaqMan™ fluorescent probe derived from the PRO197-, PRO207-, PRO226-, PRO232-, PRO243-, PRO256-, PRO269-, PRO274-, PRO304-, PRO339-, PRO1558-, PRO779, PRO1185-, PRO1245-, PRO1759-, PRO5775-, PRO7133-, PR07168-, PRO5725-, PRO202-, PRO206-, PRO264-, PRO313-, PRO342-, PRO542-, PRO773-, PRO861-, PRO1216-, PRO1686-, PRO1800-, PRO3562-, PRO9850-, PRO539-, PRO4316- or PRO4980-encoding gene. Regions of PRO197, PRO207, PRO226, PRO232, PRO243, PR0256, PRO269, PR0274, PRO304, PRO339, PRO1558, PR0779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 which are most likely to contain unique nucleic acid sequences and which are least likely to have spliced out introns are preferred for the primer and probe derivation, e.g., 3'-untranslated regions. The sequences for the primers and probes (forward, reverse and probe) used for the PRO197, PRO207, PR0226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 gene amplification analysis were as follows:

PRO197 (DNA22780-1078):

22780.tm.f:

5'-GCCATCTGGAAACTTGTGGAC-3'    (SEQ ID NO: 133)

22780.tmp:

5'-AGAAGACCACGACTGGAGAAGCCCCC-3'    (SEQ ID NO: 134)

22780.tmr.

5'-AGCCCCCCTGACTCAG-3'    (SEQ ID NO:135)

PRO207 (DNA30879-1152):

30879.tmf:

5'-GACCTGCCCCTCCCTCTAGA-3'    (SEQ ID NO:136)

30879.tm.p:

5'-CTGCCTGGGCCTGTTCACGTGTT-3'    (SEQ ID NO: 137)

30879.tm.r

5'-GGAATACTGTATTTATGTGGATGGA-3'    (SEQ ID NO:138)

PRO226 (DNA33460-1166):

33460.3utr-5:

5'-GCAATAAAGGGAGAAAGAAAGTCCT-3'    (SEQ ID NO: 139)

33460.3utr-probe.rc:

5'-TGACCCGCCCACCTCAGCCA-3'      (SEQ ID NO: 140)

33460.3utr.3b:

    5'-GCCTGAGGCTTCCTGCAGT-3'      (SEQ ID NO:141)

<u>PRO232 (DNA34435-1140):</u>

34435.3utr-5:

    5'-GCCAGGCCTCACATTCGT-3'      (SEQ ID NO: 142)

34435.3utr-probe:

    5'-CTCCCTGAATGGCAGCCTGAGCA-3'      (SEQ ID NO:143)

34435.3utr-3:

    5'-AGGTGTTTATTAAGGGCCTACGCT-3'      (SEQ ID NO: 144)

<u>PRO243 (DNA35917-1207):</u>

35917.tm.f:

    5'-CCAGTGCCTTTGCTCCTCTG -3'      (SEQ ID NO: 145)

35917.tm.p:

    5'-TGCCTCTACTCCCACCCCCACTACCT-3'      (SEQ ID NO: 146)

35917.tm.r:

    5'-TGTGGAGCTGTGGTTCCCA -3'      (SEQ ID NO: 147)

<u>PRO256 (DNA35880-1160):</u>

35880.3utr-5:

    5'-TGTCCTCCCGAGCTCCTCT-3'      (SEQ ID NO:148)

35880.3utr-probe:

    5'-CCATGCTGTGCGCCCAGGG-3'      (SEQ ID NO:149)

35880.3utr-3:

    5'-GCACAAACTACACAGGGAAGTCC-3'      (SEQ ID NO: 150)

<u>PRO269 (DNA38260-1180):</u>

38260.tm.f:

    5'-CAGAGCAGAGGGTGCCTTG-3'      (SEQ ID NO:151)

38260.tm.p:

    5'-TGGCGGAGTCCCCTCTTGGCT-3'      (SEQ ID NO:152)

38260.tm.r:

    5'-CCCTGTTTCCCTATGCATCACT-3'      (SEQ ID NO: 153)

PRO274 (DNA39987-1184):

   39987.tm.f:

    5'-GGACGGTCAGTCAGGATGACA-3'     (SEQ ID NO:154)

   39987.tm.p:

    5'-TTCGGCATCATCTCTTCCCTCTCCC-3'     (SEQ ID NO: 155)

   39987.tm.r:

    5'-ACAAAAAAAAGGGAACAAAATACGA-3'    (SEQ ID NO:156)

PRO304 (DNA39520-1217):

   39520.tm.f:

    5'-TCAACCCCTGACCCTTTCCTA-3'     (SEQ ID NO:157)

   39520.tm.p:

    5'-GGCAGGGGACAAGCCATCTCTCCT-3'     (SEQ ID NO: 18)

   39520.tm.r:

    5'-GGGACTGAACTGCCAGCTTC -3'     (SEQ ID NO: 159)

PRO339 (DNA43466-1225):

   43466.tm.f1:

    5'-GGGCCCTAACCTCATTACCTTT-3'     (SEQ ID NO: 160)

   43466.tm.p1:

    5'-TGTCTGCCTCAGCCCCAGGAAGG-3'     (SEQ ID NO: 161)

   43466.tm.r1:

    5'-TCTGTCCACCATCTTGCCTTG -3'     (SEQ ID NO: 162)

PRO1558 (DNA71282-1668):

   71282.tm.f1:

    5'-ACTGCTCCGCCTACTACGA -3'     (SEQ ID NO:163)

   71282.tm.p1:

    5'-AGGCATCCTCGCCGTCCTCA -3'     (SEQ ID NO:164)

   71282.tm.r1:

5'-AAGGCCAAGGTGAGTCCAT-3'     (SEQ ID NO: 165)

71282.tm.f2:

5'-CGAGTGTGTGCGAAACCTAA -3'     (SEQ ID NO:166)

71282.tm.p2:

5'-TCAGGGTCTACATCAGCCTCCTGC -3'     (SEQ ID NO: 167)

71282.tm.r2:

5'-AAGGCCAAGGTGAGTCCAT-3'     (SEQ ID NO:168)

PRO779 (DNA58801-1052):

58801.tm.f1:

5'-CCCTATCGCTCCAGCCAA -3'     (SEQ ID NO:169)

58801.tm.p1:

5'-CGAAGAAGCACGAACGAATGTCGAGA -3'     (SEQ ID NO: 170)

58801.tm.r1:

5'-CCGAGAAGTTGAGAAATGTCTTCA-3'     (SEQ ID NO: 171)

PRO1185 (DNA62881-1515):

62881.tm.f1:

5'-ACAGATCCAGGAGAGACTCCACA -3'     (SEQ ID NO: 172)

62881.tm.p1:

5'-AGCGGCGCTCCCAGCCTGAAT -3'     (SEQ ID NO:173)

62881.tm.r1:

5'-CATGATMGTCCTCAGTTCCATC -3'     (SEQ ID NO:174)

PRO1245 (DNA64884-1527):

64884.tm.f1:

5'-ATAGAGGGCTCCCAGAAGTG -3'     (SEQ ID NO: 175)

64884.tm.p1:

5'-CAGGGCCTTCAGGGCCTTCAC-3' (SEQ ID NO:176)

64884.tm.r1:

5'-GGTCAGCCAAACACTGTCA-3'     (SEQ ID NO: 177)

64884.tm.f2:

5'-GGGGCCCTGACAGTGTT -3'        (SEQ ID NO:178)

64884.tm.p2:

5'-CTGAGCCGAGACTGGAGCATCTACAC-3'        (SEQ ID NO:179)

64884.tm.r2:

5'-GTGGGCAGCGTCTTGTC-3'        (SEQ ID NO: 180)

PRO1759 (DNA76531-1701):

76531.tm.f1:

5'-CCTACTGAGGAGCCCTATGC -3'        (SEQ ID NO:181)

76531.tm.p1:

5'-CCTGAGCTGTAACCCCACTCCAGG -3'        (SEQ ID NO:182)

76531.tm.r1:

5'-AGAGTCTGTCCCAGCTATCTTGT -3'        (SEQ ID NO:183)

PRO5775 (DNA96869-2673):

96869.tm.f1:

5'-GGGGAACCATTCCAACATC -3'        (SEQ ID NO: 184)

96869.tm.p1:

5'-CCATTCAGCAGGGTGAACCACAG -3'        (SEQ ID NO: 185)

96869.tm.r1:

5'-TCTCCGTGACCATGAACTTTG-3'        (SEQ ID NO: 186)

PRO7133 (DNA128451-2739):

128451.tm.f1 :

5'-TTAGGGAATTTGGTGCTCAA -3'        (SEQ ID NO: 187)

128451.tm.p1:

5'-TTGCTCTCCTTGCTCTTCCCC -3'        (SEQ ID NO:188)

128451.tm.r1:

5'-TCCTGCAGTAGGTATTTTCAGTTT -3'        (SEQ ID NO:189)

PRO7168 (DNA102846-2742):

102846.tm.f1:

5'-GAGCCGGTGGTCTCAAAC-3'        (SEQ ID NO: 190)

102846.tm.p1:

    5'-CCGGGGGTCCTAGTCCCCTTC-3'       (SEQ ID NO:191)

102846.tm.r1:

    5'-TTTACTGCTGCGCTCCAA-3'       (SEQ ID NO:192)

PRO5725 (DNA92265-2669):

92265.tm.f1:

    5'-CAGCTGCAGTGTGGGAAT -3'       (SEQ ID NO:193)

92265.tm.p1:

    5'-CACTACAGCAAGAAGCTCGCCAGG -3'       (SEQ ID NO: 194)

92265.tm.r1:

    5'-CGCACAGAGTGTGCAAGTTAT -3'       (SEQ ID NO: 195)

PRO202 (DNA30869):

30869.tm.f:

    5'-CGGAAGGAGGCCAACCA-3'       (SEQ ID NO: 196)

30869.tm.p:

    5'-CGACAGTGCCATCCCCACCTTCA-3'       (SEQ ID NO:197)

30869.tm.r:

    5'-TTCTTTCTCCATCCCTCCGA-3'       (SEQ ID NO: 198)

PRO206 (DNA34405):

34405.tm.f:

    5'-GCATGGCCCCAACGGT -3'       (SEQ ID NO:199)

34405.tm.p:

    5'-CACGACTCAGTATCCATGCTCTTGACCTTGT-3'       (SEQ ID NO:200)

34405.tm.r:

    5'-TGGCTGTAAATACGCGTGTTCT-3'       (SEQ ID NO:201)

PRO264 (DNA36995):

36995.3tm.5:

    5'-CCTGTGAGATTGTGGATGAGAAGA-3'       (SEQ ID NO:202)

36995.3tm-probe:

5'-CCACACCAGCCAGACTCCAGTTGACC-3' (SEQ ID NO:203)

36995.3tm-3:

5'-GGGTGGTGCCCTCCTGA-3' (SEQ ID NO:204)

PRO313 (DNA43320):

43320.tm.f:

5'-CCATTGTTCAGACGTTGGTCA-3' (SEQ ID NO:205)

43320.tm.p:

5'-CTCTGTTAACTCTAAGATTCCTAAGGCATGCTGTGTC -3' (SEQ ID NO:206)

43320.tm.r:

5'-ATCGAGATAGCACTGAGTTCTGTCG -3' (SEQ ID NO:207)

PRO342 (DNA38649):

38649-tm.f:

5'-CTCGGCTCGCGAAACTACA-3' (SEQ ID NO:208)

38649.tm.p:

5'-TGCCCGCACAGACTTCTACTGCCTG-3' (SEQ ID NO:209)

38649.tm.r:

5'-GGAGCTACATATCATCCTTGGACA-3' (SEQ ID NO:210)

38649.tm.f2:

5'-GAGATAAACGACGGGAAGCTCTAC-3' (SEQ ID NO:211)

38649.tm.p2:

5'-ACGCCTACGTCTCCTACAGCGACTGC-3' (SEQ ID NO:212)

38649.tm.r2:

5'-GCTGCGGCTTTAGGATGAAGT-3' (SEQ ID NO:213)

PRO542 (DNA56505):

56505.tm.f1:

3'-CCTTGGCCTCCATTTCTGC -3' (SEQ ID NO:214)

56505.tm.p1:

5'-TGCTGCTCAGGCCCATGCTATGAGT-3' (SEQ ID NO:215)

56505.tm.r1:

5'-GGGTGTAGTCCAGAACAGCTAGAGA-3'　　　(SEQ ID NO:216)

PRO773 (DNA48303):

48303.tm.f1:

　　5'-CCCATTCCCAGCTTCTTG-3'　　　(SEQ ID NO:217)

48303.tm.p1 :

　　5'-CTCAGAGCCAAGGCTCCCCAGA -3'　　　(SEQ ID NO:218)

48303.tm.r1:

　　5'-TCAAGGACTGAACCATGCTAGA -3'　　　(SEQ ID NO:219)

PRO861 (DNA50798):

50798.tm.f1:

　　5'-ACCATGTACTACGTGCCAGCTCTA -3'　　　(SEQ ID NO:220)

50798.tm.p1:

　　5'-ATTCTGACTTCCTCTGATTTTGGCATGTGG -3'　　　(SEQ ID NO:221)

50798.tm.r1:

　　5'-GGCTTGAACTCTTATAGGAGTGT-3'　　　(SEQ ID NO:222)

PRO1216 (DNA66489):

66489.tm.f1:

　　5'-CTAACTGCCCAGCTCCAAGAA -3'　　　(SEQ ID NO:223)

65489.tm.p1 :

　　5'-TCACAGCACTCTCCAGGCACCTCAA -3'　　　(SEQ ID NO:224)

66489.tm.r1:

　　5'-TCTGGGCCACAGATCCAGTT-3'　　　(SEQ ID NO:225)

PRO1686 (DNA80896):

80896.tm.f1:

　　5'-GCTCAGCCCTAGACCCTGACTT -3'　　　(SEQ ID NO:226)

80896.tm.p1: .

　　5'-CAGGCTCAGCTGCTGTTGTAACCTCAGTAATG -3'　　　(SEQ ID NO:227)

80896.tm.r1:

　　5'-CGTGGACAGCAGGAGCCT-3'　　　(SEQ ID NO:228)

PRO1800 (DNA35672-2508):

 35672.tm.f1:

  5'-ACTCGGGATTCCTGCTGTT-3'  (SEQ ID NO:229)

 35672.tm.r1:

  5'-GGCCTGTCCTGTGTTCTCA-3'  (SEQ ID NO:230)

 35672.tm.p1:

  5'-AGGCCTTTACCCAAGGCCACAAC-3'  (SEQ ID NO:231)

PRO3562 (DNA96791):

 96791.tm.f1:

  5'-GACCCACGCGCTACGAA -3'  (SEQ ID NO:232)

 96791.tm.p1:

  5'-CGGTCTCGTTCATGGACGTCAACAG -3'  (SEQ ID NO:233)

 96791.tm.r1:

  5'-GGTCCACGGTTCTCCAGGT -3'  (SEQ ID NO:234)

PRO9850 (DNA58725):

 58725.tm.f1:

  5'-ATGATTGGTAGGAAATGAGGTAAAGTACT-3'  (SEQ ID NO:235)

 58725.tm.p1:

  5'-CCATCTTTCTGGGCACATTGAGGAACTG -3'  (SEQ ID NO:236)

 58725.tm.r1:

  5'-TGATCTAGAACTTAAACTTTGGAAAACAAC-3'  (SEQ ID NO:237)

PRO539 (DNA47465-1561):

 47465.tm.f1:

  5'-TCCCACCACTTACTTCCATGAA-3'  (SEQ ID NO:238)

 47465.tm.r1:

  5'-ATTGTCCTGAGATTCGAGCAAGA-3'  (SEQ ID NO:239)

 47465.tm.p1:

  5'-CTGGTACCCAATTGCCGCCTTGT-3'  (SEQ ID NO:240)

PRO4316 (DNA94713-2561):

94713.tm.f1:

    5'-GGTCACCTGTGGGACCTT-3'      (SEQ ID NO:241)

94713.tm.r1:

    5'-TGCACCTGACAGACAAAGC-3'      (SEQ ID NO:242)

94713.tm.p1:

    5'-TCCCTCACTCCCCTCCCTCCTAGT-3'      (SEQ ID NO:243)

<u>PRO4980 (DNA97003-2649):</u>

97003.tm.f1:

    5'-AAGCCTTTGGGTCACACTCT-3'      (SEQ ID NO:244)

97003.tm.r1:

    5'-TGGTCCACTGTCTCGTTCA-3'      (SEQ ID NO:245)

97003.tm.p1:

    5'-CCGAGCTTCCTGTCCCTTTTTCTG-3'      (SEQ ID NO:246)

The 5' nuclease assay reaction is a fluorescent PCR-based technique which makes use of the 5' exonuclease activity of Taq DNA polymerase enzyme to monitor amplification in real time. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

The 5' nuclease procedure is run on a real-time quantitative PCR device such as the ABI Prism 7700TM Sequence Detection. The system consists of a thermocycler, laser, charge-coupled device (CCD) camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5' Nuclease assay data are initially expressed as Ct, or the threshold cycle. This is defined as the cycle at which the reporter signal accumulates above the background level of fluorescence. The $\Delta$Ct values are used as quantitative measurement of the relative number of starting copies of a particular target sequence in a nucleic acid sample when comparing cancer DNA results to normal human DNA results.

Table 6 describes the stage, T stage and N stage of various primary tumors which were used to screen the PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 compounds of the invention.

Table 6
Primary Lung and Colon Tumor Profiles

| Primary Tumor | Stage | Other Stage | Dukes Stage | T Stage | N Stage |
|---|---|---|---|---|---|
| Human lung tumor AdenoCa (SRCC724) [LT1] | IIA | | | T1 | N1 |
| Human lung tumor SqCCa (SRCC725) [LT1a] | IIB | | | T3 | N0 |
| Human lung tumor AdenoCa (SRCC726) [LT2] | IB | | | T2 | N0 |

(continued)

Primary Lung and Colon Tumor Profiles

| Primary Tumor | Stage | Other Stage | Dukes Stage | T Stage | N Stage |
|---|---|---|---|---|---|
| Human lung tumor AdenoCa (SRCC727) [LT3] | IIIA | | | T1 | N2 |
| Human lung tumor AdenoCa (SRCC728) [LT4] | IB | | | T2 | N0 |
| Human lung tumor SqCCa (SRCC729) [LT6] | IB | | | T2 | N0 |
| Human lung tumor Aden/SqCCa (SRCC730) [LT7] | IA | | | T1 | N0 |
| Human lung tumor AdenoCa (SRCC731) [LT9] | IB | | | T2 | N0 |
| Human lung tumor SqCCa (SRCC732) [LT10] | IIB | | | T2 | N1 |
| Human lung tumor SqCCa (SRCC733) [LT11] | IIA | | | T1 | N1 |
| Human lung tumor AdenoCa (SRCC734) [LT12] | IV | | | T2 | N0 |
| Human lung tumor AdenoSqCCa (SRCC735)[LT13] | IB | | | T2 | N0 |
| Human lung tumor SqCCa (SRCC736) [LT15] | IB | | | T2 | N0 |
| Human lung tumor SqCCa (SRCC737) [LT16] | IB | | | T2 | N0 |
| Human lung tumor SqCCa (SRCC738) [LT17] | IIB | | | T2 | N1 |
| Human lung tumor SqCCa (SRCC739) [LT18] | IB | | | T2 | N0 |
| Human lung tumor SqCCa (SRCC740) [LT19] | IB | | | T2 | N0 |
| Human lung tumor LCCa (SRCC741) [LT21] | IIB | | | T3 | N1 |
| Human lung AdenoCa (SRCC811) [LT22] | IA | | | T1 | N0 |
| Human colon AdenoCa (SRCC742) [CT2] | | M1 | D | pT4 | N0 |
| Human colon AdenoCa (SRCC743) [CT3] | | | B | pT3 | N0 |
| Human colon AdenoCa (SRCC 744) [CT8] | | | B | T3 | N0 |
| Human colon AdenoCa (SRCC745) [CT10] | | | A | pT2 | N0 |
| Human colon AdenoCa (SRCC746) [CT12] | | MO, R1 | B | T3 | N0 |
| Human colon AdenoCa (SRCC747) [CT4] | | pMO, RO | B | pT3 | pN0 |
| Human colon AdenoCa (SRCC748) [CT15] | | M1, R2 | D | T4 | N2 |
| Human colon AdenoCa (SRCC749) [CT16] | | pMO | B | pT3 | pN0 |
| Human colon AdenoCa (SRCC750) [CT17] | | | C1 | pT3 | pN1 |
| Human colon AdenoCa (SRCC751) [CT1] | | MO, R1 | B | pT3 | N0 |
| Human colon AdenoCa (SRCC752) [CT4] | | | B | pT3 | M0 |
| Human colon AdenoCa (SRCC753) [CT5] | | G2 | C1 | pT3 | pN0 |
| Human colon AdenoCa (SRCC754) [CT6] | | pMO, RO | B | pT3 | pN0 |
| Human colon AdenoCa (SRCC755) [CT7] | | G1 | A | pT2 | pN0 |
| Human colon AdenoCa (SRCC756) [CT9] | | G3 | D | pT4 | pN2 |
| Human colon AdenoCa (SRCC757) [CT11] | | | B | T3 | N0 |
| Human colon AdenoCa (SRCC758) [CT18] | | MO, RO | B | pT3 | pN0 |

DNA Preparation:

DNA was prepared from cultured cell lines, primary tumors, and normal human blood. The isolation was performed using purification kit, buffer set and protease and all from Qiagen, according to the manufacturer's instructions and the description below.

*Cell culture lysis:*

Cells were washed and trypsinized at a concentration of $7.5 \times 10^8$ per tip and pelleted by centrifuging at 1000 rpm for 5 minutes at 4°C, followed by washing again with 1/2 volume of PBS and recentrifugation. The pellets were washed a third time, the suspended cells collected and washed 2x with PBS. The cells were then suspended into 10 ml PBS. Buffer C1 was equilibrated at 4°C. Qiagen protease #19155 was diluted into 6.25 ml cold $ddH_20$ to a final concentration of 20 mg/ml and equilibrated at 4°C. 10 ml of G2 Buffer was prepared by diluting Qiagen RNAse A stock (100 mg/ml) to a final concentration of 200 $\mu$g/ml.

Buffer CI (10 ml, 4°C) and ddH2O (40 ml, 4°C) were then added to the 10 ml of cell suspension, mixed by inverting and incubated on ice for 10 minutes. The cell nuclei were pelleted by centrifuging in a Beckman swinging bucket rotor at 2500 rpm at 4˚C for 15 minutes. The supernatant was discarded and the nuclei were suspended with a vortex into 2 ml Buffer Cl (at 4˚C) and 6 ml ddH$_2$O, followed by a second 4˚C centrifugation at 2500 rpm for 15 minutes. The nuclei were then resuspended into the residual buffer using 200 μl per tip. G2 buffer (10 ml) was added to the suspended nuclei while gentle vortexing was applied. Upon completion of buffer addition, vigorous vortexing was applied for 30 seconds. Qiagen protease (200 μl, prepared as indicated above) was added and incubated at 50˚C for 60 minutes. The incubation and centrifugation were repeated until the lysates were clear (*e.g.*, incubating additional 30-60 minutes, pelleting at 3000 x g for 10 min., 4˚C).

*Solid human tumor sample preparation and lysis:*

Tumor samples were weighed and placed into 50 ml conical tubes and held on ice. Processing was limited to no more than 250 mg tissue per preparation (1 tip/preparation). The protease solution was freshly prepared by diluting into 6.25 ml cold ddH$_2$O to a final concentration of 20 mg/ml and stored at 4˚C. G2 buffer (20 ml) was prepared by diluting DNAse A to a final concentration of 200 mg/ml (from 100 mg/ml stock). The tumor tissue was homogenated in 19 ml G2 buffer for 60 seconds using the large tip of the polytron in a laminar-flow TC hood in order to avoid inhalation of aerosols, and held at room temperature. Between samples, the polytron was cleaned by spinning at 2 x 30 seconds each in 2L ddH$_2$0, followed by G2 buffer (50 ml). If tissue was still present on the generator tip, the apparatus was disassembled and cleaned.

Qiagen protease (prepared as indicated above, 1.0 ml) was added, followed by vortexing and incubation at 50˚C for 3 hours. The incubation and centrifugation were repeated until the lysates were clear (e.g., incubating additional 30-60 minutes, pelleting at 3000 x g for 10 min., 4˚C).

*Human blood preparation and lysis:*

Blood was drawn from healthy volunteers using standard infectious agent protocols and citrated into 10 ml samples per tip. Qiagen protease was freshly prepared by dilution into 6.25 ml cold ddH$_2$O to a final concentration of 20 mg/ml and stored at 4˚C. G2 buffer was prepared by diluting RNAse A to a final concentration of 200 μg/ml from 100 mg/ml stock. The blood (10 ml) was placed into a 50 ml conical tube and 10 ml Cl buffer and 30 ml ddH$_2$O (both previously equilibrated to 4˚C) were added, and the components mixed by inverting and held on ice for 10 minutes. The nuclei were pelleted with a Beckman swinging bucket rotor at 2500 rpm, 4˚C for 15 minutes and the supernatant discarded. With a vortex, the nuclei were suspended into 2 ml Cl buffer (4˚C) and 6 ml ddH$_2$O (4˚C). Vortexing was repeated until the pellet was white. The nuclei were then suspended into the residual buffer using a 200 μl tip. G2 buffer (10 ml) was added to the suspended nuclei while gently vortexing, followed by vigorous vortexing for 30 seconds. Qiagen protease was added (200 μl) and incubated at 50˚C for 60 minutes. The incubation and centrifugation were repeated until the lysates were clear (e.g., incubating additional 30-60 minutes, pelleting at 3000 x g for 10 min., 4˚C).

*Purification of cleared lysates:*

(1) Isolation of genomic DNA:

Genomic DNA was equilibrated (1 sample per maxi tip preparation) with 10 ml QBT buffer. QP elution buffer was equilibrated at 50˚C. The samples were vortexed for 30 seconds, then loaded onto equilibrated tips and drained by gravity. The tips were washed with 2 x 15 ml QC buffer. The DNA was eluted into 30 ml silanized, autoclaved 30 ml Corex tubes with 15 ml QF buffer (50˚C). Isopropanol (10.5 ml) was added to each sample, the tubes covered with parafin and mixed by repeated inversion until the DNA precipitated. Samples were pelleted by centrifugation in the SS-34 rotor at 15,000 rpm for 10 minutes at 4˚C. The pellet location was marked, the supernatant discarded, and 10 ml 70% ethanol (4˚C) was added. Samples were pelleted again by centrifugation on the SS-34 rotor at 10,000 rpm for 10 minutes at 4˚C. The pellet location was marked and the supernatant discarded. The tubes were then placed on their side in a drying rack and dried 10 minutes at 37˚C, taking care not to overdry the samples.

After drying, the pellets were dissolved into 1.0 ml TE (pH 8.5) and placed at 50˚C for 1-2 hours. Samples were held overnight at 4˚C as dissolution continued. The DNA solution was then transferred to 1.5 ml tubes with a 26 gauge needle on a tuberculin syringe. The transfer was repeated 5x in order to shear the DNA. Samples were then placed at 50˚C for 1-2 hours.

(2) Quantitation of genomic DNA and preparation for gene amplification assay:

The DNA levels in each tube were quantified by standard A$_{260}$/ A$_{280}$ spectrophotometry on a 1:20 dilution (5 μl DNA + 95 μl ddH$_2$O) using the 0.1 ml quartz cuvettes in the Beckman DU640 spectrophotometer. A$_{260}$/A$_{280}$ ratios

were in the range of 1.8-1.9. Each DNA sample was then diluted further to approximately 200 ng/ml in TE (pH 8.5). If the original material was highly concentrated (about 700 ng/$\mu$l), the material was placed at 50˚C for several hours until resuspended.

Fluorometric DNA quantitation was then performed on the diluted material (20-600 ng/ml) using the manufacturer's guidelines as modified below. This was accomplished by allowing a Hoeffer DyNA Quant 200 fluorometer to warm-up for about 15 minutes. The Hoechst dye working solution (#H33258, 10$\mu$l, prepared within 12 hours of use) was diluted into 100 ml 1 x TNE buffer. A 2 ml cuvette was filled with the fluorometer solution, placed into the machine, and the machine was zeroed. pGEM 3Zf(+) (2 $\mu$l, lot #360851026) was added to 2 ml of fluorometer solution and calibrated at 200 units. An additional 2 $\mu$l of pGEM 3Zf(+) DNA was then tested and the reading confirmed at 400 +/- 10 units. Each sample was then read at least in triplicate. When 3 samples were found to be within 10% of each other, their average was taken and this value was used as the quantification value.

The fluorometricly determined concentration was then used to dilute each sample to 10 ng/$\mu$l in ddH$_2$O. This was done simultaneously on all template samples for a single TaqMan™ plate assay, and with enough material to run 500-1000 assays. The samples were tested in triplicate with Taqman™ primers and probe both B-actin and GAPDH on a single plate with normal human DNA and no-template controls. The diluted samples were used provided that the CT value of normal human DNA subtracted from test DNA was +/- 1 Ct. The diluted, lot-qualified genomic DNA was stored in 1.0 ml aliquots at -80˚C. Aliquots which were subsequently to be used in the gene amplification assay were stored at 4˚C. Each 1 ml aliquot is enough for 8-9 plates or 64 tests.

*Gene amplification assay:*

The PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562. PRO9850, PRO539, PRO4316 or PRO4980 compounds of the invention were screened in the following primary tumors and the resulting $\Delta$Ct values are reported in Table 7A-7C.

Table 7A

| ΔCt values in lung and colon primary tumor and cell line models | | | | | | | | | | | | | | |
|---|---|---|---|---|---|---|---|---|---|---|---|---|---|---|
| Primary Tumor | PRO197 | PRO207 | PRO226 | PRO232 | PRO243 | PRO256 | PRO269 | PRO274 | PRO304 | PRO339 | PRO1558 | PR0779 | PRO1195 | PRO1245 |
| HF-000 631 | - | - | - | - | - | - | - | - | - | - | - | - | - | - |
| HF-000 641 | - | - | - | - | - | - | - | - | - | - | - | - | - | - |
| HF-000 643 | - | - | - | - | - | - | - | - | - | - | - | - | - | - |
| HF-000 840 | - | - | - | - | - | - | - | - | - | - | 1.39 | 1.51 | - | - |
| HF-000 842 | - | - | - | - | - | - | - | - | - | - | 1.24 | - | - | - |
| HBL100 | - | - | - | - | - | - | - | - | - | - | - | - | - | - |
| MB435 | - | - | - | - | - | - | - | - | - | - | - | - | - | - |
| T47D | - | - | - | - | - | - | - | - | - | - | - | - | - | - |
| MB468 | - | - | - | - | - | - | - | - | - | - | - | - | - | - |
| MB175 | - | - | - | - | - | - | - | - | - | - | - | - | - | - |
| MB361 | - | - | - | - | - | - | - | - | - | - | - | - | - | - |
| BT20 | - | - | - | - | - | - | - | - | - | - | - | - | - | - |
| MCF7 | - | - | - | - | - | - | - | - | - | - | - | - | - | - |
| SKBR3 | - | - | - | - | - | - | - | - | - | - | - | - | - | - |
| SW480 | - | 1.85 | 2.14 | - | - | - | - | - | - | - | -. | 1.87<br>1.56 | - | - |
| SW620 | - | 1.96 | 2.67 | - | - | 1.23 | - | - | - | - | | 1.13<br>1.38<br>1.21 | - | - |
| Colo320 | - | 1.09 | - | - | - | - | - | - | - | - | - | 1.18 | - | - |
| HT29 | - | - | 2.15 | - | - | 1.58 | - | - | - | - | - | 1.03<br>1.90 | - | - |

EP 1 626 084 B1

107

(continued)

ΔCt values in lung and colon primary tumor and cell line models

| Primary Tumor | PRO197 | PRO207 | PRO226 | PRO232 | PRO243 | PRO256 | PRO269 | PRO274 | PRO304 | PRO339 | PRO1558 | PRO779 | PRO1195 | PRO1245 |
|---|---|---|---|---|---|---|---|---|---|---|---|---|---|---|
| HM7 | - | - | 1.23 | - | - | - | - | - | - | - | - | 1.33 | - | - |
| WiDr | - | - | 2.21 | - | - | 1.35 | - | - | - | - | - | 1.35 | - | - |
| HCT116 | - | 1.83 | 2.13 | - | - | 1.35 | - | - | - | - | - | 2.24 1.70 | - | - |
| SKCOI | - | 1.13 | 1.94 | - | - | - | - | - | - | - | - | 1.11 | - | - |
| SW403 | - | - | 1.81 | - | - | - | - | - | - | - | - | - | - | - |
| LS174T | - | - | - | - | - | - | - | - | - | - | - | 1.18 | - | - |
| Colo205 | - | - | - | - | - | - | - | - | - | - | - | - | - | - |
| HCT15 | - | - | - | - | - | - | - | - | - | - | - | - | - | - |
| HCC 2998 | - | - | - | - | - | - | - | - | - | - | - | - | - | - |
| KM12 | - | - | - | - | - | - | - | - | - | - | - | - | - | - |
| A549 | - | - | - | - | - | - | - | - | - | - | - | - | - | - |
| Caln-1 | - | - | - | - | - | - | - | - | - | - | - | - | - | - |
| Caln-6 | - | - | - | - | - | - | - | - | - | - | - | - | - | - |
| H157 | - | - | - | - | - | - | - | - | - | - | - | - | - | - |
| H441 | - | - | - | - | - | - | - | - | - | - | - | - | - | - |
| H460 | - | - | - | - | - | - | - | - | - | - | - | - | - | - |
| SKMES1 | - | - | - | - | - | - | - | - | - | - | - | - | - | - |
| SW900 | - | - | - | - | - | - | - | - | - | - | - | - | - | - |
| H522 | - | - | - | - | - | - | - | - | - | - | - | - | - | 1.10 |
| H810 | - | - | - | - | - | - | - | - | - | - | - | - | - | - |
| SRCC 1094 | - | - | - | - | - | - | - | - | - | - | - | - | - | - |
| SRCC 1095 | - | - | - | - | - | - | - | - | - | - | - | - | - | - |
| SRCC 1096 | - | - | - | - | - | - | - | - | - | - | - | - | - | - |
| SRCC 1097 | - | - | - | - | - | - | - | - | - | - | - | - | - | - |
| SRCC 1098 | - | - | - | - | - | - | - | - | - | - | - | - | - | - |

(continued)

ΔCt values in lung and colon primary tumor and cell line models

| Primary Tumor | PRO197 | PRO207 | PRO226 | PRO232 | PRO243 | PRO256 | PRO269 | PRO274 | PRO304 | PRO339 | PRO1558 | PRO0779 | PRO1195 | PRO1245 |
|---|---|---|---|---|---|---|---|---|---|---|---|---|---|---|
| SRCC 1099 | - | - | - | - | - | - | - | - | - | - | - | - | - | - |
| SRCC 1100 | - | - | - | - | - | - | - | - | - | - | - | - | - | - |
| SRCC 1101 | - | - | - | - | - | - | - | - | - | - | - | - | - | - |
| HF-000 545 | - | - | - | - | - | - | - | - | - | - | - | - | - | - |
| HF-000 499 | - | - | - | - | - | - | - | - | - | - | - | - | - | - |
| HF-000 539 | - | - | - | - | - | - | - | - | - | - | - | - | - | - |
| HF-000 575 | - | - | - | - | - | - | - | - | - | - | - | - | - | - |
| HF-000 698 | - | - | - | - | - | - | - | - | - | - | - | - | - | - |
| HF-000 756 | - | - | - | - | - | - | - | - | - | - | - | - | - | - |
| HF-000 762 | - | - | - | - | - | - | - | - | - | - | - | - | - | - |
| HF-000 789 | - | - | - | - | - | - | - | - | - | - | - | - | - | - |
| HF-000 795 | - | - | - | - | - | - | - | - | - | - | 1.01 | - | - | - |
| HF-000 811 | - | - | - | - | - | - | - | - | - | - | - | - | - | - |
| HF-000 755 | - | - | - | ⋮ | - | - | - | - | - | - | - | - | - | - |
| CT2 | - | - | 1.15 | - | - | - | - | - | - | - | - | 1.83<br>2.41<br>2.28<br>2.91 | 1.73 | - |
| CT3 | - | 1.29 | 1.26 | - | - | - | - | - | - | - | - | 1.06<br>1.14<br>1.72 | - | - |
| CT8 | - | 133 | - | - | - | - | - | - | - | - | - | 1.01<br>1.03<br>1.20 | - | - |
| CT10 | --- | --- | --- | --- | --- | --- | --- | --- | --- | --- | --- | 1.03 | --- | --- |
| CT12 | --- | --- | 1.20 | --- | --- | --- | --- | --- | --- | --- | --- | 1.05<br>1.15 | --- | --- |
| CT14 | --- | --- | 1.38 | --- | 1.14 | --- | --- | --- | --- | --- | --- | 1.01 | --- | --- |

(continued)

| ΔCt values in lung and colon primary tumor and cell line models | | | | | | | | | | | | | |
|---|---|---|---|---|---|---|---|---|---|---|---|---|---|
| Primary Tumor | PRO197 | PRO207 | PRO226 | PRO232 | PRO243 | PRO256 | PRO269 | PRO274 | PRO304 | PRO339 | PRO1558 | PR0779 | PRO1195 | PRO1245 |
| | | | | | | | | | | | | 1.14<br>1.20 | | |
| CT15 | --- | 1.26 | 1.07 | --- | --- | --- | --- | --- | --- | --- | --- | 1.14<br>1.12 | --- | 1.00 |
| CT16 | --- | --- | --- | --- | --- | --- | --- | --- | --- | --- | --- | 1.14<br>1.22 | --- | --- |
| CT17 | --- | --- | --- | --- | --- | --- | --- | --- | --- | --- | --- | 1.12<br>1.17 | --- | --- |
| CT1 | --- | 1.10 | --- | 2.41 | --- | --- | --- | --- | --- | --- | --- | 1.02<br>1.69<br>1.54<br>1.28<br>1.15 | --- | --- |
| CT4 | --- | 1.13 | 1.11 | 105 | --- | --- | --- | --- | --- | --- | --- | 1.19<br>1.22<br>1.12 | --- | --- |
| CT5 | --- | 1.14 | 1.12 | 1.59 | 1.17 | --- | --- | --- | --- | --- | --- | 1.62<br>2.02<br>2.24<br>2.32<br>2.36<br>1.75 | --- | --- |
| CT6 | --- | --- | --- | --- | --- | --- | --- | --- | --- | --- | --- | 1.17 | --- | --- |
| CT7 | --- | --- | --- | 1.00 | --- | --- | --- | --- | --- | --- | --- | 1.00<br>1.04 | --- | --- |
| CT9 | --- | --- | --- | 1.13 | --- | --- | --- | --- | --- | --- | --- | 1.05 | --- | --- |
| CT11 | --- | 132 | 1.35 | 1.92 | --- | --- | --- | --- | --- | --- | --- | 1.27<br>1.73<br>1.82<br>1.89<br>1.93 | --- | --- |

110

(continued)

| Primary Tumor | PRO197 | PRO207 | PRO226 | PRO232 | PRO243 | PRO256 | PRO269 | PRO274 | PRO304 | PRO339 | PRO1558 | PR0779 | PRO1195 | PRO1245 |
|---|---|---|---|---|---|---|---|---|---|---|---|---|---|---|
| | | | | | | | | | | | | 1.43 | | |
| CT18 | --- | --- | --- | 1.29 | --- | --- | --- | --- | --- | --- | --- | --- | --- | --- |
| CT25 | --- | --- | --- | --- | --- | --- | --- | --- | --- | --- | --- | --- | --- | --- |
| CT28 | --- | --- | --- | --- | --- | --- | --- | --- | --- | --- | --- | --- | --- | --- |
| CT35 | --- | --- | --- | --- | --- | --- | --- | --- | --- | --- | --- | --- | --- | --- |
| HF-000611 | --- | --- | --- | --- | --- | --- | --- | --- | --- | --- | --- | --- | --- | --- |
| HF-000613 | --- | --- | --- | --- | --- | --- | --- | --- | --- | --- | --- | --- | --- | --- |
| HF-001291 | --- | --- | --- | --- | --- | --- | --- | --- | --- | --- | --- | --- | --- | --- |
| HF-00t293 | --- | --- | --- | --- | --- | --- | --- | --- | --- | --- | --- | --- | --- | --- |
| HF-001294 | --- | --- | --- | --- | --- | --- | --- | --- | --- | --- | 1.50 | --- | --- | --- |
| HF-001295 | --- | --- | --- | --- | --- | --- | --- | --- | --- | --- | --- | --- | --- | --- |
| HF-001296 | --- | --- | --- | --- | --- | --- | --- | --- | --- | --- | 2.88 | --- | --- | --- |
| HF-001297 | --- | --- | --- | --- | --- | --- | --- | --- | --- | --- | --- | --- | --- | --- |
| HF-001299 | --- | --- | --- | --- | --- | --- | --- | --- | --- | --- | 1.37 | --- | --- | --- |
| HF-001300 | --- | --- | --- | --- | --- | --- | --- | --- | --- | --- | --- | --- | --- | --- |
| LT7 | --- | --- | 1.12 | --- | --- | --- | 1.04 | --- | --- | 1.08 | --- | --- | --- | --- |
| LT27 | --- | --- | --- | --- | --- | --- | --- | --- | --- | --- | --- | --- | --- | --- |
| LT13 | 1.40 | 1.26 | 1.10 | --- | 1.05 | --- | 1.27 | --- | 1.29 | 1.04 | --- | 1.69 | --- | 3.84 |

ΔCt values in lung and colon primary tumor and cell line models

| ΔCt values in lung and colon primary tumor and cell line models | | | | | | | | | | | | | | |
|---|---|---|---|---|---|---|---|---|---|---|---|---|---|---|
| Primary Tumor | PRO197 | PRO207 | PRO226 | PRO232 | PRO243 | PRO256 | PRO269 | PRO274 | PRO304 | PRO339 | PRO1558 | PR0779 | PRO1195 | PRO1245 |
| | | 1.29 | 1.10 | | | | | | | | | 2.79 2.42 1.44 | | |
| LT1 | --- | --- | --- | --- | --- | --- | --- | --- | --- | --- | --- | --- | --- | --- |
| LT2 | --- | --- | --- | --- | --- | --- | --- | --- | --- | --- | --- | --- | --- | --- |
| LT3 | 1.50 | 1.14 | 1.59 | --- | 1.08 | --- | --- | --- | --- | 1.17 | --- | 1.65 1.19 1.17 | 1.01 | --- |
| LT4 | --- | --- | 1.11 | --- | --- | --- | --- | 1.24 | --- | --- | --- | --- | --- | --- |
| LT9 | 1.25 | --- | 1.36 | --- | --- | --- | 1.80 | --- | --- | 1.03 | --- | 1.27 | --- | --- |
| LT12 | --- | --- | --- | 2.40 | 1.20 | --- | 1.14 | --- | 1.15 | 1.26 | --- | 1.03 2.09 1.99 1.20 | --- | --- |
| LT22 | --- | --- | --- | --- | --- | --- | --- | --- | --- | --- | --- | --- | --- | --- |
| LT30 | --- | --- | --- | --- | --- | --- | --- | --- | --- | --- | --- | --- | 1.58 | --- |
| LT33 | --- | --- | --- | --- | --- | --- | --- | --- | --- | --- | --- | --- | --- | --- |
| L18 | --- | --- | --- | --- | --- | --- | --- | --- | --- | --- | --- | --- | --- | --- |
| LT21 | 1.10 | 1.12 | 1.17 | --- | --- | --- | --- | --- | --- | --- | --- | 1.00 | --- | --- |
| LT1a | --- | --- | 1.39 | --- | --- | --- | --- | --- | --- | --- | --- | 1.46 1.04 | --- | --- |
| LT6 | 1.39 | --- | --- | --- | --- | --- | --- | --- | --- | --- | --- | 1.75 1.25 | --- --- | --- --- |
| LT10 | 1.03 | --- | --- | --- | --- | --- | --- | --- | --- | --- | --- | 1.50 | --- | --- |
| LT11 | 1.65 | 1.33 | 1.28 | --- | 1.34 | --- | 1.14 | --- | 1.51 | 1.39 | - | 1.77 | --- | --- |

EP 1 626 084 B1

(continued)

ΔCt values in lung and colon primary tumor and cell line models

| Primary Tumor | PRO197 | PRO207 | PRO226 | PRO232 | PRO243 | PRO256 | PRO269 | PRO274 | PRO304 | PRO339 | PRO1558 | PRO0779 | PRO1195 | PRO1245 |
|---|---|---|---|---|---|---|---|---|---|---|---|---|---|---|
| LT15 | 1.22 | 1.59<br>1.22<br>1.18 | 1.01<br>1.04 | 1.86 | 2.34<br>1.72 | --- | 1.36 | --- | 1.34 | --- | --- | 1.39<br>1.48<br>2.30<br>3.73<br>3.31<br>1.89 | --- | 1.01 |
| LT16 | --- | --- | --- | --- | 1.24<br>1.64 | --- | --- | 1.00 | 1.00 | --- | --- | 1.89<br>1.50<br>1.38 | --- | 1.98 |
| LT17 | 1.68 | 1.32<br>1.57 | 1.26<br>1.57 | 1.35 | 1.27 | --- | 1.42 | --- | 1.68 | 1.63 | --- | 1.08<br>1.95<br>1.51<br>1.50 | --- | --- |
| LT18 | --- | --- | --- | 1.04 | --- | --- | --- | 1.61 | --- | --- | --- | 1.00 | --- | --- |
| LT19 | --- | 1.16<br>1.58 | 1.08<br>1.25 | 1.21 | 1.39 | --- | 1.60 | --- | 1.15 | --- | --- | 3.49<br>3.21<br>3.73 | --- | --- |
| LT26 | --- | --- | --- | --- | --- | --- | --- | --- | --- | --- | --- | --- | 1.66 | --- |
| LT28 | --- | --- | --- | --- | --- | --- | --- | --- | --- | --- | --- | --- | --- | --- |
| LT29 | --- | --- | --- | --- | --- | --- | --- | --- | --- | --- | --- | --- | --- | --- |
| LT31 | --- | --- | --- | --- | --- | --- | --- | --- | --- | --- | --- | --- | --- | --- |
| HF-000 854 | --- | --- | --- | --- | --- | --- | --- | --- | --- | --- | --- | --- | --- | --- |
| HF-000 855 | --- | --- | --- | --- | --- | --- | --- | --- | --- | --- | --- | --- | --- | --- |

(continued)

| Primary Tumor | PRO197 | PRO207 | PRO226 | PRO232 | PRO243 | PRO256 | PRO269 | PRO274 | PRO304 | PRO339 | PRO1558 | PR0779 | PRO1195 | PRO1245 |
|---|---|---|---|---|---|---|---|---|---|---|---|---|---|---|
| ΔCt values in lung and colon primary tumor and cell line models | | | | | | | | | | | | | | |
| HF-000 856 | --- | --- | --- | --- | --- | --- | --- | --- | --- | --- | --- | --- | --- | --- |
| HF-000 831 | --- | --- | --- | --- | --- | --- | --- | --- | --- | --- | --- | --- | --- | --- |
| HF-000 832 | --- | --- | --- | --- | --- | --- | --- | --- | --- | --- | --- | --- | --- | --- |
| HF-000 550 | --- | --- | --- | --- | --- | --- | --- | --- | --- | --- | --- | --- | --- | --- |
| HF-000 551 | --- | --- | --- | --- | --- | --- | --- | --- | --- | --- | --- | --- | --- | --- |
| HF-000 733 | --- | --- | --- | --- | --- | --- | --- | --- | --- | --- | --- | --- | --- | --- |
| HF-000 716 | --- | --- | --- | --- | --- | --- | --- | --- | --- | --- | --- | --- | --- | --- |

Table 7B

| ΔCt values in lung and colon primary tumor and cell line models | | | | | | | | | | | | | | |
|---|---|---|---|---|---|---|---|---|---|---|---|---|---|---|
| Primary Tumor | PRO1759 | PRO5775 | PRO7133 | PRO7168 | PRO5725 | PRO202 | PRO206 | PR0264 | PR0313 | PRO342 | PRO542 | PRO773 | PRO861 | PRO1216 |
| HF-000 631 | --- | 1.97 1.70 | --- | 1.43 | --- | --- | --- | --- | --- | --- | --- | --- | --- | --- |
| HP-000 641 | --- | 1.90 1.87 | --- | --- | 1.17 1.03 | --- | --- | --- | --- | --- | --- | --- | --- | --- |
| HF-000 643 | --- | 1.13 1.21 | --- | --- | --- | --- | --- | --- | --- | --- | --- | --- | --- | --- |
| HF-000 840 | 1.11 | 3.64 3.55 | 2.11 | 2.65 | 1.82 2.20 1.99 | --- | --- | --- | --- | 1.35 | --- | --- | --- | --- |
| HF-000 842 | --- | 2.56 2.42 2.12 2.88 | --- | 1.73 | --- | --- | --- | --- | --- | 1.13 | --- | --- | --- | --- |
| HBL100 | --- | --- | --- | --- | --- | --- | --- | --- | --- | --- | --- | --- | --- | --- |
| MB435s | --- | --- | --- | --- | --- | --- | --- | --- | --- | --- | --- | --- | --- | --- |
| T47D | --- | --- | --- | --- | --- | --- | --- | --- | --- | --- | --- | --- | --- | --- |
| MB468 | --- | --- | --- | --- | --- | --- | --- | --- | --- | --- | --- | --- | --- | --- |
| MB175 | --- | --- | --- | --- | --- | --- | --- | --- | --- | --- | --- | --- | --- | --- |
| MB361 | --- | --- | --- | --- | --- | --- | --- | --- | --- | --- | --- | --- | --- | --- |
| BT20 | --- | --- | --- | --- | --- | --- | --- | --- | --- | --- | --- | --- | --- | --- |
| MCF7 | --- | --- | --- | --- | --- | --- | --- | --- | --- | --- | 1.14 | --- | --- | --- |
| SKBR3 | --- | --- | --- | --- | --- | --- | --- | --- | --- | --- | --- | --- | --- | --- |
| SW480 | --- | --- | --- | --- | --- | --- | --- | --- | --- | --- | 1.35 | --- | --- | --- |
| SW620 | --- | --- | --- | --- | --- | --- | --- | --- | 1.03 | 2.09 | 1.17 | --- | --- | --- |

115

(continued)

ΔCt values in lung and colon primary tumor and cell line models

| Cell line | | | | |
|---|---|---|---|---|
| Colo320 | --- | --- | 1.31 | 1.13 |
| HT29 | --- | --- | 1.97 | 3.08, 2.59, 3.24, 2.68, 2.77 |
| HM7 | --- | --- | --- | --- |
| WiDr | 2.42 | --- | --- | 3.35, 3.15, 2.59, 2.94, 3.03, 2.99 |
| HCT116 | 1.71 | --- | --- | 2.09, 2.01, 2.12, 1.87, 1.98, 2.07 |
| SKCO1 | --- | --- | --- | 1.71, 2.00, 1.97, 1.64, 1.82 |
| SW403 | 1.14 | --- | --- | 1.73, 1.15, 1.64, 1.17, 1.28 |
| LS174T | 1.16 | --- | --- | 1.41 |

(continued)

| ΔCt values in lung and colon primary tumor and cell line models | | | | | | | | | | | | | | |
|---|---|---|---|---|---|---|---|---|---|---|---|---|---|---|
| Colo205 | --- | --- | --- | --- | --- | --- | --- | --- | --- | --- | --- | 1.41 | --- | --- |
| HCT15 | --- | --- | --- | --- | --- | --- | --- | --- | --- | --- | --- | --- | --- | --- |
| HCC 2998 | --- | --- | --- | --- | --- | --- | --- | --- | --- | --- | --- | --- | --- | --- |
| KM12 | --- | --- | --- | --- | --- | --- | --- | --- | --- | --- | --- | --- | --- | --- |
| A549 | --- | --- | --- | --- | --- | --- | --- | --- | --- | --- | --- | --- | --- | --- |
| Calu-1 | --- | --- | --- | --- | --- | --- | --- | --- | --- | 1.21 | --- | --- | --- | --- |
| Calu-6 | --- | --- | --- | --- | --- | --- | --- | --- | --- | --- | --- | --- | --- | --- |
| H157 | --- | --- | --- | --- | --- | --- | --- | --- | --- | --- | --- | --- | --- | --- |
| H441 | --- | --- | --- | --- | --- | --- | --- | --- | --- | 1.65 | 1.15 | 1.51 | 1.71 | --- |
| H460 | --- | --- | --- | --- | --- | --- | --- | --- | --- | --- | --- | --- | --- | --- |
| SKMES1 | --- | --- | --- | --- | --- | --- | --- | --- | --- | --- | --- | --- | --- | --- |
| SW900 | --- | --- | --- | --- | --- | --- | --- | --- | --- | --- | --- | --- | --- | --- |
| H522 | --- | --- | --- | --- | --- | --- | --- | --- | --- | --- | --- | --- | 1.02 | --- |
| H810 | --- | --- | --- | --- | --- | --- | --- | --- | --- | --- | --- | --- | --- | --- |
| SRCC 1094 | --- | --- | --- | --- | --- | --- | --- | --- | --- | --- | --- | --- | --- | --- |
| SRCC 1095 | --- | --- | --- | --- | --- | --- | --- | --- | --- | --- | --- | --- | --- | --- |
| SRCC 1096 | --- | --- | --- | --- | --- | --- | --- | --- | --- | --- | --- | --- | --- | --- |
| SRCC 1097 | --- | --- | --- | --- | --- | --- | --- | --- | --- | --- | --- | --- | --- | --- |
| SRCC 1098 | --- | --- | --- | --- | --- | --- | --- | --- | --- | --- | --- | --- | --- | --- |
| SRCC 1099 | --- | --- | --- | --- | --- | --- | --- | --- | --- | --- | --- | --- | --- | --- |

117

(continued)

| ΔCt values in lung and colon primary tumor and cell line models | | | | | | | | | | | | | |
|---|---|---|---|---|---|---|---|---|---|---|---|---|---|
| SRCC 1100 | --- | --- | --- | --- | --- | --- | --- | --- | --- | --- | --- | --- | --- | --- |
| SRCC 1101 | --- | --- | --- | --- | --- | --- | --- | --- | --- | --- | --- | --- | --- | --- |
| HF-000 545 | --- | --- | --- | --- | --- | --- | --- | --- | --- | --- | --- | --- | --- | --- |
| HF-000 499 | --- | --- | --- | --- | --- | --- | --- | --- | --- | --- | --- | --- | --- | --- |
| HF-000 539 | --- | --- | --- | --- | --- | --- | --- | --- | --- | --- | --- | --- | --- | --- |
| HF-000 575 | --- | --- | --- | --- | --- | --- | --- | --- | --- | --- | --- | --- | --- | --- |
| HF-000 698 | --- | --- | --- | --- | --- | --- | --- | --- | --- | --- | --- | --- | --- | --- |
| HF-000 756 | --- | --- | --- | --- | --- | --- | --- | --- | --- | --- | --- | --- | --- | --- |
| HF-000 762 | --- | 2.01 1.04 | --- | --- | 1.26 1.04 | --- | --- | --- | --- | --- | --- | --- | --- | --- |
| HF-000 789 | --- | 1.30 1.12 | --- | --- | --- | --- | --- | --- | --- | --- | --- | --- | --- | --- |
| HF-000 795 | 1.32 | --- | 1.08 | --- | 1.02 1.28 1.10 | --- | --- | --- | --- | --- | --- | --- | --- | --- |
| HF-000 811 | --- | 1.82 1.80 | 1.09 | --- | --- | --- | --- | --- | --- | --- | --- | --- | --- | --- |
| HF-000 755 | --- | --- | --- | --- | --- | --- | --- | --- | --- | --- | --- | --- | --- | --- |
| CT2 | --- | --- | --- | --- | --- | --- | 1.21 | --- | 1.75 | 3.04 | --- | --- | 2.40 2.35 | --- |
| CT3 | --- | --- | --- | --- | --- | --- | --- | --- | --- | 1.21 | --- | --- | 1.52 | --- |

EP 1 626 084 B1

(continued)

| ΔCt values in lung and colon primary tumor and cell line models | | | | | | | | | | | | | |
|---|---|---|---|---|---|---|---|---|---|---|---|---|---|
| | | | | | | | | | | | | 1.39 | |
| CT8 | --- | --- | --- | --- | --- | --- | --- | --- | --- | 1.21 | --- | --- | 1.55 | --- |
| CT10 | --- | --- | --- | --- | --- | --- | 1.06 | --- | --- | 1.81 | 1.13 | --- | 1.97 1.33 | --- |
| CT12 | --- | --- | --- | --- | --- | --- | 1.06 | --- | --- | 1.41 | 1.08 1.17 | --- | 1.36 | 1.18 |
| CT14 | --- | --- | --- | --- | --- | --- | 1.29 | --- | --- | 1.61 | 1.41 | --- | 1.75 1.17 | --- |
| CT15 | --- | --- | --- | --- | --- | --- | 1.32 | --- | --- | 1.41 | --- | 1.04 | 1.75 | --- |
| CT16 | --- | --- | --- | --- | --- | --- | 1.59 | --- | --- | 1.39 | --- | 1.37 | 1.11 | |
| CT17 | --- | --- | --- | --- | --- | --- | --- | --- | --- | 1.19 | --- | 1.34 | 1.11 | --- |
| CT1 | --- | --- | --- | --- | --- | --- | --- | --- | 1.28 | 1.61 | --- | --- | 1.09 1.22 | --- |
| CT4 | --- | --- | --- | --- | --- | --- | --- | --- | 1.57 | 1.58 | --- | --- | 1.16 | --- |
| CT5 | --- | --- | --- | --- | --- | --- | 1.23 | --- | 2.01 | 2.29 | 1.06 | --- | 1.95 | 1.21 |
| CT6 | --- | --- | --- | --- | --- | --- | --- | --- | --- | 1.20 | --- | --- | --- | --- |
| CT7 | --- | --- | --- | --- | --- | --- | --- | --- | --- | --- | --- | --- | 1.14 | --- |
| CT9 | --- | --- | --- | --- | --- | --- | --- | --- | 1.56 | 1.00 | 1.03 | --- | 1.00 | --- |
| CT11 | --- | --- | --- | --- | --- | --- | --- | --- | 2.12 | 2.27 | --- | --- | 1.88 | --- |
| CT18 | --- | --- | --- | --- | --- | --- | 1.33 | --- | --- | --- | --- | --- | --- | --- |
| CT25 | --- | --- | --- | --- | --- | --- | --- | --- | --- | --- | --- | --- | --- | --- |
| CT28 | --- | --- | --- | --- | --- | --- | --- | --- | --- | --- | --- | --- | --- | --- |
| CT35 | --- | --- | --- | --- | --- | --- | --- | --- | --- | --- | --- | --- | --- | --- |
| HF-000 611 | --- | --- | --- | --- | --- | --- | --- | --- | --- | --- | --- | --- | --- | --- |
| HF-000 613 | --- | --- | --- | --- | --- | --- | --- | --- | --- | --- | --- | --- | --- | --- |

119

| ΔCt values in lung and colon primary tumor and cell line models | | | | | | | | | | | | | | |
|---|---|---|---|---|---|---|---|---|---|---|---|---|---|---|
| HF-00 1291 | --- | --- | --- | --- | --- | --- | --- | --- | --- | --- | --- | --- | --- | --- |
| HF-00 1293 | --- | 2.12 2.09 | --- | --- | --- | --- | --- | --- | --- | --- | --- | --- | --- | --- |
| KF-00 1294 | --- | 215 1.99 | --- | --- | --- | --- | --- | --- | --- | 1.57 | --- | --- | --- | --- |
| HF-00 1295 | --- | 1.99 2-15 | --- | --- | 1.10 | --- | --- | --- | --- | --- | --- | --- | --- | --- |
| HF-00 1296 | 1.51 | 4.62 4.78 | 1.71 | --- | 1.22 | --- | --- | --- | --- | 3.15 | --- | --- | --- | --- |
| HF-00 1297 | --- | --- | --- | --- | --- | --- | --- | --- | --- | --- | --- | --- | --- | --- |
| HF-00 1299 | --- | 1.92 1.95 | --- | --- | --- | --- | --- | --- | --- | --- | --- | --- | --- | --- |
| HF-00 1300 | --- | --- | --- | --- | --- | --- | --- | --- | --- | --- | --- | --- | --- | --- |
| LT7 | --- | --- | --- | --- | --- | 1.50 1.79 | --- | --- | --- | 1.25 | 1.11 | --- | --- | 1.15 |
| LT27 | --- | --- | --- | --- | --- | --- | --- | --- | --- | --- | --- | --- | --- | --- |
| LT13 | --- | --- | --- | --- | --- | 1.64 | --- | --- | --- | 1.34 2.85 2.12 | 1.38 | 2.98 | 1.33 | --- |
| LT1 | --- | --- | --- | --- | --- | 1.29 1.15 | --- | --- | --- | --- | --- | --- | --- | --- |
| LT2 | --- | --- | --- | --- | --- | --- | --- | --- | --- | --- | --- | --- | --- | --- |
| LT3 | --- | --- | --- | --- | --- | 1.67 1.66 | --- | 1.82 | --- | 1.89 1.71 | --- | --- | --- | --- |
| LT4 | --- | --- | --- | --- | --- | 1.21 | --- | 1.43 | --- | --- | --- | --- | --- | --- |

EP 1 626 084 B1

120

| ΔCt values in lung and colon primary tumor and cell line models | | | | | | | | | | | | | | |
|---|---|---|---|---|---|---|---|---|---|---|---|---|---|---|
| LT9 | --- | --- | --- | --- | --- | 1.30 | --- | 1.13 | 1.19 | 1.51 | --- | --- | --- | --- |
| LT12 | --- | --- | --- | --- | --- | 1.73 | --- | --- | 1.03 | 2.02<br>1.74 | 1.31<br>1.41 | --- | 1.18<br>1.38 | 1.02 |
| LT22 | --- | --- | --- | --- | --- | --- | --- | --- | --- | --- | --- | --- | --- | --- |
| LT30 | --- | --- | --- | --- | --- | --- | --- | --- | --- | --- | --- | --- | --- | --- |
| LT33 | --- | --- | --- | --- | --- | --- | --- | --- | --- | --- | --- | --- | --- | --- |
| LT8 | --- | --- | --- | --- | --- | --- | --- | --- | --- | --- | --- | --- | 1.00 | --- |
| LT21 | --- | --- | --- | --- | --- | --- | --- | --- | --- | 1.00 | 1.19 | --- | --- | --- |
| LT1a | --- | --- | --- | --- | --- | 1.26<br>1.24 | --- | 1.28 | --- | 1.72<br>1.29 | --- | --- | 1.19 | --- |
| LT6 | --- | --- | --- | --- | --- | 1.75<br>1.34 | --- | 1.62 | --- | 2.01 | --- | --- | --- | --- |
| LT10 | --- | --- | --- | --- | --- | --- | --- | --- | --- | 202<br>1.06 | 2.79 | --- | --- | --- |
| LT11 | --- | --- | --- | --- | --- | 1.31 | --- | --- | --- | 1.08<br>1.88<br>1.93 | --- | --- | 1.03 | --- |
| LT15 | --- | --- | --- | --- | --- | 1.63 | --- | --- | --- | 2.12<br>3.16<br>2.80 | --- | --- | 1.28 | ---<br>---<br>--- |
| LT16 | --- | --- | --- | --- | --- | 1.30 | ---- | --- | 2.48 | 1.05 | 1.32 | 2.19 | 1.33 | --- |
| LT17 | --- | --- | --- | --- | --- | 1.74 | --- | 1.72 | --- | 1.12<br>2.26<br>1.77 | 1.00<br>1.45 | --- | --- | --- |
| LT18 | --- | --- | --- | --- | --- | --- | --- | --- | --- | --- | 1.21 | --- | --- | --- |
| LT19 | --- | --- | --- | --- | --- | 1.98 | --- | --- | 2.10 | 3.47 3.02 | 1.35 | --- | --- | --- |
| LT26 | --- | --- | --- | --- | --- | --- | --- | --- | --- | --- | --- | --- | --- | --- |

EP 1 626 084 B1

| ΔCt values in lung and colon primary tumor and cell line models | | | | | | | | | | | | | |
|---|---|---|---|---|---|---|---|---|---|---|---|---|---|
| LT28 | --- | --- | --- | --- | --- | --- | --- | --- | --- | --- | --- | --- | --- | --- |
| LT29 | --- | --- | --- | --- | --- | --- | --- | --- | --- | --- | --- | --- | --- | --- |
| LT31 | --- | --- | --- | --- | --- | --- | --- | --- | --- | --- | --- | --- | --- | --- |
| HF-000 854 | --- | --- | --- | --- | --- | --- | --- | --- | --- | --- | --- | --- | --- | --- |
| HF-000 855 | --- | --- | --- | --- | --- | --- | --- | --- | --- | --- | --- | --- | --- | --- |
| HF-000 856 | --- | --- | --- | --- | --- | --- | --- | --- | --- | --- | --- | --- | --- | --- |
| HP-000 831 | --- | --- | --- | --- | --- | --- | --- | --- | --- | --- | --- | --- | --- | --- |
| HF-000 832 | --- | --- | --- | --- | --- | --- | --- | --- | --- | --- | --- | --- | --- | --- |
| HP-000 550 | --- | --- | --- | --- | --- | --- | --- | --- | --- | --- | --- | --- | --- | --- |
| HF-000 551 | --- | --- | --- | --- | --- | --- | --- | --- | --- | --- | --- | --- | --- | --- |
| HP-000 733 | --- | --- | --- | --- | --- | --- | --- | --- | --- | --- | --- | --- | --- | --- |
| HF-000 716 | --- | --- | --- | --- | --- | --- | --- | --- | --- | --- | --- | --- | --- | --- |

Table 7C

| ΔCt values in lung and colon primary tumor and cell line models | | | | | | | |
|---|---|---|---|---|---|---|---|
| Primary Tumor | PRO1686 | PRO1800 | PRO3562 | PRO9850 | PRO539 | PRO4316 | PRO4980 |
| HF-000631 | --- | --- | --- | --- | --- | --- | --- |
| HF-000641 | --- | --- | --- | --- | --- | --- | --- |
| HF-000643 | --- | --- | --- | --- | --- | --- | --- |
| HF-000840 | 1.61 | --- | 1.87 | --- | --- | 2.34 | 1.01 |
| HF-000842 | 1.11 | --- | --- | --- | --- | --- | --- |
| HBL100 | --- | --- | --- | --- | --- | --- | --- |
| MB43Ss | --- | --- | --- | --- | --- | --- | --- |
| T47D | --- | --- | --- | --- | --- | --- | --- |
| MB468 | --- | --- | --- | --- | --- | --- | --- |
| MB175 | --- | --- | --- | --- | --- | --- | --- |
| MB361 | --- | --- | --- | --- | --- | --- | --- |
| BT20 | --- | --- | --- | --- | --- | --- | --- |
| MCF7 | --- | --- | --- | --- | --- | --- | --- |
| SKBR3 | --- | --- | --- | --- | --- | --- | --- |
| SW480 | --- | --- | --- | --- | --- | --- | --- |
| SW620 | --- | --- | 1.08 | --- | --- | --- | --- |
| Colo320 | --- | 1.16 | - | --- | --- | --- | --- |
| HT29 | --- | --- | --- | --- | --- | --- | --- |
| HM7 | --- | --- | --- | --- | --- | --- | --- |
| WiDr | --- | --- | --- | --- | --- | --- | --- |
| HCT116 | --- | --- | 1.26 1.15 | --- | --- | --- | --- |
| SKCO1 | --- | --- | --- | --- | --- | --- | --- |
| SW403 | --- | --- | --- | --- | --- | --- | --- |
| LS174T | --- | --- | --- | --- | --- | --- | --- |
| Colo205 | --- | --- | --- | --- | --- | --- | --- |
| HCT15 | --- | --- | --- | --- | --- | --- | --- |
| HCC2998 | --- | --- | --- | --- | --- | --- | --- |
| KM12 | --- | --- | --- | --- | --- | --- | --- |
| A549 | --- | --- | --- | --- | --- | --- | --- |
| Calu-1 | --- | --- | --- | --- | --- | --- | --- |
| Calu-6 | --- | --- | --- | --- | --- | --- | --- |
| H157 | --- | --- | --- | --- | --- | --- | --- |
| H441 | --- | --- | --- | --- | --- | --- | --- |
| H460 | --- | --- | --- | --- | --- | --- | --- |
| SKMBS1 | --- | --- | --- | --- | --- | --- | --- |
| SW900 | --- | --- | --- | --- | --- | --- | --- |

(continued)

| ΔCt values in lung and colon primary tumor and cell line models | | | | | | | |
|---|---|---|---|---|---|---|---|
| Primary Tumor | PRO1686 | PRO1800 | PRO3562 | PRO9850 | PRO539 | PRO4316 | PRO4980 |
| H522 | --- | --- | 2.93 | --- | --- | --- | --- |
| H810 | --- | --- | ---- | --- | --- | --- | --- |
| SRCC 1094 | --- | --- | --- | --- | --- | --- | --- |
| SRCC 1095 | --- | --- | --- | --- | --- | --- | --- |
| SRCC 1096 | --- | --- | --- | --- | --- | --- | --- |
| SRCC 1097 | --- | --- | --- | --- | --- | --- | --- |
| SRCC 1098 | --- | --- | --- | --- | --- | --- | --- |
| SRCC 1099 | --- | --- | --- | --- | --- | --- | --- |
| SRCC 1100 | --- | --- | --- | --- | --- | --- | --- |
| SRCC 1101 | --- | --- | --- | --- | --- | --- | --- |
| hF-000545 | --- | --- | 1.05 | --- | --- | --- | --- |
| HF-000499 | --- | --- | --- | --- | --- | --- | --- |
| HF-000539 | --- | --- | 2.10 | --- | --- | --- | --- |
| HF-000373 | --- | --- | --- | --- | --- | --- | --- |
| FH-000698 | --- | --- | --- | --- | --- | --- | --- |
| HF-000756 | --- | --- | --- | --- | --- | --- | --- |
| HF-000762 | --- | --- | --- | --- | --- | --- | --- |
| HF-000789 | --- | --- | --- | --- | --- | --- | --- |
| HF-000795 | 1.13 | --- | --- | --- | --- | 1.06 | --- |
| HF-000811 | --- | --- | --- | --- | --- | --- | --- |
| HF-000755 | --- | --- | --- | --- | --- | --- | --- |
| CT2 | 1.38 | 1.50 | --- | --- | --- | --- | --- |
| CT3 | --- | --- | --- | --- | 1.17 | --- | --- |
| CT8 | --- | --- | --- | --- | --- | --- | --- |
| CT10 | 1.32 | --- | --- | 1.10 | 1.16 | --- | --- |
| CT12 | 1.20 | --- | --- | --- | 1.19 | --- | --- |
| CT14 | --- | 1.62 | --- | --- | --- | --- | --- |
| CT15 | --- | 1.48 1.08 | 1.01 | 1.23 | 1.03 | --- | --- |
| CT16 | --- | --- | --- | 1.49 | --- | --- | --- |
| CT17 | --- | --- | --- | --- | --- | --- | --- |
| CT1 | 1.50 | --- | --- | 1.00 | --- | --- | --- |

(continued)

| ΔCt values in lung and colon primary tumor and cell line models | | | | | | | |
|---|---|---|---|---|---|---|---|
| Primary Tumor | PRO1686 | PRO1800 | PRO3562 | PRO9850 | PRO539 | PRO4316 | PRO4980 |
| CT4 | 1.75 | --- | --- | 1.25 | --- | --- | --- |
| CT5 | 2.32 | 1.10 | --- | 1.49 | --- | --- | --- |
| CT6 | 1.13 | --- | --- | 1.04 | --- | --- | --- |
| CT7 | --- | --- | --- | 1.15 | --- | --- | --- |
| CT9 | --- | --- | --- | --- | --- | --- | --- |
| CT11 | 2.76 | 1.20 | --- | 1.33 | 1.12 | --- | --- |
| CT18 | --- | --- | --- | --- | --- | --- | --- |
| CT25 | --- | --- | --- | --- | --- | --- | --- |
| CT28 | --- | --- | --- | --- | --- | --- | --- |
| CT35 | --- | --- | --- | --- | --- | --- | --- |
| HF-000611 | --- | --- | --- | --- | --- | --- | --- |
| HF-000613 | --- | --- | --- | --- | --- | --- | --- |
| HF-001291 | --- | --- | --- | --- | --- | --- | --- |
| HF-001293 | --- | --- | --- | --- | --- | --- | --- |
| HF-001294 | 1.69 | --- | --- | --- | --- | --- | 1.14 |
| HF-001295 | --- | --- | --- | --- | --- | --- | --- |
| HF-001296 | 3.08 | --- | --- | --- | --- | --- | 1.87 |
| HF-001297 | --- | --- | --- | --- | --- | --- | --- |
| HF-001299 | 1.11 | --- | --- | --- | --- | --- | 1.11 |
| HF-001300 | --- | --- | --- | --- | --- | --- | --- |
| LT7 | --- | --- | --- | --- | --- | --- | --- |
| LT27 | --- | --- | --- | --- | --- | --- | --- |
| LT13 | 1.42 | 1.27 2.18 2.22 1.70 | 3.94 3.57 | 1.19 | 1.64 1.08 | --- | --- |
| LT1 | --- | --- | --- | --- | --- | --- | --- |
| LT2 | --- | --- | --- | --- | --- | --- | --- |
| LT3 | --- | --- | --- | --- | --- | --- | --- |
| LT4 | --- | --- | --- | --- | --- | --- | --- |
| LT9 | --- | --- | --- | --- | --- | --- | --- |
| LT12 | --- | 134 2.28 2.03 | --- | 1.32 | 1.23 | | |
| LT22 | --- | --- | --- | --- | --- | --- | --- |
| LT30 | --- | --- | --- | --- | --- | --- | --- |
| LT33 | --- | --- | --- | --- | --- | --- | --- |
| LT8 | --- | --- | --- | --- | --- | --- | --- |

(continued)

| ΔCt values in lung and colon primary tumor and cell line models | | | | | | | |
|---|---|---|---|---|---|---|---|
| Primary Tumor | PRO1686 | PRO1800 | PRO3562 | PRO9850 | PRO539 | PRO4316 | PRO4980 |
| L121 | --- | 1.30 | --- | --- | 1.32 | --- | --- |
| LT1a | --- | --- | --- | --- | --- | --- | --- |
| LT6 | --- | --- | --- | --- | --- | --- | --- |
| LT10 | --- | --- | --- | --- | --- | --- | --- |
| LT11 | 1.12 | 1.03 1.65 1.59 | --- | 1.35 | --- | --- | --- |
| LT15 | 1.67 | 1.70 2.23 1.93 | --- | 1.61 | 1.78 1.10 | --- | --- |
| LT16 | --- | 1.00 1.05 1.09 | 2.64 2.25 | --- | --- | --- | --- |
| LT17 | 1.59 | 1.94 1.63 | --- | --- | 1.94 1.01 | --- | --- |
| LT18 | 1.07 | 1.12 | --- | --- | --- | --- | --- |
| LT19 | --- | 2.51 2.18 | --- | --- | 1.16 | --- | --- |
| LT16 | --- | --- | --- | --- | --- | --- | --- |
| LT28 | --- | --- | --- | --- | --- | --- | --- |
| LT29 | --- | --- | --- | --- | --- | --- | --- |
| LT31 | --- | --- | --- | --- | --- | --- | --- |
| HF-000854 | --- | --- | --- | --- | --- | --- | --- |
| HF-000855 | --- | --- | --- | --- | --- | --- | --- |
| HF-000856 | --- | --- | --- | --- | --- | --- | --- |
| HF-000831 | --- | --- | --- | --- | --- | --- | --- |
| HF-000832 | --- | --- | --- | --- | --- | --- | --- |
| HF-000550 | --- | --- | --- | --- | --- | --- | --- |
| HF-000551 | --- | --- | --- | --- | --- | --- | --- |
| HF-000733 | --- | --- | 2.03 | --- | --- | --- | --- |
| HF-000716 | --- | --- | 1.83 | --- | --- | --- | --- |

DISCUSSION AND CONCLUSION:

PRO197 (DNA22780-1078):

The ΔCt values for DNA22780-1078 in a variety of tumors are reported in Table 7A. A ACt of >1 was typically used as the threshold value for amplification scoring, as this represents a doubling of gene copy. Table 7A indicates that significant amplification of nucleic acid DNA22780-1078 encoding PRO197 occurred in primary lung tumors: LT13, LT3, LT9, LT21, LT6, LT10, LT11, LT15, and LT17.

Because amplification of DNA22780-1078 occurs in various lung tumors, it is highly probable to play a significant role in tumor formation or growth. As a result, antagonists (e.g., antibodies) directed against the protein encoded by DNA22780-1078 (PRO0197) would be expected to have utility in cancer therapy.

PRO207 (DNA30879-1152):

The ΔCt values for DNA30879-1152 in a variety of tumors are reported in Table 7A. A ΔCt of >1 was typically used as the threshold value for amplification scoring, as this represents a doubling of gene copy. Table 7A indicates that significant amplification of nucleic acid DNA30879-1152 encoding PR0207 occurred: (1) in primary lung tumors: LT13, LT3, LT21, LT11, LT15, LT17, and LT19; (2) in primary colon tumors: CT3. CT10, CT15, CT1, CT4, C75, and CT11; and (3) in colon tumor cell lines: SW480, SW620, Colo320, HCT116, and SKCO1.

Because amplification of DNA30879-1152 occurs in various tumors, it is highly probable to play a significant role in tumor formation or growth. As a result, antagonists (e.g., antibodies) directed against the protein encoded by DNA30879-1152 (PR0207) would be expected to have utility in cancer therapy.

PRO226 (DNA33460-1166):

The ΔCt values for DNA33460-1166 in a variety of tumors are reported in Table 7A. A ΔCt of >1 was typically used as the threshold value for amplification scoring, as this represents a doubling of gene copy. Table 7A indicates that significant amplification of nucleic acid DNA33460-1166 encoding PR0226 occurred: (1) in primary lung tumors: LT7, LT13, LT3, LT4, LT9, L721, LT1a, LT11, LT15, LT17, and LT19; (2) in primary colon tumors: CT2, CT3, CT12, CT14, CT15, CT4, CT5, and CT11; and (3) in colon tumor cell lines: SW480, SW620. HT29; HM7, WiDr, HCT116, SKCO1, and SW403.

Because amplification of DNA33460-1166 occurs in various tumors, it is highly probable to play a significant role in tumor formation or growth. As a result, antagonists (e.g., antibodies) directed against the protein encoded by DNA33460-1166 (PRO226) would be expected to have utility in cancer therapy.

PRO232 (DNA34435-1140):

The ΔCt values for DNA34435-1140 in a variety of tumors are reported in Table 7A. A ΔCt of >1 was typically used as the threshold value for amplification scoring, as this represents a doubling of gene copy. Table 7A indicates that significant amplification of nucleic acid DNA34435-1140 encoding PR0232 occurred: (1) in primary lung tumors: LT12, LT15, LT17, LT18, and LT19; and (2) in primary colon tumors: CT1, CT4, CT5, CT7, CT9, CT11 and CT18.

Because amplification of DNA34435-1140 occurs in various tumors, it is highly probable to play a significant role in tumor formation or growth. As a result, antagonists (e.g., antibodies) directed against the protein encoded by DNA34435-1140 (PRO232) would be expected to have utility in cancer therapy.

PRO243 (DNA35917-1207):

The ΔCt values for DNA35917-1207 in a variety of tumors are reported in Table 7A. A ΔCt of >1 was typically used as the threshold value for amplification scoring, as this represents a doubling of gene copy. Table 7A indicates that significant amplification of nucleic acid DNA35917-1207 encoding PR0243 occurred: (1) in primary lung tumors: LT13, LT3, LT12, LT11, LT15, LT16, LT17, and LT19; and (2) in primary colon tumors: CT14 and CT5.

Because amplification of DNA35917-1207 occurs in various tumors, it is highly probable to play a significant role in tumor formation or growth. As a result, antagonists (e.g., antibodies) directed against the protein encoded by DNA35917-1207 (PRO243) would be expected to have utility in cancer therapy.

PRO256 (DNA35880-11601:

The ΔCt values for DNA35880-1160 in a variety of tumors are reported in Table 7A. A ΔCt of >1 was typically used as the threshold value for amplification scoring, as this represents a doubling of gene copy. Table 7A indicates that significant amplification of nucleic acid DNA35880-1160 encoding PR0256 occurred in colon tumor cell lines: SW620, HT29, WiDr, and HCT116.

Because amplification of DNA35880-1160 occurs in various tumors, it is highly probable to play a significant role in tumor formation or growth. As a result, antagonists (e.g., antibodies) directed against the protein encoded by DNA35880-1160 (PRO256) would be expected to have utility in cancer therapy.

PRO269 (DNA38260-1180):

The ΔCt values for DNA38260-1180 in a variety of tumors are reported in Table 7A. A ΔCt of >t was typically used as the threshold value for amplification scoring, as this represents a doubling of gene copy. Table 7A indicates that significant amplification of nucleic acid DNA38260-1180 encoding PR0269 occurred in primary lung tumors: LT7, LT13, LT9, LT12, LT11, LT15, LT17, and LT19.

Because amplification of DNA38260-1180 occurs in various lung tumors, it is highly probable to play a significant role in tumor formation or growth. As a result, antagonists (e.g., antibodies) directed against the protein encoded by DNA38260-1180 (PR0269) would be expected to have utility in cancer therapy.

PRO274 (DNA39987-1194):

The ΔCt values for DNA39987-1184 in a variety of tumors are reported in Table 7A. A ACt of >1 was typically used as the threshold value for amplification scoring, as this represents a doubling of gene copy. Table 7A indicates that significant amplification of nucleic acid DNA39987-1184 encoding PRO274 occurred in primary lung tumors: LT4, LT16, and LT18.

Because amplification of DNA39987-1184 occurs in various lung tumors, it is highly probable to play a significant role in tumor formation or growth. As a result, antagonists (e.g., antibodies) directed against the protein encoded by DNA39987-1184 (PRO274) would be expected to have utility in cancer therapy.

PRO304(DNA39520-12171:

The ΔCt values for DNA39520-1217 in a variety of tumors are reported in Table 7A. A ΔCt of >1 was typically used as the threshold value for amplification scoring, as this represents a doubling of gene copy. Table 7A indicates that significant amplification of nucleic acid DNA39520-1217 encoding PR0304 occurred in primary lung tumors: LT13, LT12, LT11, LT15, LT16, LT17 and LT19.

Because amplification of DNA39520-1217 occurs in various lung tumors, it is highly probable to play a significant role in tumor formation or growth. As a result, antagonists (e.g., antibodies) directed against the protein encoded by DNA39520-1217 (PR0304) would be expected to have utility in cancer therapy.

PRO339 (DNA43466-1225):

The ΔCt values for DNA43466-1225 in a variety of tumors are reported in Table 7A. A ΔCt of >1 was typically used as the threshold value for amplification scoring, as this represents a doubling of gene copy. Table 7A indicates that significant amplification of nucleic acid DNA43466-1225 encoding PRO339 occurred in primary lung tumors: LT7, LT13, LT3, LT9, LT12, LT11, and LT17.

Because amplification of DNA43466-1225 occurs in various lung tumors, it is highly probable to play a significant role in tumor formation or growth. As a result, antagonists (e.g., antibodies) directed against the protein encoded by DNA43466-1225 (PR0339) would be expected to have utility in cancer therapy.

PRO1558 (DKA71282-1668):

The ΔCt values for DNA71282-1668 in a variety of tumors are reported in Table 7A. A ΔCt of >1 was typically used as the threshold value for amplification scoring, as this represents a doubling of gene copy. Table 7A indicates that significant amplification of nucleic acid DNA71282-1668 encoding PRO1558 occurred: (1) in primary lung tumors: HF-000840, HF-000842, HF-001294, HF-001296 and HP-001299; and (2) in colon tumor center HF-000795.

Because amplification of DNA71282-1668 occurs in various tumors, it is highly probable to play a significant role in tumor formation or growth. As a result, antagonists (e.g., antibodies) directed against the protein encoded by DNA71282-1668 (PRO1558) would be expected to have utility in cancer therapy.

PRO779 (DNA58801-1052):

The ΔCt values for DNA58801-1052 in a variety of tumors are reported in Table 7A. A ΔCt of >1 was typically used as the threshold value for amplification scoring, as this represents a doubling of gene copy. Table 7A indicates that significant amplification of nucleic acid DNA58801-1052 encoding PR0779 occurred: (1) in primary lung tumors: LT13, LT3, LT9, LT12, LT21, LT1-a, LT6, LT10, LT11, LT15, LT16, LT17, LT18, LT19, and HF-000840; (2) in primary colon tumors: CT2, CT3, CT8, CT10, CT12, CT14, CT15, CT16, CT17, CT1, CT4, CT5, CT6, CT7, CT9, and CT11; and (3) in colon tumor cell lines: SW480, SW620, Colo320, HT29, HM7, WiDr, HCT116, SKCO1, and GS174T.

Because amplification of DNA58801-1052 occurs in various tumors, it is highly probable to play a significant role in tumor formation or growth. As a result, antagonists (e.g., antibodies) directed against the protein encoded by DNA58801-1052 (PR0779) would be expected to have utility in cancer therapy.

PRO1185 (DNA62881-1515):

The ΔCt values for DNA62881-1515 in a variety of tumors are reported in Table 7A. A ΔCt of >1 was typically used as the threshold value for amplification scoring, as this represents a doubling of gene copy. Table 7A indicates that significant amplification of nucleic acid DNA62881-1515 encoding PRO1185 occurred: (1) in primary lung tumors: LT3, LT30 and LT26; and (2) in primary colon tumor CT2.

Because amplification of DNA62881-1515 occurs in various tumors, it is highly probable to play a significant role in tumor formation or growth. As a result, antagonists (e.g., antibodies) directed against the protein encoded by DNA62881-1515 (PRO1185) would be expected to have utility in cancer therapy.

PRO1245 (DNA64884-1527):

The ΔCt values for DNA64884-1527 in a variety of tumors are reported in Table 7A. A ΔCt of >1 was typically used as the threshold value for amplification scoring, as this represents a doubling of gene copy. Table 7A indicates that significant amplification of nucleic acid DNA64884-1527 encoding PRO1245 occurred: (1) in primary lung tumors: LT13, LT15 and LT16; (2) in lung tumor cell line H522: and (3) in primary colon tumor CT15.

Because amplification of DNA64884-1527 occurs in various tumors, it is highly probable to play a significant role in tumor formation or growth. As a result, antagonists (e.g., antibodies) directed against the protein encoded by DNA64884-1527 (PRO1245) would be expected to have utility in cancer therapy.

PRO1759 (DNA76531-1701):

The ΔCt values for DNA76531-1701 in a variety of tumors are reported in Table 7B. A ΔCt of >1 was typically used as the threshold value for amplification scoring, as this represents a doubling of gene copy. Table 7B indicates that significant amplification of nucleic acid DNA76531-1701 encoding PRO1759 occurred: (1) in primary lung tumors: HF-000840 and HF-001296; and (2) in primary colon tumor center HF-000795.

Because amplification of DNA76531-1701 occurs in various tumors, it is highly probable to play a significant role in tumor formation or growth As a result, antagonists (e.g., antibodies) directed against the protein encoded by DNA76531-1701 (PRO1759) would be expected to have utility in cancer therapy.

PRO5775 (DNA96869-2673):

The ΔCt values for DNA96869-2673 in a variety of tumors are reported in Table 7B. A ACt of >1 was typically used as the threshold value for amplification scoring, as this represents a doubling of gene copy. Table 7B indicates that significant amplification of nucleic acid DNA96869-2673 encoding PR05775 occurred: (1) in primary lung tumors,: HF-000631, HF-000641, HF-000643, HP-000840, HF-000842, HF-001293, HF-001294, **HF-**001295, HP-001296 and HF-001299; and (2) in primary colon tumor centers: HF-000762, HF-000789, and HF-000811.

Because amplification of DNA96869-2673 occurs in various tumors, it is highly probable to play a significant role in tumor formation or growth. As a result, antagonists (e.g., antibodies) directed against the protein encoded by DNA96869-2673 (PRO5775) would be expected to have utility in cancer therapy.

PRO7133 (DNA128451-2739):

The ΔCt values for DNA128451-2739 in a variety of tumors are reported in Table 7B. A ΔCt of >1 1 was typically used as the threshold value for amplification scoring, as this represents a doubling of gene copy. Table 7B indicates that significant amplification of nucleic acid DNA128451-2739 encoding PRO7133 occurred: (1) in primary lung tumors: HF-000840 and HF-001296; and (2) in primary colon tumor centers: HF-000795 and HF-000811.

Because amplification of DNA 128451-2739 occurs in various tumors, it is highly probable to play a significant role in tumor formation or growth. As a result, antagonists (e.g., antibodies) directed against the protein encoded by DNA128451-2739 (PRO7133) would be expected to have utility in cancer therapy.

PRO7168 (DNA102846-2742):

The ΔCt values for DNA 102846-2742 in a variety of tumors are reported in Table 7B. A ΔCt of >1 was typically used as the threshold value for amplification scoring, as this represents a doubling of gene copy. Table 7B indicates that significant amplification of nucleic acid DNA102846-2742 encoding PR07168 occurred in primary lung tumors: HF-000631, HF-000840 and HF-000842.

Because amplification of DNA102846-2742 occurs in various tumors, it is highly probable to play a significant role in tumor formation or growth. As a result, antagonists (e.g., antibodies) directed against the protein encoded by DNA102846-2742 (PR07168) would be expected to have utility in cancer therapy.

PRO5725 (DNA92265-2669):

The ΔCt values for DNA9226S-2669 in a variety of tumors are reported in Table 7B. A ACt of >1 was typically used as the threshold value for amplification scoring, as this represents a doubling of gene copy. Table 7B indicates that significant amplification of nucleic acid DNA92265-2669 encoding PRO5725 occurred: (1) in primary lung tumors: HF-000641, HF-000840, HF-001295, and HF-001296; and (2) in primary colon tumor centers: HF-000762 and HF-000795.

Because amplification of DNA92265-2669 occurs in various tumors, it is highly probable to play a significant role in tumor formation or growth. As a result, antagonists (e.g., antibodies) directed against the protein encoded by DNA92265-2669 (PRO5725) would be expected to have utility in cancer therapy.

PRO202 (DNA30869):

The ΔCt values for DNA30869 in a variety of tumors are reported in Table 7B. A ΔCt of >1 was typically used as the threshold value for amplification scoring, as this represents a doubling of gene copy. Table 7B indicates that significant amplification of nucleic acid DNA30869 encoding PR0202 occurred in primary lung tumors: LT7, LT13, LT1, LT3, LT4, LT9, LT12, LT1a, LT6, LT11, LT15, LT16, LT17, and LT19.

Because amplification of DNA30869 occurs in various lung tumors, it is highly probable to play a significant role in tumor formation or growth. As a result, antagonists (e.g., antibodies) directed against the protein encoded by DNA30869 (PRO202) would be expected to have utility in cancer therapy.

PRO206 (DNA34405):

The ΔCt values for DNA34405 in a variety of tumors are reported in Table 7B. A ΔCt of >1 was typically used as the threshold value for amplification scoring, as this represents a doubling of gene copy. Table 7D indicates that significant amplification of nucleic acid DNA34405 encoding PR0206 occurred in primary colon tumors: CT2, CT10, CT12, CT14, CT15, CT16, CT5, and CT18.

Because amplification of DNA34405 occurs in various colon tumors, it is highly probable to play a significant role in tumor formation or growth. As a result, antagonists (e.g., antibodies) directed against the protein encoded by DNA34405 (PRO206) would be expected to have utility in cancer therapy.

PRO264 (DNA36995):

The ΔCt values for DNA36995 in a variety of tumors are reported in Table 7B. A ΔCt of >1 was typically used as the threshold value for amplification scoring, as this represents a doubling of gene copy. Table 7B indicates that significant amplification of nucleic acid DNA36995 encoding PRO264 occurred in primary lung tumors: LT3, LT4, LT9, LT1a, LT6, and LT17.

Because amplification of DNA36995 occurs in various colon tumors, it is highly probable to play a significant role in tumor formation or growth. As a result, antagonists (e.g., antibodies) directed against the protein encoded by DNA36995 (PRO264) would be expected to have utility in cancer therapy.

PRO313 (DNA43320):

The ΔCt values for DNA43320 in a variety of tumors are reported in Table 7B. A ΔCt of >1 was typically used as the threshold value for amplification scoring, as this represents a doubling of gene copy. Table 7B indicates that significant amplification of nucleic acid DNA43320 encoding PR0313 occurred: (1) in primary lung tumors: LT9, LT12, LT16, and LT19; (2) in primary colon tumors: CT2, CT1, CT4, CT5, CT9, and CT11; and (3) in colon tumor cell line SW620.

Because amplification of DNA43320 occurs in various tumors, it is highly probable to play a significant role in tumor formation or growth. As a result, antagonists (e.g., antibodies) directed against the protein encoded by DNA43320 (PR0313) would be expected to have utility in cancer therapy.

PRO342 (DNA38649):

The ΔCt values for DNA38649 in a variety of tumors are reported in Table 7B. A ΔCt of >1 was typically used was the threshold value for amplification scoring, as this represents a doubling of gene copy. Table 7B indicates that significant amplification of nucleic acid DNA38649 encoding PR0342 occurred: (1) in primary lung tumors: LT7, LT13, LT3, LT9, LT12, LT21, LT1a, LT6, LT10, LT11, LT15, LT16, LT17, LT19, HF-000840, HF-000842, HF-001294, and HF-001296; (2) in primary colon tumors: CT2, CT3, CT8, CT10, CT12, CT14, CT15, CT16, CT17, CT1, CT4, CT5, CT6, CT9, and CT11; (3) in lung tumor cell lines: Calu-1 and H441; and (4) in colon tumor cell lines: SW620 and LS174T.

Because amplification of DNA38649 occurs in various tumors, it is highly probable to play a significant role in tumor formation or growth. As a result, antagonists (*e.g.*, antibodies) directed against the protein encoded by DNA38649 (PR0342) would be expected to have utility in cancer therapy.

PRO542 (DNA56505):

The ΔCt values for DNA56505 in a variety of tumors are reported in Table 7B. A ΔCt of >1 was typically used as the threshold value for amplification scoring, as this represents a doubling of gene copy. Table 7B indicates that significant amplification of nucleic acid DNA56505 encoding PRO542 occurred: (1) in primary lung tumors: LT7, LT13, LT12, LT21, LT10, LT16, LT17, LT18, and LT19; (2) in primary colon tumors: CT10, CT12, CT14, CT5, and CT9; (3) in lung tumor cell line H441; (4) in colon tumor cell lines: SW480, SW620, HT29, WiDr, HCT116, SKCO1, SW403, and LS174T; and (5) in breast tumor cell lines: HBL100 and MCF7.

Because amplification of DNA56505 occurs in various tumors, it is highly probable to play a significant role in tumor formation or growth. As a result, antagonists (e.g., antibodies) directed against the protein encoded by DNA56505 (PRO542) would be expected to have utility in cancer therapy.

PRO773 (DNA48303):

The ΔCt values for DNA48303 in a variety of tumors are reported in Table 7B. A ΔCt of >1 was typically used as the threshold value for amplification scoring, as this represents a doubling of gene copy. Table 7B indicates that significant amplification of nucleic acid DNA48303 encoding PRO773 occurred: (1) in primary lung tumors: LT13 and LT16; (2) in primary colon tumors: CT15. CT16 and CT17; (3) in colon tumor cell lines: Colo320, HT29, and Colo205; and (4) in lung tumor cell line H441.

Because amplification of DNA48303 occurs in various tumors, it is highly probable to play a significant role in tumor formation or growth. As a result, antagonists (e.g., antibodies) directed against the protein encoded by DNA48303 (PR0773) would be expected to have utility in cancer therapy.

PRO861 (DNA50798):

The ΔCt values for DNA50798 in a variety of tumors are reported in Table 7B. A ΔCt of >1 was typically used as the threshold value for amplification scoring, as this represents a doubling of gene copy. Table 7B indicates that significant amplification of nucleic acid DNA50798 encoding PRO861 occurred: (1) in primary lung tumors: LT13, LT12, LT8, LT1a, LT11, LT15 and LT16; (2) in primary colon tumors: CT2, CT3, CT8, CT10, CT12, CT14, CT15, CT16, CT17, CT1, CT4, CT5, CT7, CT9, and CT11; and (3) in lung tumor cell lines: H441 and H522

Because amplification of DNA50798 occurs in various tumors, it is highly probable to play a significant role in tumor formation or growth. As a result, antagonists (*e.g.*, antibodies) directed against the protein encoded by DNA50798 (PRO861) would be expected to have utility in cancer therapy.

PRO1216 (DNA66489):

The ΔCt values for DNA66489 in a variety of tumors are reported in Table 7B. A ΔCt of >1 was typically used as the threshold value for amplification scoring, as this represents a doubling of gene copy. Table 7B indicates that significant amplification of nucleic acid DNA 66489 encoding PRO1216 occurred: (1) in primary lung tumors: LT7, and LT12; (2) in primary colon tumors: CT12 and CT5; and (3) in colon tumor cell lines: WiDr, HCT116, SW403, and LS174T.

Because amplification of DNA66489 occurs in various tumors, it is highly probable to play a significant role in tumor formation or growth. As a result, antagonists (e.g., antibodies) directed against the protein encoded by DNA66489 (PRO1216) would be expected to have utility in cancer therapy.

PRO1686 (DNA80896):

The ΔCt values for DNA80896 in a variety of tumors are reported in Table 7C. A ΔCt of >1 was typically used as the threshold value for amplification scoring, as this represents a doubling of gene copy. Table 7C indicates that significant amplification of nucleic acid DNA80896 encoding PRO1686 occurred: (1) in primary lung tumors: LT13, LT11, LT15, LT17, LT18, HF-000840, HF-000842, HF-001294, HF-001296, and HF-001299; (2) in primary colon tumors: CT2, dT10, CT12, CT1, CT4, CT5, CT6, and CT11; and (3) colon tumor center HF-000795.

Because amplification of DNA80896 occurs in various tumors, it is highly probable to play a significant role in tumor formation or growth. As a result, antagonists (e.g., antibodies) directed against the protein encoded by DNA80896 (PRO1686) would be expected to have utility in cancer therapy.

PRO1800 (DNA35672-2508):

The ΔCt values for DNA35672-2509 in a variety of tumors are reported in Table 7C. A ΔCt of >1 was typically used as the threshold value for amplification scoring, as this represents a doubling of gene copy. Table 7C indicates that significant amplification of nucleic acid DNA35672-2508 encoding PR01800 occurred: (1) in primary lung tumors: LT13, LT12, LT21, LT11, LT15, LT16, LT17, LT18, and LT19; (2) in primary colon tumors: CT2, CT14, CT15, CT5, and CT11; and (3) in colon tumor cell line Colo320.

Because amplification of DNA35672-2508 occurs in various tumors, it is highly probable to play a significant role in tumor formation or growth. As a result, antagonists (e.g., antibodies) directed against the protein encoded by DNA35672-2508 (PRO1800) would be expected to have utility in cancer therapy.

PRO3562 (DNA96791):

The ΔCt values for DNA96791 in a variety of tumors are reported in Table 7C. A ΔCt of >1 was typically used as the threshold value for annplification scoring, as this represents a doubling of gene copy. Table 7C indicates that significant amplification of nucleic acid DNA96791 encoding PRO3562 occurred: (1) in primary lung tumors: LT13, LT16, and HP-000840; (2) in primary colon tumor GT15; (3) in colon tumor center HP-000539: (4) in lung tumor cell line H522; (5) in colon tumor cell lines: SW620 and HCT116: (6) in breast tumour HF-000545; and (7) in testes tumors: HP-000733 and HF-000716.

Because amplification of DNA96791 occurs in various tumors, it is highly probable to play a significant role in tumor formation or growth. As a result, antagonists (*e.g.*, antibodies) directed against the protein encoded by DNA96791 (PR03562) would be expected to have utility in cancer therapy.

PR09850 (DNA58725):

The ΔCt values for DNA58725 in a variety of tumors are reported in Table 7C. A ΔCt of > 1 was typically used as the threshold value for amplification scoring, as this represents a doubling of gene copy. Table 7C indicates that significant amplification of nucleic acid DNA58725 encoding PRO9850 occurred: (1) in primary lung tumors: LT13, LT12, LT11, and LT15; and (2) in primary colon tumors: CR10, CT15, CT16, CT1, CT4, CT5, CT6, CT7, and CT11.

Because amplification of DNA58725 occurs in various tumors, it is highly probable to play a significant role in tumor formation or growth. As a result, antagonists (*e.g.*, antibodies) directed against the protein encoded by DNA58725 (PR09850) would be expected to have utility in cancer therapy.

PRO539 (DNA47465-1561):

The ΔCt values for DNA47465-1561 in a variety of tumors are reported in Table 7C. A ΔCt of >1 was typically used as the threshold value for amplification scoring, as this represents a doubling of gene copy. Table 7C indicates that significant amplification of nucleic acid DNA47465-1561 encoding PR0539 occurred: (1) in primary lung tumors: LT13, LT12, LT21, LT15, LT17, and LT19; and (2) in primary colon tumors: CT3, CT10, CT12, CT15, and CT11.

Because amplification of DNA47465-1561 occurs in various tumors, it is highly probable to play a significant role in tumor formation or growth. As a result, antagonists (e.g., antibodies) directed against the protein encoded by DNA47465-1561 (PRO539) would be expected to have utility in cancer therapy.

PRO4316 (DNA94713-2561):

The ΔCt values for DNA94713-2561 in a variety of tumors are reported in Table 7C. A ΔCt of >1 was typically used as the threshold value for amplification scoring, as this represents a doubling of gene copy. Table 7C indicates that significant amplification of nucleic acid DNA94713-2561 encoding PRO4316 ocurred: (1) in primary lung tumor HF-000840; and (2) in primary colon tumor center HF-000795.

Because amplification of DNA94713-2561occurs in various tumors, it is highly probable to play a significant role in tumor formation or growth. As a result, antagonists (e.g., antibodies) directed against the protein encoded by DNA94713-2561 (PRO4316) would be expected to have utility in cancer therapy.

PRO4980 (DNA97003-2649):

The ΔCt values for DNA97003-2649 in a variety of tumors are reported in Table 7C. A ΔCt of >1 was typically used as the threshold value for amplification scoring, as this represents a doubling of gene copy. Table 7C indicates that significant amplification of nucleic acid DNA97003-2649 encoding PRO4980 ocurred in primary lung tumors: HF-000840. HF-001294, HF-001296 and HP-001299.

Because amplification of DNA97003-2649 occurs in various lung tumors, it is highly probable to play a significant role in tumor formation or growth. As a result, antagonists (*e.g.*, antibodies) directed against the protein encoded by DNA97003-2649 (PRO4980) would be expected to have utility in cancer therapy.

EXAMPLE 27

*In situ* Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis, and aid in chromosome mapping.

*In situ* hybridization was performed following an optimized version of the protocol by Lu and Gillett, Cell Vision, 1: 169-176 (1994), using PCR-generated $^{33}$P-labeled riboprobes. Briefly, formalin-fixed, paraffin-embedded human tissues were sectioned, deparaffinized, deproteinated in proteinase K (20 g/ml) for 15 minutes at 37˚C, and further processed for *in situ* hybridization as described by Lu and Gillett, *supra.* A ([33]-P)UTP-labeled antisense riboprobe was generated from a PCR product and hybridized at 55 ˚C overnight. The slides were dipped in Kodak NTB2™ nuclear track emulsion and exposed for 4 weeks.

$^{33}$P-Riboprobe synthesis

6.0 μl (125 mCi) of $^{33}$P-UTP (Amersham BF 1002, SA<2000 Ci/mmol) were speed-vacuum dried. To each tube containing dried [33]p-UTP, the following ingredients were added:

2.0 μl 5x transcription buffer
1.0 μl DTT (100 mM)
2.0 μl NTP mix (2.5 mM: 10 μl each of 10 mM GTP, CTP & ATP + 10μl $H_2O$)
1.0 μl UTP (50 μM)
1.0 μl RNAsin
1.0 μl DNA template (1 μg)
1.0 μl $H_2O$
1.0 μl RNA polymerase (for PCR products T3 = AS, T7 = S, usually)

The tubes were incubated at 37 ˚C for one hour. A total of 1.0 μl RQ1 DNase was added, followed by incubation at 37˚C for 15 minutes. A total of 90 μl TE (10 mM Tris pH 7.6/1 mM EDTA pH 8.0) was added, and the mixture was pipetted onto DE81 paper. The remaining solution was loaded in a MICROCON-50™ ultrafiltration unit, and spun using program 10 (6 minutes). The filtration unit was inverted over a second tube and spun using program 2 (3 minutes). After the final recovery spin, a total of 100 μl TE was added, then 1 μl of the final product was pipetted on DE81 paper and counted in 6 ml of BIOFLUOR II™.

The probe was run on a TBF/urea gel. A total of 1-3 μl of the probe or 5 μl of RNA Mrk III was added to 3 μl of loading buffer. After heating on a 95 ˚C heat block for three minutes, the gel was immediately placed on ice. The wells of gel were flushed, and the sample was loaded and run at 180-250 volts for 45 minutes. The gel was wrapped in plastic wrap (SARAN™ brand) and exposed to XAR film with an intensifying screen in a -70˚C freezer one hour to overnight.

$^{33}$P-Hybridization

A. *Pretreatment of frozen sections*

The slides were removed from the freezer, placed on aluminum trays, and thawed at room temperature for 5 minutes. The trays were placed in a 55˚C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ H$_2$O). After deproteination in 0.5 $\mu$g/ml proteinase K for 10 minutes at 37˚C (12.5 $\mu$l) of 10 mg/ml stock in 250 ml prewarmed RNAse-free RNAse buffer), the sections were washed in 0.5 x SSC for 10 minutes at room temperature. The sections were dehydrated in 70%, 95%, and 100% ethanol, 2 minutes each.

B. *Pretreatment of paraffin-embedded sections*

The slides were deparaffinized, placed in SQ H$_2$O, and rinsed twice in 2 x SSC at room temperature, for 5 minutes each time. The sections were deproteinated in 20 $\mu$g/ml proteinase K (500 $\mu$l of 10 mglml in 250 ml RNase-free RNase buffer, 37˚C,15 minutes) for human embryo tissue, or 8 x proteinase K (100 $\mu$l in 250 ml Rnase buffer, 37˚C, 30 minutes) for formalin tissues. Subsequent rinsing in 0.5 x SSC and dehydration were performed as described above.

C. *Prehybridization*

The slides were laid out in a plastic box lined with Box buffer (4 x SSC, 50% formamide) - saturated filter paper. The tissue was covered with 50 $\mu$l of hybridization buffer(3.75 g dextran sulfate + 6 ml SQ H$_2$O), vortexed, and heated in the microwave for 2 minutes with the cap loosened. After cooling on ice,18.75 ml formamide, 3.75 ml 20 x SSC, and 9 ml SQ H$_2$O were added, and the tissue was vortexed well and incubated at 42˚C for 1-4 hours.

D. *Hybridization*

1.0 x 10$^6$ cpm probe and 1.0 $\mu$l tRNA (50 mg/ml stock) per slide were heated at 95˚C for 3 minutes. The slides were cooled on ice, and 48 $\mu$l hybridization buffer was added per slide. After vortexing, 50 $\mu$l $^{33}$P mix was added to 50 $\mu$l prehybridization on the slide. The slides were incubated overnight at 55˚C.

E. *Washes*

Washing was done for 2x 10 minutes with 2xSSC. EDTA at room temperature (400 ml 20 x SSC + 16 ml 0.25 M EDTA. V$_r$=4L), followed by RNAseA treatment at 37˚C for 30 minutes (500 $\mu$l of 10 mg/ml in 250 ml Rnase buffer = 20 $\mu$g/ml), The slides were washed 2 x10 minutes with 2 x SSC, EDTA at room temperature. The stringency wash conditions were as follows: 2 hours at 55˚C, 0.1 x SSC, EDTA (20 ml 20 x SSC + 16 ml EDTA, V$_r$=4L).

F. *Oligonucleotides*

*In situ* analysis was performed on six of the DNA sequences disclosed herein. The oligonucleotides employed for these analyses are as follows:

(1) PRO197 (DNA22780-1078):

DNA22780.p1:

5'-GAA TTC TAA TAC GAC TCA CTA TAG GGC CGC CACCGCCGTGCTACTGA-3' (SEQ ID NO:247)

DNA22780.p2:

5'-CTA TGA AATTAA CCCTCACTA AAG GGA TGC AGG CGG CTGACATTGTGA-3'(SEQ ID NO:248)

(2) PRO207 (DNA30879-1152):

DNA30879.p1:

5'-GGA TTC TAA TAC GAC TCA CTA TAG GGC TCC TGC GCC TTT CCT GAA CC-3' (SEQ ID NO:249)

DNA30879.p2:

5'-CTA TGA AAT TAA CCC TCA CTA AAG GGA GAC CCA TCC TTG CCC ACA GAG-3' (SEQ ID NO:250)

(3) PRO226 (DNA33460-1166):

DNA33460.p1:

5'-GGA TTC TAA TAC GAC TCA CTA TAG GGC CAG CAC TGC CGG GATGTC AAC-3'(SEQ ID NO:25 1)

DNA33460.p2:

5'-CTA TGA AAT TAA CCC TCA CTA AAG GGA GTTTGG GCC TCG GAG CAG TG-3' (SEQ ID NO:2S2)

(4) PRO232 (DNA34435-1140):

DNA34435.p1:

5'-GGATCC TAA TAC GAC TCA CTA TAG GGC ACC CAC GCG TCC GGC TGC TT-3' (SEQ ID NO:2S3)

DNA34435.p2:

5'-CTA TGA AATTAA CCC TCA CTA AAG GGA CGG GGG ACA CCA CGG ACC AGA-3' (SEQ IDNO:254)

(5) PRO243 (CDNA35917-1207):

DNA35917.p1:

5'-GGATTCTAATACGACTCACTA TAGGGCAAGGAGCCGGGA CCC AGG AGA-3'(SEQ ID NO:255)

DNA35917.p2:

5'-CTA TGA AAT TAA CCC TCA CTA AAG GGA GGG GGC CCTTGG TGC TGA GT-3' (SEQ ID NO:256)

(6) PRO342 (DNA38649):

DNA38649.p1:

5'-GGA TTC TAA TAC GAC TCA CTA TAG GGC GGG GCC TTC ACC TGC TCC ATC-3'(SEQ IDNO:257)

DNA38649.p2:

5'-CTA TGA AATTAA CCCTCA CTA AAG GGA GCTGCGTCTGGG GGT CTC CTT-3'(SEQ ID NO:258)

G. *Results*

(1) PRO197 (DNA22780-1078) (NL2):

A moderate to intense signal was seen over benign but reactive stromal cells in inflamed appendix. These cells typically have large nuclei with prominent nucleoli. An intense signal was present over a small subset (<5%) of tumor cells in mammary ductal adenocarcinoma, and in peritumoral stromal cells. The histological appearance of the positive cells was not notably different than the adjacent negative cells. A very focal positive signal was found over tumor and/or stromal cells in renal cell carcinoma adjacent to necrotic tissue. No signal was seen in pulmonary adenocarcinoma.

(2) PRO207 (DNA30879-1152) (Apo 2L homolog):

Low level expression was observed over a chondrosarcoma, and over one other soft-tissue sarcoma. All other tissues were negative.
Human fetal tissues examined (E12-E16 weeks) included: placenta, umbilical cord, liver, kidney, adrenals, thyroid, lungs, heart, great vessels, oesophagus, stomach, small intestine, spleen, thymus, pancreas, brain, eye, spinal cord, body wall, pelvis and lower limb.
Adult human tissues examined included: kidnay (normal and end-stage), adrenals, myocardium, spleen, lymph node, pancreas, lung, skin, eye (including retina), bladder, and liver (normal, cirrhotic, and acute failure).
Non-human primate tissues examined included:

Chimp tissues: salivary gland, stomach, thyroid, parathyroid, tongue, thymus, ovary, and lymph node.
Rhesus monkey tissues: cerebral cortex, hippocampus, cerebellum, and penis.

(3) PRO226 (CDNA33460-1166)(EGF homolog):

A specific signal was observed over cells in loose connective tissue immediately adjacent to developing extra ocular muscle in the fetal eye. Moderate expression was also seen over soft-tissue sarcoma.

(4) PRO232 (DNA34435-1140) (stem cell antigen homolog):

*Expression pattern in human and fetal tissues*

Strong expression was seen in prostatic epithelium and bladder epithelium, with lower level of expression in bronchial epithelium, Low level expression was seen in a number of sites, including among others, bone, blood, chondrosarcoma, adult heart and fetal liver. All other tissues were negative.

*Expression in urothelium of the ureter of renal pelvis, and urethra of rhesus penis*

Expression was observed in the epithelium of the prostate, the superficial layers of the urethelium of the urinary bladder, the urethelium lining the renal pelvis, and the urethelium of the ureter (in one out of two experiments). The urethra of a rhesus monkey was negative; it was unclear whether this represents a true lack of expression by the urethra, or if it is the result of a failure of the probe to cross react with rhesus tissue. The findings in the prostate and the bladder were similar to those previously described using an isotopic detection technique. Expression of the mRNA for this antigen was not prostate epithelial specific. The antigen may serve as a useful marker for urethelial derived tissues. Expression in the superficial, post-mitotic cells of the urinary tract epithelium also suggests that it is unlikely to represent a specific stem cell marker, as this would be expected to be expressed specifically in basal epithelium.

*PSCA in prostate and bladder carcinoma*

Six samples of prostate and bladder cancer of various grades, one sample each of normal renal pelvis, ureter, bladder; prostate (including seminal vesicle) and penile ureter, and pellets of LNCaP andPC3 prostate cancer cell lines were analyzed: each sample was hybridized with sense and anti-sense probes for PSCA, and with anti-sense probe only for beta-actin (mRNA integrity control).
Normal transitional epithelium of the renal pelvis, ureter, and bladder, and stratified columnar epithelium of penile urethra were all positive for PSCA; of these, the superficial (umbrella) cells of the bladder and renal pelvis were most intensely positive. Normal prostatic glandular epithelium was variably positive for PSCA; moderately to strong positive glands occurred in close proximity to negative glands within the same tissue section. All positive epithelia (bladder and prostate) showed more intense expression in the transitional or prostatic epithelium. Seminal vesicle epithelium and all other tissues (neural, vascular, fibrous stroma, renal parenchyma) do not express PSCA.
Prostatic tumor cells are generally PSCA-negative; no detectable expression was noted in LNCaP and PC3 cells and in three of six tissue samples; moderately to weakly positive cells occurred only in three of six prostate tumor samples. PSCA-negative prostate tumor samples showed beta-actin expression consistent with adequate mRNA preservation.
Papillary transitional carcinoma cells (five of six cases) were moderately or strongly positive for PSCA. One of six tumors (a case of invasive poorly differiated TCC) showed only focally positive cells.

*PSCA and PSA expression in additional prostate and bladder carcinoma specimen*

Thirteen samples of prostate cancer (all moderately to poorly differentiated adenocarcinoma), one sample of prostate without tumor, and bladder transitional cell carcinoma of various grades (eight well-differentiated, three moderately differentiated, two poorly differentiated) were hybridized with sense and anti-sense probes for PSCA and with anti-sense probe only for beta-actin (mRNA integrity control). As an additonal control, the fourteen prostate cases were hybridized with an anti-sense probe to PSA, as were the six sections of prostate CA from the previous sudy.
One case of prostate cancer (#127) showed uniform high expression of PSCA. Two cases of prostate CA (#399, #403) showed only focal high levels of PSCA expression, and one case (#124) showed focal moderate expression, all with marked gland-to-gland variability. Most areas of these three cases, and all areas of the other nine cases showed uniformly weak or absent PSCA expression. The low PSCA signals were not due to mRNA degradation: all cases of prostate CA negative for PSCA were positive for PSA and/or beta-actin.

All eleven well- or moderately well-differentiated transitional carcinomas of the bladder were uniformly moderately or strongly positive for PSCA. Two tumors, both poorly differentiated TCC, were negative or only weakly positive.

These results confirm the previously described studies. In these two studies, nineteen prostate CA cases were examined: one of nineteen showed uniformly high expression; six of nineteen showed focal high expression in a minority of tumor cells; twelve of nineteen were negative oronly weakly positive. In contrast, these two studies included nineteen bladder TCC cases, the majority of which were uniformly moderately or strongly PSCA-positive. All sixteen well-or moderately well-differentiated TCC cases were positive; three poorly differentiated cases were negative or only weakly positive.

(5) PRO243 (DNA35917-1207) (Chordin homolog):

Faint expression was observed at the cleavage line in the developing synovial joint forming between the femoral head and acetabulum (hip joint). If this pattern of expression were observed at sites of joint formation elsewhere, it might explain the facial and limb abnormalities observed in the Cornelia de Lange syndrome.

Additional sections of human fetal face, head, limbs and mouse embryos were also examined. No expression was seen in any of the mouse tissues. Expression was only seen with the anti-sense probe.

Expression was observed adjacent to developing limb and facial bones in the periosteal mesenchyme. The expression was highly specific and was often adjacent to areas undergoing vascularization. The distribution is consistent with the observed skeletal abnormalities in the Cornelia de Lange syndrome. Expression was also observed in the developing temporal and occipital lobes of the fetal brain, but was not observed elsewhere. In addition, expression was seen in the ganglia of the developing inner ear.

(6) PRO342 (DNA38649)(IL-1 receptor homolog):

This DNA was expressed in many tissues and in many cell types. In the fetus, expression was seen in the inner aspect of the retina, in dorsal root ganglia, in small intestinal epithelium, thymic medulla and spleen. In the adult, expression was seen in epithelium of renal tubules, hepatocytes in the liver and urinary bladder. Expression was also present in infiltrating inflammatory cells and in an osteosarcoma. In chim, expression was seen on gastric epithelium, salivary gland and thymus. None of the other tissues examined showed evidence of specific expression.

Fetal tissues examined (E12-E16 weeks) included: liver, kidney, adrenals, lungs, heart, great vessels, oesophagus, stomach, spleen, gonad, spinal cord and body wall. Adult human tissues examined included: liver, kidney, stomach, bladder, prostate, lung, renal cell carcinoma, osteosarcoma, hepatitis and hepatic cirrhosis. Chimp tissues examined included: thyroid, nerve, tongue, thymus, adrenal gastric mucosa and salivary gland. Rhesus tissues examined included Rhesus brain.

In addition, eight squamous and eight adenocarcinomas of the lung were examined. Expression was observed in all tumors, although the level of expression was variable. Based on signal intensity, tumors were divided into high and low expressers. Three of the tumors (two adenocarcinomas: 96-20125 and 96-3686, and one squamous carcinoma: 95-6727) were categorized as high expressers. Moderate expression was also seen in normal benign bronchial epithelium and in lymphoid infiltrates, a finding consistent with previous observations that this receptor is widely expressed in most specimens.

EXAMPLE 28

Use of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202 PRO206, PRO264, PRO313, PRO342, PRO542, PRO773, PRO861, PRO1216, PRO1686, PRO1800. PRO3562, PRO9850, PRO539, PRO4316 or PRO4980 as a hybridization probe

The following method describes use of a nucleotide sequence encoding a PRO197, PRO207, PRO226, PRO232, PRO243, PRO256, PR0269, PRO274, PR0304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PR0861, PRO1216, PRO1686, PRO1800, PRO3562, PRO9850, PRP539, PRO4316 or PRO4980 polypeptide as a hybridization probe.

DNA comprising the coding sequence of a full-length or mature "PRO197", "PRO207", "PRO226", "PRO232". "PRO243". "PRO256", "PRO269", "PRO274", "PRO304", "PRO339", "PRO1558", "PR0779", "PRO1185", "PRO1245", "PRO1759", "PRO5775", "PRO7133", "PRO7168", "PRO5725", "PRO202", "PRO206", "PRO264", "PRO313", "PRO342", "PRO542", "PRO773", "PRO861", "PRO1216", "PRO1686", "PRO1800", "PRO3562", "PRO9850", "PRO539", "PRO4316" or "PRO4980" polypeptide as disclosed herein and/or fragments thereof may be employed as a probe to screen for homologous DNAs (such as those encoding naturally-occurring variants of PRO197, PR0207, PRO226, PR0232, PRO243, PRO256, PRO269, PRO274, PRO304, PRO339, PRO1558, PRO779, PRO1185, PRO1245, PRO1759, PRO5775, PRO7133, PRO7168, PRO5725, PR0202, PRO206, PRO264, PR0313, PRO342, PRO542, PRO773, PRO0861, PRO1216, PRO1686, PRO 1800, PR03562, PRO9850, PRO539, PRO4316 or PRO4980) in human tissue cDNA libraries or human tissue genomic libraries.

Hybridization and washing of filters containing either library DNAs is performed under the following thigh stringency conditions. Hybridization of radiolabeled PRO197-,PRO207-,PRO226-, PRO232-, PRO243-, PRO256-PRO269-, PRO274-, PRO304-, PR0339-, PRO1558-, PRO779-, PRO1185-, PRO1245-, PRO1759-, PRO5775-, PR07133-, PR07168-, PRO5725-. PRO202-, PRO206-, PRO264-, PRO313-, PRO342-, PRO542-, PR0773-, PRO861-, PRO1216-, PRO1686-, PRO1800-, PRO3562-, PR09850-, PRO539-, PRO4316- or PRO4980-derived probe to the filters is performed in a solution of 50% formamide, 5x SSC, 0.1 % SDS, 0.1% sodium pyrophosphate, 50 mM sodium phosphate, pH 6.8, 2x Denhardt's solution, and 10% dextran sulfate at 42˚C for 20 hours. Washing of the filters is performed in an aqueous solution of 0.1x SSC and 0.1% SDS at 42˚C.

DNAs having a desired sequence identity with the DNA encoding full-length native sequence PRO197, PRO207, PRO226, PRO232, PRO243, PRO256, PR0269, PR0274, PRO304, PRO339, PRO1558, PRO779, PRO1185, PRO1245, PRO1759, PRO5775, PR07133, PRO7168, PRO5725, PR0202, PRO206, PRO264, PR0313, PRO342, PRO542, PRO773, PRO861, PRO1216, PRO1686, PRO1800, PRO3562, PRO9850, PRO539, PRO4316 or PRO4980 can then be identified using standard techniques known in the art.

EXAMPLE 29

Expression of PRO197, PRO207, PRO226, PRO232, PRO243, PRO256, PRO269, PRO274, PRO304 PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 Polypeptides in *E. coli.*

This example illustrates preparation of an unglycosylated form of PRO197, PRO207, PRO226, PRO232, PRO243, PRO256, PR0269, PRO274, PR0304, PR0339, PRO1558, PRO779, PRO1185, PRO1245, PRO1759, PRO5775, PRO7133, PRO7168, PRO5725, PRO202, PR0206, PRO264, PR0313, PRO342, PRO542, PRO773, PRO861, PRO1216, PRO1686, PRO1800, PR03562, PRO9850, PRO539, PRO4316 or PR04980 by recombinant expression in *E. coli.*

The DNA sequence encoding the PRO polypeptide of interest is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli; see* Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a poly-His leader (including the first six STII codons, poly-His sequence, and enterokinase cleavage site), the PRO197, PRO207, PRO226, PRO232, PRO243, PRO256, PRO269, PRO274, PRO304, PRO339, PRO1558, PR0779, PRO1185, PRO1245, PRO1759, PRO5775, PR07133, PRO7168, PRO5725, PRO202, PRO206, PRO264, PRO313, PR0342, PRO542, PRO773, PRO861, PRO1216, PRO1686, PRO1800, PRO3562, PRO9850, PRO539, PRO4316 or PRO4980 coding region, lambda transcriptional terminator, and an argU gene.

The ligation mixture is then used to transform a selected *E. coli* strain using the methods described in Sambrook *et al., supra.* Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation The cell pellet obtained by the centrifugation can be solubilized using various agents known in the art, and the solubilized PRO197, PRO207, PRO226, PR0232, PRO243, PR0256, PRO269, PRO274, PRO304, PRO339, PRO1558, PR0779, PRO1185, PRO1245, PRO1759, PRO5775, PRO7133, PRO7168, PRO5725, PRO202, PRO206, PRO264. PR0313, PRO342, PRO542, PR0773, PRO861, PRO1216, PRO1686, PRO1800, PR03562, PR09850, PRO539, PRO4316 or PRO4980 protein can then be purified using a metal chelating column under conditions that allow tight binding of the protein.

PRO197, PRO207, PRO1185, PRO5725, PRO202, and PR03562 were successfully expressed in *E. coli* in a poly-His tagged form using the following procedure. The DNA encoding PRO197, PR0207, PRO1185, PRO5725, PRO202, and PR03562 was initially amplified using selected PCR primers. The primers contained restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences were then ligated into an expression vector, which was used to transform an *E. coli* host based on strain 52 (W3110 fuhA(tonA) Ion galE rpoHts(htpRts) clpP(lacIq). Transformants were first grown in LB containing 50 mg/ml carbenicillin at 30˚C with shaking until an O.D. of 3-5 at 600 nm was reached. Cultures were then diluted 50-100 fold into CRAP media (prepared by mixing 3.57 g $(NH_4)_2SO_4$, 0.71 g sodium citrate•$2H_2O$, 1.07 g KCl, 5.36 g Difco yeast extract, 5.36g Sheffield hycase SF in 500 ml water, as well as 110 mM MPOS, pH 7.3, 0.55% (w/v) glucose and 7 mM $MgSO_4$) and grown for approximately 20-30 hours at 30˚C with shaking. Samples were removed to verify expression by SDS-PAGE analysis, and the bulk culture was centrifuged to pellet the cells. Cell pellets were frozen until purification and refolding.

*E. coli* paste from 0.5 to 1 L fermentations (6-10 g pellets) was resuspended in 10 volumes (w/v) in 7 M guanidine, 20 mM Tris, pH 8 buffer. Solid sodium sulfite and sodium tetrathionate were added to make final concentrations of 0.1M and 0.02 M, respectively, and the solution was stirred overnight at 4˚C. This step results in a denatured protein with all cysteine residues blocked by sulfitolization. The solution was centrifuged at 40,000 rpm in a Beckman Ultracentifuge for 30 min. The supernatant was diluted with 3-5 volumes of metal chelate column buffer (6 M guanidine, 20 mM Tris. pH 7.4) and filtered through 0.22 micron filters to clarify. The clarified extract was loaded onto a 5 ml Qiagen Ni $^{2+}$-NTA metal chelate column equilibrated in the metal chelate column buffer. The column was washed with additional buffer containing 50 mM imidazole (Calbiochem, Utrol grade), pH 7.4. The proteins were eluted with buffer containing 250 mM imidazole. Fractions containing the desired protein were pooled and stored at 4 ˚C. Protein concentration was estimated by its absorbance at 280 nm using the calculated extinction coefficient based on its amino acid sequence.

The protein was refolded by diluting sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes were chosen so that the final protein concentration was between 50 to 100 micrograms/ml. The refolding solution was stirred gently at 4 ˚C for 12-36 hours. The refolding reaction was quenched by the addition of TFA to a final concentration of 0.4% (pH of approximately 3). Before further purification of the protein, the solution was filtered through a 0.22 micron filter and acetonitrile was added to 2-10% final concentration. The refolded protein was chromatographed on a Poros R1/H reversed phase column using a mobile buffer of 0.1% TFA with elution with a gradient of acetonitrile from 10 to 80%. Aliquots of fractions with $A_{280}$ absorbance were analyzed on SDS polyacrylamide gels and fractions containing homogeneous refolded protein were pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO197. PRO207, PRO1185, PR05725, PRO202, and PR03562 protein were pooled and the acetonitrile removed using a gentle stream of nitrogen directed at the solution. Proteins were formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

EXAMPLE 30

Expression of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542 PRO773, PRO861, PRO1216, PRO1686, PRO1800, PRO3562, PRO9850, PRO539, PRO4316 or PRO4980 in mammalian cells

This example illustrates preparation of a potentially glycosylated form of PRO197, PRO207, PRO226. PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PR0542, PRO773, PRO861, PRO1216, PRO1686, PRO1800, PRO3562, PRO9850, PRO539, PRO4316 or PRO4980 by recombinant expression in mammalian cells.

The vector, pRK5 (*see* EP 307,247, published March 15, 1989), is employed as the expression vector. Optionally, the PRO197, PRO207, PRO226, PR0232, PRO243, PRO256, PRO269, PRO274, PRO304, PRO339, PRO1558, PR0779, PRO1185, PRO1245, PRO1759, PRO5775, PRO7133, PRO7168, PRO5725, PR0202, PRO206, PRO264, PRO313, PRO342, PRO542, PRO773, PRO861, PRO1216, PRO1686, PRO1800, PRO3562, PR09850, PRO539, PRO4316 or PRO4980 DNA is ligated into pRK5 with selected restriction enzymes to allow insertion of the PRO197, PRO207, PRO226, PRO232, PRO243, PRO256, PRO269, PRO274, PRO304, PRO339, PRO1558, PR0779, PRO1185, PRO1245, PRO1759, PR05775, PR07133, PR07168, PR05725, PRO202, PRO206, PRO264, PRO313, PRO342, PRO542, PRO773, PRO861, PRO1216, PRO1686, PRO1800, PRO3562, PRO9850, PRO539, PRO4316 or PR04980 DNA using ligation methods such as described in Sambrook *et al., supra.* The resulting vector is called pRK5-PRO197, pRK5-PRO207, pRK5-PRO226, pRK5-PRO232, pRK5-PRO243, pRK5-PRO256, pRK5-PRO269, pRK5-PRO274, pRK5-PRO304, pRK5-PRO339, pRK5-PRO1558, pRK5-PRO779, pRK5-PRO1185, pRK5-PRO1245, pRK5-PRO1759, pRK5-PRO5775, pRK5-PRO7133, pRK5-PRO7168, pRK5-PRO5725, pRK5-PRO202, pRK5-PRO206, pRK5-PRO264, pRK5-PRO313, pRK5-PRO342, pRK5-PRO542, pRK5-PRO773, pRK5-PRO861, pRK5-PRO1216, pRK5-PRO1686, pRK5-PRO1800, pRK5-PRO3562, pRK5-PRO9850, pRK5-PRO539, pRK5-PRO4316 or pRK5-PRO4980.

In one embodiment, the selected host cells may be 293 cells. Human 293 cells (ATCC CCL 1573.) are grown to confluence in tissue culture plates in medium such as DMEM supplemented with fetal calf serum and optionally, nutrient components and/or antibiotics. About $10\mu g$ pRK5-PRO197, pRK5-PRO207, pRK5-PRO226, pRK5-PRO231, pRK5-PRO243, pRK5-PRO256, pRK5-PRO269, pRK5-PRO274, pRK5-PRO304, pRK5-PRO339, pRK5-PRO1558, pRK5-PRO779, pRK5-PRO1185, pRK5-PRO1245, pRK5-PRO1759, pRK5-PRO5775, pRK5-PRO7133, pRK5-PRO7168, pRK5-PRO5725, pRK5-PRO202, pRK5-PRO206, pRK5-PRO264, pRK5-PRO313, pRK5-PRO342, pRK5-PRO542, pRK5-PRO773, pRK5-PRO861, pRK5-PRO1216, pRK5-PRO1686, pRK5-PRO1800, pRK5-PRO3562, pRK5-PRO9850, pRK5-PRO539, pRK5-PRO4316 or pRK5-PRO4980 DNA is mixed with about 1 $\mu g$ DNA encoding the VA RNA gene [Thimmappaya et al., Cell, 31:543 (1982)] and dissolved in 500 $\mu l$ of 1 mM Tris-HCl, 0.1 mM EDTA, 0.227 M $CaCl_2$. To this mixture is added, dropwise, 500 $\mu l$ of 50 mM HEPES (pH 7.35), 280 mM NaCl, 1.5 mM $NaPO_4$, and a precipitate is allowed to form for 10 minutes at 25˚C. The precipitate is suspended and added to the 293 cells and allowed to settle for about four hours at 37˚C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium(alone) or culture medium containing 200 $\mu Ci/ml$ $^{35}$S-cysteine and 200 $\mu Ci/ml$ $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of the PRO197, PRO207. PRO226, PRO232, PRO243, PRO256, PRO269, PRO274. PR0304, PR0339, PRO1558, PRO779. PRO1185, PRO1245, PRO1759, PRO5775, PRO7133, PRO7168. PRO5725, PRO202, PRO206, PRO264, PRO313, PRO342, PRO542, PRO773, PRO861, PRO1216, PRO1686, PRO1800, PRO3562, PRO9850, PRO539, PRO4316 or PR04980 polypeptide. The cultures containing transfected cells may undergo further incubation (in serum free medium) and the medium is tested in selected bioassays.

In an alternative technique, PRO197, PRO207, PRO226, PRO232, PRQO243, PRO256, PRO269, PRO274, PRO304, PRO339, PRO1558, PRO779, PRO1185, PRO1245, PRO1759, PRO5775, PRO7133, PRO7168, PR05725, PRO202, PRO206, PRO264, PR0313, PR0342, PR0542, PRO773, PR0861, PRO1216, PRO1686, PRO1800, PR03562, PRO9850, PR0539, PR04316 or PRO4980 DNA may be introduced into 293 cells transiently using the dextran sulfate method described by Somparyrac et al., Proc. Natl. Acad. Sci.,12:7575 (1981). 293 cells are grown to maximal density in a spinner flask and 700 $\mu g$ pRK5-PRO197, pRK5-PRO207, pRK5-PRO226, pRK5-PRO232, pRK5-PRO243, pRK5-PRO256, pRK5-PRO269, pRK5-PRO274, pRK5-PRO304, pRK5-PRO339, pRK5-PRO1558, pRK5-PRO779, pRK5-PRO1185, pRK5-PRO1245, pRK5-PRO1759, pRK5-PRO5775, pRK5-PRO7133, pRK5-PRO7168, pRK5-PRO5725, pRK5-PRO202, pRK5-PRO206, pRK5-PRO264, pRK5-PRO313, pRK5-PRO342, pRK5-PRO542, pRK5-PRO773, pRK5-PRO861, pRK5-PRO1216, pRK5-PRO1686, pRK5-PRO1800, pRK5-PRO3562, pRK5-PRO9850, pRK5-PRO539, pRK5-PRO4316 or pRK5-PRO4980 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 $\mu g/ml$ bovine insulin and 0.1 $\mu g/ml$ bovine transferrin. After about four days, the conditioned media is centrifuged and filtered to remove cells and debris. The sample containing expressed PRO197, PRO207, PR0226, PRO232, PRO243, PRO256, PRO269, PRO274, PRO304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PR0773, PR0861, PRO1216, PRO 1686, PRO 1800, PR03562, PRO9850, PRO539, PRO4316 or PRO4980 can then be concentrated and purified by any selected method, such as dialysis and/or column chromatography.

In another embodiment PRO197, PR0207, PR0226, PRO232, PRO243, PRO256, PRO269, PR0274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PR04316 or PRO4980 can be expressed in CHO cells. The pRK5-PRO197. pRK5-PRO207, pRK5-PRO226, pRK5-PRO232, pRK5-PRO243, pRK5-PRO256, pRK5-PRO269, pRK5-PRO274, pRK5-PRO304, pRK5-PRO339, pRK5-PRO1558, pRK5-PRO779, pRK5-PRO1185, pRK5-PRO1245, pRK5-PRO1759, pRK5-PRO5775, pRK5-PRO7133, pRK5-PRO7168, pRK5-PRO5725, pRK5-PRO202, pRK5-PRO206, pRK5-PRO264, pRK5-PRO313, pRK5-PRO342, pRK5-PRO542, pRK5-PRO773, pRK5-PRO851, pRK5-PRO1216, pRK5-PRO1686, pRK5-PRO1800, pRK5-PRO3562, pRK5-PRO9850, pRK5-PRO539, pRK5-PRO4316 or pRK5-PRO4980 vector can be transfected into CHO cells using known reagents such as $CaPO_4$ or DEAE-dextran. As described above, the cell cultures can be incubated, and the medium replaced with culture medium (alone) or medium containing a radiolabel such as $^{35}$S-methionine. After determining

the presence of the PRO197, PRO207, PRO226, PRO232, PRO243, PRO256, PRO269, PRO274, PRO304, PRO339, PRO1558, PR0779, PRO1185, PRO1245, PRO1759, PRO5775, PR07133, PRO7168, PRO5725, PR0202, PR0206, PRO264, PRO313, PRO342, PRO542, PRO773, PRO861, PRO1216, PRO 1686, PRO1800, PRO3562, PRO9850, PRO539, PR04316 or PRO4980 polypeptide, the culture medium may be replaced with serum free medium. Preferably, the cultures are incubated for about 6 days, and then the conditioned medium is harvested. The medium containing the expressed PRO197, PR0207, PR0226, PR0232, PRO243, PRO256, PRO269, PRO274, PR0304, PRO339, PRO1558, PRO779, PRO1185, PRO1245, PRO1759, PRO5775, PRO7133, PRO7168, PRO5725, PRO202, PR0206, PRO264, PRO313, PRO342, PR0542, PRO773, PRO861, PRO1216, PRO1686, PRO1800, PRO3562, PRO9850, PR0539, PRO4316 or PR04980 can then be concentrated and purified by any selected method.

Epitope-tagged PRO197, PR0207, PR0226, PRO232, PR0243, PRO256. PRO269, PRO274, PRO304, PR0339, PRO1558, PR0779, PRO1185, PRO1245, PRO1759, PRO5775, PRO7133, PRO7168, PRO5725. PRO202, PRO206, PRO264, PR0313, PRO342, PR0542, PR0773, PRO861, PRO1216, PRO1686, PRO1800, PRO3562, PRO9850. PR0539, PR04316 or PR04980 may also be expressed in host CHO cells. The PRO1977, PRO207, PRO226, PRO232, PR0243, PRO256, PRO269, PRO274, PRO304, PR0339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PR04316 or PRO4980 may be subcloned out of the pRK5 vector. The subclone insert can undergo PCR to fuse in frame with a selected epitope tag such as a poly-His tag into a Baculovirus expression vector. The poly-His tagged PRO197, PRO207, PRO226, PRO237, PRO243, PRO256, PRO269, PRO274, PRO304, PRO339, PRO1558, PRO779, PRO1185, PRO1245, PRO1759, PRO5775, PRO7133, PR07168, PRO5725, PRO202, PRO206, PRO264, PRO313, PRO342, PRO542, PRO773, PRO861, PRO1216, PRO1686, PRO1800, PRO3562, PRO9850. PRO539, PRO4316 or PR04980 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO197, PRO207, PRO226, PR0232, PRO243. PRO256, PRO269, PRO274, PRO304, PR0339, PRO1558, PRO779, PRO1185, PRO 1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 can then be concentrated and purified by any selected method, such as by $Ni^{2+}$-chelate affinity chromatography. Expression in CHO and/or COS cells may also be accomplished by a transient expression procedure.

PRO197, PRO226, PRO256, PRO202, PRO264, PRO542, PRO773 and PRO861 were expressed in CHO cells by a stable expression procedure, whereas PRO256, PRO264 and PRO861 were expressed in CHO cells by a transient procedure. Stable expression in CHO cells was performed using the following procedure. The proteins were expressed as an IgG construct (immunoadhesin), in which the coding sequences for the soluble forms (e.g.. extracellular domains) of the respective proteins were fused to an IgG1 constant region sequence containing the hinge, CH2 and CH2 domains and/or in a poly-His tagged form.

Following PCR amplifications, the respective DNAs were subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used for expression in CHO cells is as described in Lucas et al., Nucl. Acids Res., 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA were introduced into approximately 10 million CHO cells using commercially available transfection reagents Superfect® (Qiagen), Dosper® or Fugene® (Boehringer Mannheim). The cells were grown as described in Lucas *et al., supra.* Approximately 3 x 10' cells are frozen in an ampule for further growth and production as described below.

The ampules containing the plasmid DNA were thawed by placement into a water bath and mixed by vortexing. The contents were pipetted into a centrifuge tube containing 10 mls of media and centrifuged at 1000 rpm for 5 minutes. The supernatant was aspirated and the cells were resuspended in 10 ml of selective media (0.2 $\mu$m filtered PS20 with 5% 0.2 $\mu$m diafiltered fetal bovine serum). The cells were then aliquoted into a 100 film spinner containing 90 ml of selective media. After 1-2 days, the cells were transferred into a 250 ml spinner filled with 150 ml selective growth medium and incubated at 37°C. After another 2-3 days. 250 mL 500 ml and 2000 ml spinners were seeded with $3x10^5$ cells/ml. The cell media was exchanged with fresh media by centrifugation and resuspension in production medium. Although any suitable CHO media may be employed, a production medium described in US Patent No. 5,122,469, issued June 16.1992 was actually used. 3L production spinner was seeded at 1.2 x $10^6$ cells/ml. On day 0, the cell number and pH were determined. On day 1, the spinner was sampled and sparging with filtered air was commenced. On day 2, the spinner was sampled, the temperature shifted to 33°C, and 30 ml of 500 g/L glucose and 0.6 ml of 10% antifoam (e.g., 35% polydimethylsiloxane emulsion, Dow Corning 365 Medical Grade Emulsion) added. Throughout the production, the pH was adjusted as necessary to keep at around 7.2. After 10 days, or until viability dropped below 70%, the cell culture was harvested by centrifugation and filtered through a 0.22 $\mu$m filter. The filtrate was either stored at 4˚C or immediately loaded onto columns for purification.

For the poly-His tagged constructs, the proteins were purified using a Ni $^{2+}$-NTA column (Qiagen). Before purification, imidazole was added to the conditioned media to a concentration of 5 mM. The conditioned media was pumped onto a 6 ml Ni $^{2+}$NTA column equilibrated in 20 mM Hepes, pH 7.4, buffer containing 0.3 M NaCl and 5 mM imidazole at a flow rate of 4-5 ml/min. at 4˚C. After loading, the column was washed with additional equilibration buffer and the protein eluted with equilibration buffer containing 0.25 M imidazole. The highly purified protein was subsequently desalted into a storage buffer containing 10 mM Hepes, 0.14 M NaCl and 4% mannitol, pH 6.8, with a 25 ml G25 Superfine (Pharmacia) column and stored at -80˚C.

Immunoadhesin (Fc containing) constructs were purified from the conditioned media as follows. The conditioned medium was pumped onto a 5 ml Protein A column (Pharmacia) which had been equilibrated in 20 mM Na phosphate buffer, pH 6.8. After loading, the column was washed extensively with equilibration buffer before elution with 100 mM citric acid, pH 3.5. The eluted protein was immediately neutralized by collecting 1 ml fractions into tubes containing 275 $\mu$l of 1 M Tris buffer, pH 9. The highly purified protein was subsequently desalted into storage buffer as described above for the poly-His tagged proteins. The homogeneity was assessed by SDS polyacrylamide gels and by N-terminal amino acid sequencing by Edman degradation.

EXAMPLE 32

Expression of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 in Yeast

The following method describes recombinant expression of PRO197, PRO207, PRO226, PRO232, PRO243, PRO256, PRO269, PR0274, PR0304, PR0339, PRO1558, PR0779, PRO1185, PRO1245, PRO1759, PRO5775, PR07133, PRO7168, PRO5725, PRO202, PRO206, PRO264, PR0313, PRO342, PRO542, PRO773, PRO861, PRO1216, PRO1686, PRO1800, PRO3562, PR09850, PRO539, PRO4316 or PRO4980 in yeast

First, yeast expression vectors are constructed for intracellular production or secretion of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PR07168, PRO5725, PRO202, PR0206, PR0264, PR0313, PRO342, PRO542, PR0773, PRO861, PRO1216, PRO 1686, PRO 1800, PRO3562, PRO9850, PRO539, PR04316 or PRO4980 from the ADH2/GAPDH promoter. DNA encoding PRO197, PRO207, PRO226, PR0232, PRO243, PR0256, PR0269, PR0274, PRO304, PR0339, PRO1558, PR0779, PRO1185, PRO1245, PRO1759, PRO5775, PR07133, PRO7168, PRO5725, PRO202, PRO206, PRO264, PR0313, PRO342, PRO542, PRO773. PRO861,PRO1216, PRO1686, PRO1800, PRO3562, PRO9850, PR0539, PRO4316 or PRO4980 and the promoter is inserted into suitable restriction enzyme sites in the selected plasmid to direct intracellular expression of PRO197, PRO207, PR0226, PR0232, PR0243, PRO256, PRO269, PRO274, PR0304, PR0339, PRO1558, PR0779, PRO1185, PRO1245, PRO1759, PRO5775, PRO7133, PRO7168, PR05725, PRO202, PRO206, PR0264, PRO313, PRO342, PR0542, PRO773, PRO861, PRO1216, PRO1686, PRO1800, PR03562, PRO9850, PR0539, PR04316 or PRO4980, For secretion, DNA encoding PRO197, PRO207, PR0226, PRO232, PRO243, PRO256, PRO269, PRO274, PRO304, PRO339, PRO1558, PRO779, PRO1185, PRO1245, PRO1759, PR05775, PR07133, PRO7168, PRO5725, PRO202, PRO206, PRO264, PRO313, PRO342, PRO542, PRO773, PRO861, PRO1216, PRO1686, PRO1800, PR03562, PRO9850, PR0539, PR04316 or PRO4980 can be cloned into the selected plasmid, together with DNA encoding the ADH2/GAPDH promoter, a native PRO197, PRO207, PRO226, PRO232, PRO243, PRO256, PR0269, PR0274, PRO304, PRO339, PRO1558, PRO779, PRO1185, PRO1245, PRO1759, PRO5775, PRO7133, PR07168, PRO5725, PR0202, PRO206, PRO264, PR0313, PR0342, PR0542, PR0773, PRO861, PRO1216, PRO1686, PRO1800, PRO3562, PR09850, PRO539, PR04316 or PRO4980 signal peptide or other mammalian signal peptide, or, for example, a yeast alpha-factor or invertase secretory signal/leader sequence, and linker sequences (if needed) for expression of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PR0313, PRO342, PRO542, PRO773, PRO861, PRO1216, PRO1686, PRO1800, PRO3562, PRO9850, PRO539, PRO4316 or PR04980.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE. followed by staining of the gels with Coomassie Blue stain.

Recombinant PRO197, PRO0207, PRO226, PRO232, PRO243, PRO256, PRO269, PRO274, PR0304, PRO339, PRO1558, PRO779, PRO1185, PRO1245, PRO1759, PRO5775, PRO7133, PRO7168, PRO5725, PRO202, PRO206, PRO264, PR0313, PRO342, PR0542, PR0773, PRO861, PRO1216, PRO1686, PRO1800, PR03562, PR09850, PRO539, PRO4316 or PR04980 can subsequently be isolated and purified by removing the yeast cells from the fermentation medium by centrifugation and then concentrating the medium using selected cartridge filters. The concentrate containing PRO197, PRO207, PRO226, PRO232, PRO243, PRO256, PRO269, PRO274, PR0304, PR0339, PRO1558, PRO779, PRO1185, PRO1245, PRO1759, PRO5775, PRO7133, PR07168, PRO5725, PRO202, PRO206, PRO264, PR0313, PRO342, PRO542, PRO773, PRO861, PRO1216, PRO1686, PRO1800, PRO3562, PRO9850, PRO539, PRO4316 or PRO4980 may further be purified using selected column chromatography resins.

EXAMPLE 33

Expression of PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980 in Baculovirus-infected Insect Cells

The following method describes recombinant expression in Baculovirus-infected insect cells.

The sequence coding for PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PR05725, PRO202, PRO206, PRO264, PR0313, PRO342, PR0542, PR0773, PRO861, PRO1216, PRO1686, PRO1800, PR03562, PRO9850, PRO539, PRO4316 or PR04980 is fused upstream of an epitope tag contained within a baculovirus expression vector. Such epitope tags include poly-His tags and immunoglobulin tags (like Fc regions of IgG). A variety of plasmids may be employed, including plasmids derived from commercially available plasmids such as pVL1393 (Novagen). Briefly, the sequence encoding PRO197, PR0207, PRO226, PRO232, PRO243, PRO256, PRO269, PRO274, PRO304, PRO339, PRO1558, PRO779, PRO1185, PRO1245, PRO1759, PR05775, PR07133, PRO7168, PRO5725, PRO202, PR0206, PR0264, PR0313, PRO342. PR0542, PRO773. PRO861, PRO1216, PRO1686, PRO1800, PRO3562, PRO9850, PRO539, PRO4316 or PRO4980 or the desired portion of the coding sequence of PRO197, PRO207, PR226, PRO232, PRO243, PRO256, PRO269, PRO274, PR0304, PR0339, PRO1558, PR0779, PRO1185, PRO1245, PRO1759, PRO5775, PRO7133, PRO7168, PRO5725, PRO202, PRO206, PRO264, PR0313, PR0342, PRO542, PR0773, PRO861, PRO1216, PRO1686, PRO1800, PRO3562, PRO9850, PRO539, PRO4316 or PRO4980 [such as the sequence encoding the extracellular domain of a transmembrane protein or the sequence encoding the mature protein if the protein is extracellular] is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodopterafrugiperda* ("Sf9") cells (ATCC CRL 1711) using lipofectin (commercially available from GIBCO-BRL). After 4 - 5 days of incubation at 28˚C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-His tagged PRO197, PRO207, PRO226, PRO232, PRO243, PR0256, PR0269, PR0274, PR0304, PR0339, PRO1558, PRO779, PRO1185, PRO1245, PRO1759, PRO5775, PRO7133, PR07168, PRO5725. PRO202, PR0206, PRO264, PR0313, PRO342, PRO542, PRO773, PRO861, PRO1216, PRO1686, PRO1800, PRO3562, PRO9850, PRO539, PRO4316 or PRO4980 can then be purified, for example, by $Ni^{2+}$-chelate affinity chromatography as follows. Extracts are prepared from recombinant virus-infected Sf9 celts as described by Rupert et al., Nature 362:175-179 (1993). Briefly, Sf9 cells are washed, resuspended in sonication buffer (25 ml Hepes, pH 7.9; 12.5 mM $MgCl_2$; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate. 300 mM NaCl, 10% glycerol, pH 7.8) and filtered through a 0.45 $\mu$m filter. A $Ni^{2+}$-NTA agarose column (commercially available from Qiagen) is prepared with a bed volume of 5 ml, washed with 25 ml of water and equilibrated with 25 ml of loading buffer. The filtered cell extract is loaded onto the column at 0.5 ml per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM imidazole gradient in the secondary wash buffer. One ml fractions are collected and analyzed by SDS-PAGE and silver staining or Western blot with $Ni^{2+}$-NTA-conjugated to alkaline phosphatase (Qiagen). Fractions containing the eluted $His_{10}$-tagged PRO197, PRO207, PRO226, PRO232, PRO243, PRO256. PR0269, PR0274, PR0304, PRO339, PRO1558, PRO779, PRO1185, PRO1245, PRO1759, PRO5775, PRO7133, PRO7168, PRO5725, PR0202, PRO206, PRO264, PR0313, PRO342, PRO542, PRO773, PRO861, PRO1216, PRO1686, PRO1800, PR03562, PRO9850, PRO539, PRO4316 or PR04980, respectively, are pooled and dialyzed against loading buffer.

Alternatively, purification of the IgG tagged (or Fc tagged) PRO197, PRO207, PR0226, PRO232, PR0243, PRO256, PR0269, PR0274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PR03562, PRO9850, PRO539, PRO4316 or PRO4980 can be performed using known chromatography techniques, including for instance, Protein A or protein G column chromatography.

While expression is actually performed in a 0.5-2 L scale, it can be readily scaled up for larger (e.g., 8 L) preparations. The proteins are expressed as an IgG construct (immunoadhesin), in which the protein extracellular region is fused to an IgG 1 constant region sequence containing the hinge, CH2 and CH3 domains and/or in poly His tagged forms.

Following PCR amplification, the respective coding sequences are subcloned into a baculovirus expression vector (pb.PH.IgG for IgG fusions and pb.PH.His.c for poly-His tagged proteins), and the vector and Baculogold® baculovirus DNA (Pharmingen) are co-transfected into 105 *Spodoptera frugiperda* ("Sf9") cells (ATCC CRL 1711), using Lipofectin (Gibco BRL). pb.PH.IgG and pb.PH.His are modifications of the commercial available baculovirus expression vector pVL1393 (Pharmingen), with modified polylinker regions to include the His or Fc tag sequences. The cells are grown in Hink's TNM-FH medium supplemented with 10% FBS (Hyclone). Cells are incubated for 5 days at 28°C. The supernatant is harvested and subsequently used for the first viral amplification by infecting Sf9 cells in Hink's TNM-FH medium supplemented with 10% FBS at an approximate multiplicity of infection (MOI) of 10. Cells are incubated for 3 days at 28°C. The supernatant is harvested and the expression of the constructs in the baculovirus expression vector is determined by batch binding of l ml of supernatant to 25 ml of Ni $^{2+}$-NTA beads (QIAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The first viral amplification supernatant is used to infect a spinner culture (500 ml) of Sf9 cells grown in ESF-921 medium (Expression Systems LLC) at an approximate MOI of 0.1. Cells are incubated for 3 days at 28°C. The supernatant is harvested and filtered. Batch binding and SDS-PAGE analysis are repeated, as necessary, until expression of the spinner culture is confirmed.

The conditioned medium from the transfected cells (0.5 to 3 L) is harvested by centrifugation to remove the cells and filtered through 0.22 micron filters. For the poly-His tagged constructs, the protein construct is purified using a Ni $^{2+}$-NTA column (Qiagen). Before purification, imidazole is added to the conditioned media to a concentration of 5 mM. The conditioned media is pumped onto a 6 ml Ni $^{2+}$-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc containing) constructs of proteins are purified from the conditioned media as follows. The conditioned media is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 ml of 1 M Tris buffer, pH 9. The highly purified protein is subsequently desalted into storage buffer as described above for the poly-His tagged proteins. The homogeneity of the proteins is verified by SDS polyacrylamide gel (PEG) electrophoresis and N-terminal amino acid sequencing by Edman degradation.

PRO256, PRO269, PRO1245, PR0264 and PRO542 were expressed in Baculovirus -infected Sf9 insect cells by the above procedure.

Alternatively, a modified baculovirus procedure may be used incorporating high 5 cells. In this procedure, the DNA encoding the desired sequence is amplified with suitable systems, such as Pfu (Stratagene), or fused upstream (5'-of) of an epitope tag contained with a baculovirus expression vector. Such epitope tags include poly-His tags and immunoglobulin tags (like Fc regions of IgG). A variety of plasmids may be employed, including plasmids derived from commercially available plasmids such as pIE1-1 (Novagen). The pIE1-1 and pIE1-2 vectors are designed for constitutive expression of recombinant proteins from the baculovirus ie1 promoter in stably-transformed insect cells. The plasmids differ only in the orientation of the multiple cloning sites and contain all promoter sequences known to be important for ie1-mediated gene expression in uninfected insect cells as well as the hr5 enhancer element. pIE1-1 and pIE1-2 include the translation initiation site and can be used to produce fusion proteins. Briefly, the desired sequence or the desired portion of the sequence (such as the sequence encoding the extracellular domain of a transmembrane protein) is amplified by PCR with primers complementary to the 5' and 3' regions. The 5' primer may incorporate flanking (selected) restriction enzyme sites. The product is then digested with those selected restriction enzymes and subcloned into the expression vector.. For example, derivatives of pIE1-1 can include the Fc region of human IgG (pb.PH.IgG) or an 8 histidine (pb.PH.His) tag downstream (3'-of) the desired sequence. Preferably, the vector construct is sequenced for confirmation.

High 5 cells are grown to a confluency of 50% under the conditions of 27 °C, no CO$_2$, NO pen/strep. For each 150 mm plate, 30 $\mu$g of pIE based vector containing the sequence is mixed with 1 ml Ex-Cell medium (Media: Ex-Cell 401 + 1/100 L-Glu JRH Biosciences #14401-78P (note: this media is light sensitive)), and in a separate tube, 100 μl of CellFectin (CellFECTIN (GibcoBRL#10362-010) (vortexed to mix)) is mixed with 1 ml of Ex-Cell medium. The two solutions are combined and allowed to incubate at room temperature for 15 minutes. 8 ml of Ex-Cell media is added to the 2 ml of DNA/CellFECTIN mix and this is layered on high 5 cells that have been washed once with Ex-Cell media. The plate is then incubated in darkness for 1 hour at room temperature. The DNA/CellFECTIN mix is then aspirated, and the cells are washed once with Ex-Cell to remove excess CellFECTIN, 30 ml of fresh Ex-Cell media is added and the cells are incubated for 3 days at 28˚C. The supernatant is harvested and the expression of the sequence in the baculovirus expression vector is determined by batch binding of 1 ml of supernatant to 25 ml of Ni $^{2+}$-NTA beads (QIAGEN) for histidine tagged proteins or Protein-A Sepharose CL-4B beads (Pharmacia) for IgG tagged proteins followed by SDS-PAGE analysis comparing to a known concentration of protein standard by Coomassie blue staining.

The conditioned media from the transfected cells (0.5 to 3 L) is harvested by centrifugation to remove the cells and filtered through 0.22 micron filters. For the poly-His tagged constructs, the protein comprising the sequence is purified using a Ni$^{2+}$-NTA column (Qiagen). Before purification, imidazole is added to the conditioned media to a concentration of 5 mM. The conditioned media is pumped onto a 6 ml Ni$^{2+}$-NTA column equilibrated in 20 mM Hepes, pH 7.4, buffer containing 0.3 M NaCl and 5 mM imidazole at a flow rate of 4-5 ml/min. at 48˚C. After loading, the column is washed with additional equilibration buffer and the protein eluted with equilibration buffer containing 0.25 M imidazole. The highly purified protein is then subsequently desalted into a storage buffer containing 10 mM Hepes, 0.14 M NaCl and 4% mannitol, pH 6.8, with a 25 ml G25 Superfine (Pharmacia) column and stored at -80˚C.

Immunoadhesin (Fc containing) constructs of proteins are purified from the conditioned media as follows. The conditioned media is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 ml of 1 M Tris buffer, pH 9. The highly purified protein is subsequently desalted into storage buffer as described above for the poly-His tagged proteins. The homogeneity of the sequence is assessed by SDS polyacrylamide gels and by N-terminal amino acid sequencing by Edman degradation and other analytical procedures as desired or necessary.

PRO226, PRO232, PRO243. PRO269, PR0779, PRO202, PR0542 and PR0861 were successfully expressed by the above modified baculovirus procedure incorporating high 5 cells.

EXAMPLE 34

Preparation of Antibodies that Bind PRO197, PRO207, PRO226, PRO232, PRO243, PRO256, PRO269, PRO274, PRO304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980

This example illustrates preparation of monoclonal antibodies which can specifically bind PRO197, PR0207, PRO226, PRO232, PRO243, PRO256, PR0269, PRO274, PR0304, PRO339, PRO1558, PR0779, PRO1185, PRO1245, PRO1759. PRO5775, PRO7133, PRO7168, PR05725, PRO202, PRO206, PRO264, PRO313, PRO342, PR0542, PR0773, PRO861, PRO1216, PRO1686, PRO1800, PRO3562, PR09850, PR0539, PRO4316 or PRO4980.

Techniques for producing the monoclonal antibodies are known in the art and are described, for instance, in Goding, *supra.* Immunogens that may be employed include purified PRO197, PRO207, PRO226, PRO232, PRO243, PRO256, PR0269, PR0274, PR0304, PRO339, PRO1558, PRO779, PRO1185, PRO1245, PRO1759, PRO5775, PR07133, PR07168, PRO5725, PR0202, PRO206, PRO264, PR0313, PRO342, PRO542, PR0773, PRO861, PRO1216, PRO1686, PRO1800, PR03562, PR09850, PR0539, PR04316 or PR04980 fusion proteins containing PRO197, PRO207, PRO226, PRO232, PRO243, PRO256, PRO269, PRO274, PR0304, PR0339, PRO1558, PRO779, PRO1185. PRO1245, PRO1759, PRO5775, PRO7133, PRO7168, PRO5725, PRO202, PRO206, PRO264, PRO313, PRO0342, PR0542, PRO773, PR0861, PRO1216, PRO1686, PRO1800, PR03562, PRO9850, PRO539, PR04316 or PR04980 and cells expressing recombinant PRO197, PRO207, PR0226, PRO232, PRO243, PRO256, PR0269, PR0274, PR0304, PR0339, PRO1558, PR0779, PRO1185, PRO1245, PRO1759, PR05775, PRO7133, PRO7168, PR05725, PR0202, PR0206, PR0264, PR0313, PR0342, PR0542, PRO773, PRO861, PRO1216, PRO1686, PRO1800, PR03562, PR09850, PRO539, PR04316 or PR04980 on the cell surface. Selection of the immunogen can be made by the skilled artisan without undue experimentation.

Mice, such as Balb/c, are immunized with the PRO197, PRO207, PRO226, PRO232, PRO243, PRO256, PRO269, PRO274, PR0304, PR0339, PRO1558, PR0779, PRO1185, PRO1245, PRO1759, PRO5775, PRO7133, PR07168, PR05725, PRO202, PRO206, PR0264, PRO0313, PRO342, PRO542, PR0773, PRO861, PRO1216, PRO1686, PRO1800, PR03562, PR09850, PR0539, PRO4316 or PRO4980 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO197, anti-PRO207, anti-PRO226, anti-PRO232, anti-PRO243, anti-PRO256, anti-PR0269, anti-PRO274, anti-PRO304, anti-PRO339, anti-PRO1558, anti-PRO779, anti-PRO1185, anti-PRO1245, anti-PRO1759, anti-PRO5775, anti-PR07133, anti-PR07168, anti-PRO5725, anti. PRO202, anti-PRO206, anti-PRO264, anti-PRO313, anti-PR0342, anti-PRO542, anti-PRO773, anti-PRO861, anti-PRO1216, anti-PRO1686, anti-PRO1800, anti-PRO3562, anti-PRO9850, anti-PRO539, anti-PRO4316 or anti-PRO4980 antibodies.

After a suitable antibody titer has been detected, the animals "positive" for antibodies can be injected with a final intravenous injection of PRO197, PRO207, PR0226, PRO232, PRO243, PRO256, PR0269, PRO274, PR0304, PRO339, PRO1558, PRO779, PRO1185, PRO1245, PRO1759, PRO5775, PRO7133, PRO7168, PRO5725, PRO202, PRO206, PRO264, PRO313, PRO342, PRO542, PRO773, PRO861, PRO1216, PRO1686, PRO1800, PRO3562, PRO9850, PRO539, PRO4316 or PRO4980. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO197, PRO207, PR0226, PRO232, PRO243, PRO256, PRO269, PRO274, PR0304, PR0339, PRO1558, PRO779, PRO1185, PRO1245, PRO1759, PRO5775, PR07133, PR07168, PR05725, PRO202, PRO206, PRO264, PRO313, PRO342, PRO542, PRO773, PRO861, PRO1216, PRO1686, PRO1800, PRO3562, PRO9850, PRO539, PR04316 or PR04980. Determination of "positive" hybridoma cells secreting the desired monoclonal antibodies against PRO197, PRO207. PRO226, PRO232, PRO243, PRO256, PRO269, PRO274, PR0304, PR0339, PRO1559, PR0779, PRO11185, PRO1245, PRO1759, PRO5775, PRO7133, PRO7168, PRO5725, PR0202, PRO206, PRO264, PR0313, PR0342, PR0542, PRO773, PR0861, PRO1216, PRO1686, PRO1800, PR03562, PR09850, PRO539, PR04316 or PR04980 is within the skill in the art

The positive hybridoma cells can be injected intraperitoneally into syngeneic Balb/c mice to produce ascites containing the anti-PRO 197, anti-PR0207, anti-PRO226, anti-PR0232, anti-PRO243, anti-PRO256, anti-PRO269, anti-PRO274, anti-PRO304, anti-PRO339, anti-PRO1558, anti-PRO779, anti-PRO1185, anti-PRO1245, anti-PRO1759, anti-PRO5775, anti-PRO7133, anti-PR07168, anti-PRO5725, anti-PRO202, anti-PRO206, anti-PRO264, anti-PR0313, anti-PR0342, anti-PR0542, anti-PRO773, anti-PRO861, anti-PRO1216, anti-PRO1686, anti-PRO1800, anti-PR03562, anti-PR09850, anti-PRO539, anti-PRO4316 or anti-PRO4980 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to proteins A or protein G can be employed.

Deposit of Material:

The following materials have been deposited with the American Type CultureCollection,10801 University Blvd., Manassas, VA 20110-2209, USA (ATCC):

| Material | ATCC Deposit No.: | Deposit Date |
|---|---|---|
| DNA22780-1078 | 209284 | September 18. 1997 |
| DNA30879-1152 | 209358 | October 10, 1997 |
| DNA33460-1166 | 209376 | October 16, 1997 |
| DNA34435-1140 | 209250 | September 16, 1997 |
| DNA35917-1207 | 209508 | December 3.1997 |
| DNA35880-1160 | 209379 | October 16, 1997 |
| DNA38260-1180 | 209397 | October 17, 1997 |
| DNA39987-1184 | 209786 | April 21.1998 |
| DNA39520-1217 | 209482 | November 21, 1997 |
| DNA43466-1225 | 209490 | November 21, 1997 |
| DNA71282-1668 | 203312 | October 6, 1998 |
| DNA58801-1052 | 55820 | September 5, 1996 |
| DNA62881-1515 | 203096 | August 4, 1998 |
| DNA64884-1527 | 203155 | August 25,1998 |

(continued)

| Material | ATCC Deposit No.: | Deposit Date |
|---|---|---|
| DNA76531-1701 | 203465 | November 17, 1998 |
| DNA96869-2673 | PTA-255 | June 22, 1999 |
| DNA128451-2739 | PTA-618 | August 31, 1999 |
| DNA102846-2742 | PTA-545 | August 17,1999 |
| DNA92265-2669 | PTA-256 | June 22, 1999 |
| DNA35672-2508 | 203538 | December 15, 1998 |
| DNA47465-1561 | 203661 | February 2, 1999 |
| DNA94713-2561 | 203835 | March 9, 1999 |
| DNA97003-2649 | PTA-43 | May 11, 1999 |

These deposits were made under the provisions of the Budapest Treaty on the International Recognition of the Deposit of Microorganisms for the Purpose of Patent Procedure and the Regulations thereunder (Budapest Treaty). This assures the maintenance of a viable culture of the deposit for 30 years from the date of deposit. The deposit will be made available by the ATCC under the terms of the Budapest Treaty, and subject to an agreement between Genentech, Inc., and the ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 U.S.C. § 122 and the Commissioner's rules pursuant thereto (including 37 C.F.R. § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

The following numbered paragraphs (paras.) contain statements of broad combinations of the inventive technical features herein disclosed:-

1. An isolated antibody that binds to a PRO1800 polypeptide.
2. The antibody of Para. 1 which specifically binds to said polypeptide.
3. The antibody of Para. 1 which induces the death of a cell that expresses said polypeptide.
4. The antibody of Para. 3, wherein said cell is a cancer cell that overexpresses said polypeptide as compared to a normal cell of the same tissue type.
5. The antibody of Para. 1 which is a monoclonal antibody.
6. The antibody of Para. 5 which comprises a non-human complementarity determining region (CDR) or a human framework region (FR).
7. The antibody of Para. 1 which is labeled.
8. The antibody of Para. 1 which is an antibody fragment or a single-chain antibody.
9. A composition of matter which comprises an antibody of Para. 1 in admixture with a pharmaceutically acceptable carrier.
10. The composition of matter of Para. 9 which comprises a therapeutically effective amount of said antibody.
11. The composition of matter of Para. 9 which further comprises a cytotoxic or a chemotherapeutic agent.
12. An isolated nucleic acid molecule that encodes the antibody of Para. 1.
13. A vector comprising the nucleic acid molecule of Para. 12.
14. A host cell comprising the vector of Para. 13.
15. A method for producing an antibody that binds to a PRO1800, said method comprising culturing the host cell of Para. 14 under conditions sufficient to allow expression of said antibody and recovering said antibody from the cell culture.

16. An antagonist of a PRO1800 polypeptide.

17. The antagonist of Para. 16, wherein said antagonist inhibits tumor cell growth.

18. An isolated nucleic acid molecule that hybridizes to a nucleic acid sequence that encodes a PRO1800 polypeptide, or the complement thereof.

19. The isolated nucleic acid molecule of Para. 18, wherein said hybridization is under stringent hybridization and wash conditions.

20. A method for determining the presence of a PRO1800 polypeptide in a sample suspected of containing said polypeptide, said method comprising exposing the sample to an anti-PRO1800, antibody and determining binding of said antibody to said polypeptide in said sample.

21. The method of Para. 20, wherein said sample comprises a cell suspected of containing a PRO1800 polypeptide.

22. The method of Para. 21, wherein said cell is a cancer cell.

23. A method of diagnosing tumor in a mammal, said method comprising detecting the level of expression of a gene encoding a PRO1800 polypeptide (a) in a test sample of tissue cells obtained from the mammal, and (b) in a control sample of known normal tissue cells of the same cell type, wherein a higher expression level in the test sample, as compared to the control sample, is indicative of the presence of tumor in the mammal from which the test tissue cells were obtained.

24. A method of diagnosing tumor in a mammal, said method comprising (a) contacting an anti-PRO1800, antibody with a test sample of tissue cells obtained from the mammal, and (b) detecting the formation of a complex between said antibody and a PRO1800 polypeptide in the test sample, wherein the formation of a complex is indicative of the presence of a tumor in said mammal.

25. The method of Para. 24, wherein said antibody is detectably labeled.

26. The method of Para. 24, wherein said test sample of tissue cells is obtained from an individual suspected of having neoplastic cell growth or proliferation.

27. A cancer diagnostic kit comprising an anti-PRO1800 antibody and a carrier in suitable packaging.

28. The kit of Para. 27 which further comprises instructions for using said antibody to detect the presence of a PRO1800 polypeptide in a sample suspected of containing the same.

29. A method for inhibiting the growth of tumor cells, said method comprising exposing tumor cells that express a PRO1800, polypeptide to an effective amount of an agent that inhibits a biological activity of said polypeptide, wherein growth of said tumor cells is thereby inhibited.

30. The method of Para. 29, wherein said tumor cells overexpress said polypeptide as compared to normal cells of the same tissue type.

31. The method of Para. 29, wherein said agent is an anti-PRO1800, antibody.

32. The method of Para. 31, wherein said anti-PRO1800, antibody induces cell death.

33. The method of Para. 29, wherein said tumor cells are further exposed to radiation treatment, a cytotoxic agent or a chemotherapeutic agent.

34. A method for inhibiting the growth of tumor cells, said method comprising exposing tumor cells that express a PRO1800, polypeptide to an effective amount of an agent that inhibits the expression of said polypeptide, wherein growth of said tumor cells is thereby inhibited.

35. The method of Para. 34, wherein said tumor cells overexpress said polypeptide as compared to normal cells of the same tissue type.

36. The method of Para. 34, wherein said agent is an antisense oligonucleotide that hybridizes to a nucleic acid which encodes the PRO1800, polypeptide or the complement thereof.

37. The method of Para. 36, wherein said tumor cells are further exposed to radiation treatment, a cytotoxic agent or a chemotherapeutic agent.

38. An article of manufacture, comprising:

   a container;
   a label on the container; and
   a composition comprising an active agent contained within the container, wherein the composition is effective for inhibiting the growth of tumor cells and wherein the label on the container indicates that the composition is effective for treating conditions characterized by overexpression of a PRO1800, PRO9850, PRO539, PR04316 or PR04980 polypeptide in said tumor cells as compared to in normal cells of the same tissue type.

39. The article of manufacture of Para. 38, wherein said active agent inhibits a biological activity of and/or the expression of said PRO1800, polypeptide.

40. The article of manufacture of Para. 39, wherein said active agent is an anti-PRO1800, antibody.

41. The article of manufacture of Para. 39, wherein said active agent is an antisense oligonucleotide.

42. A method of identifying a compound that inhibits a biological or immunological activity of a PRO1800, polypeptide, said method comprising contacting a candidate compound with said polypeptide under conditions and for a time sufficient to allow the two components to interact and determining whether a biological or immunological activity of said polypeptide is inhibited.

43. The method of Para. 42, wherein said candidate compound is an anti-PRO1800, antibody.

44. The method of Para. 42, wherein said candidate compound or said PRO1800, polypeptide is immobilized on a solid support.

45. The method of Para. 44, wherein the non-immobilized component is detectably labeled.

46. A method of identifying a compound that inhibits an activity of a PRO1800, polypeptide, said method comprising the steps of (a) contacting cells and a candidate compound to be screened in the presence of said polypeptide under conditions suitable for the induction of a cellular response normally induced by said polypeptide and (b) determining the induction of said cellular response to determine if the test compound is an effective antagonist, wherein the lack of induction of said cellular response is indicative of said compound being an effective antagonist.

47. A method for identifying a compound that inhibits the expression of a PRO1800, polypeptide in cells that express said polypeptide, wherein said method comprises contacting said cells with a candidate compound and determining whether expression of said polypeptide is inhibited.

48. The method of Para. 47, wherein said candidate compound is an antisense oligonucleotide.

49. Isolated nucleic acid having at least 80% nucleic acid sequence identity to a nucleotide sequence that encodes an amino acid sequence selected from the group consisting of the amino acid sequence shown in Figure 2 (SEQ ID NO:2).

50. Isolated nucleic acid having at least 80% nucleic acid sequence identity to a nucleotide sequence selected from the group consisting of the nucleotide sequence shown in Figure 1 (SEQ ID NO:1).

51. Isolated nucleic acid having at least 80% nucleic acid sequence identity to a nucleotide sequence selected from the group consisting of the full-length coding sequence of the nucleotide sequence shown in Figure 1 (SEQ ID NO:1).

52. Isolated nucleic acid having at least 80% nucleic acid sequence identity to the full-length coding sequence of the DNA deposited under ATCC accession number

53. A vector comprising the nucleic acid of any one of Para.s 49 to 52.

54. The vector of Para. 53 operably linked to control sequences recognized by a host cell transformed with the vector.

55. A host cell comprising the vector of Para. 53.

56. The host cell of Para. 55, wherein said cell is a CHO cell.

57. The host cell of Para. 55, wherein said cell is an *E. coli.*

58. The host cell of Para. 55, wherein said cell is a yeast cell.

59. The host cell of Para. 55, wherein said cell is a Baculovirus-infected insect cell.

60. A process for producing a polypeptide comprising culturing the host cell of Para. 55 under conditions suitable for expression of said polypeptide and recovering said polypeptide from the cell culture.

61. An isolated polypeptide having at least 80% amino acid sequence identity to an amino acid sequence selected from the group consisting of the amino acid sequence shown in Figure 2 (SEQ ID NO:2).

62. An isolated polypeptide scoring at least 80% positives when compared to an amino acid sequence selected from the group consisting of the amino acid sequence shown in Figure 2 (SEQ ID NO:2).

63. An isolated polypeptide having at least 80% amino acid sequence identity to an amino acid sequence encoded by the full-length coding sequence of the DNA deposited under ATCC accession number 203538.

64. A chimeric molecule comprising a polypeptide according to any one of Para.s 61 to 63 fused to a heterologous amino acid sequence.

65. The chimeric molecule of Para. 64, wherein said heterologous amino acid sequence is an epitope tag sequence.

66. The chimeric molecule of Para. 64, wherein said heterologous amino acid sequence is a Fc region of an immunoglobulin.

67. An antibody which specifically binds to a polypeptide according to any one of Para.s 61 to 63.

68. The antibody of Para. 67, wherein said antibody is a monoclonal antibody, a humanized antibody or a single-chain antibody.

69. Isolated nucleic acid having at least 80% nucleic acid sequence identity to:

(a) a nucleotide sequence encoding the polypeptide shown in Figure 2 (SEQ ID NO:2), lacking its associated signal peptide:

(b) a nucleotide sequence encoding an extracellular domain of the polypeptide shown in Figure 2 (SEQ ID NO: 2), with its associated signal peptide; or

(c) a nucleotide sequence encoding an extracellular domain of the polypeptide shown in Figure 2 (SEQ ID NO: 2), lacking its associated signal peptide.

70. An isolated polypeptide having at least 80% amino acid sequence identity to:

(a) the polypeptide shown in Figure 2 (SEQ ID NO:2), lacking its associated signal peptide;

(b) an extracellular domain of the polypeptide shown in Figure 2 (SEQ ID NO:2), with its associated signal peptide; or

(c) an extracellular domain of the polypeptide shown in Figure 2 (SEQ ID NO:2), lacking its associated signal peptide.

**Claims**

1. A method of diagnosing colon tumor in a mammal, said method comprising detecting the level of expression of a gene encoding a PRO1800 polypeptide (a) in a test sample of colon tissue cells obtained from the mammal, and (b) in a control sample of known normal colon tissue cells of the same cell type, wherein a higher expression level in the test sample, as compared to the control sample, is indicative of the presence of tumor in the mammal from which the test tissue cells were obtained, and wherein said PRO1800 polypeptide is a polypeptide having:

   (i) the amino acid sequence shown in Figure 2,
   (ii) the amino acid sequence shown in Figure 2, lacking the signal peptide, or
   (iii) the amino acid sequence encoded by the full-length coding sequence of DNA35672-2508, deposited under ATCC accession number 203538.

2. The method of claim 1, wherein the signal peptide is from amino acids 1 to 15±5 of Figure 2.

3. A method of diagnosing colon tumor in a mammal, said method comprising (a) contacting an antibody with a test sample of colon tissue cells obtained from the mammal, and (b) detecting the formation of a complex between said antibody and a PRO1800 polypeptide in the test sample, wherein the formation of a complex is indicative of the presence of a tumor in said mammal;
   wherein the antibody is capable of binding to a polypeptide consisting of:

   (i) the amino acid sequence shown in Figure 2,
   (ii) the amino acid sequence shown in Figure 2, lacking the signal peptide, or
   (iii) the amino acid sequence encoded by the full-length coding sequence of DNA35672-2508, deposited under ATCC accession number 203538;

   and wherein the PRO1800 polypeptide is a polypeptide having:

   (i) the amino acid sequence shown in Figure 2,
   (ii) the amino acid sequence shown in Figure 2, lacking the signal peptide, or
   (iii) the amino acid sequence encoded by the full-length coding sequence of DNA35672-2508, deposited under ATCC accession number 203538.

4. The method of claim 3, wherein said PRO1800 polypeptide is a polypeptide having:

   (i) the amino acid sequence shown in Figure 2,
   (ii) the amino acid sequence shown in Figure 2, lacking the signal peptide, or
   (iii) the amino acid sequence encoded by the full-length coding sequence of DNA35672-2508, deposited under ATCC accession number 203538.

5. The method of claim 3 or claim 4, wherein the signal peptide is from amino acids 1 to 15±5 of Figure 2.

6. The method of claim 3 or claim 4, wherein said antibody is detectably labeled.

7. The method of claim 3 or claim 4, wherein said test sample of tissue cells is obtained from an individual suspected of having neoplastic cell growth or proliferation.

**Patentansprüche**

1. Verfahren zur Diagnose eines Kolon-Tumors bei einem Säugetier, wobei das Verfahren das Detektieren des Expressionsausmaßes eines Gens umfasst, das für ein PRO1800-Polypeptid kodiert, und zwar (a) in einer Testprobe an Kolongewebe-Zellen, die von dem Säugetier erhalten wurden, und (b) ein einer Kontrollprobe bekannter normaler Kolongewebe-Zellen desselben Zelltyps, worin ein höheres Expressionsausmaß in der Testprobe im Vergleich zur Kontrollprobe ein Indikator für die Gegenwart eines Tumors bei dem Säugetier ist, von dem die Testgewebe-Zellen erhalten wurden, und worin das PRO1800-Polypeptid ein Polypeptid mit Folgendem ist:

(i) der Aminosäuresequenz, die in Fig. 2 dargestellt ist,
(ii) der Aminosäuresequenz, die in Fig. 2 dargestellt ist, der das Signalpeptid fehlt, oder
(iii) der Aminosäuresequenz, für die die kodierende Sequenz voller Länge von DNA35672-2508 kodiert, hinterlegt unter der ATCC-Zugriffsnr. 203538.

**2.** Verfahren nach Anspruch 1, worin das Signalpeptid von Aminosäure 1 bis 15 $\pm$ 5 aus Fig. 2 vorliegt.

**3.** Verfahren zur Diagnose eines Kolon-Tumors bei einem Säugetier, wobei das Verfahren (a) das Kontaktieren eines Antikörpers mit einer Testprobe an Kolongewebe-Zellen, die von dem Säugetier erhalten wurden, sowie (b) das Detektieren der Bildung eines Komplexes zwischen dem Antikörper und einem PRO1800-Polypeptid in der Testprobe umfasst, worin die Bildung eines Komplexes ein Indikator der Gegenwart eines Tumors bei dem Säugetier ist; worin der Antikörper in der Lage ist, an ein Polypeptid zu binden, das aus Folgendem besteht:

(i) der Aminosäuresequenz, die in Fig. 2 dargestellt ist,
(ii) der Aminosäuresequenz, die in Fig. 2 dargestellt ist, der das Signalpeptid fehlt, oder
(iii) der Aminosäuresequenz, für die die kodierende Sequenz voller Länge von DNA35672-2508 kodiert, hinterlegt unter der ATCC-Zugriffsnr. 203538;

und worin das PRO1800-Polypeptid ein Polypeptid mit Folgendem ist:

(i) der Aminosäuresequenz, die in Fig. 2 dargestellt ist,
(ii) der Aminosäuresequenz, die in Fig. 2 dargestellt ist, der das Signalpeptid fehlt, oder
(iii) der Aminosäuresequenz, für die die kodierende Sequenz voller Länge von DNA35672-2508 kodiert, hinterlegt unter der ATCC-Zugriffsnr. 203538.

**4.** Verfahren nach Anspruch 3, worin das PRO1800-Polypeptid ein Polypeptid mit Folgendem ist:

(i) der Aminosäuresequenz, die in Fig. 2 dargestellt ist,
(ii) der Aminosäuresequenz, die in Fig. 2 dargestellt ist, der das Signalpeptid fehlt, oder
(iii) der Aminosäuresequenz, für die die kodierende Sequenz voller Länge von DNA35672-2508 kodiert, hinterlegt unter der ATCC-Zugriffsnr. 203538.

**5.** Verfahren nach Anspruch 3 oder Anspruch 4, worin das Signalpeptid von Aminosäure 1 bis 15 $\pm$ 5 aus Fig. 2 vorliegt.

**6.** Verfahren nach Anspruch 3 oder Anspruch 4, worin der Antikörper detektierbar markiert ist.

**7.** Verfahren nach Anspruch 3 oder Anspruch 4, worin die Testprobe an Gewebezellen von einem Individuum erhalten wurde, bei dem der Verdacht eines neoplastischen Zellwachstums oder einer neoplastischen Zellproliferation besteht.

**Revendications**

**1.** Méthode pour diagnostiquer une tumeur du côlon chez un mammifère, ladite méthode comprenant la détection de degré d'expression d'un gène codant pour un polypeptide PRO1800 (a) dans un échantillon d'essai de cellules de tissu du côlon obtenu à partir du mammifère et (b) dans un échantillon témoin de cellules de tissu du côlon normal connues du même type cellulaire, dans laquelle un plus haut degré d'expression dans l'échantillon d'essai, comparativement à l'échantillon témoin, indique la présence d'une tumeur chez le mammifère à partir duquel les cellules de tissu d'essai ont été obtenues, et dans laquelle ledit polypeptide PRO1800 est un polypeptide ayant :

(i) la séquence d'aminoacides représentée sur la figure 2,
(ii) la séquence d'aminoacides représentée sur la figure 2, dépourvue du peptide signal, ou

(iii) la séquence d'aminoacides codée par la séquence codante totale du DNA35672-2508, déposé sous le numéro de dépôt ATCC 203538.

**2.** Méthode suivant la revendication 1, dans laquelle le peptide signal va de l'aminoacide 1 à l'aminoacide 15 $\pm$ 5 de la figure 2.

**3.** Méthode pour diagnostiquer une tumeur du côlon chez un mammifère, ladite méthode comprenant (a) la mise en contact d'un anticorps avec un échantillon d'essai de cellules de tissu du côlon obtenu à partir du mammifère, et (b) la détection de la formation d'un complexe entre ledit anticorps et un polypeptide PR01800 dans l'échantillon d'essai, dans laquelle la formation d'un complexe indique la présence d'une tumeur chez ledit mammifère ;
dans laquelle l'anticorps est capable de lier à un polypeptide consistant en :

(i) la séquence d'aminoacides représentée sur la figure 2,
(ii) la séquence d'aminoacides représentée sur la figure 2, dépourvue du peptide signal, ou
(iii) la séquence d'aminoacides codée par la séquence codante totale du DNA35672-2508, déposé sous le numéro de dépôt ATCC numéro 203538 ;

et dans laquelle le polypeptide PR01800 est un polypeptide ayant :

(i) la séquence d'aminoacides représentée sur la figure 2,
(ii) la séquence d'aminoacides représentée sur la figure 2, dépourvue du peptide signal, ou
(iii) la séquence d'aminoacides codée par la séquence codante totale du DNA35672-2508, déposé sous le numéro de dépôt ATCC numéro 203538.

**4.** Méthode suivant la revendication 3, dans laquelle ledit polypeptide PRO1800 est un polypeptide ayant :

(i) la séquence d'aminoacides représentée sur la figure 2,
(ii) la séquence d'aminoacides représentée sur la figure 2, dépourvue du peptide signal, ou
(iii) la séquence d'aminoacides codée par la séquence codante totale du DNA35672-2508, déposé sous le numéro de dépôt ATCC numéro 203538.

**5.** Méthode suivant la revendication 3 ou la revendication 4, dans laquelle le peptide signal va de l'aminoacide 1 à l'aminoacide 15 $\pm$ 5 de la figure 2.

**6.** Méthode suivant la revendication 3 ou la revendication 4, dans laquelle ledit anticorps est marqué de manière détectable.

**7.** Méthode suivant la revendication 3 ou la revendication 4, dans laquelle ledit échantillon d'essai de cellules de tissu est obtenu à partir d'un individu supposé présenter une croissance ou prolifération cellulaire néoplasique.

FIGURE 1

```
CGGACGCGTGGGACCCATACTTGCTGGTCTGATCCATGCACAAGGCGGGGCTGCTAGGCCTCTGTGCCCGGGCTT
GGAATTCGGTGCGGATGGCCAGCTCCGGGATGACCCGCCGGGACCCGCTCGCAAATAAGGTGGCCCTGGTAACGG
CCTCCACCGACGGGATCGGCTTCGCCATCGCCCGGCGTTTGGCCCAGGACGGGGCCCATGTGGTCGTCAGCAGCC
GGAAGCAGCAGAATGTGGACCAGGCGGTGGCCACGCTGCAGGGGGAGGGGCTGAGCGTGACGGGCACCGTGTGCC
ATGTGGGGAAGGCGGAGGACCGGGAGCGGCTGGTGGCCACGGCTGTGAAGCTTCATGGAGGTATCGATATCCTAG
TCTCCAATGCTGCTGTCAACCCTTTCTTTGGAAGCATAATGGATGTCACTGAGGAGGTGTGGGACAAGACTCTGG
ACATTAATGTGAAGGCCCCAGCCCTGATGACAAAGGCAGTGGTGCCAGAAATGGAGAAACGAGGAGGCGGCTCAG
TGGTGATCGTGTCTTCCATAGCAGCCTTCAGTCCATCTCCTGGCTTCAGTCCTTACAATGTCAGTAAAACAGCCT
TGCTGGGCCTGACCAAGACCCTGGCCATAGAGCTGGCCCCAAGGAACATTAGGGTGAACTGCCTAGCACCTGGAC
TTATCAAGACTAGCTTCAGCAGGATGCTCTGGATGGACAAGGAAAAAGAGGAAAGCATGAAAGAAACCCTGCGGA
TAAGAAGGTTAGGCGAGCCAGAGGATTGTGCTGGCATCGTGTCTTTCCTGTGCTCTGAAGATGCCAGCTACATCA
CTGGGGAAACAGTGGTGGTGGGTGGAGGAACCCCGTCCCGCCTCTGAGGACCGGGAGACAGCCCACAGGCCAGAG
TTGGGCTCTAGCTCCTGGTGCTGTTCCTGCATTCACCCACTGGCCTTTCCCACCTCTGCTCACCTTACTGTTCAC
CTCATCAAATCAGTTCTGCCCTGTGAAAAGATCCAGCCTTCCCTGCCGTCAAGGTGGCGTCTTACTCGGGATTCC
TGCTGTTGTTGTGGCCTTGGGTAAAGGCCTCCCCTGAGAACACAGGACAGGCCTGCTGACAAGGCTGAGTCTACC
TTGGCAAAGACCAAGATATTTTTTCCTGGGCCACTGGTGAATCTGAGGGGTGATGGGAGAGAAGGAACCTGGAGTGG
AAGGAGCAGAGTTGCAAATTAACAGCTTGCAAATGAGGTGCAAATAAAATGCAGATGATTGCGCGGCTTTGAAAA
AAAAAA
```

**FIGURE 2**

MHKAGLLGLCARAWNSVRMASSGMTRRDPLANKVALVTASTDGIGFAIARRLAQDGAHVVVSSRKQQNVDQAVAT
LQGEGLSVTGTVCHVGKAEDRERLVATAVKLHGGIDILVSNAAVNPFFGSIMDVTEEVWDKTLDINVKAPALMTK
AVVPEMEKRGGGSVVIVSSIAAFSPSPGFSPYNVSKTALLGLTKTLAIELAPRNIRVNCLAPGLIKTSFSRMLWM
DKEKEESMKETLRIRRLGEPEDCAGIVSFLCSEDASYITGETVVVGGGTPSRL


Signal peptide:                          Amino acids 1-15

N-glycosylation site:                    Amino acids 183-187

N-myristoylation sites:                  Amino acids 43-49;80-86;191-197;213-219;
                                         272-278

Microbodies C-terminal targeting signal:
                                         Amino acids 276-280

Short-chain alcohol dehydrogenase:  Amino acids 162-199

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4675187 A
- US 4816567 A
- EP 404097 A
- WO 9311161 A
- US 4275149 A
- US 5364934 A
- WO 8705330 A
- US 4640835 A
- US 4496689 A
- US 4301144 A
- US 4670417 A
- US 4791192 A
- US 4179337 A
- US 5428130 A
- WO 8905859 A
- US 4399216 A
- DD 266710
- US 4946783 A
- EP 139383 A
- US 4943529 A
- EP 402226 A
- EP 183070 A
- EP 244234 A
- EP 394538 A
- WO 9100357 A
- US 5010182 A
- EP 362179 A
- WO 9013646 A
- EP 36776 A
- EP 73657 A
- GB 2211504 A
- EP 117060 A
- EP 117058 A
- WO 9318186 A
- US 4376110 A
- WO 9733551 A
- US 4873191 A, Hoppe and Wanger
- US 4736866 A
- US 5545807 A
- US 5545806 A
- US 5569825 A
- US 5625126 A
- US 5633425 A
- US 5661016 A
- WO 8101145 A
- WO 8807378 A
- US 4975278 A
- WO 9308829 A
- WO 9627011 A
- US 4676980 A
- WO 9100360 A
- WO 92200373 A
- EP 03089 A
- WO 9411026 A
- US 4485045 A
- US 4544545 A
- US 5013556 A
- US 3773919 A
- EP 616812 A
- WO 9715668 A2
- DE 19818620
- WO 0004135 A
- EP 307247 A
- US 5122469 A

### Non-patent literature cited in the description

- **BORING et al.** *CA Cancel J. Clin.,* 1993, vol. 43, 7
- **HUNTER.** *Cell,* 1991, vol. 64, 1129
- **BISHOP.** *Cell,* 1991, vol. 64, 235-248
- **ALITALO et al.** *Adv. Cancer Res.,* 1986, vol. 47, 235-281
- **SLAMON et al.** *Science,* 1987, vol. 235, 177-182
- **SLAMON et al.** *Science,* 1989, vol. 244, 707-712
- **SCHWAB et al.** *Genes Chromosomes cancer,* 1990, vol. 1, 181-193
- **RAVDIN ; CHAMNESS.** *Gene,* 1995, vol. 159, 19-27
- **HYNES ; STERN.** *Biochim. Biophys. Acta,* 1994, vol. 1198, 165-184
- **BASELGA et al.** *Oncology,* 1997, vol. 11 (3), 43-48
- **BASELGA et al.** *J. Clin. Oncol.,* 1996, vol. 14, 737-744
- Cell cycle regulation, oncogens, and antineoplastic drugs. **MURAKAMI et al.** The Molecular Basis of Cancer. WB Saunders, 1995, 13

- **WILMAN.** Prodrugs in Cancer Chemotherapy. *Biochemical Society Transactions,* 1986, vol. 14, 375-382
- Prodrugs: A Chemical Approach to Targeted Drug Delivery. **STELLA et al.** Directed Drug Delivery. Humana Press, 1985, 147-267
- **NIELSEN et al.** *Prot. Eng,* 1997, vol. 10, 1-6
- **VON HEINJE et al.** *Nucl. Acids Res.,* 1986, vol. 14, 4683-4690
- **ALTSCHUL et al.** *Nucleic Acids Res.,* 1997, vol. 25, 3389-3402
- **ALTSCHUL et al.** *Methods in Enzymology,* 1996, vol. 266, 460-480
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **KABAT et al.** *NIH Publ. No.91-3242,* 1991, vol. I, 647-669
- **KABA et al.** Sequences of Proteins of Immunological Interest,. 1991
- **CLOTHIA ; LESK.** *J. Mol. Biol.,* 1987, vol. 196, 901-917
- **ZAPATA et al.** *Protein Eng.,* 1995, vol. 8 (10), 1062
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **MORRISON et al.** *Proc. Natl. Acad. Sci. USA,* 1984, vol. 81, 6851-6855
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **PLUCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **CARTER et al.** *Nucl. Acids Res.,* 1986, vol. 13, 4331
- **ZOLLER et al.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415
- **CUNNINGHAM ; WELLS.** *Science,* 1989, vol. 244, 1081-1085
- **CREIGHTON.** The Proteins. W.H. Freeman & Co,
- **CHOTHIA.** *J. Mol. Biol.,* 1976, vol. 150, 1
- **T.E. CREIGHTON.** Proteins: Structure and Molecular Properties. W.H. Freeman & Co, 1983, 79-86
- **APLIN ; WRISTON.** *CRC Crit. Rev. Biochem.,* 1981, 259-306
- **HAKIMUDDIN et al.** *Arch. Biochem. Biophys.,* 1987, vol. 259, 52
- **EDGE et al.** *Anal. Biochem.,* 1981, vol. 118, 131
- **THOTAKURA et al.** *Meth. Enzymol.,* 1987, vol. 138, 350
- **FIELD et al.** *Mol. Cell. Biol.,* 1988, vol. 8, 2159-2165
- **EVAN et al.** *Molecular and Cellular Biology,* 1985, vol. 5 (1), 3610-3616
- **PABORSKY et al.** *Protein Engineering,* 1990, vol. 3 (6), 547-553
- **HOPP et al.** *BioTechnology,* 1988, vol. 6, 1204-1210
- **MARTIN et al.** *Science,* 1992, vol. 255, 192-194
- **SKINNER et al.** *J. Biol. Chem.,* 1991, vol. 266, 15163-15166
- **LUTZ-FREYERMUTH et al.** *Proc. Natl. Acad. Sci. USA,* 1990, vol. 87, 6393-6397
- **STEWART et al.** Solid-Phase Peptide Synthesis. W.H. Freeman Co, 1969
- **MERRIFIELD.** *J. Am. Chem. Soc.,* 1963, vol. 85, 2149-2154
- **SAMBROOK et al.** Molecular Cloning: A Laboratory Manual. Cold Spring Harbor Laboratory Press, 1989
- **DIEFFENBACH et al.** PCR Primer: A Laboratory Manual. Cold Spring Harbor Laboratory Press, 1995
- **SAMBROOK et al.** Mammalian Cell Biotechnology: a Practical Approach. IRL Press, 1991
- **SHAW et al.** *Gene,* 1983, vol. 23, 315
- **GRAHAM ; VAN DER EB.** *Virology,* 1978, vol. 52, 456-457
- **SOLINGEN et al.** *J. Bact.,* 1977, vol. 130, 946
- **HSIAO et al.** *Proc. Natl. Acad.-Sci. (USA),* 1979, vol. 76, 3829
- **KEOWN et al.** *Methods in Enzymology,* 1990, vol. 185, 527-537
- **MANSOUR et al.** *Nature,* 1988, vol. 336, 348-352
- **BEACH ; NURSE.** *Nature,* 1981, vol. 290, 140
- **FLEER et al.** *Bio/Technology,* 1991, vol. 9, 968-975
- **LOUVENCOURT et al.** *J. Bacteriol.,* 1983, 737
- **VANDEN BERG et al.** *Bio/Technology,* 1990, vol. 8, 135
- **SREEKRISHNA et al.** *J. Basic Microbiol.,* 1988, vol. 28, 265-278
- **CASE et al.** *Proc. Natl. Acad. Sci. USA,* 1979, vol. 76, 5259-5263
- **BALLANCE et al.** *Biochem. Biophys. Res. Commun,* 1983, vol. 112, 284-289
- **TILBURN et al.** *Gene,* 1983, vol. 26, 205-221

- **YELTON et al.** *Proc. Natl. Acad. Sci. USA,* 1984, vol. 81, 1470-1474
- **KELLY ; HYNES.** *EMBO J.,* 1985, vol. 4, 475-479
- **C. ANTHONY.** *The Biochemistry of Methylotrophs,* 1982, 269
- **GRAHAM et al.** *J. Gen. Virol.,* 1977, vol. 36, 59
- **URLAUB ; CHASIN.** *Proc. Natl. Acad. Sci. USA,* 1980, vol. 77, 4216
- **MATHER.** *Biol. Reprod.,* 1980, vol. 23, 243-251
- **URLAUB et al.** *Proc. Natl. Acad. Sci. USA.,* 1980, vol. 77, 4216
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157
- **JONES.** *Genetics,* 1977, vol. 85, 12
- **CHANG et al.** *Nature,* 1978, vol. 275, 615
- **GOEDDEL et al.** *Nature,* 1979, vol. 281, 544
- **GOEDDEL.** *Nucleic Acids Res.,* 1980, vol. 8, 4057
- **DEBOER et al.** *Proc. Natl. Acad. Sci. USA,* vol. 80, 21-25
- **HITZEMAN et al.** *J. Biol. Chem.,* 1980, vol. 255, 2073
- **HESS et al.** *J. Adv. Enzyme Reg.,* 1968, vol. 7, 149
- **HOLLAND.** *Biochemistry,* 1978, vol. 17, 4900
- **GETHING et al.** *Nature,* 1981, vol. 293, 620-625
- **MANTEI et al.** *Nature,* 1979, vol. 281, 40-46
- **THOMAS.** *Proc. Natl. Acad. Sci. USA,* 1980, vol. 77, 5201-5205
- **DEUTSCHER.** *Methods in Enzymology,* 1990, 182
- **SCOPES.** Protein Purification: Principles and Practice. Springer-Verlag, 1982
- **GRAY et al.** *Radiation Res.,* 1994, vol. 137, 275-289
- **S. GELMINI et al.** *Clin. Chem.,* 1997, vol. 43, 752
- **STEWART et al.** *Genome Research,* 1997, vol. 7, 422-433
- **ZOLA.** Monoclonal Antibodies: A Manual of Techniques. CRC Press, Inc, 1987, 147-158
- **SMALL et al.** *Mol. Cell. Biol.,* 1985, vol. 5, 642-648
- The Nude Mouse in Oncology Research. CRC Press, Inc, 1991
- **KARMALI et al.** *Br. J. Cancer,* 1983, vol. 48, 689-696
- **DREBIN et al.** *PNAS USA,* 1986, vol. 83, 9129-9133
- **WANG et al.** *Cancer Research,* 1994, vol. 54, 4726-4728
- **TOO et al.** *Cancer Research,* 1995, vol. 55, 681-684
- **DELEO et al.** *J. Exp. Med.,* 1977, vol. 146, 720
- **PALLADINO et al.** *J. Immunol,* 1987, vol. 138, 4023-4032
- **ZUPI.** *Br. J. Cancer,* 1980, vol. 41 (4), 309
- **ZACHARSKI.** *Haemostasis,* 1986, vol. 16, 300-320
- **RYGAARD ; SPANG-THOMSEN.** Proc. 6th Int. Workshop on Immune-Deficient Animals. 1989, 301
- **VAN DER PUTTEN et al.** *Proc. Natl. Acad. Sci. USA,* 1985, vol. 82, 6148-615
- **THOMPSON et al.** *Cell,* 1989, vol. 56, 313-321
- **LO.** *Mol. Cell Biol.,* 1983, vol. 3, 1803-1814
- **LAVITRANO et al.** *Cell,* 1989, vol. 57, 717-73
- **LASKO et al.** *Proc. Natl. Acad. Sci. USA,* 1992, vol. 89, 6232-636
- **THOMAS ; CAPECCHI.** *Cell,* 1987, vol. 51, 503
- **LI et al.** *Cell,* 1992, vol. 69, 915
- **BRADLEY.** Teratocarcinomas and Embryonic Stem Cells: A Practical Approach. IRL, 1987, 113-152
- **FIELDS ; SONG.** *Nature,* 1989, vol. 340, 245-246
- **CHIEN et al.** *Proc. Natl. Acad. Sci. USA,* 1991, vol. 88, 9578-9582
- **CHEVRAY ; NATHANS.** *Proc. Natl. Acad. Sci. USA,* 1991, vol. 89, 5789-5793
- **COLIGAN et al.** Current Protocols in Immun. 1991, vol. 1
- **LEE et al.** *Nucl. Acids Res.,* 1979, vol. 6, 3073
- **COONEY et al.** *Science,* 1988, vol. 241, 456
- **DERVAN et al.** *Science,* 1991, vol. 251, 1360
- **OKANO.** *Neurochem,* 1991, vol. 56, 560
- Oligodeoxynucleotides as Antisense Inhibitors of Gene Expression. CRC Press, 1988
- **ROSSI.** *Current Biology,* 1994, vol. 4, 469-471
- **KOHLER ; MILSTEIN.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON ; POLLARD.** *Anal. Biochem.,* 1980, vol. 107, 220
- **JONES et al.** *Nature,* 1986, vol. 321, 522-325
- **RIECHMANN et al.** *Nature,* 1988, vol. 332, 323-329
- **PRESTA.** *Curr. Op. Struct. Biol.,* 1992, vol. 2, 593-596
- **RIECHMANN et al.** *Nature,* 1988, vol. 332, 323-327

- **VERHOEYEN et al.** *Science,* 1988, vol. 239, 1534-1536
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **MASSEY.** *Nature,* 1987, vol. 328, 457-458
- **NEUBERGER et al.** *Nature,* 1984, vol. 312, 604-608
- **MILSTEIN ; CUELLO.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **BRENNAN et al.** *Science,* 1985, vol. 229, 81
- **SHALABY et al.** *J. Exp. Med.,* 1992, vol. 175, 217-225
- **KOSTELNY et al.** *J. Immunol.,* 1992, vol. 148 (5), 1547-1553
- **GRUBER.** *J. Immunol.,* 1994, vol. 152, 5368
- **TUTT et al.** *J. Immunol.,* 1991, vol. 147, 60
- **CARON et al.** *J. Exp. Med.,* 1992, vol. 176, 1191-1195
- **SHOPES.** *J. Immunol.,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **VITETTA et al.** *Science,* 1987, vol. 238, 1098
- **EPSTEIN et al.** *Proc. Natl. Acad. Sci. USA,* 1985, vol. 82, 3688
- **HWANG et al.** *Proc. Natl. Acad. Sci, USA,* 1980, vol. 77, 4030
- **MARTIN et al.** *J. Biol. Chem,* 1982, vol. 257, 286-288
- **GABIZON et al.** *J. National Cancer Inst.,* 1989, vol. 81 (19), 1484
- **MARASCO et al.** *Proc. Natl. Acad. Sci. USA,* 1993, vol. 90, 7889-7893
- Remington's Pharmaceutical Sciences. 1980
- Chemotherapy Service. Williams & Wilkins, 1992
- **SAMBROOK et al.** Molecular Cloning,: A Laboratory Manual. Cold Spring Harbor Press, 1989
- **AUSUBEL et al.** Current Protocols in Molecular Biolo. Green Publishing Associates and Wiley Interscience, 1989
- **INNIS et al.** PCR Protocols: A Guide to Methods and Applications. Academic Press, Inc, 1990
- **HARLOW et al.** Antibodies: A Laboratory Manual. Cold Spring Harbor Press, 1988
- **GAIT.** Oligonucleotide Synthesis. IRL Press, 1984
- **R.I. FRESHNEY.** *Animal Cell Culture,* 1987
- **COLIGAN et al.** *Current Protocols in Immunology,* 1991
- **HOLMES et al.** *Science,* 1991, vol. 253, 1278-1280
- **GURNEY et al.** *Blood,* 1995, vol. 85, 981-988
- **CHEN et al.** *Genomics,* 1993, vol. 17, 651-656
- **XU et al.** *Gen. Engin.,* 1994, vol. 16, 241-253
- **CROSS, S. ; BIRD, A.** *Curr. Opin. Genet. & Devel.,* 1995, vol. 5, 109-314
- **ALTSCHUL ; GISH.** *Methods Enzymol.,* 1996, vol. 266, 460-480
- **A. L. GURNEY et al.** *Blood,* 1995, vol. 85, 981-988
- **CHEN et al.** *Genomics,* 1993, vol. 17, 651-656
- **BARNES, W.** *Proc. Natl, Acad. Sci. USA,* 1994, vol. 91, 2216-2220
- **CHEN et al.** *Genomics,* 1993, vol. 17, 651-56
- **ALTSCHUL, S. ; GISH, W.** *Methods Enzymol.,* 1996, vol. 266, 460-480
- **NEEDLEMAN ; WUNSCH.** *J. Mol. Biol.,* 1970, vol. 48, 443-453
- **S. CROSS ; BIRD, A.** *Curr. Opin. Genet. Dev.,* 1995, vol. 5, 109-314
- **ALTSHUL et al.** *Methods in Enzymology,* 1996, vol. 266, 460-480
- **ALTSHUL et al.** *Methods in Enzymology,* 1996, vol. 266, 460-180
- **LU ; GILLETT.** *Cell Vision,* 1994, vol. 1, 169-176
- **BOLIVAR et al.** *Gene,* 1977, vol. 2, 95
- **THIMMAPPAYA et al.** *Cell,* 1982, vol. 31, 543
- **SOMPARYRAC et al.** *Proc. Natl. Acad. Sci.,* 1981, vol. 12, 7575
- **AUSUBEL et al.** Current Protocols of Molecular Biology. John Wiley and Sons, 1997
- **LUCAS et al.** *Nucl. Acids Res.,* 1996, vol. 24 (9), 1774-1779
- **O'REILLEY et al.** Baculovirus expression vectors: A Laboratory Manual. Oxford University Press, 1994
- **RUPERT et al.** *Nature,* 1993, vol. 362, 175-179